(19) Europäisches Patentamt
European Patent Office
Office européen des brevets

(11) EP 4 406 953 A1

(12) **EUROPEAN PATENT APPLICATION**
published in accordance with Art. 153(4) EPC

(43) Date of publication:
**31.07.2024 Bulletin 2024/31**

(21) Application number: **22872024.9**

(22) Date of filing: **21.09.2022**

(51) International Patent Classification (IPC):
***C07D 413/14*** *(2006.01)* ***A61K 31/4545*** *(2006.01)*
***A61K 31/496*** *(2006.01)* ***A61P 35/00*** *(2006.01)*

(52) Cooperative Patent Classification (CPC):
**A61K 31/4545; A61K 31/496; A61P 35/00; C07D 413/14**

(86) International application number:
**PCT/CN2022/120267**

(87) International publication number:
**WO 2023/045978 (30.03.2023 Gazette 2023/13)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **26.09.2021 CN 202111131041**
**09.09.2022 CN 202211104698**

(71) Applicant: **Medshine Discovery Inc.**
**Nanjing, Jiangsu 210032 (CN)**

(72) Inventors:
- **LEI, Maoyi**
  **Shanghai 200131 (CN)**
- **XU, Yu**
  **Shanghai 200131 (CN)**
- **WANG, Shaohui**
  **Shanghai 200131 (CN)**
- **LUO, Yunfu**
  **Shanghai 200131 (CN)**
- **CHEN, Shuhui**
  **Shanghai 200131 (CN)**

(74) Representative: **dompatent von Kreisler Selting Werner -**
**Partnerschaft von Patent- und Rechtsanwälten mbB**
**Deichmannhaus am Dom**
**Bahnhofsvorplatz 1**
**50667 Köln (DE)**

(54) **2,6-PIPERIDINEDIONE COMPOUND AND APPLICATION THEREOF**

(57) Provided are a 2,6-piperidincdione compound and an application thereof. Particularly provided are a compound represented by formula (II), a stereoisomer thereof, and a pharmaceutically acceptable salt thereof.

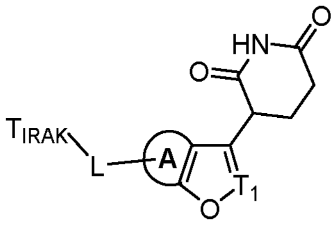

**( II )**

EP 4 406 953 A1

## Description

The present application claims right of the following priorities:

This application claims 1) the priority and rights of Chinese Patent Application No. 2021111310410 submitted to CNIPA on September 26, 2021, and 2) the priority and rights of Chinese Patent Application No. 2022111046982 submitted to CNIPA on September 9, 2022, the contents of which are incorporated herein by reference in their entireties.

TECHNICAL FIELD

The invention relates to a class of 2,6-piperidinedione compounds and an application thereof. Specifically, the invention relates to a compound of formula (II), a stereoisomer thereof, and a pharmaceutically acceptable salt thereof.

BACKGROUND

Interleukin-1 Receptor-Associated Kinase 4 (IRAK4) plays a connecting role in the signal transduction pathway between Toll-like receptor family (TLRs) and interleukin-1 receptor family (IL-1R), and receives upstream signals to activate its downstream JNK and NF-κB signal pathways, which is closely related to the development and progression of human inflammatory immune diseases and tumors.

The Toll-like receptor (TLR) signal transduction protein myeloid differentiation factor (MyD88) often mutates in various lymphomas, such as Waldenström macroglobulinemia, lymphoplasmacytic lymphoma, anti-immune large B-cell lymphoma and marginal zone lymphoma, with mutation rates of 95-97%, 79%, 50-80%, 15-29% and 6-10% respectively. IRAK4 is involved in almost all biological functions of MyD88, making it an extremely attractive drug target with unlimited potentials, especially for the treatment of MyD88-driven lymphomas.

Researches have found that IRAK4 can not only phosphorylate protein, but also form a complex with MyD88 to exert its biological functions. The activation of the JNK signaling pathway by IRAK4 requires its phosphorylation function, yet activation of the NF-κB signaling pathway does not require its phosphorylation function, which indicates that IRAK4 possesses both protein kinase and scaffold protein functions, and plays roles in signaling pathways. Therefore, traditional small-molecule kinase inhibitors targeting IRAK4 cannot completely block all biological functions of IRAK4.

Proteolysis targeting chimera (PROTAC) is a technique that applies the ubiquitin-proteasome system to target specific proteins and induce their intracellular degradation. The ubiquitin-proteasome system is the primary pathway for intracellular protein degradation, mainly responsible for the clearance of denatured, mutated, or harmful proteins as its normal physiological functions. Over 80% of intracellular protein degradation is dependent on the ubiquitin-proteasome system. PROTAC utilizes the cell's own protein destruction mechanism to clear specific targeted proteins within the cell. To date, the PROTAC technology has become increasingly mature, and can be used to target a variety of proteins, including scaffold proteins, transcription factors, enzymes, and regulatory proteins. In addition, domide-class drugs, known as immunomodulatory drugs (IMiDs), activate the E3 ubiquitin ligase complex formed with Cereblon (CRBN) to ubiquitinate the transcription factors IKZF1 and IKZF3, which are then recognized and degraded by the proteasome, thereby producing a toxic effect on tumors. CRBN, as an important target for antitumor and immunomodulatory drugs, has been proven to have definitive therapeutic effects in a variety of hematological malignancies, skin diseases such as erythema nodosum leprosum, and autoimmune diseases like systemic lupus erythematosus.

Therefore, the development of PROTAC and IMiD bifunctional molecules targeting IRAK4 can more thoroughly block all functions of IRAK4 by degrading and removing IRAK4, and thus fundamentally inhibits the IRAK4 signaling pathway comprehensively, while also possessing good CRBN modulating effects, leading to synergistic therapeutic effects. Consequently, the strategy can better exert anti-tumor effects and improve clinical treatment outcomes.

CONTENT OF THE PRESENT INVENTION

The present disclosure provides a compound of formula (II), a stereoisomer thereof, or a pharmaceutically acceptable salt thereof,

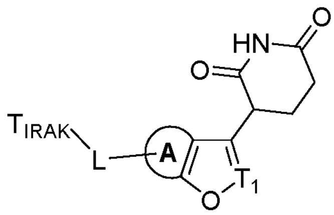

**( II )**

wherein

$T_{IRAK}$ is selected from

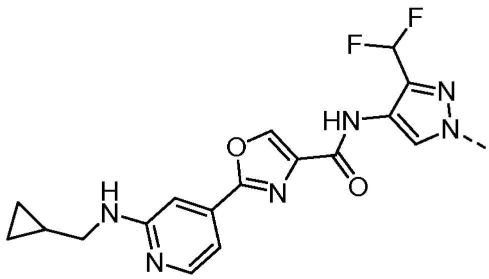

and

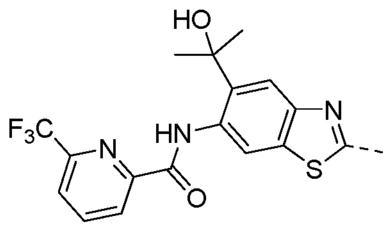

;

L is selected from $C_{2-10}$ alkylene, any 2, 3 or 4 $CH_2$ on the $C_{2-10}$ alkylene are each independently substituted by Ra, and each $C_{2-10}$ alkylene is independently and optionally substituted by 1, 2, 3, 4, 5, or 6 halogens;
Each Ra is independently selected from -N(R)-, -O-, -C(O)NH-, -$C_{3-6}$ cycloalkyl- and -4- to 8-membered heterocycloalkyl-;
R is selected from H and $C_{1-4}$ alkyl;
$T_1$ is selected from CH and N;
ring A is selected from $C_{6-10}$ aryl and 5- to 10-membered heteroaryl.

In some embodiments of the present disclosure, the Ra is independently selected from -NH-, -N($CH_3$)-, -O-, -C(O)NH-, -cyclopropyl-, -cyclobutyl-, -cyclopentyl-, -cyclohexyl-, - piperidyl-, -piperazinyl-, -azaspiro[3.3]heptyl-, -diazspiro[3.3]heptyl-, and - azabicyclic[3.1.0]hexyl-, and other variables are as defined in the present disclosure.

In some embodiments of the present disclosure, each Ra is independently selected from -NH-, -N($CH_3$)-, -O-, -C(O)NH-,

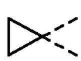, 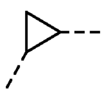, 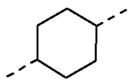, 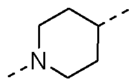, 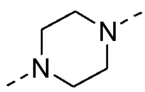,

and

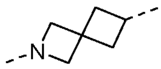,

, and other variables are as defined in the present disclosure.

In some embodiments of the present disclosure, the L is selected from $C_{4-10}$ alkylene, any 3 $CH_2$ on the $C_{4-10}$ alkylene are each independently substituted by Ra, each $C_{4-10}$ alkylene is independently and optionally substituted by 1, 2, 3, 4, 5 or 6 halogens, and other variables are as defined in the present disclosure.

In some embodiments of the present disclosure, the L is selected from -$C_{3-6}$ cycloalkyl-$CH_2$-Ra-$C_{1-3}$ alkylene-Ra-$C_{0-3}$ alkylene-, -$C_{3-6}$ cycloalkyl-$C_{1-3}$ alkylene-4- to 8-membered heterocycloalkyl-$C_{1-3}$ alkylene-Ra-, and -$C_{3-6}$ cycloalkyl-$C_{1-3}$ alkylene-4- to 8-membered heterocycloalkyl-$C_{1-3}$ alkylene-, each $C_{1-3}$ alkylene is independently and optionally substituted by 1 or 2 halogens, and other variables are as defined in the present disclosure.

In some embodiments of the present disclosure, the L is selected from- cyclohexyl-$CH_2$-N(R)-$C_{1-3}$ alkylene-O-$C_{1-3}$ alkylene-, -cyclohexyl-$CH_2$-4- to 8-membered heterocycloalkyl-$C_{1-3}$ alkylene-N (R)-, -cyclohexyl-$CH_2$-piperidyl-$C_{1-3}$ alkylene-C(O)NH-, - cyclohexyl-$CH_2$ piperidyl-$CF_2$-C(O)NH -, -cyclohexyl-$CH_2$-piperazinyl-$C_{1-3}$ alkylene-C(O)NH-, and -cyclohexyl-$CH_2$-piperazinyl-$C_{1-3}$ alkylene-, and other variables are as defined in the present disclosure.

In some embodiments of the present disclosure, the L is selected from

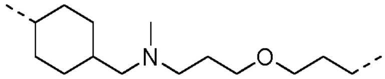, 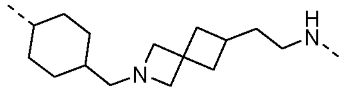, 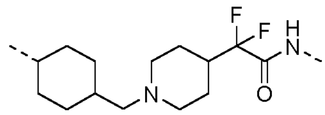,

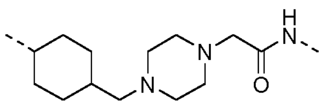, and 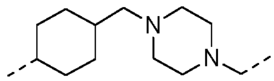,

and other variables are as defined in the present disclosure.

In some embodiments of the present disclosure, the ring A is selected from phenyl and naphthyl, and other variables are as defined in the present disclosure.

In some embodiments of the present disclosure, the structural moiety

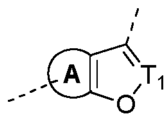

is selected from

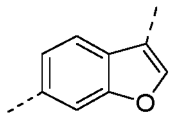, 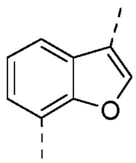, 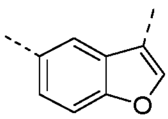, 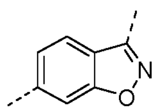, 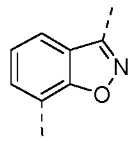,

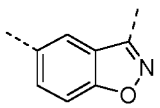, 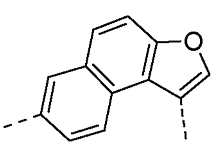, 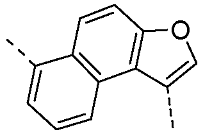, 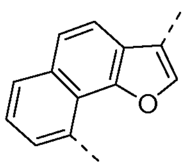, 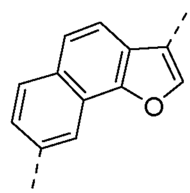,

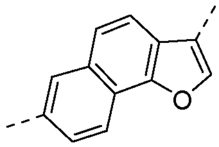, 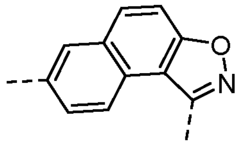, and 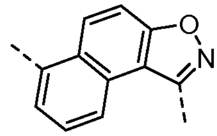,

and other variables are as defined in the present disclosure.

In some embodiments of the present disclosure, the structural moiety

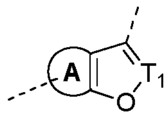

is selected from

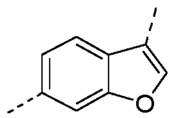, 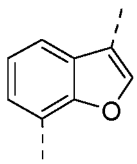, 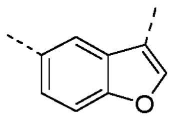, 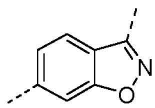, 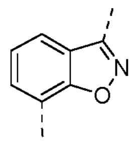,

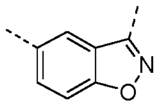, 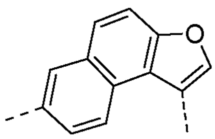, 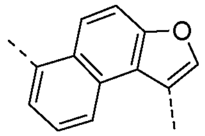, 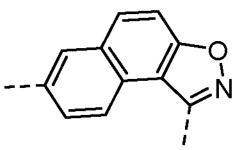, or 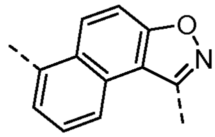,

and other variables are as defined in the present disclosure.

The present disclosure also provides compounds of formulas (II-1), (II-2), (II-3), and (II-4), a stereoisomer thereof, or a pharmaceutically acceptable salt thereof,

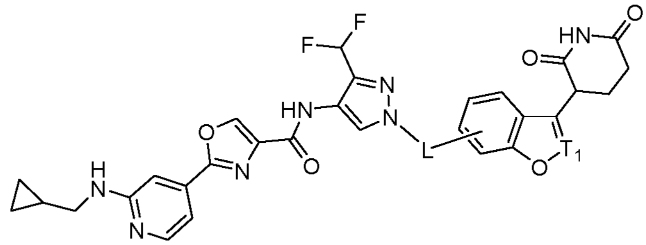

( II-1 )

,

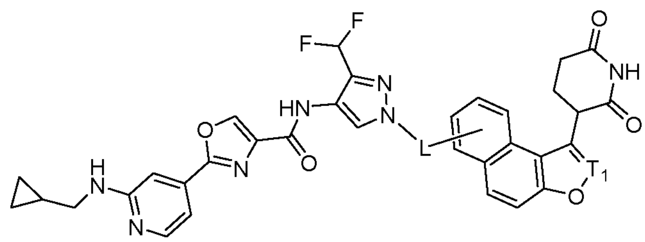

( II-2 )

,

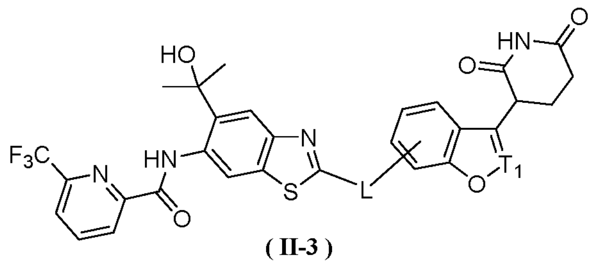

( II-3 )

,

and

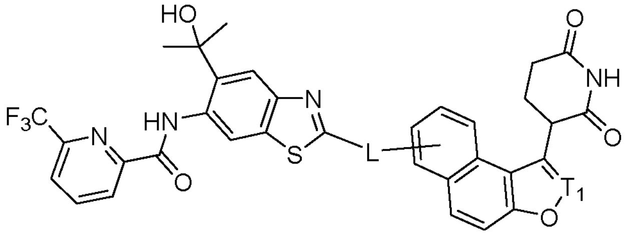

( II-4 )

,

wherein L and $T_1$ are as defined in formula (II).

The present disclosure also provides compounds of formulas (II-1a), (II-2a), (II-3a), and (II-4a), a stereoisomer thereof, or a pharmaceutically acceptable salt thereof,

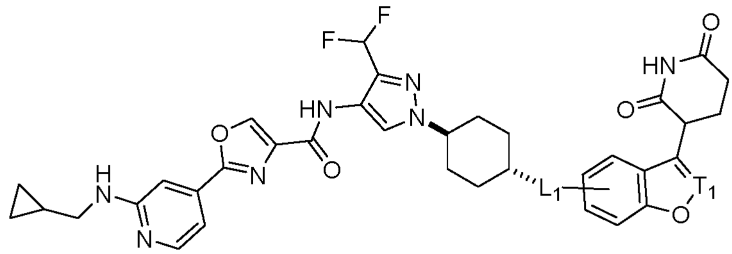

( II-1a )

,

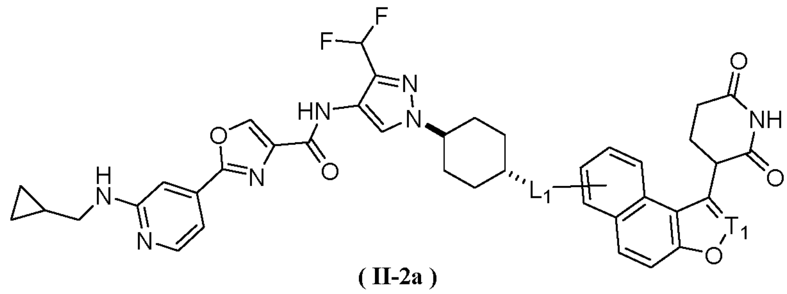

( II-2a )

,

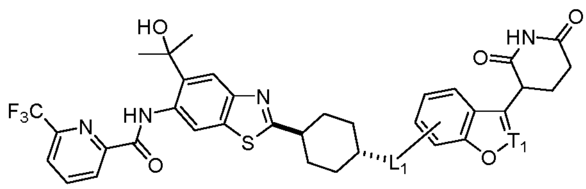

( II-3a )

, ;

and

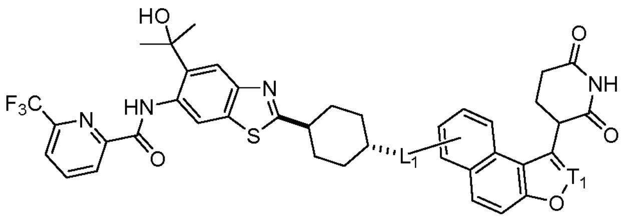

( II-4a )

,

wherein

$L_1$ is selected from -$C_{1-3}$ alkylene-Ra-$C_{1-3}$ alkylene-Ra-$C_{0-3}$ alkylene-, -$C_{1-3}$ alkylene-4- to 8-membered heterocycloalkyl-$C_{1-3}$ alkylene-Ra-, and-$C_{1-3}$ alkylene-4- to 8-membered heterocycloalkyl-$C_{1-3}$ alkylene-, and each $C_{1-3}$ alkylene is independently and optionally substituted by 1 or 2 halogen atoms;
$T_1$ and Ra are as defined in formula (II).

The present disclosure also provides compounds of formula (I), a stereoisomer thereof, or a pharmaceutically acceptable salt thereof,

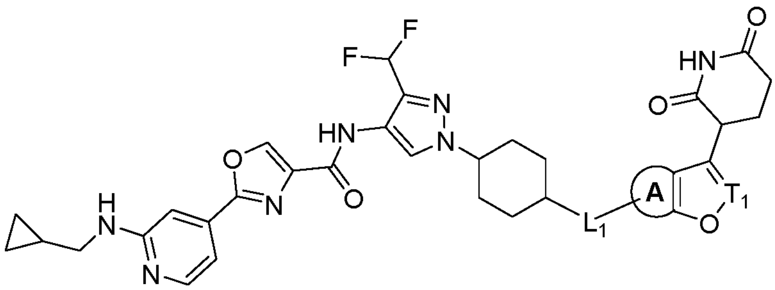

( I )

,

wherein

$L_1$ is selected from $C_{1-10}$ alkylene, wherein 1, 2, or 3 $CH_2$ are optionally substituted by Ra, and the $C_{1-10}$ alkylene is optionally substituted by 1 or 2 halogen atoms;
Ra is selected from -N(R)-, -O-, -C(O)NH-, -$C_{3-6}$ cycloalkyl-, or -4- to 8-membered heterocycloalkyl-;
R is selected from H or $C_{1-4}$ alkyl;
$T_1$ is selected from CH or N;
ring A is selected from $C_{6-10}$ aryl or 5- to 10-membered heteroaryl;
the "4- to 8-membered heterocycloalkyl and 5- to 10-membered heteroaryl" each contains 1, 2 or 3 heteroatoms independently selected from N, O, S, or Se.

In some embodiments of the present disclosure, the Ra in formula (I) is selected from -N($CH_3$)-, -O-, -C(O)NH-, -cyclopropyl- or -piperazinyl-, and other variables are as defined in the present disclosure.
In some embodiments of the present disclosure, the Ra in formula (I) is selected from -N($CH_3$)-, -O-, -C(O)NH-, or

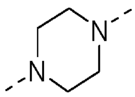

,

and other variables are as defined in the present disclosure.
In some embodiments of the present disclosure, the $L_1$ in formula (I) is selected from

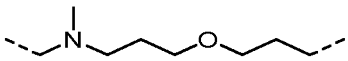, 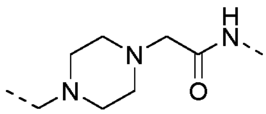, or 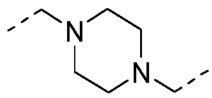,

and other variables are as defined in the present disclosure.

In some embodiments of the present disclosure, the ring A in formula (I) is selected from phenyl or naphthyl, and other variables are as defined in the present disclosure.

In some embodiments of the present disclosure, the structural moiety

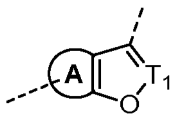

in formula (I) is selected from

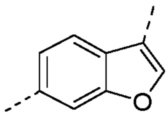, 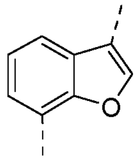, 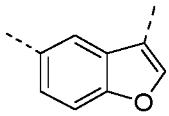, 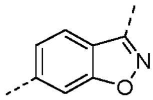,

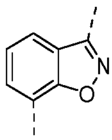, 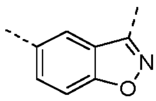, 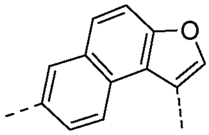, 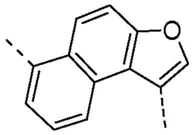, 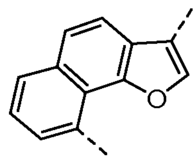, 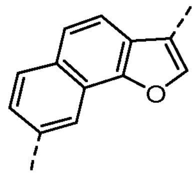,

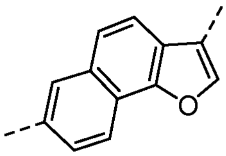, 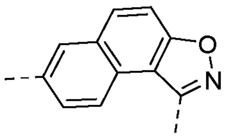, or 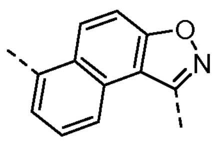,

and other variables are as defined in the present disclosure.

The present disclosure also provides compounds of formulas (I-1), (I-2), and (I-3), a stereoisomer thereof, or a pharmaceutically acceptable salt thereof,

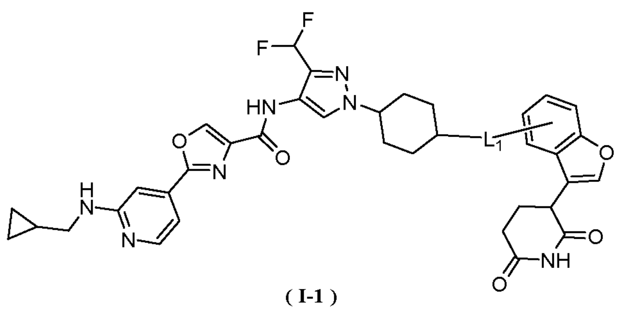

(I-1)

,

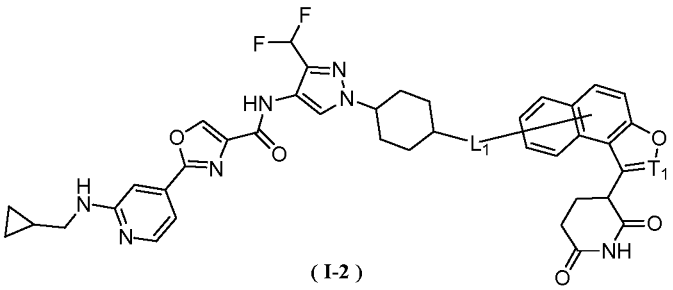

( I-2 )

, and

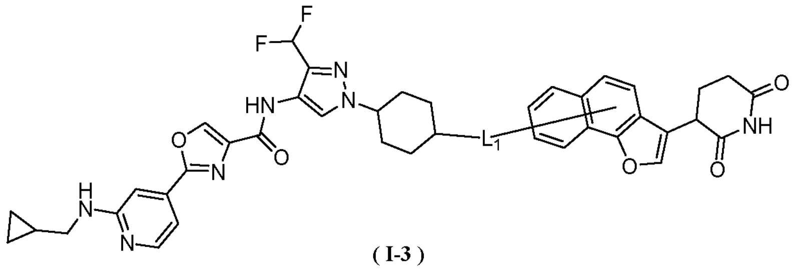

( I-3 )

,

wherein $L_1$ and $T_1$ are as defined in the present disclosure.

The present disclosure also provides a compound, a stereoisomer thereof, or a pharmaceutically acceptable salt thereof as shown below,

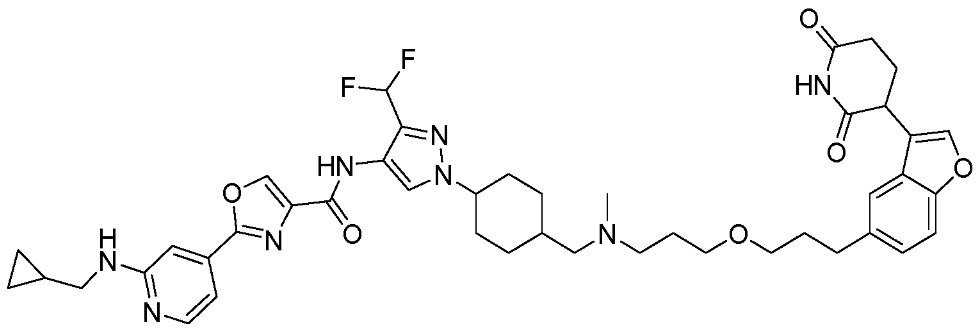

,

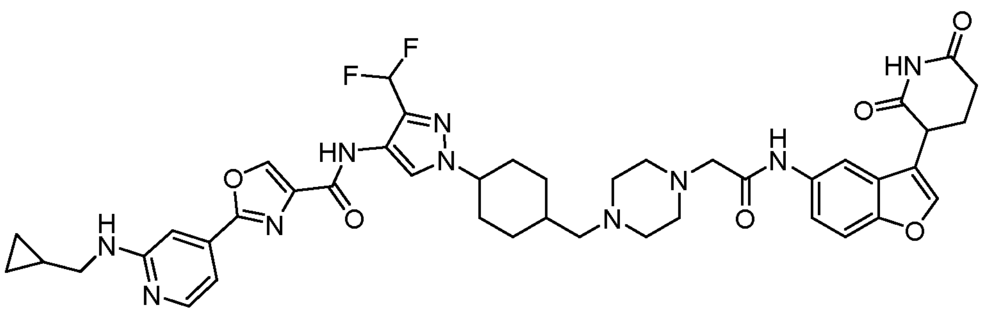

,

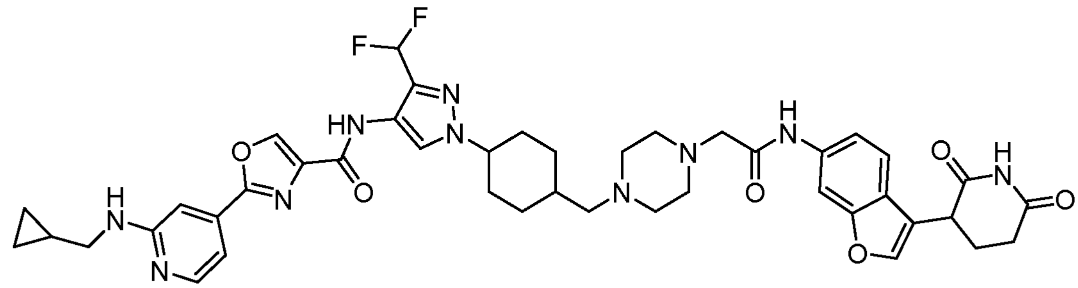

,

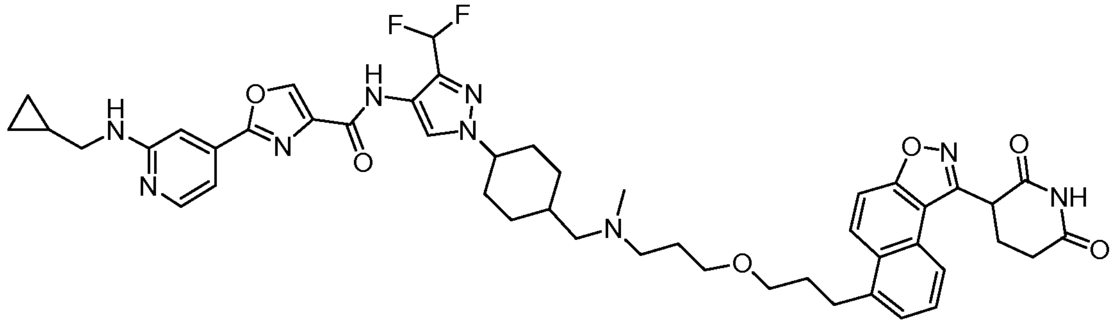

,

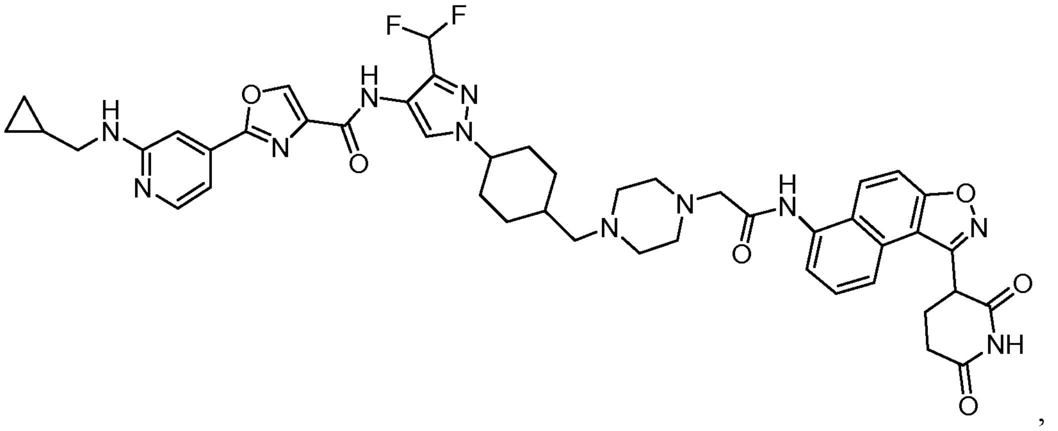

,

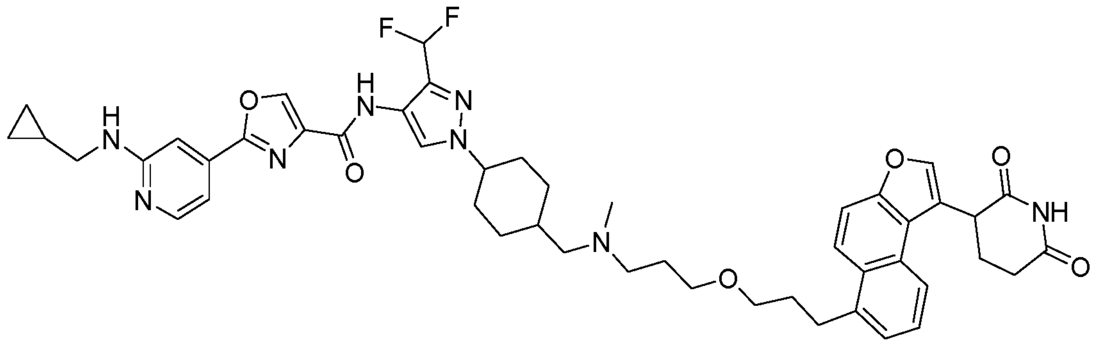

,

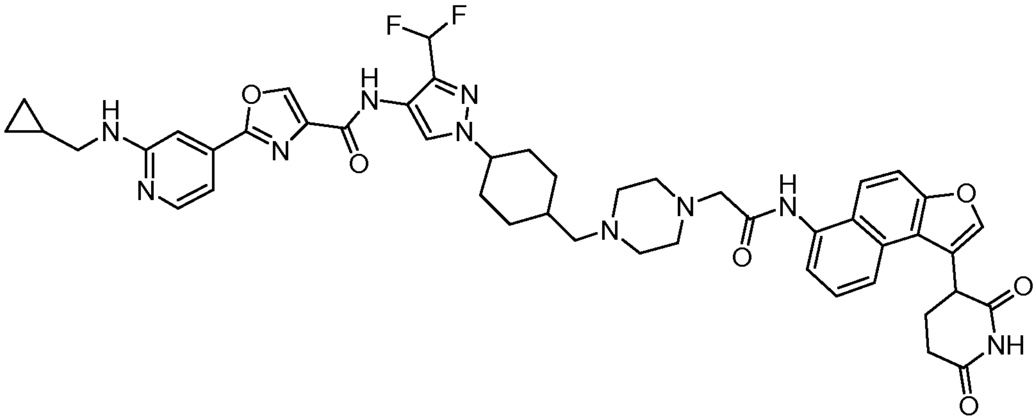

,

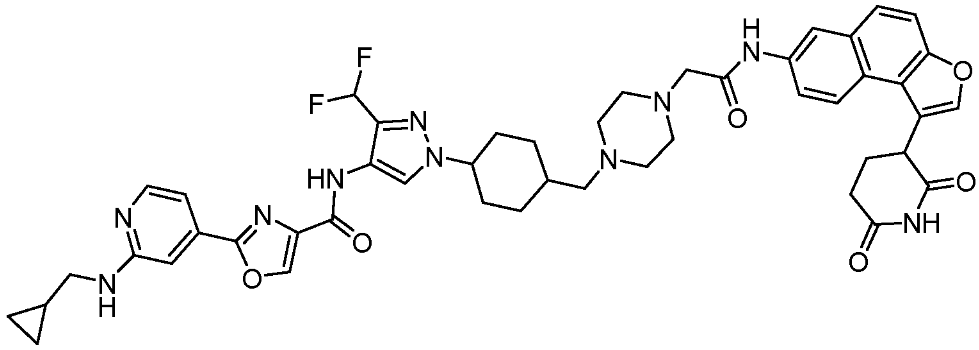

,

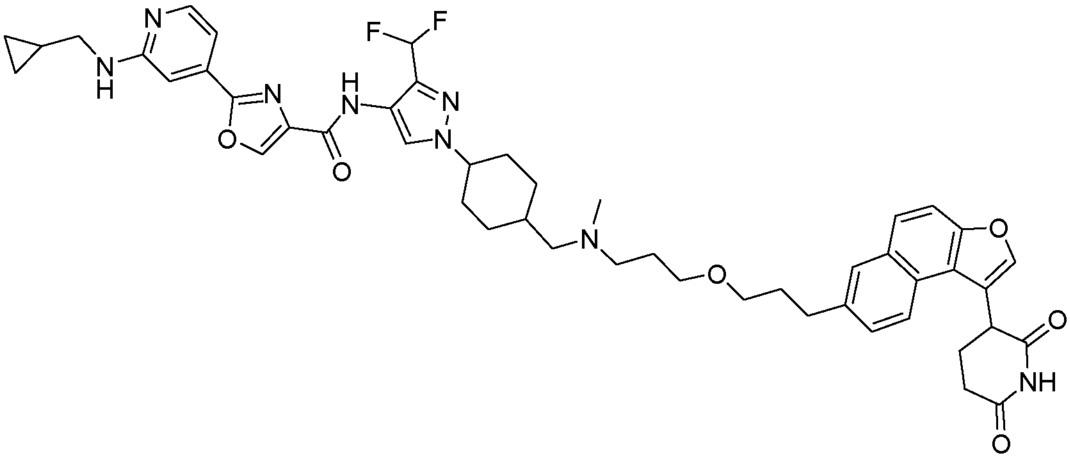

,

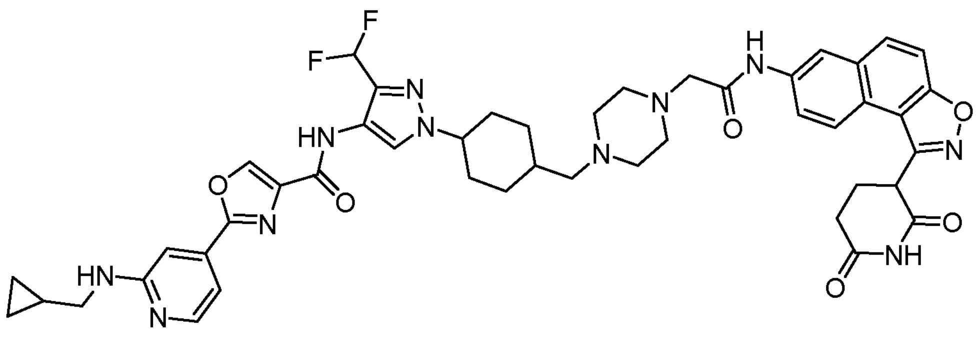

,

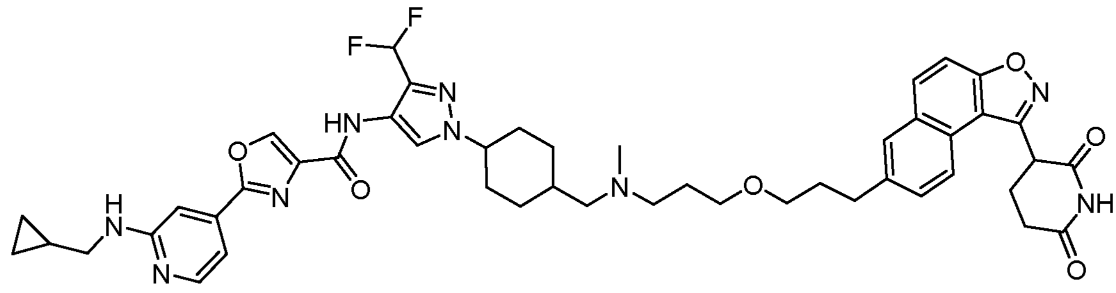

,

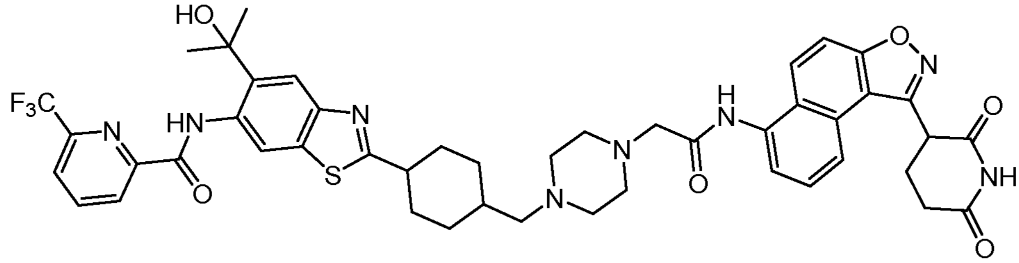

,

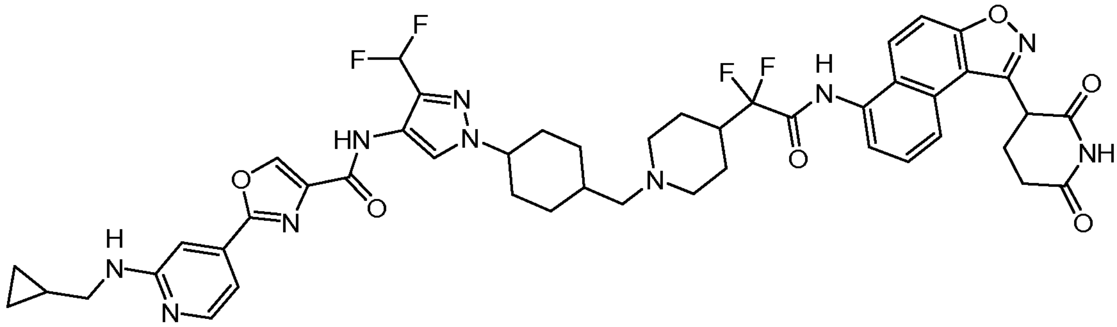

,

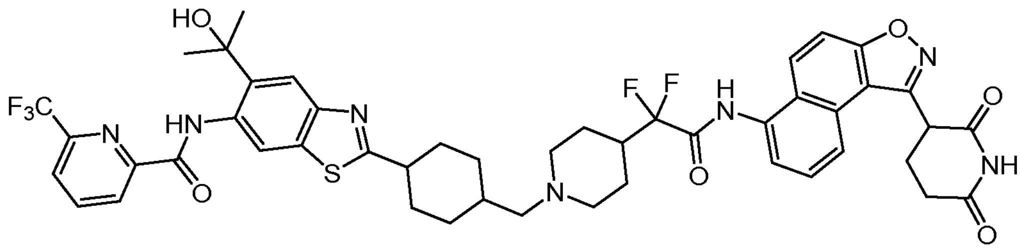

,

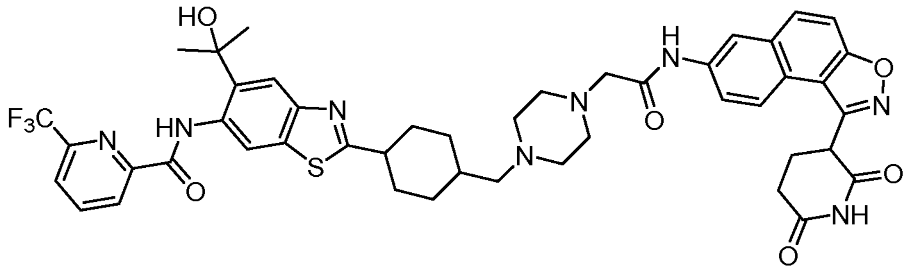

,

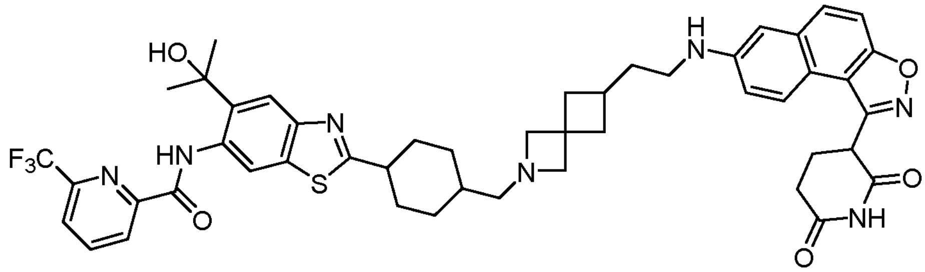

,

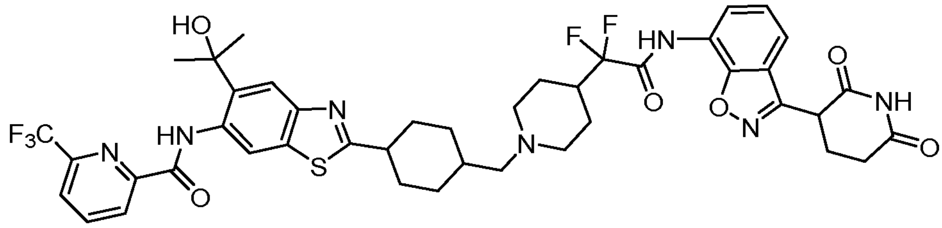

,

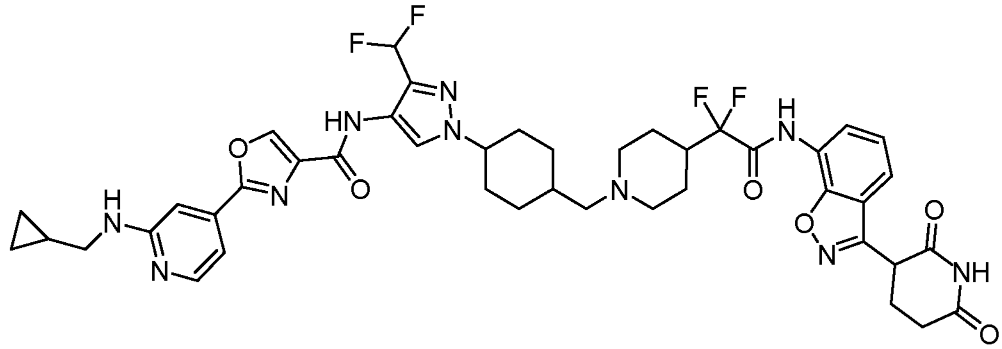

,

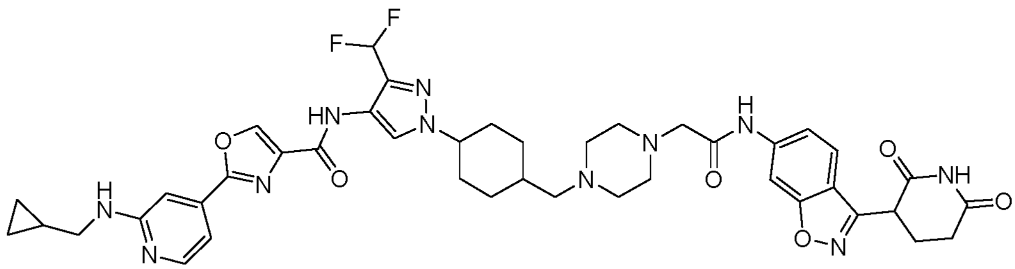

,

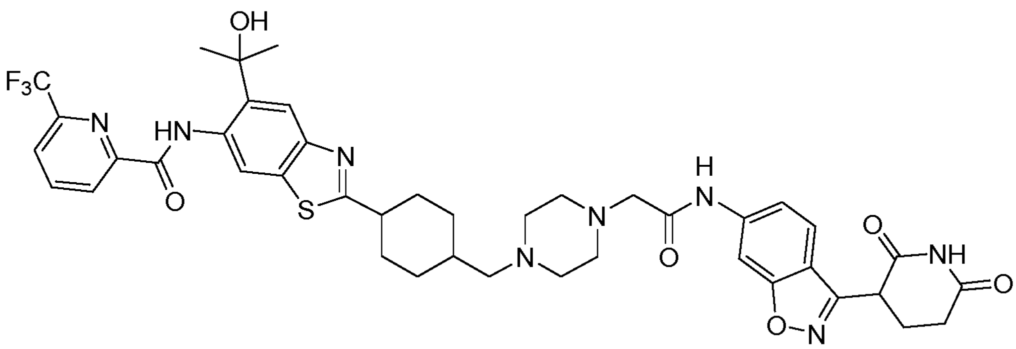

,

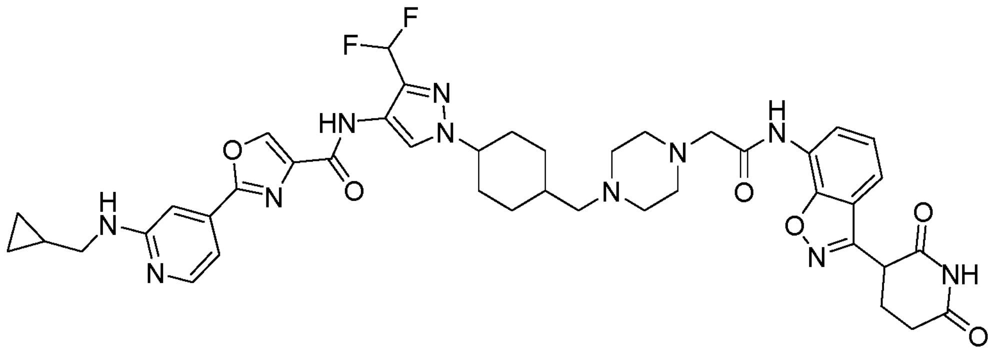,

and

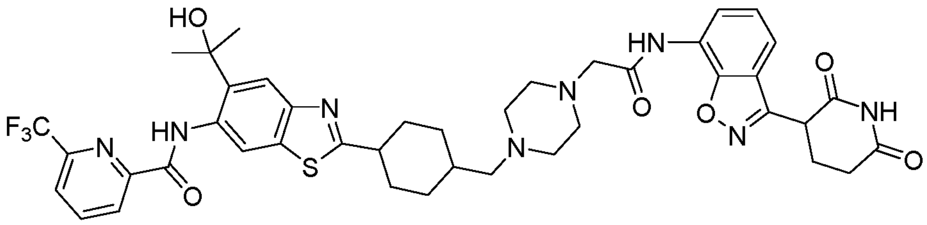.

The present disclosure also provides a compound, a stereoisomer thereof, or a pharmaceutically acceptable salt thereof as shown below,

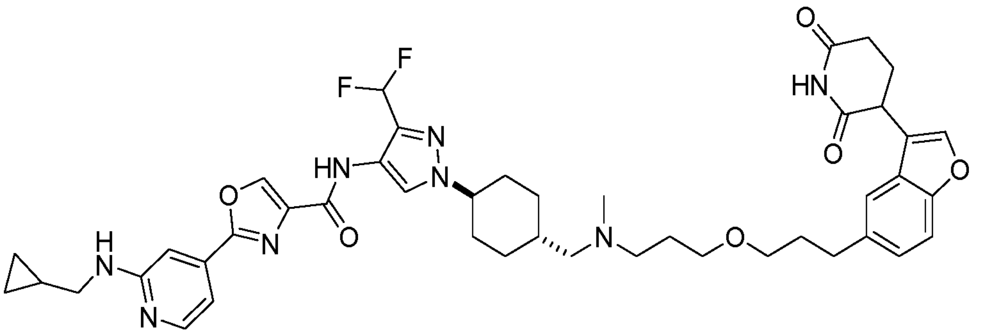,

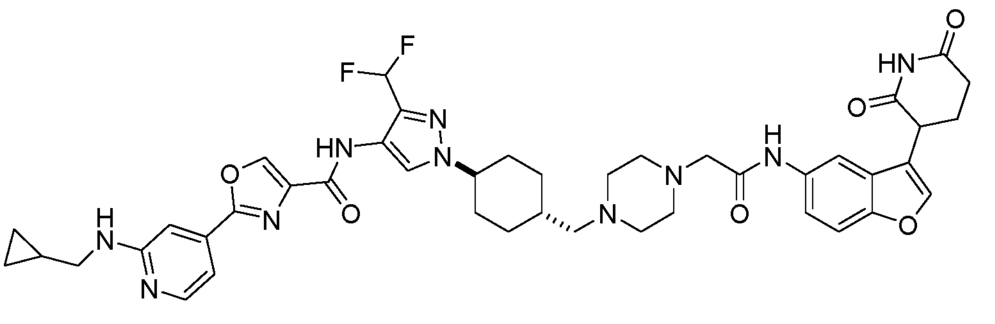,

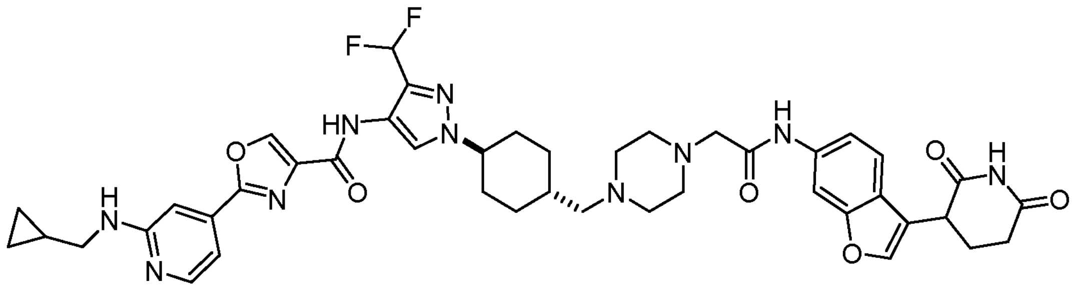

,

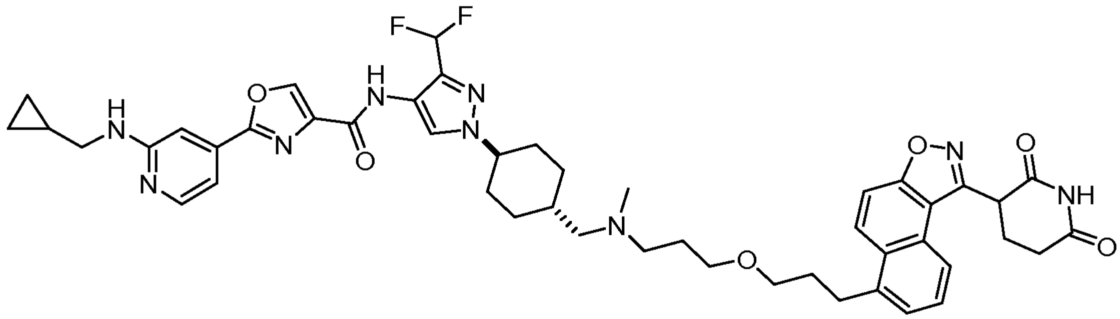

,

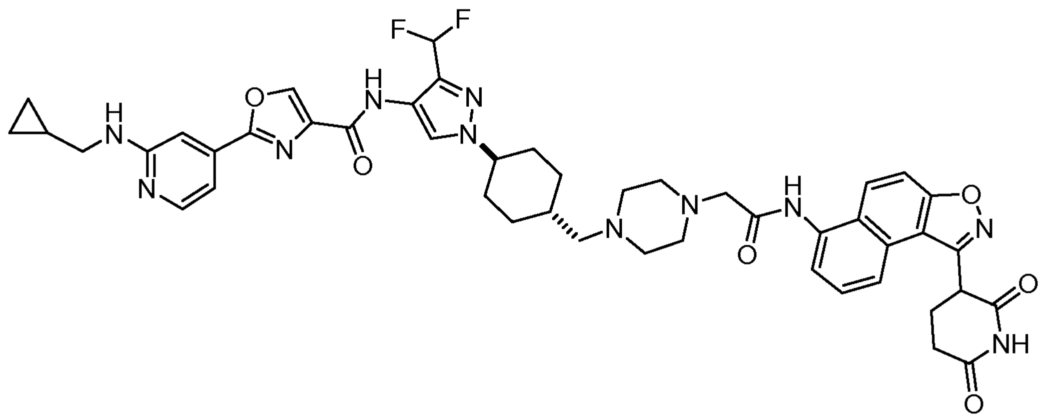

,

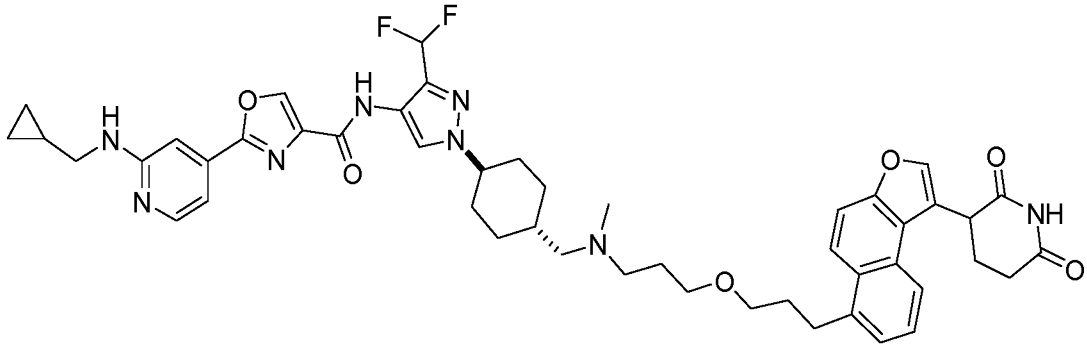

,

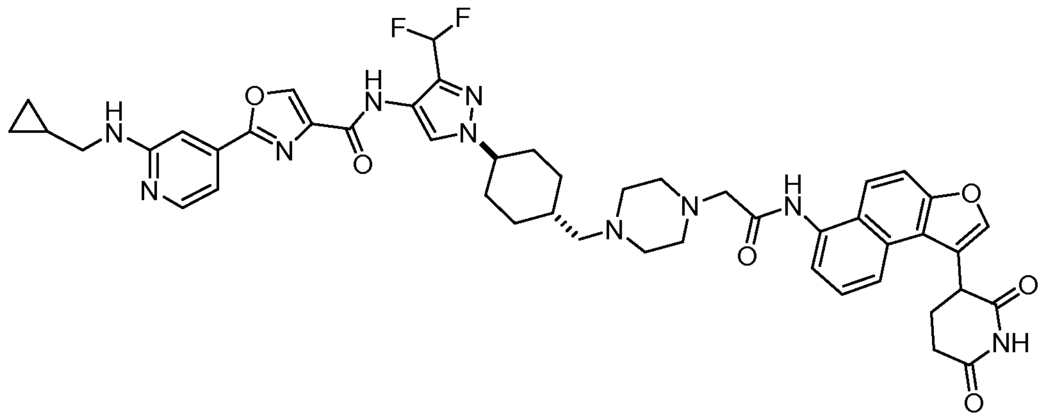

,

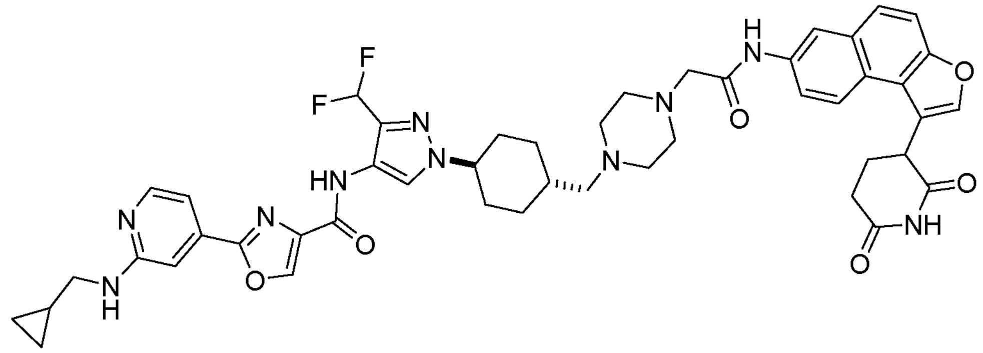

,

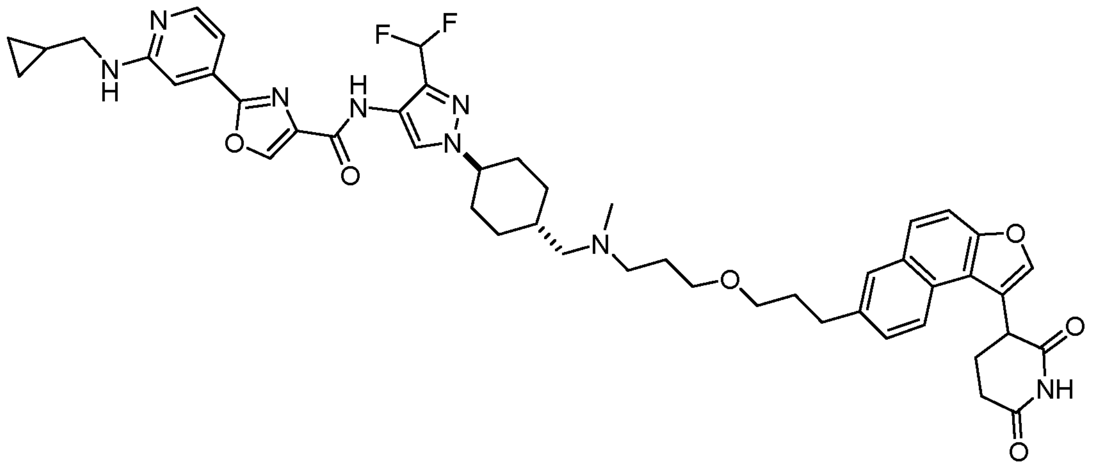

,

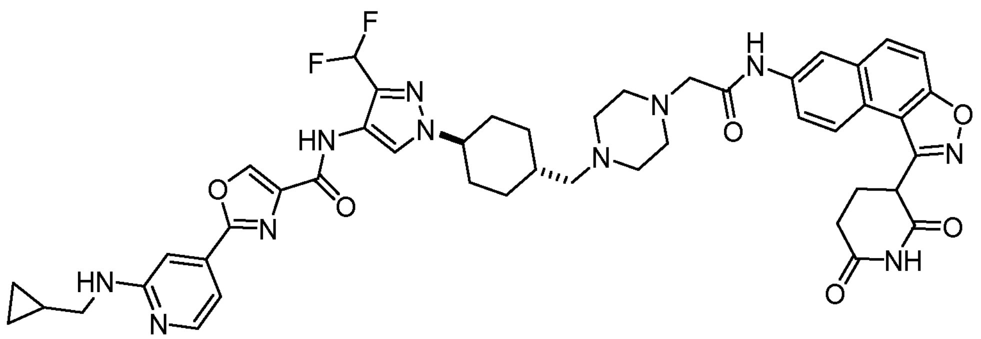

,

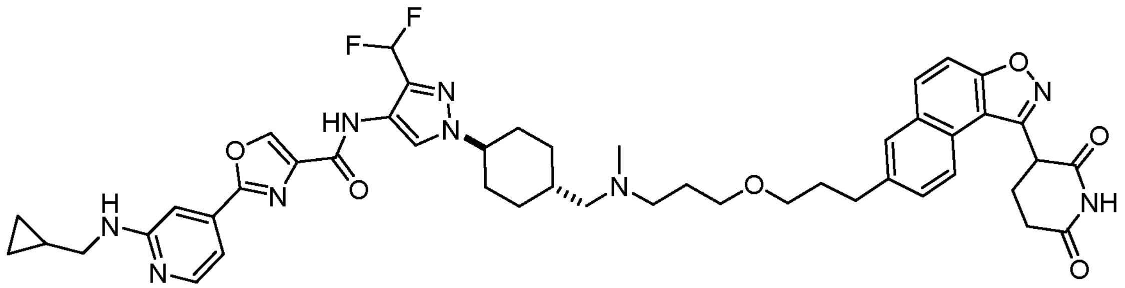

,

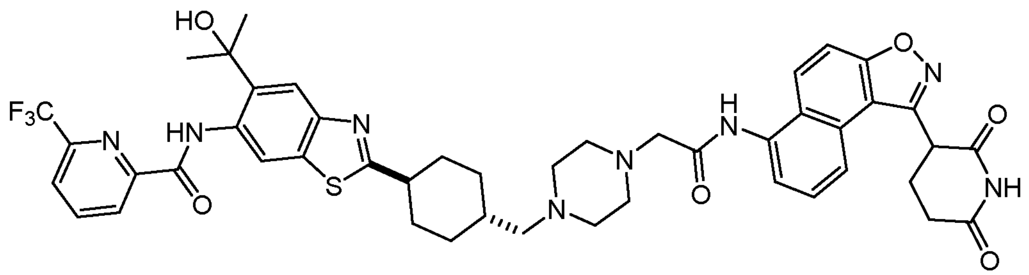

,

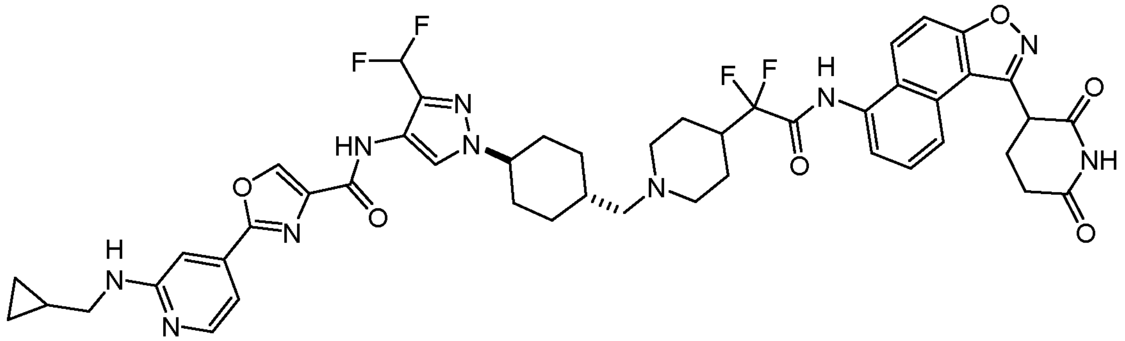

,

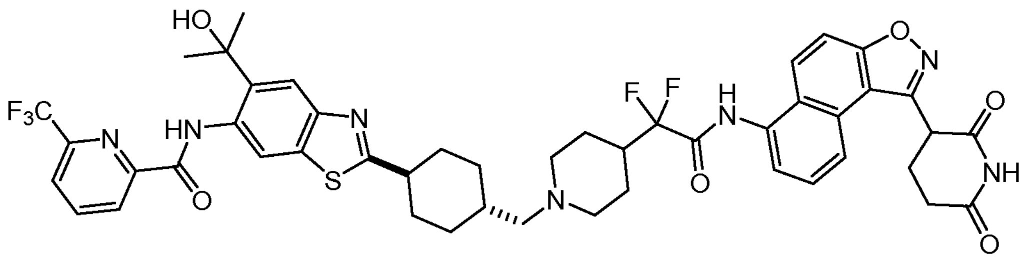

,

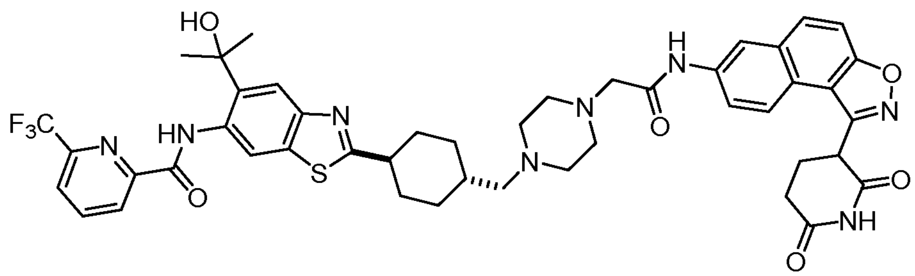

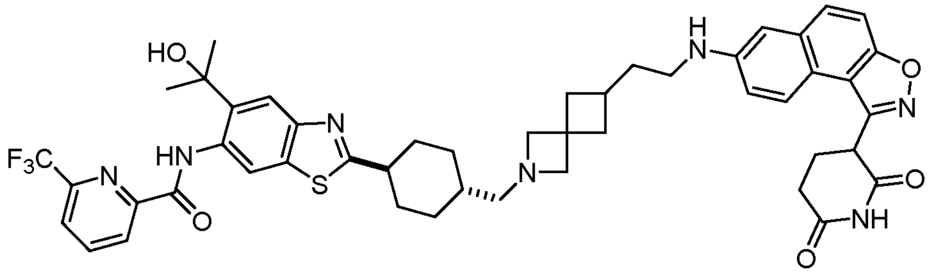

,

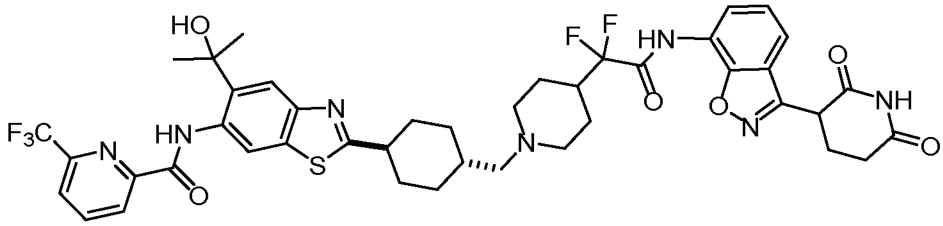

,

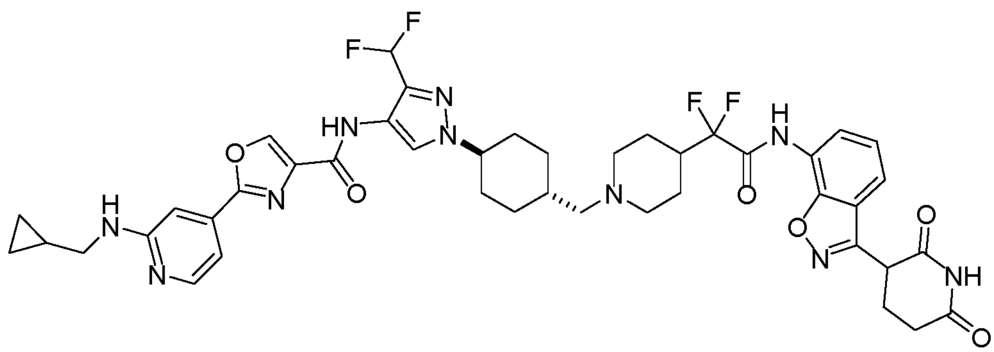

,

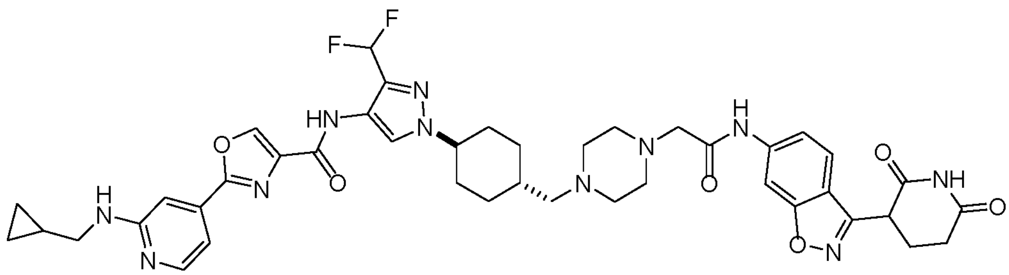

,

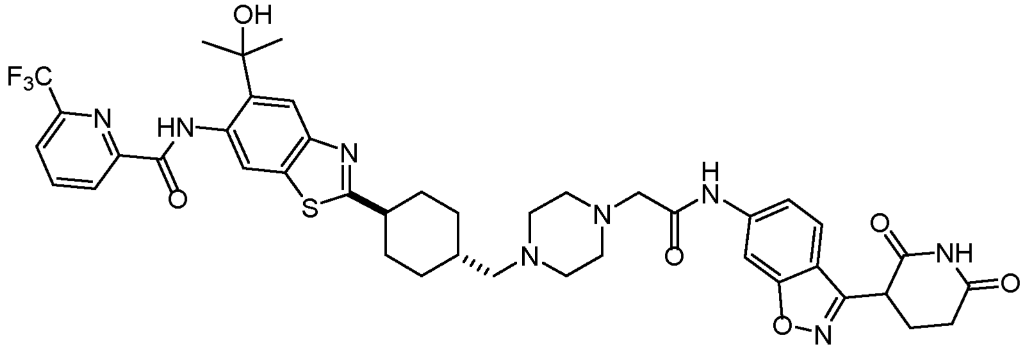

,

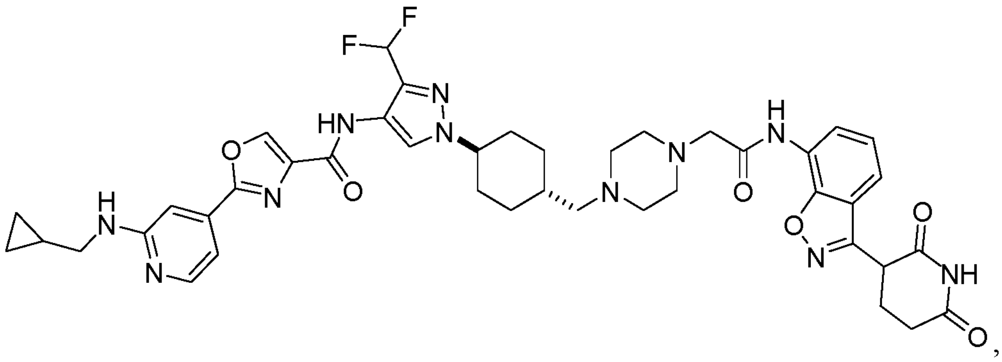

and

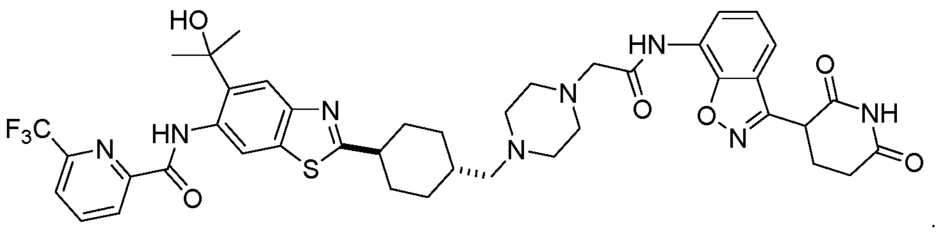

In some embodiments of the present disclosure, the compound is selected from:

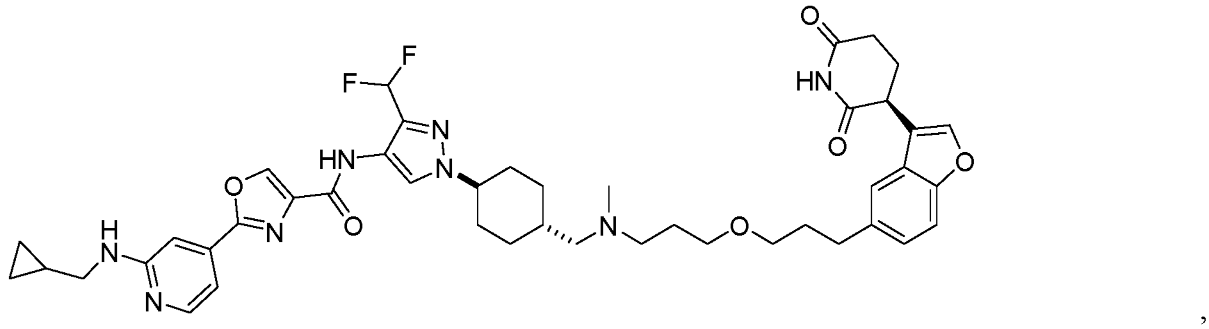

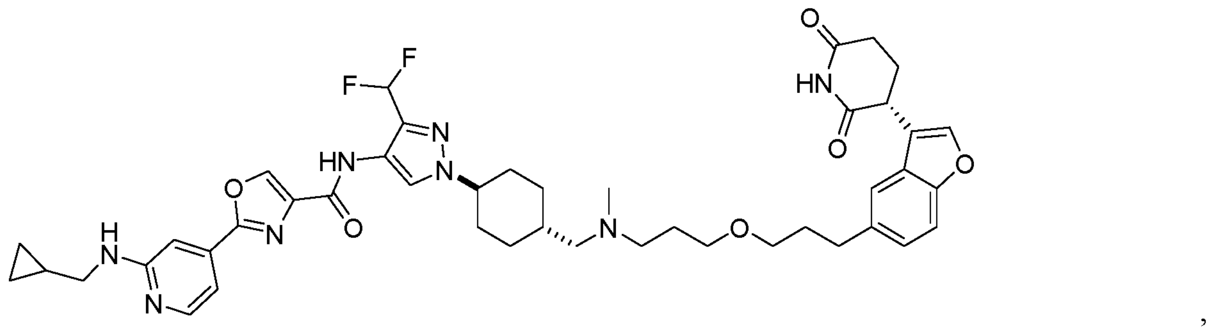

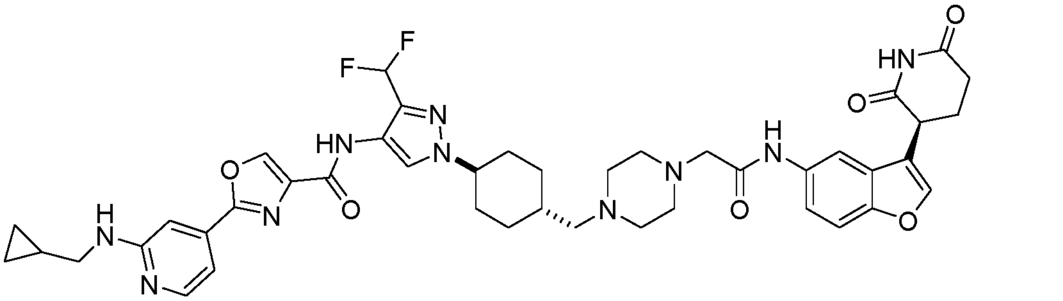

,

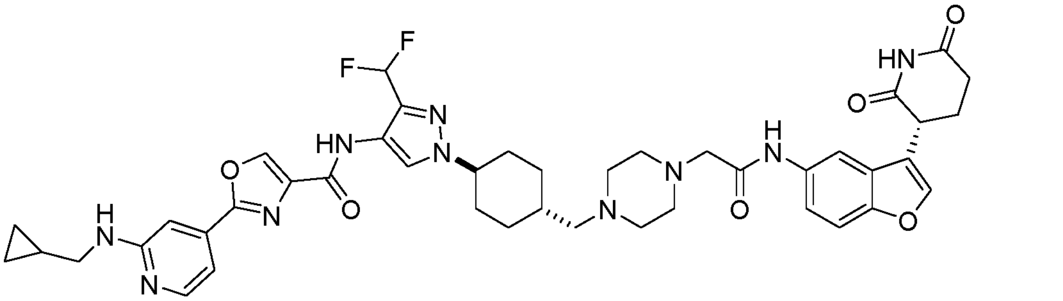

,

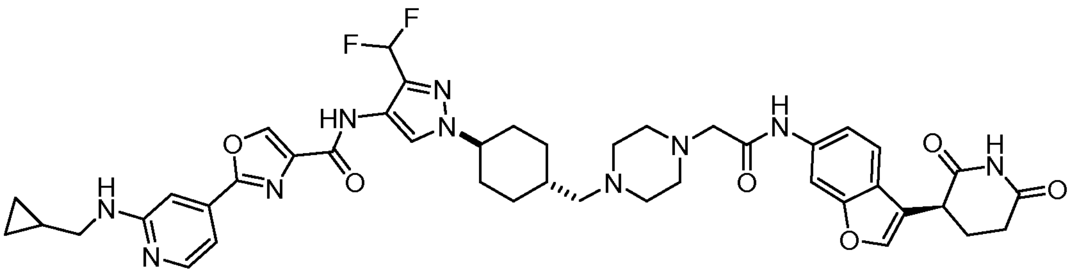

,

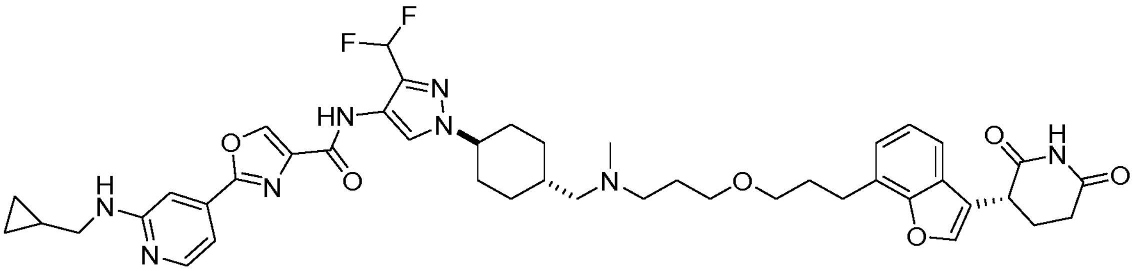

,

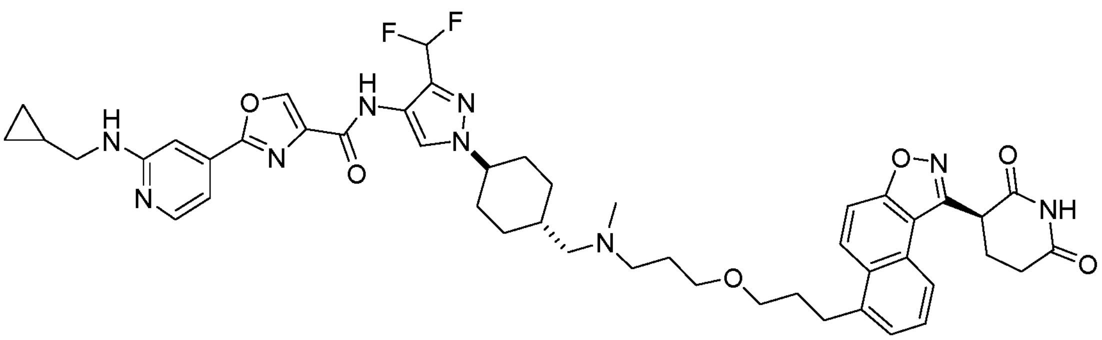

,

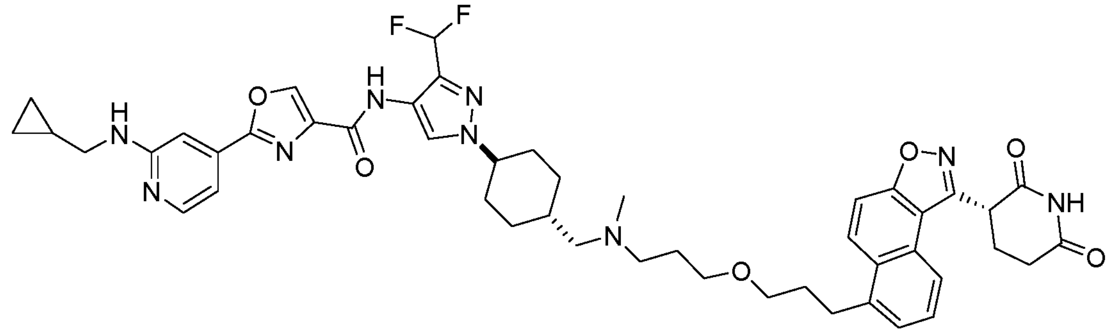

,

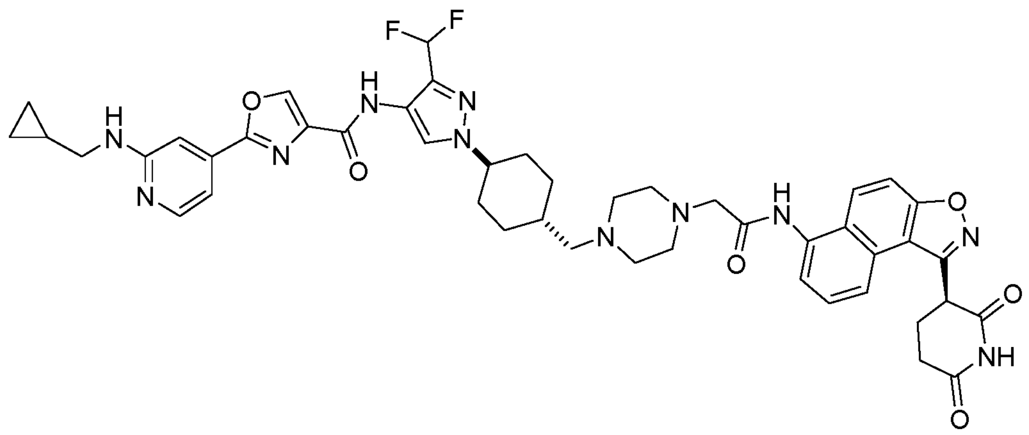

,

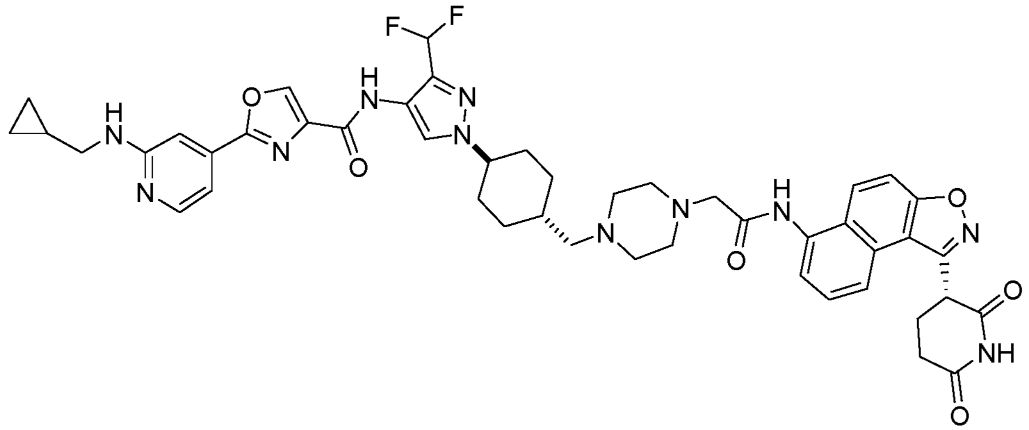

,

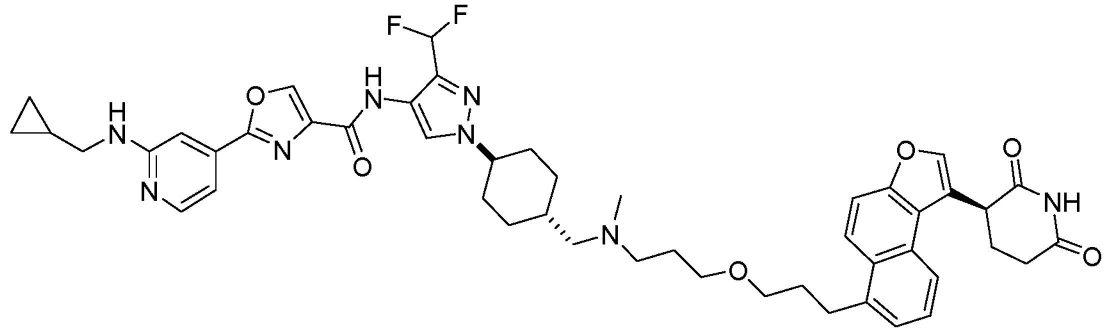

,

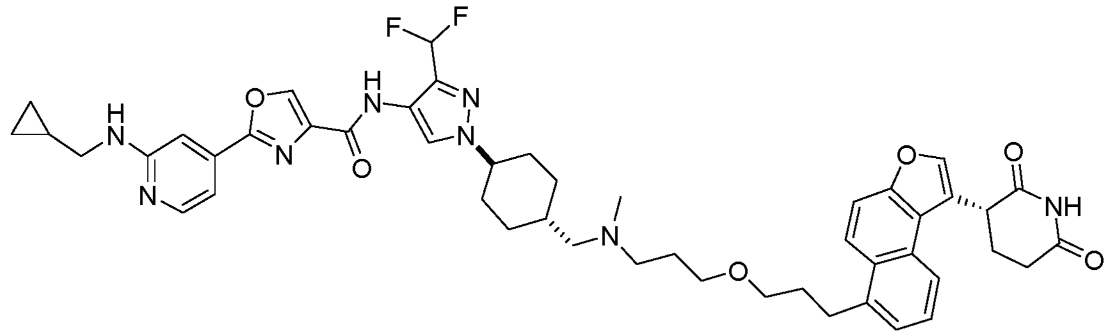

,

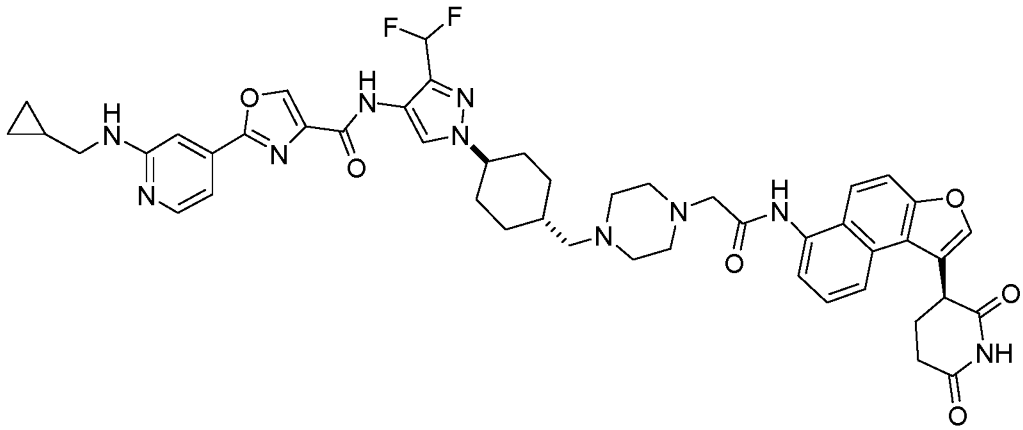

,

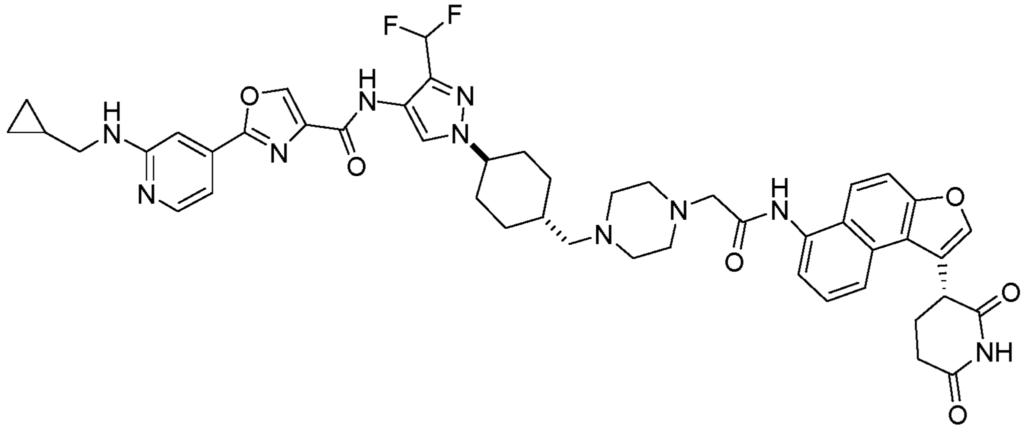

,

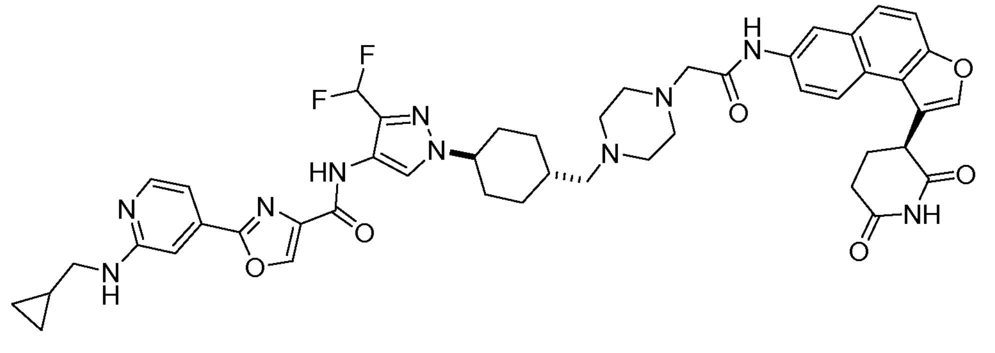

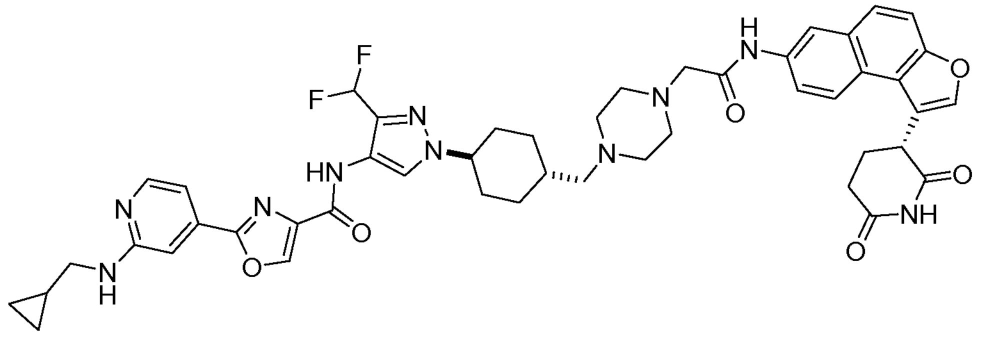

,

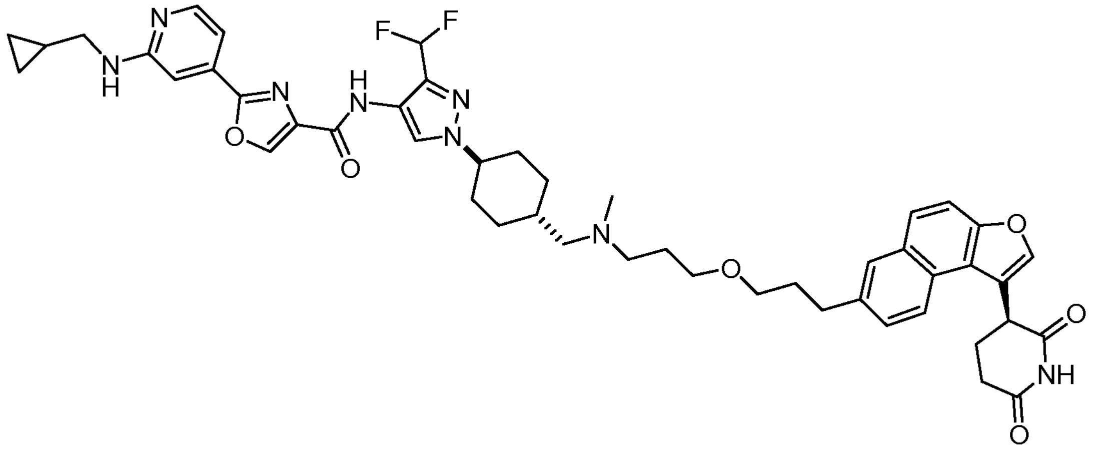

,

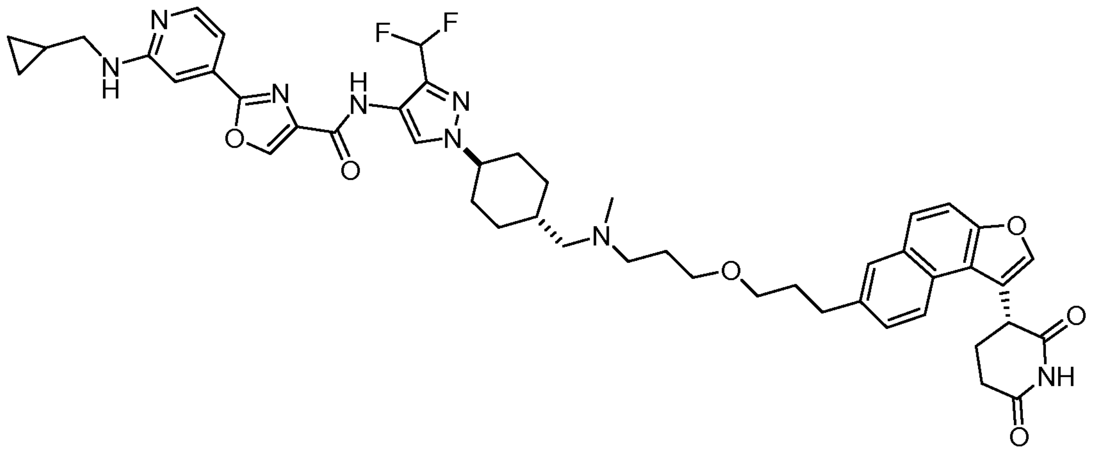

,

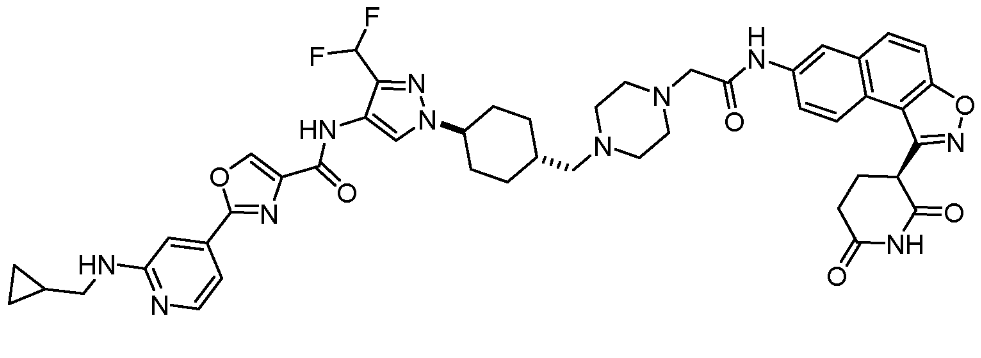

,

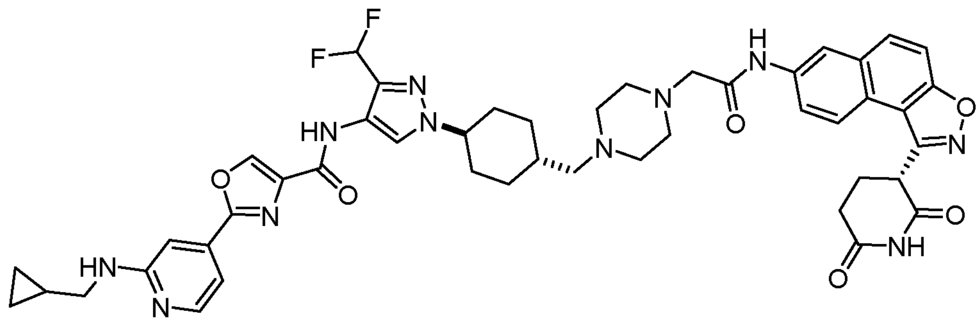

,

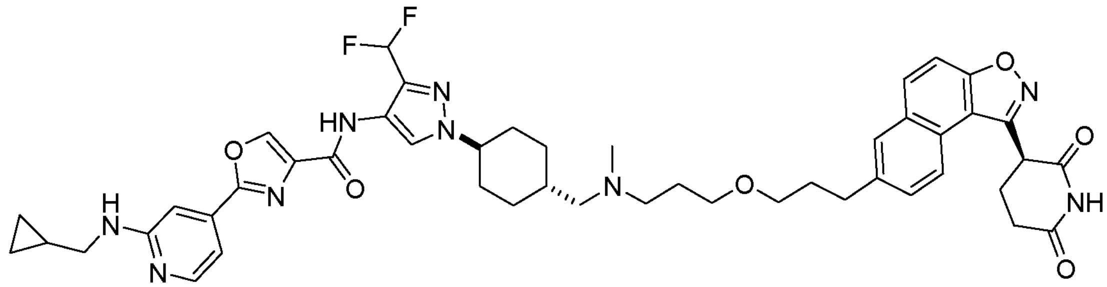

,

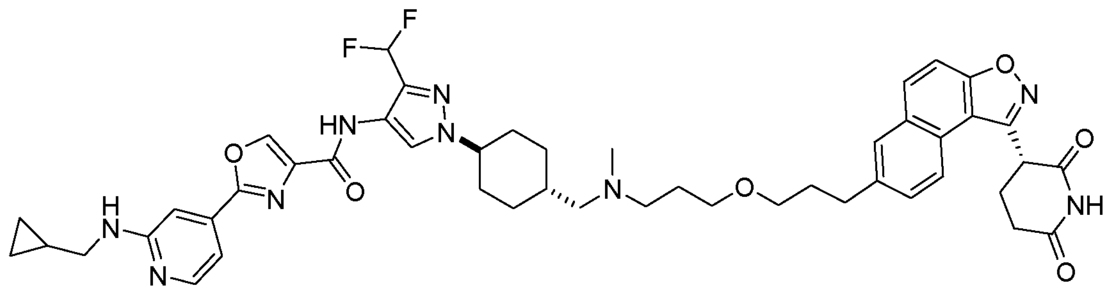

,

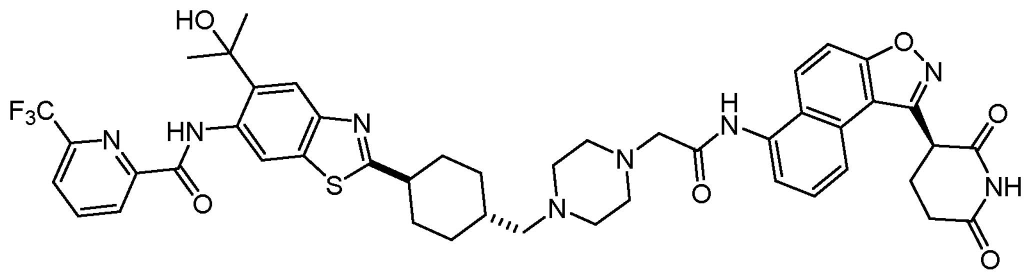

,

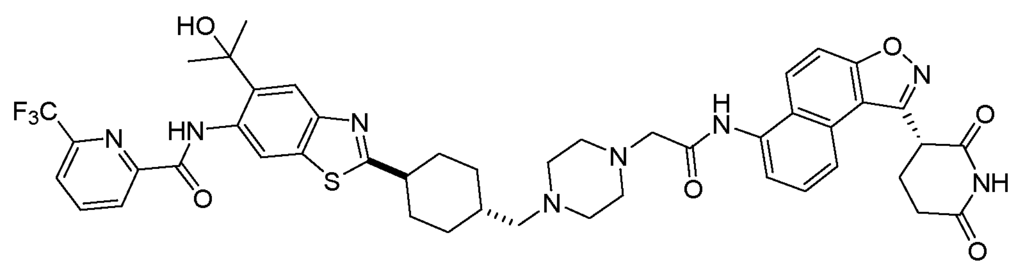

,

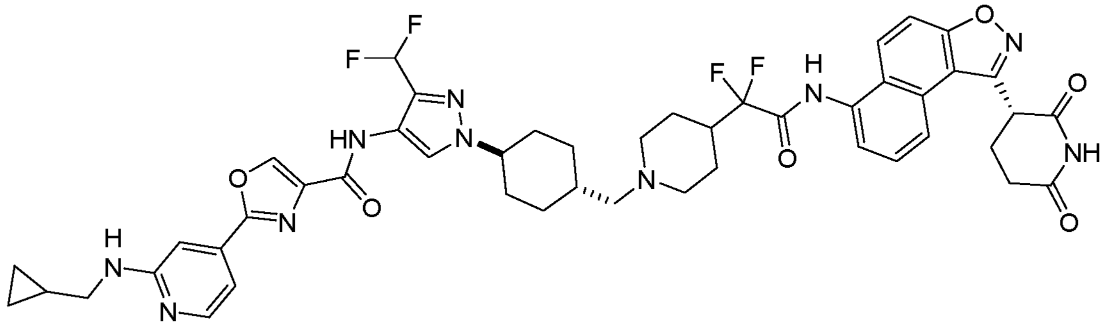

,

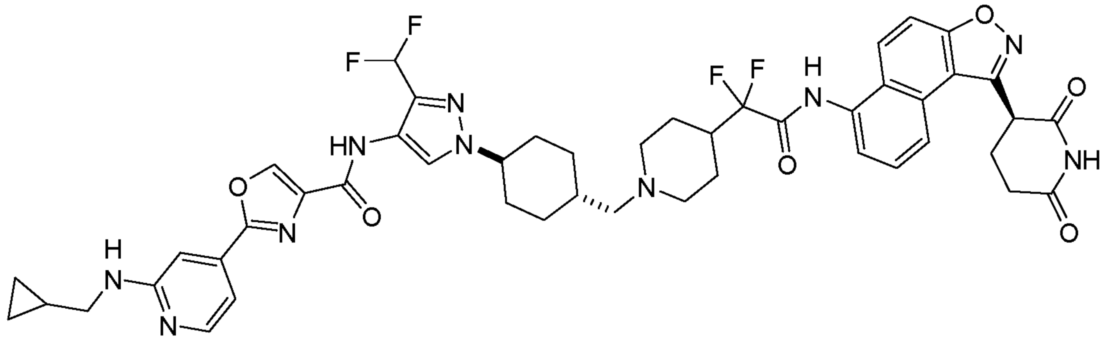

,

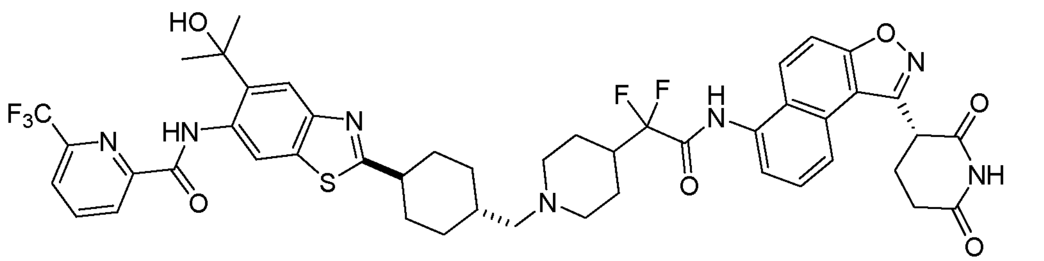

,

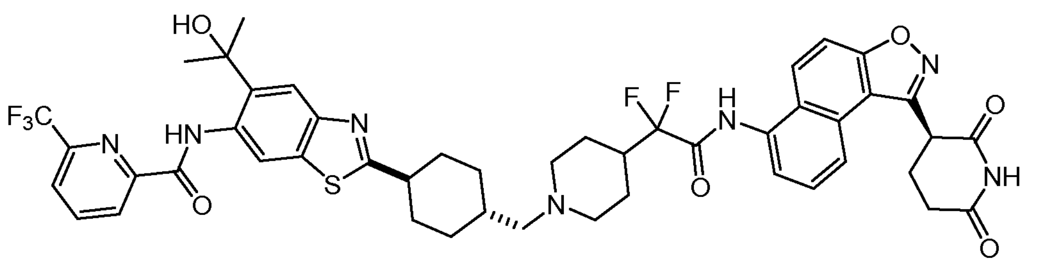

,

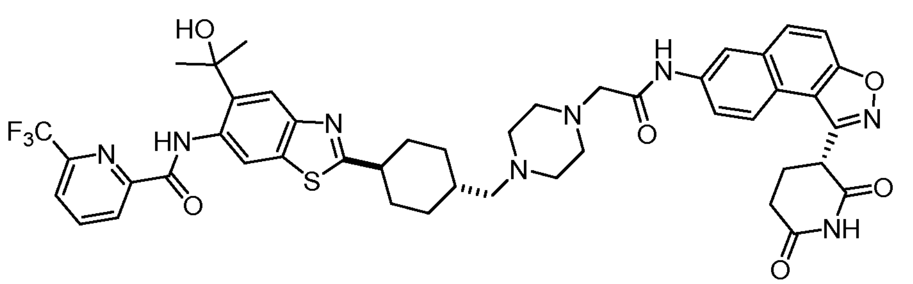

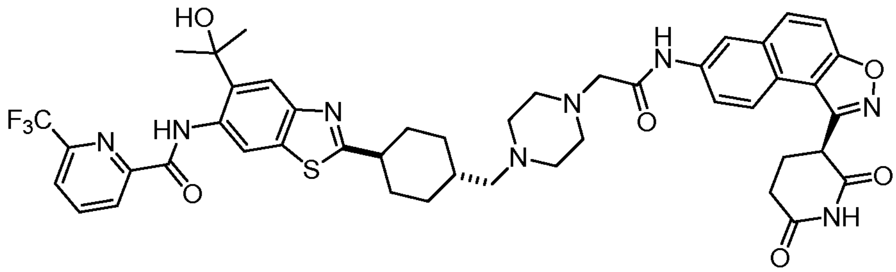

,

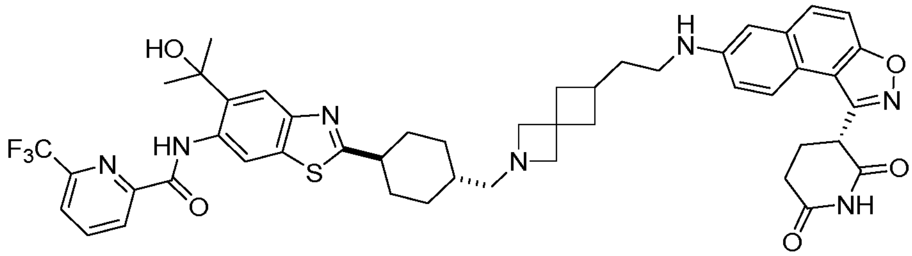

,

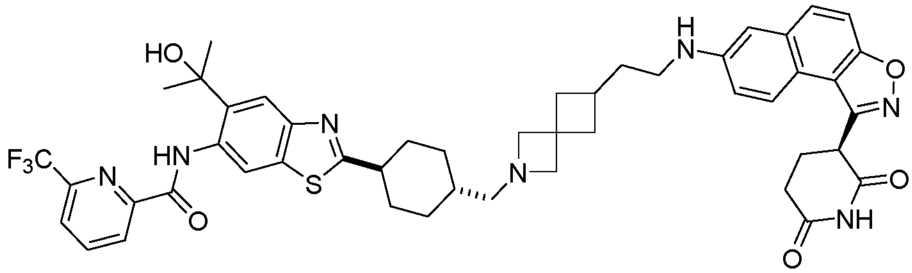

,

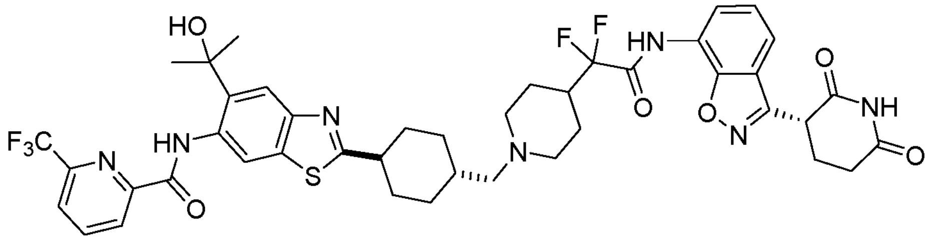

,

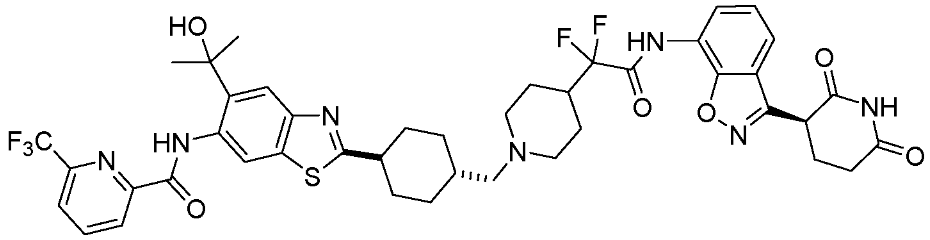

,

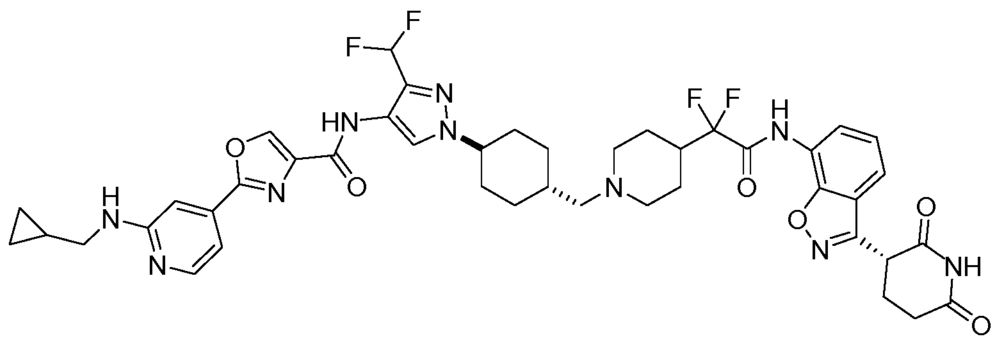

,

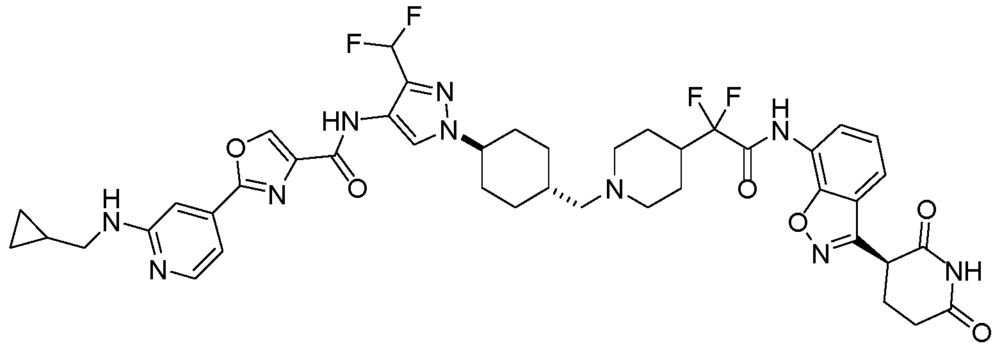

,

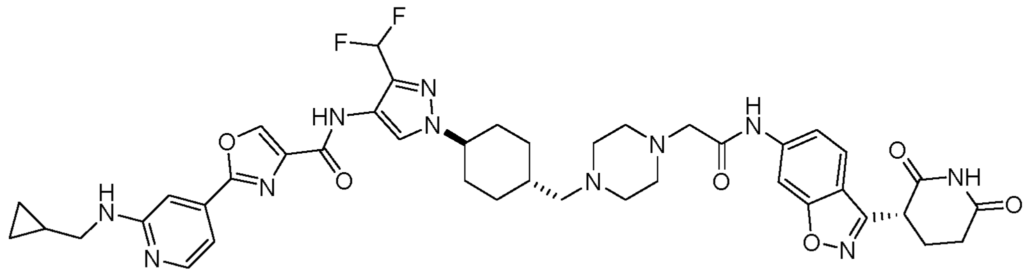

,

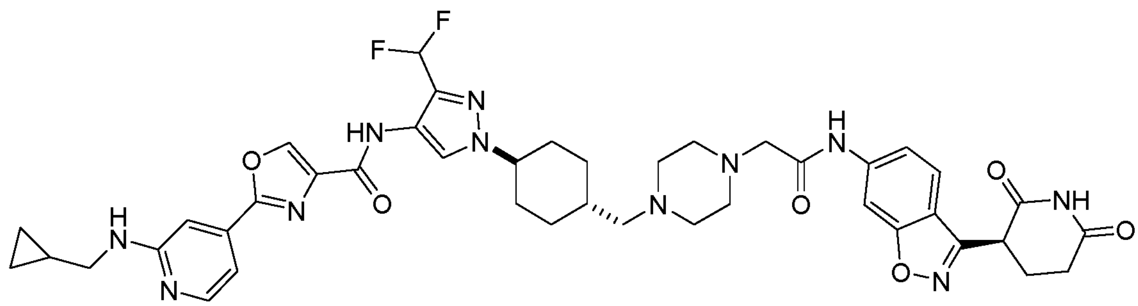

,

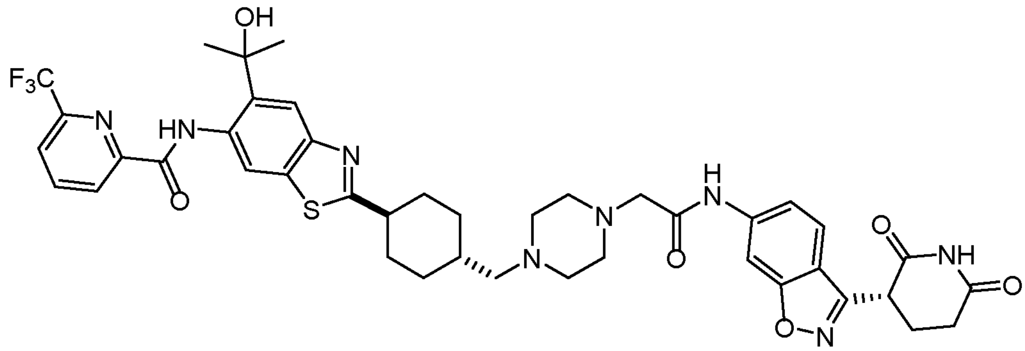

,

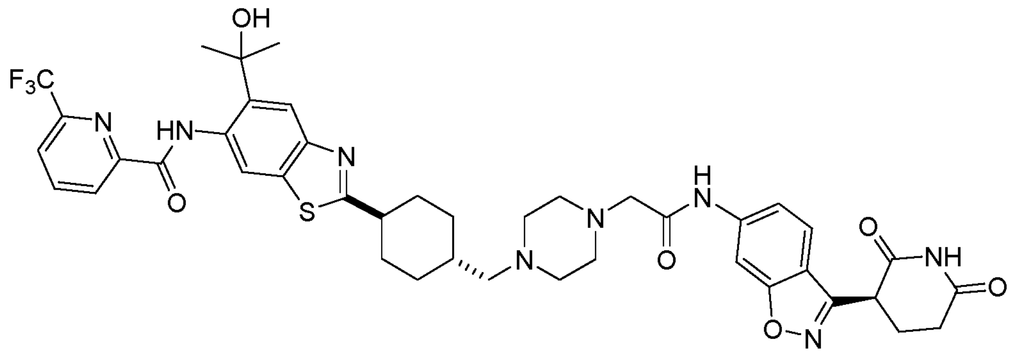

,

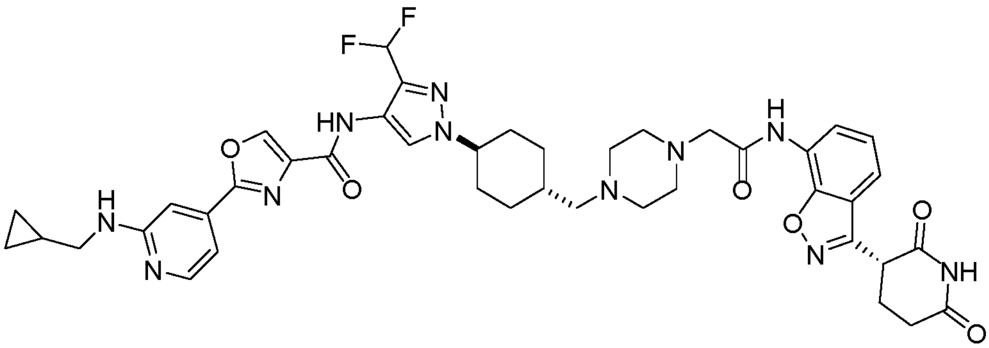

,

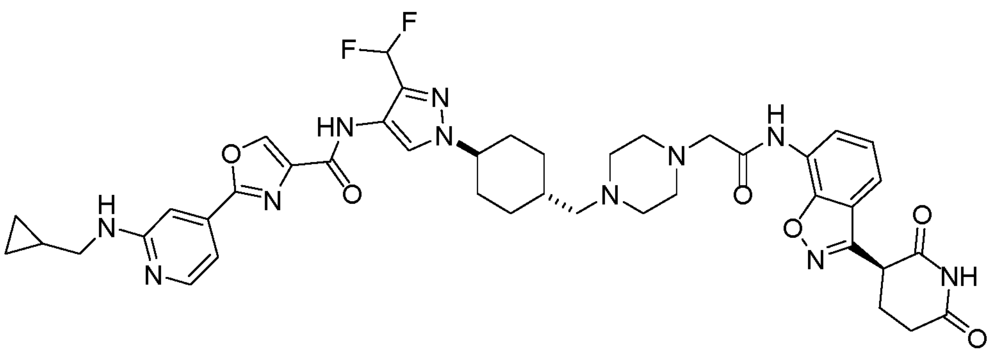

,

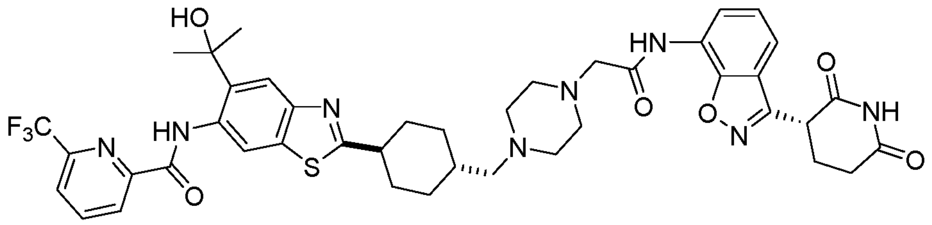

,

and

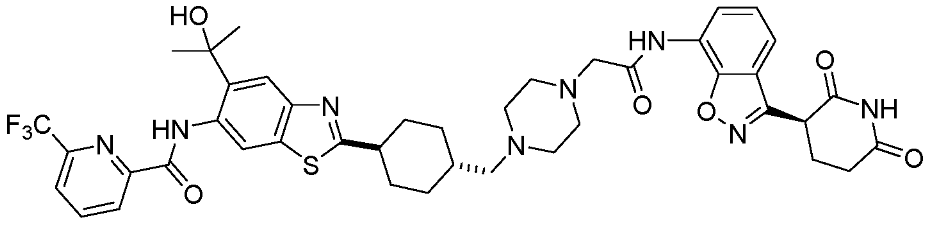

.

The present disclosure also provides a pharmaceutical composition comprising a therapeutically effective amount of the compound of the present disclosure, the stereoisomer thereof, or the pharmaceutically acceptable salt thereof.

The present disclosure also provides a use of the compound, the stereoisomer thereof, the pharmaceutically acceptable salt thereof, and the pharmaceutical composition in the manufacture of a medicament for treating tumors associated with proteolysis targeting chimeras of Interleukin-1 Receptor-Associated Kinase 4.

In some embodiments of the present disclosure, the tumor associated with proteolysis targeting chimeras of Interleukin-1 Receptor-Associated Kinase 4 is B-cell lymphoma.

**Technical effect**

The compounds of the present disclosure exhibit excellent degradation effect on target proteins IRAK4, IKZF1, and IKZF3. The compounds of the present disclosure exhibit excellent inhibitory effect on cell proliferation in lymphoma cell lines OCI-LY10, TMD-8, and SU-DHL-2. The compounds of the present disclosure exhibit high plasma exposure when administered orally. The compounds of the present disclosure exhibit superior pharmacokinetic properties in rodents like mice and rats, and non-rodents like beagles. The compounds of the present disclosure have remarkable tumor-suppressing effects and is dose-dependent in a SCID mouse xenograft tumor model using human B-cell lymphoma OCI-LY10 cells. The compounds of the present disclosure have remarkable tumor-suppressing effects in a CB17 SCID mouse model with subcutaneous xenograft tumors of human lymphoma SU-DHL-2 cells.

**Definition and description**

Unless otherwise specified, the following terms and phrases when used herein have the following meanings. A specific term or phrase should not be considered indefinite or unclear in the absence of a particular definition, but should be understood in the ordinary sense. When a trading name appears herein, it is intended to refer to its corresponding commodity or active ingredient thereof.

The term "pharmaceutically acceptable" is used herein in terms of those compounds, materials, compositions, and/or dosage forms, which are suitable for use in contact with human and animal tissues within the scope of reliable medical judgment, with no excessive toxicity, irritation, an allergic reaction, or other problems or complications, commensurate with a reasonable benefit/risk ratio.

"Pharmaceutical composition" refers to a formulation containing one or more of the compounds of the present disclosure, the stereoisomer thereof, or the pharmaceutically acceptable salts thereof, as well as other components such as physiologically/pharmaceutically acceptable carriers and excipients. The purpose of the pharmaceutical compositions is to facilitate administration to the organism and enhance the absorption of the active ingredients, and thereby exert biological activity.

The term "targeting chimera" refers to a bifunctional molecule containing two small-molecule ligands, one with high affinity for a target protein and the second for recruiting an E3 ligase. The E3 ligase ubiquitinates the protein and target it for proteolysis via the 26S proteasome.

The term "therapeutically effective amount" or "effective amount" refer to the quantity of a compound of the present disclosure that achieves the following effects: (i) treating or preventing a specific disease, condition, or disorder; (ii) reducing, improving, or eliminating one or more symptoms of a specific disease, condition, or disorder, or (iii) preventing or delaying the onset of one or more symptoms of a specific disease, condition, or disorder described herein. In the case of cancer, a therapeutically effective amount of a drug can reduce the number of cancer cells; decrease tumor size; inhibit (i.e., slow down to some extent and preferably stop) the infiltration of cancer cells into surrounding organs; inhibit (i.e., slow down to some extent and preferably stop) tumor metastasis; inhibit tumor growth to some extent; and/or alleviate one or more symptoms associated with cancer to some extent. In terms of the extent to which a drug can prevent the growth of existing cancer cells and/or kill existing cancer cells, it may have antiproliferative and/or cytotoxic properties.

The term "pharmaceutically acceptable salt" refers to a salt of the compound of the present disclosure that is prepared by reacting the compound having a specific substituent of the present disclosure with a relatively non-toxic acid or base. When the compound of the present disclosure contains a relatively acidic functional group, a base addition salt can be obtained by bringing the neutral form of the compound into contact with a sufficient amount of base in a pure solution or a suitable inert solvent. The pharmaceutically acceptable base addition salt includes a salt of sodium, potassium, calcium, ammonium, organic amine, magnesium, or similar salts. When the compound of the present disclosure contains a relatively basic functional group, an acid addition salt can be obtained by bringing the neutral form of the compound into contact with a sufficient amount of acid in a pure solution or a suitable inert solvent. Certain specific compounds of the present disclosure contain both basic and acidic functional groups, thus can be converted to any base or acid addition salt.

The pharmaceutically acceptable salt of the present disclosure can be prepared from the parent compound that contains an acidic or basic moiety by conventional chemical method. Generally, such salt can be prepared by reacting the free acid or base form of the compound with a stoichiometric amount of an appropriate base or acid in water or an organic solvent or a mixture thereof.

The term "treatment" includes the suppression, slowing, halting, or reversal of the progression or severity of existing symptoms or conditions.

Unless otherwise specified, the term "isomer" is intended to include a geometric isomer, a cis-trans isomer, a stereoisomer, an enantiomer, an optical isomer, a diastereoisomer, and a tautomeric isomer.

The compounds of the present disclosure may exist in specific geometric or stereoisomeric forms. The present disclosure contemplates all such compounds, including *cis* and *trans* isomers, (-)- and (+)-enantiomers, (R)- and (S)-enantiomers, diastereoisomers, (D)-isomers, (Z)-isomers, racemic, and other mixtures thereof, such as enantiomer or diastereomer enriched mixtures, all of which are within the scope of the present disclosure. Additional asymmetric carbon atoms may be present in substituents such as alkyl. All these isomers and their mixtures are included within the scope of the present disclosure.

Unless otherwise specified, the term "enantiomer" or "optical isomer" refers to stereoisomers that are mirror images of each other.

Unless otherwise specified, the term "*cis-trans* isomer" or "geometric isomer" is caused by the inability to rotate freely of double bonds or single bonds of ring-forming carbon atoms.

Unless otherwise specified, the term "diastereomer" refers to a stereoisomer in which a molecule has two or more chiral centers and the relationship between the molecules is not mirror images.

Unless otherwise specified, "(+)" refers to dextrorotation, "(-)" refers to levorotation, and "(±)" refers to racemic.

Unless otherwise specified, the absolute configuration of a stereogenic center is represented by a wedged solid bond (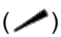) and a wedged dashed bond (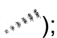); the relative configuration is represented by a straight solid bond (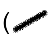) and a straight dashed bond (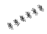), for example, trans-1, 4-dimethylcyclohexane is represented by

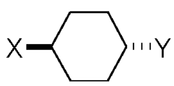

or

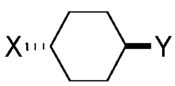

, and cis-1,4-disubstituted cyclohexane is represented by

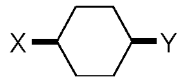

or

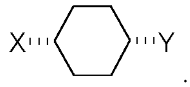

Unless otherwise specified, the terms "enriched in one isomer", "enriched in isomers", "enriched in one enantiomer", or "enriched in enantiomers" refer to the content of one of the isomers or enantiomers is less than 100%, and the content of the isomer or enantiomer is greater than or equal to 60%, or greater than or equal to 70%, or greater than or equal to 80%, or greater than or equal to 90%, or greater than or equal to 95%, or greater than or equal to 96%, or greater than or equal to 97%, or greater than or equal to 98%, or greater than or equal to 99%, or greater than or equal to 99.5%, or greater than or equal to 99.6%, or greater than or equal to 99.7%, or greater than or equal to 99.8%, or greater than or equal to 99.9%.

Unless otherwise specified, the term "isomer excess" or "enantiomeric excess" refers to the difference between the relative percentages of two isomers or two enantiomers. For example, if the content of one isomer or enantiomer is 90%, and the content of the other isomer or enantiomer is 10%, the isomer or enantiomer excess (ee value) is 80%.

Optically active (*R*)- and (*S*)-isomers, or *D* and *L* isomers can be prepared using chiral synthesis, chiral reagents, or other conventional techniques. If one kind of enantiomer of certain compound of the present disclosure is to be obtained, it can be obtained by asymmetric synthesis or derivative action of chiral auxiliary, wherein the resulting diastereomeric mixture is separated and the auxiliary group is cleaved to provide the pure desired enantiomer. Alternatively, when the molecule contains a basic functional group (such as amino) or an acidic functional group (such as carboxyl), a salt of a diastereoisomer is formed with an appropriate optically active acid or base, and then diastereomeric resolution is performed by conventional methods known in the art, and then the pure enantiomer is recovered. In addition, the enantiomer and the diastereoisomer are generally separated through chromatography which uses a chiral stationary phase and optionally combines with a chemical derivative method (such as carbamate generated from amine).

The compound of the present disclosure may contain an unnatural proportion of atomic isotope at one or more atoms that constitute the compound. For example, the compound can be radiolabeled with a radioactive isotope, such as tritium ($^{3}H$), iodine-125 ($^{125}I$), or C-14 ($^{14}C$). For another example, deuterated drugs can be formed by replacing hydrogen with deuterium, the bond formed by deuterium and carbon is stronger than that of ordinary hydrogen and carbon, compared with non-deuterated drugs, deuterated drugs have the advantages of reduced toxic and side effects, increased drug stability, enhanced efficacy, extended biological half-life of drugs, etc. All isotopic variations of the compound of the present disclosure, whether radioactive or not, are encompassed within the scope of the present disclosure.

When the enumerative linking group does not indicate the direction for linking, the direction for linking is arbitrary, for example, the linking group L contained in

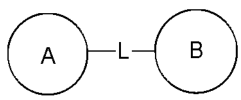

is -M-W-, then -M-W- can link ring A and ring B to form

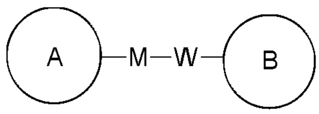

in the direction same as left-to-right reading order, and form

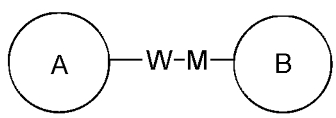

in the direction contrary to left-to-right reading order. A combination of the linking groups, substituents and/or variables thereof is allowed only when such combination can result in a stable compound.

Unless otherwise specified, when a group has one or more linkable sites, any one or more sites of the group can be linked to other groups through chemical bonds. When the linking site of the chemical bond is not positioned, and there is an H atom at the linkable site, then the number of H atoms at the site will decrease correspondingly with the number of the chemical bonds linking thereto so as to meet the corresponding valence. The chemical bond between the site and other groups can be represented by a straight solid bond (/), a straight dashed bond (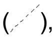), or a wavy line (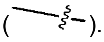). For example, the straight solid bond in -$OCH_3$ means that it is linked to other groups through the oxygen atom in the group; the straight dashed bond in

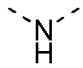

means that it is linked to other groups through the two ends of nitrogen atom in the group; the wave line in

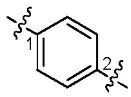

means that the phenyl group is linked to other groups through carbon atoms at position 1 and position 2; another example

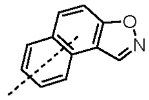

means that it can be linked to other groups through any linkable sites on the naphthalene ring by one chemical bond, including at least six types of linkage, including

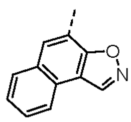, 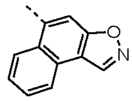,

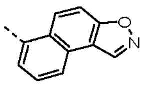, 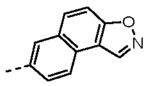, 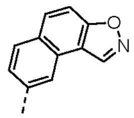, and 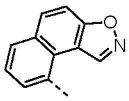.

"Optional" or "optionally" means that the subsequent event or condition may occur but not requisite, that the term includes the instance in which the event or condition occurs and the instance in which the event or condition does not occur.

The term "substituted" means one or more than one hydrogen atom on a specific atom is substituted by the substituent, including deuterium and hydrogen variables, as long as the valence of the specific atom is normal and the substituted compound is stable. When the substituent is an oxygen (i.e., =O), it means two hydrogen atoms are substituted. Positions on an aromatic ring cannot be substituted with a ketone. The term "optionally substituted" means an atom can be substituted with a substituent or not, unless otherwise specified, the type and number of the substituent may be arbitrary as long as being chemically achievable.

When any variable (such as R) occurs in the constitution or structure of the compound more than once, the definition of the variable at each occurrence is independent. Thus, for example, if a group is substituted by 0 to 2 R, the group can be optionally substituted with up to two R, wherein the definition of R at each occurrence is independent. Moreover, a combination of the substituent and/or the variant thereof is allowed only when the combination results in a

stable compound.

The term "replaced" means that a specified atom or group can be replaced by another atom or group as specified. For example, $CH_2$ in $CH_3CH_2CH_3$ can be replaced by O, S and NH to obtain $CH_3OCH_3$, $CH_3SCH_3$ and $CH_3NHCH_3$.

Unless otherwise specified, $C_{n-n+m}$ or $C_n$-$C_{n+m}$ includes any specific case of n to n+m carbons, for example, $C_{1-12}$ includes $C_1$, $C_2$, $C_3$, $C_4$, $C_5$, $C_6$, $C_7$, $C_8$, $C_9$, $C_{10}$, $C_{11}$, and $C_{12}$, and any range from n to n+m is also included, for example $C_{1-12}$ includes $C_{1-3}$, $C_{1-6}$, $C_{1-9}$, $C_{3-6}$, $C_{3-9}$, $C_{3-12}$, $C_{6-9}$, $C_{6-12}$, and $C_{9-12}$, etc.; similarly, n-membered to n+m-membered means that the number of atoms on the ring is from n to n+m, for example, 3- to 12-membered ring includes 3-membered ring, 4-membered ring, 5-membered ring, 6-membered ring, 7-membered ring, 8-membered ring, 9-membered ring, 10-membered ring, 11-membered ring, and 12-membered ring, and any range from n to n+m is also included, for example, 3- to 12-membered ring includes 3- to 6-membered ring, 3- to 9-membered ring, 5- to 6-membered ring, 5- to 7-membered ring, 6- to 7-membered ring, 6- to 8-membered ring, and 6- to 10-membered ring, etc.

Unless otherwise specified, the term "$C_{1-10}$ alkyl" refers to a linear or branched saturated hydrocarbon group consisting of 1 to 10 carbon atoms. The $C_{1-10}$ alkyl includes $C_{1-9}$, $C_{1-8}$, $C_{1-7}$, $C_{1-6}$, $C_{1-5}$, $C_{1-4}$, $C_{1-3}$, $C_{1-2}$, $C_{18}$, $C_{17}$, $C_{16}$, $C_{15}$, $C_{14}$, $C_{13}$, $C_{12}$, $C_{11}$, $C_{10}$, $C_{1-9}$, $C_8$, $C_7$, $C_6$, and $C_5$ alkyl, etc.; it can be monovalent (e.g., methyl), divalent (e.g., methylene), or multivalent (e.g., methine). Examples of $C_{1-8}$ alkyl include, but are not limited to, methyl (Me), ethyl (Et), propyl (including n-propyl and isopropyl), butyl (including n-butyl, isobutyl, s-butyl, and t-butyl), pentyl (including n-pentyl, isopentyl, and neopentyl), hexyl, heptyl, and octyl, etc. The term "$C_{1-10}$ alkylene" refers to a divalent $C_{1-10}$ alkyl.

When alkyl serves as a linking group, then "alkyl" refer to the linked alkylene group. The term "alkylene" refers to a saturated divalent hydrocarbon group obtained by removing two hydrogen atoms from a saturated linear or branched hydrocarbon. Examples of alkyl denoting a linked alkylene group include, but are not limited to, $-CH_2-$, $-CH_2CH_2-$, $-CH(CH_3)CH_2-$, $-CH(CH_2CH_3)-$, $-CH_2CH(CH_3)-$, $-CH_2CH_2CH_2-$, $-CH_2CH_2CH_2CH_2-$, etc.

Unless otherwise specified, the term "$C_{1-4}$ alkyl" refers to a linear or branched saturated hydrocarbon group consisting of 1 to 4 carbon atoms. The $C_{1-4}$ alkyl includes $C_{1-3}$ alkyl, $C_{1-2}$ alkyl, $C_2$ alkyl, $C_3$ alkyl, and methyl, etc.; it can be monovalent (such as methyl), divalent (such as methylene), or multivalent (such as methine). Examples of $C_{1-6}$ alkyl include, but are not limited to, methyl (Me), ethyl (Et), propyl (including n-propyl and isopropyl), butyl (including n-butyl, isobutyl, s-butyl, and t-butyl), etc.

Unless otherwise specified, "$C_{3-6}$ cycloalkyl" refers to a saturated cyclic hydrocarbon group consisting of 3 to 6 carbon atoms, which can be monocyclic and bicyclic, and the $C_{3-6}$ cycloalkyl includes $C_{3-5}$, $C_{4-5}$, and $C_{5-6}$ cycloalkyl, etc.; it can be monovalent, divalent, or multivalent. Examples of $C_{3-6}$ cycloalkyl include, but are not limited to, cyclopropyl, cyclobutyl, cyclopentyl, cyclohexyl, etc.

Unless otherwise specified, the term "4- to 8-membered heterocycloalkyl" by itself or in combination with other terms refers to a saturated cyclic group consisting of 4 to 8 ring atoms, wherein 1, 2, 3, or 4 ring atoms are heteroatoms independently selected from O, S, N, and Se, and the rest are carbon atoms, wherein nitrogen atoms are optionally quaternized, and nitrogen and sulfur heteroatoms can be optionally oxidized (i.e., NO and $S(O)_p$, p is 1 or 2). It includes monocyclic and bicyclic systems, wherein the bicyclic system includes a spiro ring, a fused ring, and a bridged ring. In addition, with regard to the "4- to 8-membered heterocycloalkyl", a heteroatom may occupy the connection position of the heterocycloalkyl with the rest of the molecule. The 4- to 8-membered heterocycloalkyl includes 4- to 7-membered, 4- to 6-membered, 4- to 5-membered, 4-membered, 5-membered, 6-membered heterocycloalkyl, etc. Examples of 4- to 8-membered heterocycloalkyl include, but are not limited to, azetidinyl, oxetanyl, thietanyl, pyrrolidinyl, pyrazolidinyl, imidazolidinyl, tetrahydrothienyl (including tetrahydrothiophen-2-yl, tetrahydrothiophen-3-yl, etc.), tetrahydrofuranyl (including tetrahydrofuran-2-yl, etc.), tetrahydropyranyl, piperidinyl (including 1-piperidinyl, 2-piperidinyl, 3-piperidinyl, etc.), piperazinyl (including 1-piperazinyl, 2-piperazinyl, etc.), morpholinyl (including 3-morpholinyl, 4-morpholinyl, etc.), dioxinyl, dithianyl, isoxazolidinyl, isothiazolidinyl, 1,2-oxazinyl, 1,2-thiazinyl, hexahydropyridazinyl, homopiperazinyl, homopiperidinyl, dioxacycloheptyl, etc.

Unless otherwise specified, the terms "$C_{6-10}$ aromatic ring" and "$C_{6-10}$ aryl" in the present disclosure can be used interchangeably, and the term "$C_{6-10}$ aromatic ring" or "$C_{6-10}$ aryl" refers to a cyclic hydrocarbon group consisting of 6 to 10 carbon atoms with a conjugated $\pi$-electron system, which can be a monocyclic, condensed bicyclic, or condensed tricyclic system, wherein each ring is aromatic. It can be monovalent, divalent, or multivalent, and the $C_{6-l0}$ aryl includes $C_{6-9}$, $C_9$, $C_{10}$, and $C_6$ aryl, etc. Examples of $C_{6-10}$ aryl include, but are not limited to, phenyl, naphthyl (including 1-naphthyl, 2-naphthyl, etc.).

Unless otherwise specified, the terms "5- to 10-membered heteroaryl ring" and "5- to 10-membered heteroaryl" in the present disclosure can be used interchangeably, and the term "5- to 10-membered heteroaryl" refers to a cyclic group consisting of 5 to 10 ring atoms with a conjugated $\pi$-electron system, wherein 1, 2, 3, or 4 ring atoms are heteroatoms independently selected from O, S, N, and Se, and the rest are carbon atoms. It can be a monocyclic, condensed bicyclic, or condensed tricyclic system, wherein each ring is aromatic. Here, the nitrogen atom is optionally quaternized, and nitrogen and sulfur heteroatoms can be optionally oxidized (i.e., NO and $S(O)_p$, wherein p is 1 or 2). The 5- to 10-membered heteroaryl can be linked to the rest of the molecule through a heteroatom or a carbon atom. The 5- to 10-membered heteroaryl includes 5- to 8-membered, 5- to 7-membered, 5- to 6-membered, 5-membered, and 6-membered heteroaryl, etc. Examples of the 5- to 10-membered heteroaryl include, but are not limited to, pyrrolyl (including *N*-pyrrolyl, 2-pyrrolyl, 3-pyrrolyl, etc.), pyrazolyl (including 2-pyrazolyl, 3-pyrrolyl, etc.), imidazolyl (including *N*-imidazolyl, 2-imidazolyl, 4-imidazolyl, 5-imidazolyl, etc.), oxazolyl (including 2-oxazolyl, 4-oxazolyl, 5-oxazolyl, etc.), triazolyl (1*H*-1,2,3-triazolyl, 2*H*-1,2,3-triazolyl, 1*H*-1,2,4-triazolyl, 4H-1,2,4-triazolyl, etc.), tetrazolyl, isoxazolyl (3-isoxazolyl, 4-isoxazolyl, 5-isoxazolyl, etc.), thiazolyl (including 2-thiazolyl, 4-thiazolyl, 5-thiazolyl, etc.), furyl (including 2-furyl, 3-furyl, etc.), thienyl (including 2-thienyl, 3-thienyl, etc.), pyridyl (including 2-pyridyl, 3-pyridyl, 4-pyridyl, etc.), pyrazinyl, pyrimidyl (including 2-pyrimidyl, 4-pyrimidyl, etc.), benzothiazolyl (including 5-benzothiazolyl, etc.), purinyl, benzimidazolyl (including 2-benzimidazolyl, etc.), benzoxazolyl, indolyl (including 5-indolyl, etc.), isoquinolyl (including 1-isoquinolyl, 5-isoquinolinyl, etc.), quinoxalinyl (including 2-quinoxalinyl, 5-quinoxalinyl, etc.), or quinolinyl (including 3-quinolinyl, 6-quinolinyl, etc.).

Unless otherwise specified, the term "halogen element" or "halogen" by itself or as part of another substituent refers to fluorine, chlorine, bromine, or iodine atom.

The structure of the compounds of the present disclosure can be confirmed by conventional methods known to those skilled in the art, and if the present disclosure involves an absolute configuration of a compound, then the absolute configuration can be confirmed by means of conventional techniques in the art. For example, in the case of single crystal X-ray diffraction (SXRD), diffraction intensity data are collected from the cultured single crystal using a Bruker D8 venture diffractometer with CuKα radiation as the light source and scanning mode: φ/ω scan, and after collecting the relevant data, the crystal structure is further analyzed by direct method (Shelxs97), so that the absolute configuration can be confirmed.

The compounds of the present disclosure can be prepared by a variety of synthetic methods known to those skilled in the art, including the specific embodiments listed below, the embodiments formed by their combination with other chemical synthesis methods, and equivalent alternatives known to those skilled in the art, preferred embodiments include but are not limited to the examples of the present disclosure.

The compounds of the present disclosure are named according to the conventional naming principles in the art or by ChemDraw® software, and the commercially available compounds use the supplier catalog names.

The solvent used in the present disclosure is commercially available.

The present adopts the following abbreviations: Ph stands for phenyl; Me stands for methyl; Et stands for ethyl; M means moles per liter; Boc stands for tert-butoxycarbonyl, which is an amino protecting group.

DETAILED DESCRIPTION OF THE PREFERRED EMBODIMENT

The present disclosure is described in detail by the examples below, but it does not mean that there are any adverse restrictions on the present disclosure. The present disclosure has been described in detail herein, and its specific examples have also been disclosed; for one skilled in the art, it is obvious to make various modifications and improvements to the examples of the present disclosure without departing from the spirit and scope of the present disclosure.

Reference example 1: Fragment BB-1

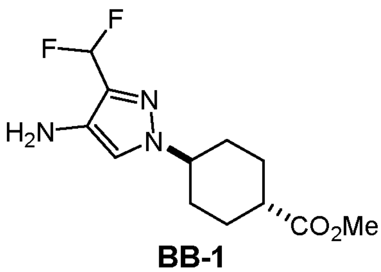

**BB-1**

Synthetic route:

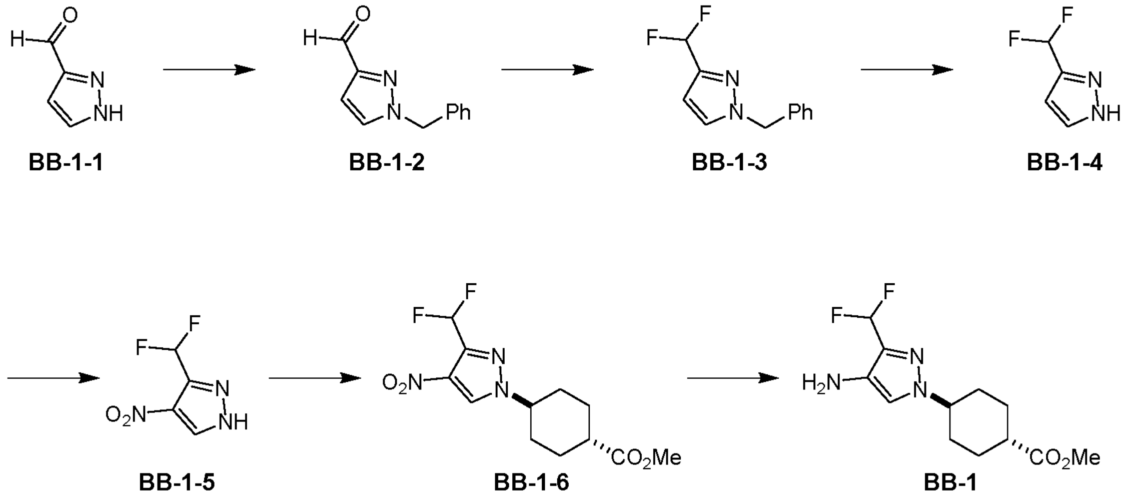

BB-1-1 BB-1-2 BB-1-3 BB-1-4

BB-1-5 BB-1-6 BB-1

Step 1: Synthesis of compound **BB-1-2**

Compound **BB-1-1** (40 g, 416.29 mmol) was dissolved in N, N-dimethylformamide (400 mL) at room temperature under nitrogen atmosphere, then benzyl bromide (74.76 g, 437.10 mmol, 51.92mL) and cesium carbonate (339.09 g, 1.04 mol) were added thereto. The reaction mixture was stirred and reacted at room temperature under nitrogen atmosphere for 2 hours. After the reaction was completed, the reaction mixture was added with water (1500 mL), and extracted with ethyl acetate (5 × 1000 mL). The organic phases were combined, washed with 10% brine (3 × 1000 mL ), dried over anhydrous sodium sulfate, and filtered, and the filtrate was concentrated under reduced pressure to remove the solvent. The resulting residue was separated by column chromatography (eluent: petroleum ether/ethyl acetate = 1/0 to 20/1, v/v) to obtain compound **BB-1-2.** MS-ESI *m/z:* 187.2 $[M+H]^+$.

Step 2: Synthesis of compound **BB-1-3**

Compound **BB-1-2** (58 g, 311.48 mmol) was dissolved in dichloromethane (500 mL) at room temperature under nitrogen atmosphere, and the mixture was cooled to 0°C, and then diethylaminosulfur trifluoride (150.62 g, 934.43 mmol, 123.46 mL) was added thereto. The reaction mixture was stirred and reacted at room temperature for 16 hours. After the reaction was completed, the mixture was cooled to 0°C. The reaction mixture was slowly dropwise added with methanol (200 mL), and quenched, The reaction mixture was concentrated under reduced pressure to remove the solvent, added with water (1000 mL), and extracted with ethyl acetate (3 × 1000 mL). The organic phases were combined, washed sequentially with saturated sodium bicarbonate solution (1000 mL) and saturated brine (1000 mL ), dried over anhydrous sodium sulfate, and filtered. The filtrate was concentrated under reduced pressure to remove the solvent. The resulting residue was separated by column chromatography (eluent: petroleum ether/ethyl acetate = 1/0 to 20/1, v/v). The crude product was added with petroleum ether (20 mL), stirred at room temperature for 2 hours, and filtered. The filter cake was rinsed with petroleum ether (10 mL), collected, and dried under vacuum to obtain compound **BB-1-3.** MS-ESI *m/z:* 209.1 $[M+H]^+$. $^1H$ NMR (400 MHz, $CDCl_3$) δ: 7.42-7.31 (m, 4H), 7.26-7.21 (m, 2H), 6.73 (t, *J*=55.2 Hz, 1H), 6.51 (t, *J*=1.0 Hz, 1H), 5.33 (s, 2H).

Step 3: Synthesis of compound **BB-1-4**

Compound **BB-1-3** (26.5 g, 127.28 mmol) was dissolved in methanol (300 mL) at room temperature, then palladium hydroxide/carbon (5 g, purity of 20%) and hydrochloric acid (2 M, 25 mL) were added thereto. The reaction mixture was heated to 60°C, and stirred and reacted under hydrogen atmosphere (50 psi) for 30 hours. After the reaction was completed, the reaction mixture was filtered through diatomite. The filter cake was rinsed with methanol (800 mL). The filtrate was collected and concentrated under reduced pressure to remove the solvent to obtain compound **BB-1-4.** $^1H$ NMR (400 MHz, DMSO_$d_6$) δ: 11.41 (s, 1H), 7.84 (d, *J*=2.0 Hz, 1H), 7.00 (t, *J*=54.8 Hz, 1H), 6.51 (t, *J*=1.2 Hz, 1H).

Step **4:** Synthesis of compound **BB-1-5**

Compound **BB-1-4** (30 g, 254.06 mmol) was dissolved in concentrated sulfuric acid (300 mL, purity of 98%) at 0°C, then nitric acid (68.950 g, 711.24 mmol, 49.25 mL, purity of 65 to 68%) was dropwise added thereto. The reaction

mixture was stirred at 0°C for 10 minutes, then heated to 115°C, and stirred and reacted for 16 hours. After the reaction was completed, the reaction mixture was slowly poured into ice water (1000 mL), and extracted with ethyl acetate (3 × 500 mL). The organic phases were combined, washed sequentially with saturated sodium bicarbonate aqueous solution (500 mL) and saturated brine (500 mL). The organic phase was dried over anhydrous sodium sulfate, and filtered. The filtrate was concentrated under reduced pressure to remove the solvent to obtain compound **BB-1-5.** MS-ESI *m/z:* 164.1 $[M+H]^+$. $^1H$ NMR (400 MHz, DMSO_$d_6$) δ: 14.39 (s, 1H), 9.00 (s, 1H), 7.31 (t, *J*=53.0 Hz, 1H).

Step **5:** Synthesis of compound **BB-1-6**

Cis-4-hydroxycyclohexanecarboxylic acid methyl ester (35 g, 221.25 mmol) was dissolved in dichloromethane (350 mL) at room temperature under nitrogen atmosphere, then triethylamine (22.39 g, 221.25 mmol, 30.79 mL) was added thereto. The mixture was cooled to 0°C, then methanesulfonyl chloride (31.99g, 279.26 mmol, 21.61 mL) was dropwise added thereto, and the reaction mixture was stirred and reacted at 0°C for 0.5 hours. After the reaction was completed, the reaction mixture was slowly added with water (300 mL), and extracted with dichloromethane (300 mL). The organic phases were combined, washed with saturated brine (300 mL), dried over anhydrous sodium sulfate, and filtered. The filtrate was concentrated under reduced pressure to obtain a crude product, which was used in the next step directly.

Compound **BB-1-5** (13.5 g, 82.78 mmol) and the crude product obtained above were dissolved in *N*,*N*-dimethylformamide (300 mL) at room temperature, then potassium carbonate (24 g, 173.65 mmol) was added thereto. The reaction mixture was heated to 80°C, and stirred and reacted for 16 hours. After the reaction was completed, the reaction mixture was cooled to room temperature, added with water (100 mL), and extracted with ethyl acetate (40 mL × 2). The organic phases were combined, dried over anhydrous sodium sulfate, and filtered. The filtrate was concentrated under reduced pressure to remove the solvent. The resulting residue was separated by column chromatography (eluent: ethyl acetate/petroleum ether = 1/3, v/v) to obtain compound **BB-1-6.** $^1H$ NMR (400 MHz, $CDCl_3$) δ: 8.22 (s, 1H), 7.12 (t, *J*=53.4 Hz, 1H), 4.25-4.16 (m, 1H), 3.72 (s, 3H), 2.46-2.37 (m, 1H), 2.36-2.28 (m, 2H), 2.27-2.20 (m, 2H), 1.89-1.77 (m, 2H), 1.72-1.60 (m, 2H).

Step **6:** Synthesis of compound **BB-1**

Wet palladium on carbon (1.5 g, purity of 10%) was added to tetrahydrofuran (200 mL) at room temperature under argon atmosphere, then compound **BB-1-6** (7.5 g, 24.73 mmol) was added thereto. The reaction mixture was stirred and reacted at room temperature under hydrogen (15 psi) atmosphere for 15 hours. After the reaction was completed, the reaction mixture was filtered. The filter cake was washed with dichloromethane (50 mL × 2), and the filtrate was concentrated under reduced pressure to remove the solvent. The resulting residue was separated by column chromatography (eluent: ethyl acetate/petroleum ether = 1/2, v/v) to obtain compound **BB-1.** MS-ESI *m/z:* 274.1 $[M+H]^+$.

**Reference example 2: Fragment BB-2**

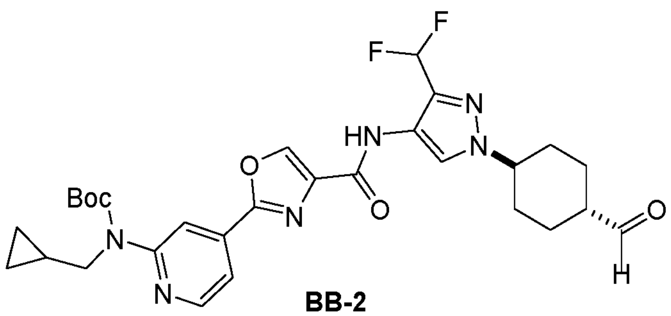

**BB-2**

Synthetic route:

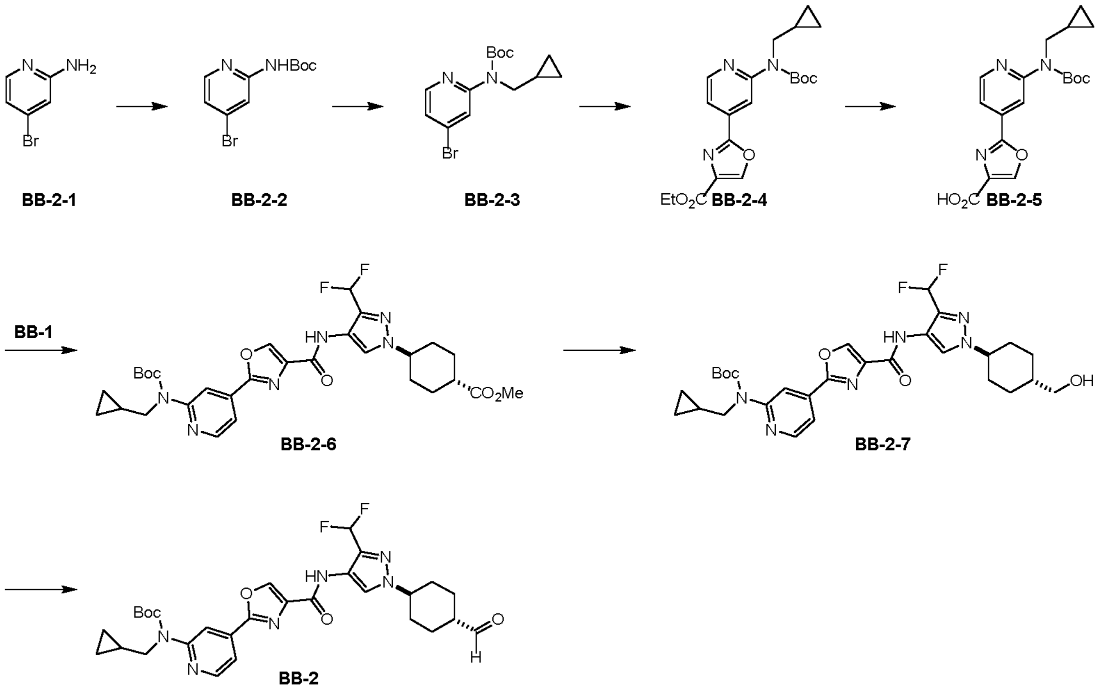


BB-2-1 BB-2-2 BB-2-3 BB-2-4 BB-2-5

BB-2-6 BB-2-7

BB-2

Step **1:** Synthesis of compound **BB-2-2**

Compound **BB-2-1** (50 g, 289.00 mmol) was dissolved in tetrahydrofuran (750 mL) at room temperature under nitrogen atmosphere, and the mixture was cooled to -5°C, and then a solution of lithium bis(trimethylsilyl)amide in n-hexane (1 M, 578.00 mL) was added thereto. The reaction mixture was stirred at -5°C for 10 minutes, and di-tert-butyl dicarbonate (63.07 g, 289.00 mmol, 66.39 mL) was added thereto. The reaction mixture was slowly warmed to room temperature, and stirred and reacted for 1 hour. After the reaction was completed, the reaction mixture was slowly added to a saturated solution of ammonium chloride (1000 mL), and extracted with ethyl acetate (1000 mL × 3). The organic phases were combined, washed with saturated brine (2000 mL × 2). The organic phase was dried over anhydrous sodium sulfate, and filtered. The filtrate was concentrated under reduced pressure to remove the solvent to obtain compound **BB-2-2.** MS-ESI *m/z*: 273.1 $[M+H]^+$, 275.1 $[M+H+2]^+$. $^1H$ NMR (400 MHz, DMSO_$d_6$) δ: 10.10 (s, 1H), 8.14 (d, *J*=5.2 Hz, 1H), 8.04 (d, *J*=1.6 Hz, 1H), 7.27 (t, *J*= 1.8, 5.4 Hz, 1H), 1.47 (s, 9H).

Step **2:** Synthesis of compound **BB-2-3**

Compound **BB-2-2** (39 g, 142.79 mmol) was dissolved in N, N-dimethylformamide (400 mL) at room temperature under nitrogen atmosphere, and the mixture was cooled to 0°C, and then sodium hydride (8.57 g, 214.19 mmol, purity of 60%) was added thereto. The reaction mixture was reacted at 0 to 5°C for 30 minutes, then bromomethyl cyclopropane (23.13 g, 171.35 mmol, 16.41 mL) was added thereto. The reaction mixture was slowly warmed to room temperature, and stirred and reacted for 15 hours. After the reaction was completed, the reaction mixture was slowly added to water (1.5 mL), and extracted with ethyl acetate (1 L × 3). The organic phases were combined, washed with saturated brine (3 L × 3). The organic phase was dried over anhydrous sodium sulfate, and filtered. The filtrate was concentrated under reduced pressure to remove the solvent. The resulting residue was separated by column chromatography (eluent: petroleum ether/ethyl acetate = 50/1 to 20/1, v/v) to obtain compound **BB-2-3.** $^1H$ NMR (400 MHz, $CDCl_3$) δ: 8.17 (d, *J*=5.2 Hz, 1H), 7.96 (d, *J*=1.6 Hz, 1H), 7.15 (dd, *J*=1.6, 5.2 Hz, 1H), 3.87 (d, *J*=7.2 Hz, 2H), 1.54 (s, 9H), 1.21-1.14 (m, 1H), 0.44-0.39 (m, 2H), 0.27-0.23 (m, 2H).

Step **3:** Synthesis of compound **BB-2-4**

Compound **BB-2-3** (47.53 g, 145.26 mmol) and ethyl oxazole-4-carboxylate (20.5 g, 145.26 mmol) were dissolved in N, N-dimethylformamide (500 mL) at room temperature under nitrogen atmosphere, then tri(o-tolyl)phosphine (8.84 g, 29.05 mmol), palladium acetate (3.26 g, 14.53 mmol), and cesium carbonate (94.66 g, 290.52 mmol) were added thereto. The reaction mixture was heated to 80°C, and stirred for 14 hours. After the reaction was completed, the reaction mixture was added to water (1 L), and extracted with ethyl acetate (1 L × 3). The organic phases were combined, washed with saturated brine (3 L × 3). The organic phases were dried over anhydrous sodium sulfate, and filtered. The

filtrate was concentrated under reduced pressure to remove the solvent. The resulting residue was separated by column chromatography (eluent: petroleum ether/ethyl acetate = 5/1 to 2/1, v/v) to obtain compound **BB-2-4.** MS-ESI *m/z:* 388.2 $[M+H]^+$. $^1$H NMR (400 MHz, $CDCl_3$) δ: 8.49 (d, *J*=5.2 Hz, 1H), 8.37 (s, 1H), 8.33 (s, 1H), 7.70 (dd, *J*=1.2, 5.2 Hz, 1H), 4.45 (q, *J*=7.0 Hz, 2H), 3.92 (d, *J*=6.8 Hz, 2H), 1.55 (s, 9H), 1.42 (t, *J*=7.2 Hz, 3H), 1.22-1.13 (m, 1H), 0.44-0.39 (m, 2H), 0.27-0.22 (m, 2H).

Step **4:** Synthesis of compound **BB-2-5**

Compound **BB-2-4** (18 g, 46.46 mmol) was dissolved in water (80 mL) and tetrahydrofuran (400 mL) at room temperature under nitrogen atmosphere, then lithium hydroxide monohydrate (5.85 g, 139.38 mmol) was added thereto. The reaction mixture was stirred and reacted at room temperature for 4 hours. After the reaction was completed. The reaction mixture was concentrated under reduced pressure, added with 2 M hydrochloric acid to adjust the pH to 2 to 3, and extracted with ethyl acetate (100 mL × 5). The organic phases were combined, dried over anhydrous sodium sulfate, and filtered. The filtrate was concentrated under reduced pressure to remove the solvent to obtain compound **BB-2-5.** $^1$H NMR (400 MHz, $CDCl_3$) δ: 8.57 (d, *J*=5.2 Hz, 1H), 8.44 (s, 1H), 8.38 (s, 1H), 7.77 (dd, *J*=1.2, 5.2 Hz, 1H), 3.94 (d, *J*=6.8 Hz, 2H), 1.55 (s, 9H), 1.20-1.11 (m, 1H), 0.45-0.39 (m, 2H), 0.29-0.20 (m, 2H).

Step 5: Synthesis of compound **BB-2-6**

Compound **BB-2-5** (5 g, 13.91 mmol) were dissolved in N, N-dimethylformamide (50 mL) at room temperature, then N, N-diisopropylethylamine (5.39 g, 41.74 mmol, 7.27mL) and 2-(7-azabenzotriazol)-N, N, N', N'-tetramethyluronium hexafluorophosphate (6.35 g, 16.70 mmol) were added thereto. The reaction mixture was stirred and reacted for 0.5 hours, then compound **BB-1** (4.18 g, 15.30 mmol) was added thereto. The reaction continued for 1.5 hours. After the reaction was completed, the reaction mixture was poured into water (500 mL), and extracted with ethyl acetate (150 mL × 2). The organic phases were combined, washed with 10% brine (150 mL × 2), dried over anhydrous sodium sulfate, and filtered. The filtrate was concentrated under reduced pressure to remove the solvent. The resulting residue was separated by column chromatography (eluent: petroleum ether/ethyl acetate = 2/1, v/v) to obtain compound **BB-2-6.** MS-ESI *m/z:* 615.4 $[M+H]^+$ $^1$H NMR (400 MHz, DMSO_$d_6$) δ: 9.77 (s, 1H), 9.00 (s, 1H), 8.59 (d, *J*=5.2 Hz, 1H), 8.29 (s, 1H), 8.19 (s, 1H), 7.68 (dd, *J*=1.2, 5.2 Hz, 1H), 7.16 (t, *J*=54.2 Hz, 1H), 4.32-4.22 (m, 1H), 3.86 (d, *J*=6.8 Hz, 2H), 3.62 (s, 3H), 2.45-2.38 (m, 1H), 2.11-1.99 (m, 4H), 1.88-1.75 (m, 2H), 1.61-1.54 (m, 1H), 1.51 (s, 9H), 1.22-1.12 (m, 1H), 0.44-0.37 (m, 2H), 0.27-0.19 (m, 2H).

Step **6:** Synthesis of compound **BB-2-7**

Compound **BB-2-6** (5 g, 8.13 mmol) was dissolved in tetrahydrofuran (50 mL) at room temperature under nitrogen atmosphere, and the mixture was cooled to -40°C, and then a solution of diisobutylaluminium hydride in toluene (1 M, 48.81 mL) was slowly added thereto. After the dropwise addition was completed, the reaction mixture was heated to 0°C, and stirred and reacted for 30 minutes. After the reaction was completed, the reaction mixture was added with 1 M sodium hydroxide solution (10 mL), then added with water (50 mL), and extracted with ethyl acetate (4 × 50 mL). The organic phases were combined, washed with saturated brine (100 mL), dried over anhydrous sodium sulfate, and filtered. The filtrate was concentrated under reduced pressure to remove the solvent. The resulting residue was purified by column chromatography (eluent: petroleum ether/ethyl acetate = 1/0 to 1/3, v/v) to obtain compound **BB-2-7.** MS-ESI *m*/*z:* 587.4 $[M+H]^+$. $^1$H NMR (400 MHz, $CDCl_3$) δ: 9.07 (s, 1H), 8.52 (d, *J*=5.2 Hz, 1H), 8.38 (s, 1H), 8.33 (s, 2H), 7.64-7.62 (m, 1H), 7.01-6.65 (m,1H) , 4.14-4.10 (m, 1H), 3.94 (d, *J*=7.2 Hz, 2H), 3.54 (d, *J*=6.4 Hz, 2H), 2.29-2.23 (m, 2H), 2.05-1.99 (m, 2H ), 1.88-1.78 (m, 2H), 1.57 (s, 9H), 1.23-1.14 (m, 4H), 0.45-0.41 (m, 2H), 0.29-0.25 (m, 2H).

Step 7: Synthesis of compound **BB-2**

Compound **BB-2-7** (1 g, 1.70 mmol) was dissolved in dichloromethane (10 mL) at room temperature under nitrogen atmosphere, then Dess-Martin periodinane (1.45 g, 3.41 mmol) was added thereto. The reaction mixture was stirred and reacted at room temperature for 1 hour. After the reaction was completed, the reaction mixture was added with a saturated solution of sodium sulfite (20 mL), and extracted with dichloromethane (3 × 20 mL). The organic phases were washed with saturated brine (30 mL), dried over anhydrous sodium sulfate, and filtered. The filtrate was concentrated under reduced pressure to remove the solvent. The resulting residue was separated by column chromatography (eluent: petroleum ether/ethyl acetate = 4/1 to 1/1, v/v) to obtain compound **BB-2.** MS-ESI *m*/*z*: 585.4 $[M+H]^+$. $^1$H NMR (400 MHz, $CDCl_3$) δ: 9.70 (s, 1H), 9.08 (s, 1H), 8.52 (d, *J*=5.6 Hz, 1H), 8.38 (s, 1H), 8.34 (s, 1H), 8.33 (s, 1H), 7.63 (dd, *J*=1.6, 5.2 Hz, 1H), 6.84 (t, *J*=54.6 Hz, 1H), 4.16-4.06 (m, 1H), 3.94 (d, *J*=6.8 Hz, 2H), 2.41-2.33 (m, 1H), 2.34-2.28 (m, 2H), 2.25-2.19 (m, 2H), 1.95-1.82 (m, 2H), 1.57 (s, 9H), 1.53-1.42 (m, 2H), 1.24-1.17(m, 1H), 0.47-0.39 (m, 2H), 0.30-0.24

(m, 2H).

**Reference example 3: Fragment BB-3**

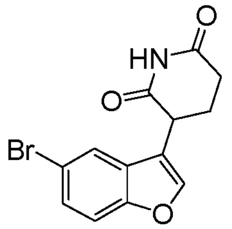

**BB-3**

Synthetic route:

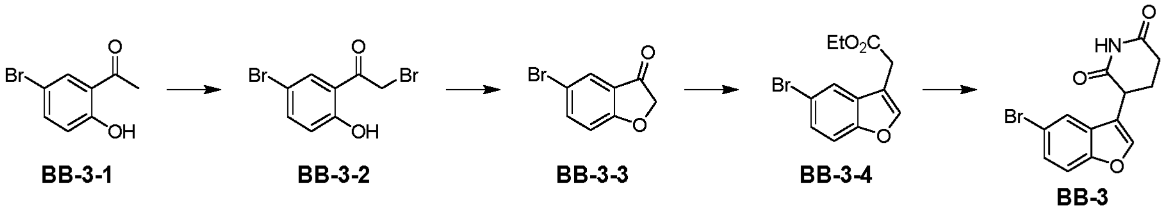

**BB-3-1** **BB-3-2** **BB-3-3** **BB-3-4** **BB-3**

Step **1:** Synthesis of compound **BB-3-2**

Compound **BB-3-1** (200 g, 930.04 mmol) was dissolved in chloroform (1000 mL) and ethyl acetate (1000 mL) at room temperature under nitrogen atmosphere, then copper bromide (415.45 g, 1.86 mol) was added thereto. The reaction mixture was heated to 90°C, and stirred and reacted for 16 hours. After the reaction was completed, the mixture was cooled to room temperature, and filtered. The filter cake was rinsed with dichloromethane (1.5 L) to obtain a solution of compound **BB-3-2,** which was used in the next step directly.

Step **2:** Synthesis of compound **BB-3-3**

The solution of compound **BB-3-2** (3.5 L) obtained above was cooled to 0°C, and then triethylamine (141.17 g, 1.40 mol, 194.18 mL) was slowly added dropwise thereto. After the dropwise addition was completed, the reaction mixture was slowly warmed to room temperature, and stirred and reacted for 2 hours. After the reaction was completed, the mixture was added with water (2 L) and the phases were separated. The aqueous phase was extracted with dichloromethane (500 mL). The organic phases were combined, washed with saturated brine (1 L × 2), dried over anhydrous sodium sulfate, and filtered. The filtrate was concentrated under reduced pressure to remove the solvent to obtain compound **BB-3-3.**

Step **3:** Synthesis of compound **BB-3-4**

Compound **BB-3-3** (198.1 g, 929.93 mmol) was dissolved in toluene (1.5 L) at room temperature under nitrogen atmosphere, and then (ethoxycarbonylmethylene)triphenylphosphorane (388.76 g, 1.12 mol) was added thereto. The reaction mixture was heated to 130°C, and stirred and reacted for 36 hours. After the reaction was completed, the mixture was cooled to room temperature, concentrated under reduced pressure to remove the solvent. The resulting residue was added with methyl tert-butyl ether (700 mL × 3), stirred at room temperature for 20 minutes, and filtered. The filter cake was rinsed with methyl tert-butyl ether (100mL), and the filtrate was collected. The filtrate was concentrated under reduced pressure to remove the solvent. The resulting residue was separated by column chromatography (eluent: petroleum ether/ethyl acetate = 1/0 to 70/1, v/v) to obtain compound **BB-3-4.** $^1$H NMR (400 MHz, $CDCl_3$) δ: 7.72 (d, *J*=2.0 Hz, 1H), 7.64 (s, 1H), 7.41 (dd, *J*=2.0 Hz, 8.8 Hz, 1H), 7.36 (d, *J*=8.8 Hz, 1H), 4.21 (q, *J*=7.0 Hz, 2H), 3.66 (d, *J*=0.8 Hz, 2H), 1.30 (t, *J*=7.2 Hz, 3H).

Step **4:** Synthesis of compound **BB-3**

Compound **BB-3-4** (3 g, 10.60 mmol) was dissolved in N, N-dimethylformamide (20 mL) at room temperature under nitrogen atmosphere, then acrylamide (903.79 mg, 12.72 mmol) and potassium tert-butoxide (1.78 g, 15.89 mmol) were sequentially added thereto. The reaction mixture was stirred and reacted at room temperature for 1 hour. After the reaction was completed, the reaction mixture was poured into 1 N hydrochloric acid (15 mL), added with water (25 mL), and extracted with ethyl acetate (35 mL × 3). The organic phases were combined, washed with saturated brine (30 mL × 2) , dried over anhydrous sodium sulfate, and filtered. The filtrate was concentrated under reduced pressure to remove the solvent. The resulting residue was separated by column chromatography (eluent: petroleum ether/ethyl acetate = 5/1 to 1/1, v/v) to obtain compound **BB-3.** MS-ESI *m/z:* 308.0 $[M+H]^+$, 310.0 $[M+H+2]^+$. $^1H$ NMR (400 MHz, DMSO_$d_6$) δ: 10.87 (s, 1H), 7.97 (s, 1H), 7.85 (d, *J*=2,0 Hz, 1H), 7.57 (d, *J*=8.4 Hz, 1H), 7.45 (dd, *J*=2.0, 8.8 Hz, 1H), 4.15 (dd, *J*=4.8, 12.4 Hz, 1H), 2.78-2.67 (m, 1H), 2.62-2.54 (m, 1H), 2.43-2.29 (m, 1H), 2.14-2.04 (m, 1H).

**Reference example 4: Fragment BB-4**

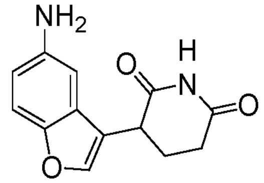

**BB-4**

Synthetic route:

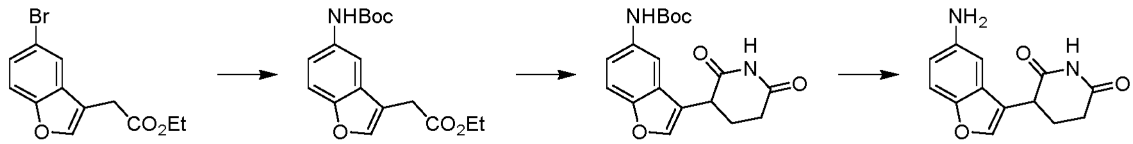

**BB-3-4** **BB-4-1** **BB-4-2** **BB-4**

Step **1:** Synthesis of compound **BB-4-1**

Compound **BB-3-4** (5 g, 17.66 mmol), tert-butyl carbamate (2.48 g, 21.19 mmol), potassium phosphate (11.25 g, 52.98 mmol), tris(dibenzylideneacetone)dipalladium (323.44 mg, 353.21 μmol) and 2-di-tert-butylphosphine-2',4',6'-triisopropyl biphenyl (299.98 mg, 706.42 μmol) were sequentially added into a mixed solvent of toluene (100 mL) and water (20 mL) at room temperature under nitrogen atmosphere. The reaction mixture was heated to 110°C, and stirred and reacted for 16 hours. After the reaction was completed, the reaction mixture was cooled to room temperature, added with water (100 mL), and filtered. The filter cake was rinsed with ethyl acetate (100 mL), and the filtrate was combined and allowed to stand for phase separation. The organic phase was collected. The organic phase was dried over anhydrous sodium sulfate, and filtered. The filtrate was concentrated under reduced pressure to remove the solvent. The resulting residue was separated by column chromatography (eluent: ethyl acetate/petroleum ether = 1/5, v/v) to obtain compound **BB-4-1.** $^1H$ NMR (400 MHz, $CDCl_3$) δ: 7.71 (br s, 1H), 7.62 (s, 1H), 7.37 (d, *J*=8.8 Hz, 1H), 7.15 (dd, *J*=2.0 Hz, 8.8 Hz, 1H), 6.58 (s, 1H), 4.20 (q, *J*=7.2 Hz, 2H), 3.67 (q, *J*=1.2 Hz, 2H), 1.53 (s, 9H), 1.29 (t, *J*=7.2 Hz, 3H).

Step **2:** Synthesis of compound **BB-4-2**

Compound **BB-4-1** (5.6 g, 17.54 mmol) and acrylamide (1.50 g, 21.05 mmol) were dissolved in N, N-dimethylformamide (100 mL) at room temperature, and then potassium tert-butoxide (1.8 g, 16.04 mmol) was added thereto. The reaction mixture was stirred and reacted at room temperature for 2 hours, and an additional amount of potassium tert-butoxide (0.6 g, 5.35 mmol) was added thereto. The reaction mixture was further stirred and reacted at room temperature for 0.5 hours, After the reaction was completed, the reaction mixture was poured into 1 M dilute hydrochloric acid (300 mL), stirred for 10 minutes, and filtered. The filter cake was rinsed with water (100 mL), and collected. The resulting filter cake was separated by column chromatography (eluent: ethyl acetate/petroleum ether = 1/1, v/v) to obtain compound **BB-4-2.** $^1$H NMR (400 MHz, $CDCl_3$) δ: 8.32 (br s, 1H), 7.72 (br s, 1H), 7.53 (s, 1H), 7.39 (d, *J*=8.8 Hz, 1H), 7.12 (dd, *J*=2.0 Hz, 8.8 Hz, 1H), 6.71 (br s, 1H), 3.97 (t, *J*=7.6 Hz, 1H), 2.82-2.63 (m, 2H), 2.39-2.29 (m, 2H), 1.53 (s, 9H).

Step 3: Synthesis of hydrochloride of compound **BB-4**

Compound **BB-4-2** (2.3 g, 6.68 mmol) was dissolved in ethyl acetate (20 mL) at room temperature, then a solution of hydrochloric acid in ethyl acetate (4 M, 40 mL) was added thereto. The reaction mixture was stirred and reacted at room temperature for 16 hours. After the reaction was completed, the reaction mixture was filtered. The filter cake was rinsed with ethyl acetate (20 mL), collected, and dried under vacuum to obtain the hydrochloride of compound **BB-4.** $^1$H NMR (400 MHz, $D_2O$) δ: 7.85 (s, 1H), 7.67 (d, *J*=8.8 Hz, 1H), 7.56 (d, *J*=1.6 Hz, 1H), 7.34 (dd, *J*=2.0 Hz, 8.8 Hz, 1H), 4.24 (dd, *J*=5.2 Hz, 12.4 Hz, 1H), 2.91-2.74 (m, 2H), 2.50-2.37 (m, 1H), 2.35-2.25 (m, 1H).

**Reference example 5: Fragment BB-5**

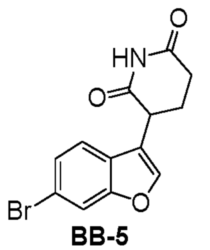

**BB-5**

Synthetic route:

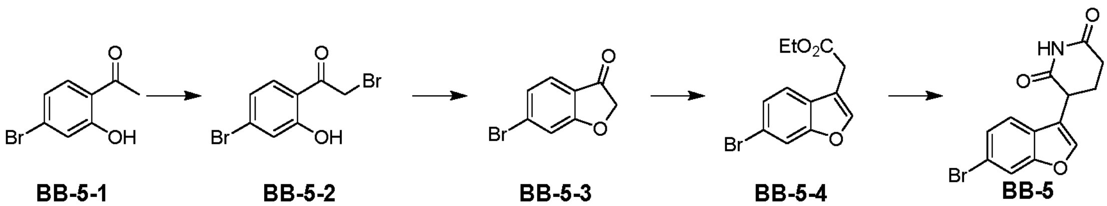

**BB-5-1** **BB-5-2** **BB-5-3** **BB-5-4** **BB-5**

Step **1:** Synthesis of compound **BB-5-2**

Compound **BB-5-1** (200 g, 930.04 mmol) was dissolved in chloroform (1 mL) and ethyl acetate (1 L) at room temperature under nitrogen atmosphere, then copper bromide (415.45 g, 1.86 mol) was added thereto. The reaction mixture was heated to 90°C, and stirred and reacted for 12 hours. After the reaction was completed, the mixture was cooled to room temperature, and filtered. The filter cake was rinsed with dichloromethane (300 mL × 2). The filtrate was collected to obtain a solution of compound **BB-5-2,** which was used in the next step directly.

Step **2:** Synthesis of compound **BB-5-3**

The solution of compound **BB-5-2** (273 g, 928.76 mmol) obtained above was cooled to 0°C, and then triethylamine (140.97 g, 1.39 mol, 193.91 mL) was slowly added dropwise thereto. After the dropwise addition was completed, the reaction mixture was slowly warmed to room temperature, and stirred and reacted for 1 hour. After the reaction was completed, the mixture was added with water (600 mL) and extracted. The phases were separated. The organic phase was washed with saturated brine (1 L), dried over anhydrous sodium sulfate, and filtered. The filtrate was concentrated under reduced pressure to remove half of the solvent, and added with toluene (500 mL). The mixture was continued to be concentrated under reduced pressure to remove the residual low-boiling solvent to obtain a solution of compound **BB-5-3** in toluene.

Step **3:** Synthesis of compound **BB-5-4**

Toluene (2 L) was added to half of the solution of the compound **BB-5-3** in toluene at room temperature under nitrogen atmosphere, and then (ethoxycarbonylmethylene)triphenylphosphorane (161.90 g, 464.73 mmol) was added thereto. The reaction mixture was heated to 130°C, and stirred and reacted for 20 hours. After the reaction was completed, the mixture was cooled to room temperature, and the two batches were combined for processing. The mixture was concentrated under reduced pressure to remove the solvent. The resulting residue was added with methyl tert-butyl ether (800 mL), stirred at room temperature for 30 minutes, and filtered. The filter cake was rinsed with methyl tert-butyl ether (100 mL × 2). The filtrate was concentrated under reduced pressure to remove the solvent. The resulting residue was separated by column chromatography (eluent: petroleum ether/ethyl acetate = 100/1 to 10/1, v/v) to obtain compound **BB-5-4.** $^1H$ NMR (400 MHz, $CDCl_3$) δ: 7.94 (s, 1H), 7.88 (s, 1H), 7.56 (d, *J*=8.4 Hz, 1H), 7.44 (d, *J*=8.4 Hz, 1H), 4.11 (q, *J*=7.2 Hz, 2H), 3.79 (s, 2H), 1.19 (t, *J*=7.2 Hz, 3H).

Step **4:** Synthesis of compound **BB-5**

Compound **BB-5-4** (5.00 g, 17.66 mmol) was dissolved in N, N-dimethylformamide (50 mL) at room temperature under nitrogen atmosphere, then acrylamide (1.51 g, 21.19 mmol) and potassium tert-butoxide (2.97 g, 26.49 mmol) were sequentially added thereto. The reaction mixture was stirred and reacted at room temperature for 2 hours. After the reaction was completed, the mixture was poured into 1 M dilute hydrochloric acid (100 mL), and extracted with ethyl acetate (30 mL × 2). The organic phases were combined, washed with 10% brine (100 mL × 2), dried over anhydrous sodium sulfate, and filtered. The filtrate was concentrated under reduced pressure to remove the solvent. The resulting residue was separated by column chromatography (eluent: ethyl acetate/petroleum ether = 1/1, v/v) to obtain compound **BB-5.** MS-ESI *m/z:* 308.0 $[M+H]^+$, 310.0 $[M+H+2]^+$. $^1H$ NMR (400 MHz, DMSO_$d_6$) δ: 10.91 (s, 1H), 7.94 (s, 1H), 7.89 (d, *J*=1.6 Hz, 1H), 7.56 (d, *J*=8.4 Hz, 1H), 7.42 (dd, *J*=1.6 Hz, 8.0 Hz, 1H), 4.15 (dd, *J*=4.8 Hz, 12.0 Hz, 1H), 2.80-2.68 (m, 1H), 2.58 (dt, *J*=4.0 Hz, 17.2 Hz, 1H), 2.38-2.25 (m, 1H), 2.15-2.05 (m, 1H).

**Reference example 6: Fragment BB-6**

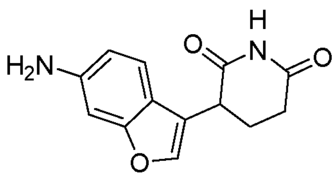

**BB-6**

Synthetic route:

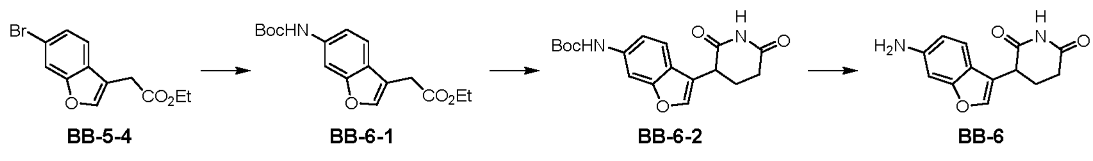

Step **1:** Synthesis of compound **BB-6-1**

Compound **BB-5-4** (10 g, 35.32 mmol), tert-butyl carbamate (4.97 g, 42.39 mmol), potassium phosphate (22.49 g, 105.96 mmol), tris(dibenzylideneacetone)dipalladium (646.88 mg, 706.42 μmol) and 2-di-tert-butylphosphine-2',4',6'-triisopropyl biphenyl (599.95 mg, 1.41 mmol) were dissolved in a mixed solvent of toluene (100 mL) and water (20 mL) at room temperature under nitrogen atmosphere. The reaction mixture was heated to 110°C, and stirred and reacted for 12 hours. After the reaction was completed, the mixture was cooled to room temperature, added with water (100 mL), and extracted with ethyl acetate (70 mL × 3). The organic phases were combined, washed with saturated brine

(70 mL × 2), dried over anhydrous sodium sulfate, and filtered. The filtrate was concentrated under reduced pressure to remove the solvent. The residue was added with n-heptane (70 mL), stirred at room temperature for 10 minutes, and filtered. The filter cake was collected. The filter cake was again added with n-heptane (50 mL), stirred at room temperature for 10 minutes, and filtered. The filter cake was rinsed with n-heptane (10 mL × 3), collected, and dried under vacuum to obtain compound **BB-6-1.** MS-ESI *m/z:* 264.2 $[M-55]^+$. $^1H$ NMR (400 MHz, $CDCl_3$) δ: 7.77 (s, 1H), 7.56 (s, 1H), 7.44 (d, *J*=8.4 Hz, 1H), 7.06 (dd, *J*=1.6 Hz, 8.4 Hz, 1H), 6.58 (s, 1H), 4.19 (q, *J*=7.0 Hz, 2H), 3.66 (s, 2H), 1.54 (s, 9H), 1.27 (t, *J*=7.2 Hz, 3H).

Step **2:** Synthesis of compound **BB-6-2**

Compound **BB-6-1** (9.8 g, 30.69 mmol) and acrylamide (2.62 g, 36.83 mmol) were dissolved in N, N-dimethylformamide (100 mL) at room temperature, and then potassium tert-butoxide (6.20 g, 55.24 mmol) was added thereto. The reaction mixture was stirred and reacted at room temperature for 1 hour. After the reaction was completed, the reaction mixture was poured into 0.5 M hydrochloric acid (120 mL), then added with water (300 mL), and extracted with ethyl acetate (150 mL × 3). The organic phases were combined, washed with saturated brine (150 mL), dried over anhydrous sodium sulfate, and filtered. The filtrate was concentrated under reduced pressure to remove the solvent. The resulting residue was added with methyl tert-butyl ether (70 mL), stirred at room temperature for 0.5 hours, and filtered. The filter cake was rinsed with methyl tert-butyl ether (10 mL × 3), collected, and dried under vacuum to obtain compound **BB-6-2.** $^1H$ NMR (400 MHz, DMSO_$d_6$) δ: 10.88 (s, 1H), 9.47 (s, 1H), 7.78 (s, 2H), 7.43 (d, *J*=8.8 Hz, 1H), 7.23 (dd, *J*= 1.4 Hz, 8.6 Hz, 1H), 4.07 (dd, *J*=4.8 Hz, 12.0 Hz, 1H), 2.78-2.65 (m, 1H), 2.62-2.53 (m, 1H), 2.35-2.23 (m, 1H), 2.15-2.06 (m, 1H), 1.49 (s, 9H).

Step **3:** Synthesis of hydrochloride of compound **BB-6**

Compound **BB-6-2** (6.6 g, 19.17 mmol) was dissolved in dichloromethane (20 mL) at room temperature, then a solution of hydrochloric acid in ethyl acetate (4 M, 150 mL) was added thereto. The reaction mixture was stirred and reacted at room temperature for 4 hours. After the reaction was completed, the mixture was concentrated under reduced pressure to remove the solvent to obtain the hydrochloride of compound **BB-6.** $^1H$ NMR (400 MHz, DMSO_$d_6$) δ: 10.92 (s, 1H), 10.06 (s, 2H), 7.98 (s, 1H), 7.66 (d, *J*=8.4 Hz, 1H), 7.57 (d, *J*=1.6 Hz, 1H), 7.21 (dd, *J*=1.8 Hz, 8.2 Hz, 1H), 4.16 (dd, *J*=4.8 Hz, 12.0 Hz, 1H), 2.81-2.69 (m, 1H), 2.63-2.54 (m, 1H), 2.39-2.26 (m, 1H), 2.16-2.07 (m, 1H).

**Reference example 7: Fragment BB-7**

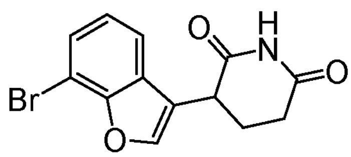

**BB-7**

Synthetic route:

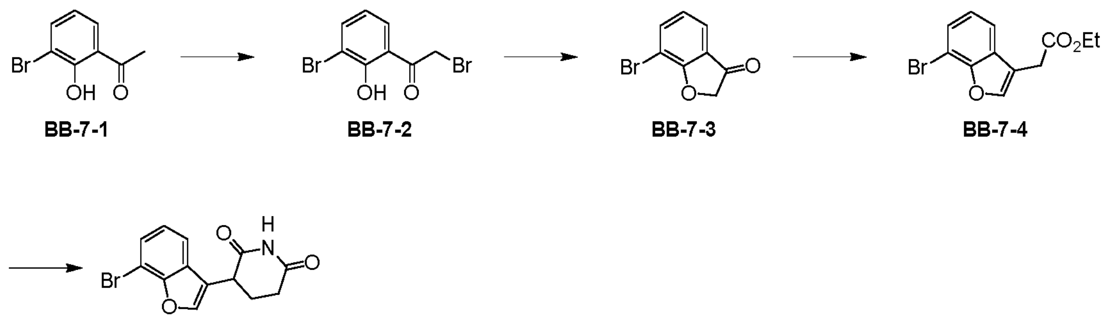

BB-7-1 BB-7-2 BB-7-3 BB-7-4

BB-7

Step **1:** Synthesis of compound **BB-7-2**

Compound **BB-7-1** (11.39 g, 52.97 mmol) was dissolved in chloroform (100 mL) and ethyl acetate (100 mL) at room temperature under nitrogen atmosphere, then copper bromide (23.66 g, 105.93 mmol) was added thereto. The reaction mixture was heated to 110°C, and stirred and reacted for 16 hours. After the reaction was completed, the mixture was cooled to room temperature, and filtered. The filtrate was concentrated under reduced pressure to remove the solvent, then the residue was added with water (20 mL), and extracted with ethyl acetate (20 mL × 3). The organic phases were combined, dried over anhydrous sodium sulfate, and filtered. The filtrate was concentrated under reduced pressure to remove the solvent. The resulting residue was separated by column chromatography (eluent: petroleum ether/ethyl acetate = 1/0 to 100/1, v/v) to obtain compound **BB-7-2.** $^1$H NMR (400 MHz, $CDCl_3$) δ: 12.39 (s, 1H), 7.81 (dd, *J*=1.2 Hz, 8.0 Hz, 1H), 7.76 (dd, *J*=1.2 Hz, 8.0 Hz, 1H), 6.88 (t, *J*=8.0 Hz, 1H), 4.47 (s, 2H).

Step **2:** Synthesis of compound **BB-7-3**

Compound **BB-7-2** (6.86 g, 18.44 mmol) was dissolved in dichloromethane (80 mL) at 0°C, and then triethylamine (1.87 g, 18.44 mmol) was slowly added dropwise thereto. The reaction mixture was slowly warmed to room temperature, and stirred and reacted for 1 hour. After the reaction was completed, the reaction mixture was directly concentrated under reduced pressure to remove the solvent to obtain a crude product of compound **BB-7-3,** which was used in the next step directly.

Step 3: Synthesis of compound **BB-7-4**

The crude product of compound **BB-7-3** (3.93 g, 18.45 mmol) was dissolved in toluene (80 mL) at room temperature under nitrogen atmosphere, and then (ethoxycarbonylmethylene)triphenylphosphorane (9.64 g, 27.67 mmol) was added thereto. The reaction mixture was heated to 130°C, and stirred and reacted for 36 hours. After the reaction was completed, the reaction mixture was cooled to room temperature, concentrated under reduced pressure to remove the solvent. Then the residue was added with water (100 mL), and extracted with ethyl acetate (50 mL × 3). The organic phases were combined, washed with saturated brine (50 mL), dried over anhydrous sodium sulfate, and filtered. The filtrate was concentrated under reduced pressure to remove the solvent. The resulting residue was separated by column chromatography (eluent: petroleum ether/ethyl acetate = 1/0 to 100/1, v/v) to obtain compound **BB-7-4.** $^1$H NMR (400 MHz, $CDCl_3$) δ: 7.72 (s, 1H), 7.52 (dd, *J*=0.8 Hz, 7.6 Hz, 1H), 7.48 (dd, *J*=0.8 Hz, 7.6 Hz, 1H), 7.15 (t, *J*=7.6 Hz, 1H), 4.20 (q, *J*=7.2 Hz, 2H), 3.70 (d, *J*=0.8 Hz, 2H), 1.28 (t, *J*=7.2 Hz, 3H).

Step **4:** Synthesis of compound **BB-7**

Compound **BB-7-4** (4.77 g, 16.85 mmol) was dissolved in N, N-dimethylformamide (30 mL) at 0°C under nitrogen atmosphere, then acrylamide (1.20 g, 16.85 mmol) and potassium tert-butoxide (1.89 g, 16.85 mmol) were sequentially added thereto. The reaction mixture was stirred and reacted at 0°C for 1 hour. After the reaction was completed, the mixture was poured into saturated ammonium chloride solution (50 mL), producing a large amount of white solid. The mixture was filtered and the solid was collected. The solid was slurried with methanol (20 mL × 2) at room temperature and filtered. The filter cake was collected, and dried under vacuum to obtain compound **BB-7.** $^1$H NMR (400 MHz, DMSO_$d_6$) δ: 10.92 (s, 1H), 8.04 (s, 1H), 7.62 (dd, *J*=0.8 Hz, 7.6 Hz, 1H), 7.56 (d, *J*=7.6 Hz, 1H), 7.21 (t, *J*=7.6 Hz, 1H), 4.17 (dd, *J*=4.8 Hz, 12.0 Hz, 1H), 2.79-2.70 (m, 1H), 2.63-2.55 (m, 1H), 2.40-2.27 (m, 1H), 2.16-2.08 (m, 1H).

**Reference example 8: Fragment BB-8**

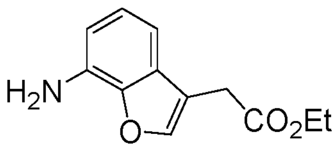

**BB-8**

Synthetic route:

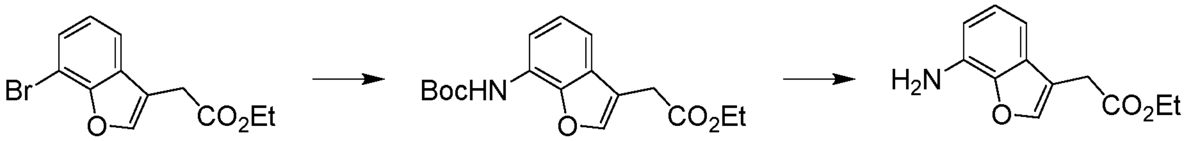

**BB-7-4** **BB-8-1** **BB-8**

Step **1:** Synthesis of compound **BB-8-1**

Compound **BB-7-4** (2 g, 7.06 mmol) was dissolved in toluene (50 mL) and water (5 mL) at room temperature under nitrogen atmosphere, and then tris(dibenzylideneacetone)dipalladium (647 mg, 706.42 μmol), 2-di-tert-butylphosphino-2',4',6'-triisopropyl biphenyl (450 mg, 1.06 mmol), potassium phosphate (6 g, 28.26 mmol), and tert-butyl carbamate (1.66 g, 14.13 mmol) were sequentially added thereto. The reaction mixture was heated to 100°C, and stirred and reacted for 16 hours. After the reaction was completed, the mixture was cooled to room temperature. The reaction mixture was poured into water (20 mL) and extracted with ethyl acetate (10 mL × 3). The organic phases were combined, washed with saturated brine (10 mL), dried over anhydrous sodium sulfate, and filtered. The filtrate was concentrated under reduced pressure to remove the solvent, and the resulting residue was separated by column chromatography (eluent: petroleum ether/ethyl acetate = 10/0 to 10/1, v/v) to obtain compound **BB-8-1.** $^1$H NMR (400 MHz, $CDCl_3$) δ: 7.93 (br s, 1H), 7.62 (s, 1H), 7.24-7.20 (m, 2H), 6.97 (s, 1H), 4.20 (q, *J*=6.8 Hz, 2H), 3.69 (d, *J*=0.8 Hz, 2H), 1.56 (s, 9H), 1.28 (t, *J*=7.2 Hz, 3H).

Step 2: Synthesis of hydrochloride of compound **BB-8**

Compound **BB-8-1** (1.32 g, 4.13 mmol) was dissolved in ethyl acetate (3 mL) at room temperature, then a solution of hydrochloric acid in ethyl acetate (4 M, 15 mL) was added thereto. The reaction mixture was stirred and reacted at room temperature for 2 hours. After the reaction was completed, the reaction mixture was concentrated under reduced pressure to remove the solvent directly to obtain the hydrochloride of compound **BB-8.**

**Reference example 9: Fragment BB-9**

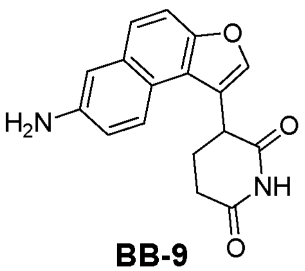

**BB-9**

Synthetic route:

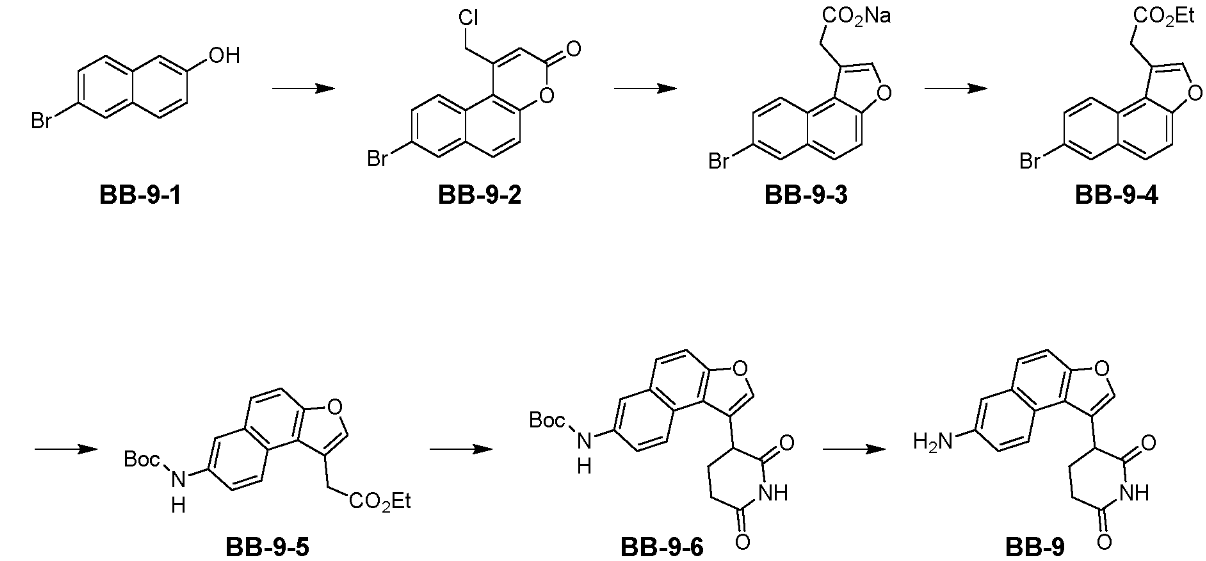

**BB-9-1** **BB-9-2** **BB-9-3** **BB-9-4**

**BB-9-5** **BB-9-6** **BB-9**

Step **1:** Synthesis of compound **BB-9-2**

Concentrated sulfuric acid (103.08 g, 1.03 mol, 56.02 mL, purity of 98%) was slowly added dropwise to a mixture of compound **BB-9-1** (25 g, 112.07 mmol) and ethyl 4-chloroacetoacetate (18.45 g, 112.07 mmol) at 0°C, with internal temperature controlled at 0 to 5°C. After the dropwise addition was completed, the reaction mixture was warmed to room temperature, and stirred and reacted for 12 hours. The four batches were combined for processing. After the reaction was completed, the reaction mixture was slowly poured into ice water (3 L) with stirring, stirred at room temperature for 10 minutes, and filtered. The filter cake was collected and dried under vacuum to obtain compound **BB-9-2.** MS-ESI *m/z:* 323.0 $[M+H]^+$, 325.0 $[M+H+2]^+$.

Step **2:** Synthesis of compound **BB-9-3**

Sodium hydroxide (17.65 g, 441.36 mmol) was dissolved in water (700 mL) at room temperature, then compound **BB-9-2** (54.40 g, 110.34 mmol, purity of 65.63%) was added thereto. The reaction mixture was heated to 80°C, and stirred and reacted for 12 hours. The three batches were combined for processing. After the reaction was completed, the mixture was cooled to room temperature, and filtered. The filter cake was washed with water (500 mL), collected, and dried under vacuum to obtain compound **BB-9-3.** $^1$H NMR (400 MHz, DMSO_$d_6$) $\delta$: 8.50 (d, *J*=9.2 Hz, 1H), 8.26 (d, *J*=2.4 Hz, 1H), 7.84 (s, 1H), 7.76 (d, *J*=1.6 Hz, 2H), 7.63 (dd, *J*=2.0 Hz, 8.8 Hz, 1H), 3.50 (s, 2H).

Step **3:** Synthesis of compound **BB-9-4**

Compound **BB-9-3** (33.16 g, 101.37 mmol) was dissolved in ethanol (300 mL) at room temperature, then concentrated sulfuric acid (27.19 g, 271.68 mmol, 14.78 mL, purity of 98%) was slowly added dropwise thereto. The reaction mixture was heated to 80°C, and stirred and reacted for 12 hours. The two batches were combined for processing. After the reaction was completed, the mixture was cooled to room temperature and concentrated under reduced pressure to remove the solvent. The resulting residue was added with water (600 mL) and extracted with ethyl acetate (200 mL $\times$ 2). The organic phases were combined, sequentially washed with an aqueous solution of sodium hydroxide (2 M, 300 mL) and saturated brine (500 mL), dried over anhydrous sodium sulfate, and filtered. The filtrate was concentrated under reduced pressure to remove the solvent to obtain compound **BB-9-4.** $^1$H NMR (400 MHz, $CDCl_3$) $\delta$ : 8.13-8.06 (m, 2H), 7.78 (s, 1H), 7.69-7.60 (m, 3H), 4.23 (q, *J*=7.2 Hz, 2H), 4.03 (s, 2H), 1.27 (t, *J*=7.2 Hz, 3H).

Step **4:** Synthesis of compound **BB-9-5**

Compound **BB-9-4** (1 g, 3.00 mmol), tris(dibenzylideneacetone)dipalladium (275 mg, 300.14 $\mu$mol), 2-di-tert-butylphosphino-2',4',6'-triisopropyl biphenyl (191.18 mg, 450.21 mmol), potassium phosphate (2.55 g, 12.01 mmol), and

tert-butyl carbamate (527.41 mg, 4.50 mmol) were dissolved in a mixed solvent of toluene (50 mL) and water (5 mL) at room temperature under nitrogen atmosphere. The reaction mixture was heated to 100°C, and stirred and reacted for 12 hours. After the reaction was completed, the mixture was cooled to room temperature. The reaction mixture was poured into water (20 mL) and extracted with ethyl acetate (10 mL × 3). The organic phases were combined, washed with saturated brine (10 mL), dried over anhydrous sodium sulfate, and filtered. The filtrate was concentrated under reduced pressure to remove the solvent. The resulting residue was separated by column chromatography (eluent: petroleum ether/ethyl acetate = 10/0 to 10/1, v/v) to obtain intermediate **BB-9-5.** MS-ESI *m/z*: 392.2 $[M+Na]^+$. $^1H$ NMR (400 MHz, $CDCl_3$) δ: 8.14 (d, *J*=9.2 Hz, 1H), 8.12 (br s, 1H), 7.74 (s, 1H), 7.67 (d, *J*=9.2 Hz, 1H), 7.62 (d, *J*=8.8 Hz, 1H), 7.45 (dd, *J*=2.2 Hz, 9.0 Hz, 1H), 6.64 (br s, 1H), 4.22 (q, *J*=7.0 Hz, 2H), 4.04 (s, 2H), 1.57 (s, 9H), 1.27 (t, *J*=7.2 Hz, 3H).

Step 5: Synthesis of compound **BB-9-6**

Compound **BB-9-5** (1.13 g, 2.74 mmol, purity of 89.57%) and acrylamide (234 mg, 3.29 mmol) were dissolved in N, N-dimethylformamide (10 mL) at 0°C under nitrogen atmosphere, and then potassium tert-butoxide (368.93 mg, 3.29 mmol) was added thereto. The reaction mixture was stirred and reacted at 0°C for 1 hour. After the reaction was completed, the reaction mixture was slowly poured into an aqueous solution of saturated ammonium chloride (30 mL), and extracted with ethyl acetate (15 mL × 3). The organic phases were combined, washed with saturated brine (10 mL), dried over anhydrous sodium sulfate, and filtered. The filtrate was concentrated under reduced pressure to remove the solvent. The resulting residue was added with methanol (10 mL), stirred at room temperature for 0.5 hours, and filtered. The filter cake was collected and dried under vacuum to obtain compound **BB-9-6.** MS-ESI *m/z:* 417.0 $[M+Na]^+$. $^1H$ NMR (400 MHz, $CDCl_3$) δ: 8.13 (br s, 1H), 8.08 (br s, 1H), 7.90 (d, *J*=8.8 Hz, 1H), 7.71 (d, *J*=9.2 Hz, 1H), 7.67-7.62 (m, 2H), 7.48 (dd, *J*=2.4 Hz, 8.8 Hz, 1H), 6.67 (br s, 1H), 4.47 (dd, *J*=5.4 Hz, 8.6 Hz, 1H), 2.85-2.71 (m, 2H), 2.55-2.40 (m, 2H), 1.57 (s, 9H).

Step 6: Synthesis of hydrochloride of compound **BB-9**

Compound **BB-9-6** (421 mg, 1.07 mmol) was dissolved in hydrochloric acid/dioxane solution (4 M, 20 mL) at room temperature. The reaction mixture was stirred and reacted at room temperature for 12 hours. After the reaction was completed, the reaction mixture was cooled, then poured into a saturated solution of ammonium chloride (100 mL), and extracted with ethyl acetate (10 mL × 3). The organic phases were combined, washed with saturated brine (10 mL), dried over anhydrous sodium sulfate, filtered, and evaporated to dryness by rotary evaporation. The resulting residue was separated by column chromatography (eluent: petroleum ether/ethyl acetate = 1/1, v/v) to obtain the hydrochloride of compound **BB-9.** MS-ESI *m/z:* 294.9 $[M+H]^+$. $^1H$ NMR (400 MHz, DMSO_$d_6$) δ: 10.95 (br s, 1H), 8.23 (d, *J*=9.2 Hz, 1H), 8.04 (s, 1H), 7.88-7.81 (m, 3H), 7.48-7.42 (m, 1H), 4.66 (dd, *J*=4.4 Hz, 12.4 Hz, 1H), 2.92-2.81 (m, 1H), 2.69-2.60 (m, 1H), 2.46-2.38 (m, 1H), 2.31-2.23 (m, 1H).

**Reference example 10: Fragment BB-10**

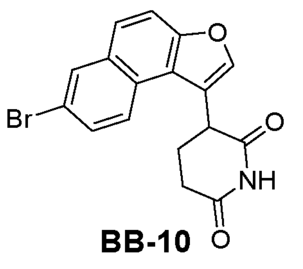

**BB-10**

Synthetic route:

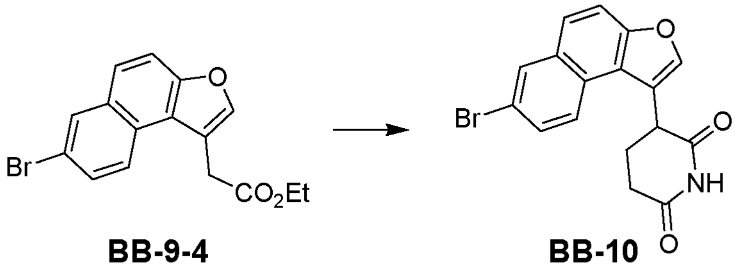

**BB-9-4** **BB-10**

Synthesis of compound **BB-10**

Compound **BB-9-4** (1 g, 3.00 mmol) was dissolved in N, N-dimethylformamide (10 mL) at room temperature under nitrogen atmosphere, then acrylamide (256 mg, 3.60 mmol) and potassium tert-butoxide (404.15 mg, 3.60 mmol) were sequentially added thereto. The reaction mixture was stirred and reacted at room temperature for 1 hour. After the reaction was completed, the reaction mixture was slowly poured into a saturated aqueous solution of ammonium chloride (100 mL), stirred at room temperature for 1 hour, and filtered. The filter cake was collected. The resulting filter cake was added with methanol (20 mL), stirred at room temperature for 0.5 hours, and filtered. The filter cake was collected, and dried under vacuum to obtain compound **BB-10.** MS-ESI *m/z:* 357.7 $[M+H]^+$, 359.7 $[M+H+2]^+$. $^1H$ NMR (400 MHz, $CDCl_3$) $\delta$: 8.15 (d, *J*=2.0 Hz, 1H), 8.06 (br s, 1H), 7.87 (d, *J*=8.8 Hz, 1H), 7.72-7.65 (m, 4H), 4.47 (dd, *J*=5.2 Hz, 9.6 Hz, 1H), 2.89-2.74 (m, 2H), 2.58-2.41 (m, 2H).

**Reference example 11: Fragment BB-11**

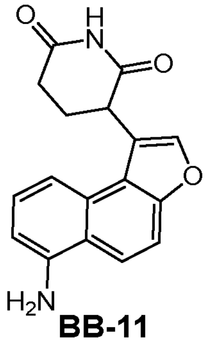

**BB-11**

Synthetic route:

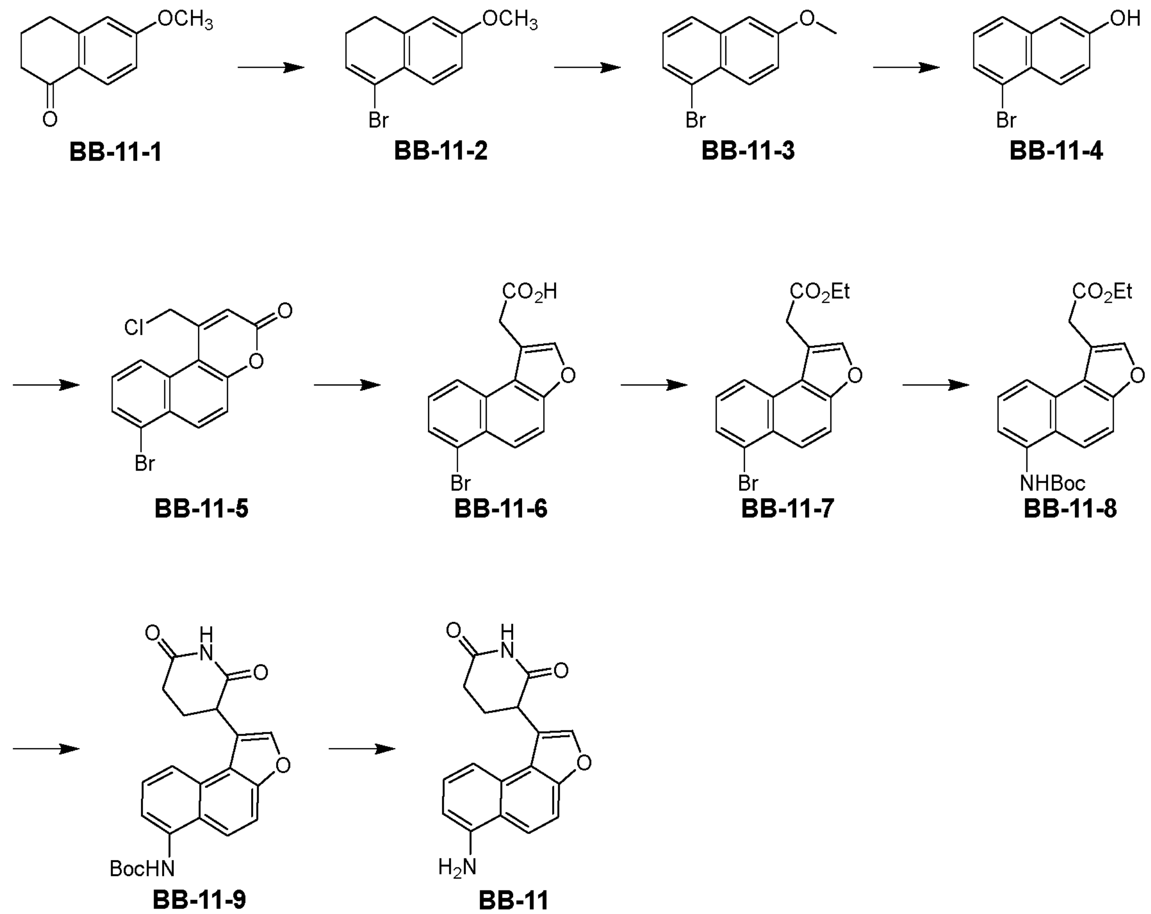

BB-11-1 BB-11-2 BB-11-3 BB-11-4

BB-11-5 BB-11-6 BB-11-7 BB-11-8

BB-11-9 BB-11

Step **1:** Synthesis of compound **BB-11-2**

Triphenyl phosphite (149.14 g, 480.67 mmol) was dissolved in dichloromethane (1.3 L) at room temperature under nitrogen atmosphere. The mixture was cooled to -70°C, and liquid bromine (83.80 g, 524.37 mmol, 27.03 mL) was dropwise added thereto. After the dropwise addition was completed, a solution of triethylamine (57.48 g, 568.07 mmol, 79.07 mL) and a solution of compound **BB-11-1** (77 g, 436.98 mmol) in dichloromethane (200 mL) were sequentially added dropwise to the mixture. After the dropwise addition was completed, the reaction mixture was slowly warmed to room temperature, and stirred and reacted for 15 hours. After the reaction was completed, the reaction mixture was slowly poured into a saturated aqueous solution of sodium sulfite (1.5 L), stirred for 10 minutes, and extracted with dichloromethane (1 L). The organic phases were washed with saturated brine (1 L), dried over anhydrous sodium sulfate, and filtered. The filtrate was concentrated under reduced pressure to remove the solvent. The resulting residue was separated by column chromatography (eluent: petroleum ether) to obtain compound **BB-11-2.**

Step **2:** Synthesis of compound **BB-11-3**

Compound **BB-11-2** (87 g, 363.85 mmol) was dissolved in toluene (1 L) at room temperature under nitrogen atmosphere. The mixture was cooled to 0°C, and 2,3-dichloro-5,6-dicyano-p-benzoquinone (90.86 g, 400.24 mmol) was slowly added thereto in batches. The reaction mixture was slowly warmed to room temperature, and stirred and reacted for 15 hours. After the reaction was completed, a saturated aqueous solution of sodium sulfite (2 L) was added dropwise. The mixture was stirred for 10 minutes, added with 1 N sodium hydroxide aqueous solution (1 L), and extracted with ethyl acetate (500 mL × 3). The organic phases were combined, washed with saturated brine (1 L), dried over anhydrous sodium sulfate, and filtered. The filtrate was concentrated under reduced pressure to remove the solvent. The resulting residue was added with petroleum ether (500 mL), stirred for 10 minutes, and filtered. The filter cake was rinsed with petroleum ether (50 mL × 2). The filtrate was collected, and concentrated under reduced pressure to remove the solvent. The resulting residue was separated by column chromatography (eluent: petroleum ether) to obtain compound **BB-11-3.** $^1H$ NMR (400 MHz, $CDCl_3$) δ: 8.12 (d, *J*=9.2 Hz, 1H), 7.68 (d, *J*=8.4 Hz, 1H), 7.60 (d, *J*=7.6 Hz, 1H), 7.26-7.20 (m, 2H), 7.11 (d, *J*=2.4 Hz, 1H), 3.92 (s, 3H).

Step **3:** Synthesis of compound **BB-11-4**

Compound **BB-11-3** (21.4 g, 90.26 mmol) was dissolved in dichloromethane (250 mL) at room temperature under nitrogen atmosphere. The mixture was cooled to 0°C, and boron tribromide (27.13 g, 108.31 mmol, 10.44 mL) was slowly added dropwise thereto. After the dropwise addition was completed, the reaction mixture was slowly warmed to room temperature, and stirred and reacted for 3 hours. After the reaction was completed, the reaction mixture was poured into ice water (500 mL) and extracted with dichloromethane (200 mL). The organic phase was washed with saturated brine (500 mL), dried over anhydrous sodium sulfate, and filtered. The filtrate was concentrated under reduced pressure to remove the solvent to obtain compound **BB-11-4.** $^1$H NMR (400 MHz, $CDCl_3$) δ: 8.15 (d, *J*=8.8 Hz, 1H), 7.65-7.59 (m, 2H), 7.25 (t, *J*=7.8 Hz, 1H), 7.19 (dd, *J*=2.4 Hz, 9.2 Hz, 1H), 7.14 (d, *J*=2.4 Hz, 1H), 5.03 (s, 1H).

Step **4:** Synthesis of compound **BB-11-5**

Compound **BB-11-4** (20 g, 89.66 mmol) was dissolved in methanesulfonic acid (200 mL) at room temperature, and ethyl 4-chloroacetoacetate (22.14 g, 134.49 mmol) was added dropwise thereto. The reaction mixture was stirred and reacted at room temperature for 15 hours. After the reaction was completed, the reaction mixture was poured into ice water (1 L), stirred at room temperature for 10 minutes, and filtered. The filter cake was rinsed with water (200 mL × 3), collected, and dried under vacuum to obtain compound **BB-11-5.** $^1$H NMR (400 MHz, DMSO_$d_6$) δ: 8.58 (d, *J*=9.2 Hz, 1H), 8.49 (d, *J*=9.2 Hz, 1H), 8.02 (d, *J*=7.2 Hz, 1H), 7.74 (d, *J*=9.2 Hz, 1H), 7.65 (dd, *J*=7.6 Hz, 8.4 Hz, 1H), 6.93 (s, 1H), 5.41 (s, 2H).

Step **5:** Synthesis of compound **BB-11-6**

Compound **BB-11-5** (29 g, 89.63 mmol) was added to an aqueous solution of sodium hydroxide (2 M, 300 mL) at room temperature. The reaction mixture was heated to 80°C, and stirred and reacted for 3 hours. After the reaction was completed, the mixture was cooled to room temperature, added with water (200 mL), added with 6 N dilute hydrochloric acid to adjust the pH to 4, and extracted with ethyl acetate (300 mL × 3). The organic phases were combined, washed with saturated brine (200 mL), dried over anhydrous sodium sulfate, and filtered. The filtrate was concentrated under reduced pressure to remove the solvent. The resulting residue was added with methyl tert-butyl ether (50 mL), stirred at room temperature for 10 minutes, and filtered. The filter cake was rinsed with methyl tert-butyl ether (10 mL × 2), collected, and dried under vacuum to obtain compound **BB-11-6.** MS-ESI *m/z:* 305.0 $[M+H]^+$, 306.9 $[M+H+2]^+$. $^1$H NMR (400 MHz, DMSO_$d_6$) δ : 12.68 (br s, 1H), 8.24 (d, *J*=8.4 Hz, 1H), 8.12 (t, *J*=4.6 Hz, 2H), 7.96 (d, *J*=9.2 Hz, 1H), 7.90 (d, *J*=7.6 Hz, 1H), 7.54 (t, *J*=7.8 Hz, 1H), 4.09 (s, 2H).

Step 6: Synthesis of compound **BB-11-7**

Compound **BB-11-6** (18 g, 58.99 mmol) was dissolved in ethanol (180 mL) at room temperature, then concentrated sulfuric acid (5.31 g, 53.09 mmol, 2.89 mL, purity of 98%) was added thereto. The reaction mixture was heated to 80°C, and stirred and reacted for 15 hours. After the reaction was completed, the mixture was cooled to room temperature, and concentrated under reduced pressure to remove the solvent. The residue was added with ethyl acetate (300 mL) and a saturated aqueous solution of sodium bicarbonate (500 mL), and extracted. The phases were separated. The organic phase was washed with saturated brine (300 mL), dried over anhydrous sodium sulfate, and filtered. The filtrate was concentrated under reduced pressure to remove the solvent. The resulting residue was added with petroleum ether (50 mL), stirred at room temperature for 10 minutes, and filtered. The filter cake was rinsed with petroleum ether (20 mL × 2), collected, and dried under vacuum to obtain compound **BB-11-7.** $^1$H NMR (400 MHz, $CDCl_3$) δ: 8.22 (t, *J*=8.4 Hz, 2H), 7.84-7.78 (m, 2H), 7.75 (d, *J*=9.2 Hz, 1H), 7.41 (dd, *J*=7.6 Hz, 8.4 Hz, 1H), 4.23 (q, *J*=7.0 Hz, 2H), 4.06 (d, *J*=0.8 Hz, 2H), 1.26 (t, *J*=7.2 Hz, 3H).

Step 7: Synthesis of compound **BB-11-8**

Compound **BB-11-7** (5 g, 15.01 mmol), tris(dibenzylideneacetone)dipalladium (961.96 mg, 1.05 mmol), 2-di-tert-butylphosphino-2',4',6'-triisopropyl biphenyl (892.16 mg, 2.10 mmol), potassium phosphate (12.74 g, 60.03 mmol), and tert-butyl carbamate (2.64 g, 22.51 mmol) were dissolved in a mixed solvent of toluene (50 mL) and water (10 mL) at room temperature under nitrogen atmosphere. The reaction mixture was heated to 100°C, and stirred and reacted for 15 hours. After the reaction was completed, the mixture was cooled to room temperature, and filtered. The filter cake was rinsed with ethyl acetate (30 mL × 3). The filtrate was washed with saturated brine (100 mL), dried over anhydrous sodium sulfate, and filtered. The filtrate was concentrated under reduced pressure to remove the solvent. The resulting residue was added with methyl tert-butyl ether (50 mL), stirred at room temperature for 10 minutes, and filtered. The

filter cake was rinsed with methyl tert-butyl ether (10 mL × 2), collected, and dried under vacuum to obtain compound **BB-11-8.**

Step **8:** Synthesis of compound **BB-11-9**

Compound **BB-11-8** (4.2 g, 11.37 mmol) and acrylamide (888.93 mg, 12.51 mmol) were dissolved in N, N-dimethylformamide (40 mL) at room temperature under nitrogen atmosphere. The mixture was cooled to 0°C. Potassium tert-butoxide (2.55 g, 22.74 mmol) dissolved in N, N-dimethylformamide (10 mL) was added dropwise thereto. The reaction mixture was warmed to room temperature, and stirred and reacted for 2 hours. After the reaction was completed, the reaction mixture was poured into 0.2 N hydrochloric acid (200 mL), and extracted with ethyl acetate (100 mL × 3). The organic phases were combined, washed with saturated brine (100 mL), dried over anhydrous sodium sulfate, and filtered. The filtrate was concentrated under reduced pressure to remove the solvent. The resulting residue was added with dichloromethane (20 mL), stirred at room temperature for 10 minutes, and filtered. The filter cake was rinsed with dichloromethane (10 mL), collected, and dried under vacuum to obtain compound **BB-11-9.** $^1$H NMR (400 MHz, DMSO_$d_6$) δ: 10.94 (s, 1H), 9.28 (s, 1H), 8.05-7.92 (m, 3H), 7.79 (d, *J*=9.6 Hz, 1H), 7.58-7.47 (m, 2H), 4.68 (dd, *J*=4.4 Hz, 12.0 Hz, 1H), 2.95-2.81 (m, 1H), 2.70-2.56 (m, 1H), 2.47-2.34 (m, 1H), 2.33-2.22 (m, 1H), 1.49 (s, 9H).

Step **9:** Synthesis of hydrochloride of compound **BB-11**

Compound **BB-11-9** (1.3 g, 3.30 mmol) was dissolved in ethyl acetate (5 mL) at room temperature, then a solution of hydrochloric acid in ethyl acetate (4 M, 50 mL) was added thereto. The reaction mixture was stirred and reacted at room temperature for 3 hours. After the reaction was completed, the reaction mixture was concentrated under reduced pressure to remove the solvent to obtain the hydrochloride of compound **BB-11.**

**Reference example 12: Fragment BB-12**

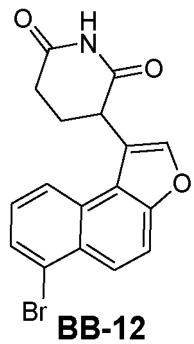

**BB-12**

Synthetic route:

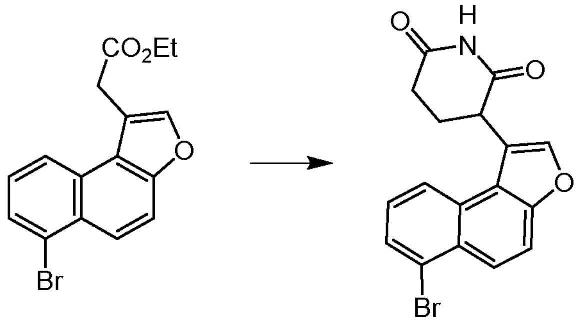

**BB-11-7** **BB-12**

Synthesis of compound **BB-12**

Acrylamide (234.67 mg, 3.30 mmol) and compound **BB-11-7** (1 g, 3.00 mmol) were dissolved in N, N-dimethylformamide (15 mL) at room temperature under nitrogen atmosphere, then potassium tert-butoxide (370.47 mg, 3.30 mmol) was added thereto. The reaction mixture was stirred and reacted at room temperature for 0.5 hours. After the reaction was completed, the reaction mixture was poured into 0.1 N hydrochloric acid (10 mL) and extracted with dichloromethane (5 mL ×2). The organic phases were combined, washed with saturated brine (5 mL), dried over anhydrous sodium sulfate, and filtered. The filtrate was concentrated under reduced pressure to remove the solvent. The resulting residue was added with dichloromethane (5 mL), stirred at room temperature for 1 hour, and filtered. The filter cake was rinsed with dichloromethane (1 mL), collected, and dried under vacuum to obtain compound **BB-12.** MS-ESI *m/z:* 358.1 $[M+H]^+$, 360.1 $[M+H+2]^+$.

**Reference example 14: Fragment BB-14**

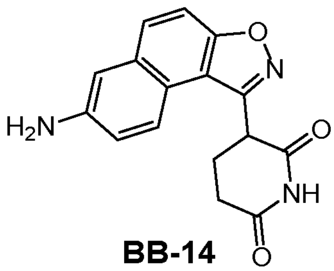

**BB-14**

Synthetic route:

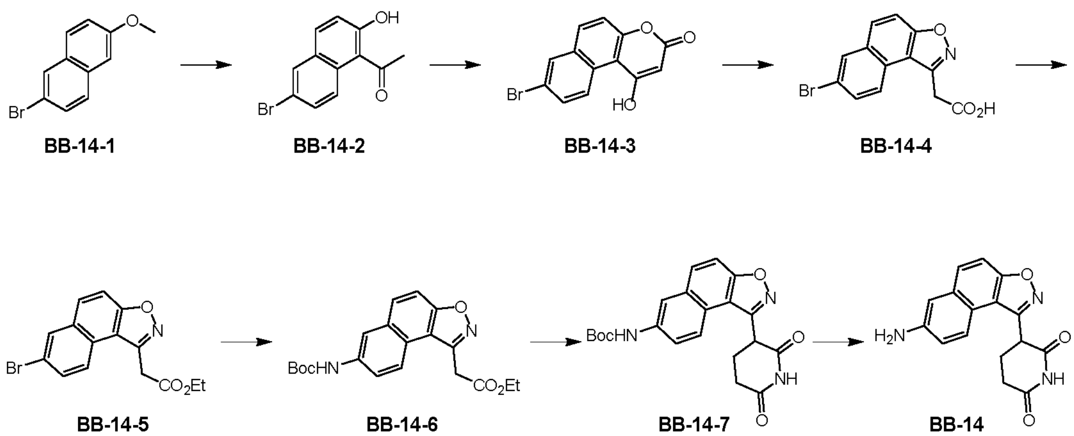

**BB-14-1** **BB-14-2** **BB-14-3** **BB-14-4**

**BB-14-5** **BB-14-6** **BB-14-7** **BB-14**

Step **1:** Synthesis of compound **BB-14-2**

Compound **BB-14-1** (150 g, 632.67 mmol) was dissolved in dichloromethane (3 L) at room temperature under nitrogen atmosphere, then acetyl chloride (49.66 g, 632.67 mmol, 45.15 mL) was added thereto. The reaction mixture was cooled to 5 to 15°C, and aluminum trichloride (177.16 g, 1.33 mol) was then added thereto in batches. The reaction mixture was warmed to room temperature, and stirred and reacted for 4 hours. Additional aluminum trichloride (29.53 g, 221.43 mmol) was added, and the reaction mixture was further stirred and reacted at room temperature for 12 hours. After the reaction was completed, the reaction mixture was slowly poured into ice water (3 L) and extracted. The phases were separated. The aqueous phase was again extracted with dichloromethane (2 L × 2). The organic phases were combined, washed with saturated brine (6 L × 2), dried over anhydrous sodium sulfate, and filtered. The filtrate was concentrated under reduced pressure to remove the solvent to obtain compound **BB-14-2.** MS-ESI *m/z:* 265.1 $[M+H]^+$, 267.1 $[M+H+2]^+$.

Step **2:** Synthesis of compound **BB-14-3**

Compound **BB-14-2** (200 g, 754.43 mmol) and dimethyl carbonate (271.83 g, 3.02 mol, 254.04 mL) was dissolved in tetrahydrofuran (2 L) at room temperature under nitrogen atmosphere. The mixture was cooled to 0°C, and potassium tert-butoxide (507.93 g, 4.53 mol) was slowly added thereto. The reaction mixture was heated to 70°C, and stirred and reacted for 12 hours. After the reaction was completed, the mixture was cooled to room temperature, concentrated under reduced pressure to remove the solvent. The resulting residue was added with ice water (4 L), and added with 6 N hydrochloric acid to adjust the pH to 2 to 3, resulting in the precipitation of a large amount of solid. The mixture was filtered. The filter cake was sequentially rinsed with water (1 L) and methyl tert-butyl ether (1 L ), collected, and dried under vacuum to obtain compound **BB-14-3.** MS-ESI m/z: 291.0 $[M+H]^+$, 293.0 $[M+H+2]^+$. $^1H$ NMR (400 MHz, DMSO_$d_6$) δ: 9.18 (d, *J*=9.2 Hz, 1H), 8.30 (d, *J*=1.2 Hz, 1H), 8.16 (d, *J*=8.8Hz, 1H), 7.79 (d, *J*=7.6 Hz, 1H), 7.56 (d, *J*=8.8 Hz, 1H), 5.83 (s, 1H).

Step 3: Synthesis of compound **BB-14-4**

Compound **BB-14-3** (200 g, 687.06 mmol), sodium acetate (309.99 g, 3.78 mol), and hydroxylamine hydrochloride (262.59 g, 3.78 mol) were dissolved in ethanol (2 L) at room temperature. The reaction mixture was heated to 80°C, and stirred and reacted for 12 hours. After the reaction was completed. The mixture was cooled to room temperature, poured into water (2 L), and concentrated under reduced pressure to remove the solvent. The mixture was added with 2 N hydrochloric acid to adjust the pH to 2 to 3, and extracted with ethyl acetate/tetrahydrofuran = 1/1 (2 L × 3, v/v). The organic phases were combined, dried over anhydrous sodium sulfate, and filtered. The filtrate was concentrated under reduced pressure to remove the solvent. The resulting residue was added with methyl tert-butyl ether (600 mL), stirred at room temperature for 1 hour, and filtered. The filter cake was collected, and dried under vacuum to obtain compound **BB-14-4.** MS-ESI *m*/*z*: 306.0 $[M+H]^+$, 308.0 $[M+H+2]^+$.

Step 4: Synthesis of compound **BB-14-5**

Concentrated sulfuric acid (33.38 g, 333.49 mmol, 18.14 mL, purity of 98%) was dissolved in ethanol (1.3 L) at room temperature, then compound **BB-14-4** (135 g, 373.32 mmol, purity of 84.65%) was added thereto. The reaction mixture was heated to 75°C, and stirred and reacted for 16 hours. After the reaction was completed, the mixture was cooled to room temperature, and concentrated under reduced pressure to remove the solvent. The resulting residue was added with ethanol(600 mL), stirred at room temperature for 5 minutes, and filtered. The filter cake was rinsed with ethanol (100 mL × 2), collected, and dried under vacuum to obtain compound **BB-14-5.** MS-ESI *m*/*z*: 334.1 $[M+H]^+$, 336.0 $[M+H+2]^+$. $^1H$ NMR (400 MHz, DMSO_$d_6$) δ: 8.47 (d, *J*=1.6 Hz, 1H), 8.19 (d, *J*=9.2 Hz, 1H), 8.08 (d, *J*=8.8 Hz, 1H), 8.00 (d, *J*=9.2 Hz, 1H), 7.89 (dd, *J*=2.0 Hz, 8.8 Hz, 1H), 4.51 (s, 2H), 4.14 (q, *J*=7.2 Hz, 2H), 1.16 (t, *J*=7.2 Hz, 3H).

Step 5: Synthesis of compound **BB-14-6**

Compound **BB-14-5** (15 g, 44.89 mmol), tris(dibenzylideneacetone)dipalladium (1.44 g, 1.57 mmol), 2-di-tert-butylphosphino-2',4',6'-triisopropyl biphenyl (1.33 g, 3.14 mmol), potassium phosphate (38.11 g, 179.55 mmol), and tert-butyl carbamate (7.89 g, 67.33 mmol) were sequentially added to a mixed solvent of toluene (150 mL) and water (30 mL) at room temperature under nitrogen atmosphere. The reaction mixture was heated to 100°C, and stirred and reacted for 12 hours. After the reaction was completed, the mixture was cooled to room temperature, added with water (100 mL) and ethyl acetate (150 mL), and extracted. The phases were separated. The aqueous phase was again extracted with ethyl acetate (200 mL × 3). The organic phases were combined, washed with saturated brine (200 mL × 3), dried over anhydrous sodium sulfate, and filtered. The filtrate was concentrated under reduced pressure to remove the solvent. The resulting residue was separated by column chromatography (eluent: petroleum ether/ethyl acetate = 20/1 to 1/1, v/v) to obtain compound **BB-14-6.** MS-ESI *m*/*z:* 371.2 $[M+H]^+$. $^1H$ NMR (400 MHz, $CDCl_3$) δ: 8.20 (br s, 1H), 8.03 (d, *J*=8.8 Hz, 1H), 7.90 (d, *J*=9.2 Hz, 1H), 7.67 (d, *J*=9.2 Hz, 1H), 7.53 (dd, *J*=2.0 Hz, 8.8 Hz, 1H), 6.75 (s, 1H), 4.31 (s, 2H), 4.21 (q, *J*=7.2 Hz, 2H), 1.57 (s, 9H), 1.20 (t, *J*=7.2 Hz, 3H).

Step 6: Synthesis of compound **BB-14-7**

Compound **BB-14-6** (7.3 g, 19.71 mmol) and acrylamide (1.54 g, 21.68 mmol) was dissolved in tetrahydrofuran (70 mL) at room temperature under nitrogen atmosphere, The mixture was cooled to 0°C, and potassium tert-butoxide (2.43 g, 21.68 mmol) was added thereto. The reaction mixture was warmed to room temperature, and stirred and reacted for 1 hour. After the reaction was completed, the reaction mixture was poured into 0.1 N hydrochloric acid (30 mL), and extracted with ethyl acetate (80 mL × 3). The organic phases were combined, washed with saturated brine (50 mL ×

2), dried over anhydrous sodium sulfate, and filtered. The filtrate was concentrated under reduced pressure to remove the solvent. The resulting residue was added with dichloromethane (5 mL), stirred at room temperature for 5 minutes, and filtered. The filter cake was rinsed with dichloromethane (3 mL), collected, and dried under vacuum to obtain compound **BB-14-7.** MS-ESI *m/z:* 396.2 $[M+H]^+$. $^1H$ NMR (400 MHz, DMSO_$d_6$) δ: 11.12 (s, 1H), 9.66 (s, 1H), 8.36 (s, 1H), 8.13 (d, *J*=8.8 Hz, 1H), 8.07 (d, *J*=8.8 Hz, 1H), 7.85 (d, *J*=9.2 Hz, 1H), 7.68 (dd, *J*=2.0 Hz, 8.8 Hz, 1H), 5.00 (dd, *J*=4.8 Hz, 11.6 Hz, 1H), 2.89-2.76 (m, 1H), 2.68-2.60 (m, 1H), 2.59-2.53 (m, 1H), 2.42-2.30 (m, 1H), 1.52 (s, 9H).

Step 7: Synthesis of hydrochloride of compound **BB-14**

Compound **BB-14-7** (1 g, 2.53 mmol) was dissolved in ethyl acetate (5 mL) at room temperature, then a solution of hydrochloric acid in ethyl acetate (4 M, 20 mL) was added thereto. The reaction mixture was stirred and reacted at room temperature for 1 hour. After the reaction was completed, the reaction mixture was filtered. The filter cake was rinsed with ethyl acetate (3 mL× 3), collected, and dried under vacuum to obtain the hydrochloride of compound **BB-14.** MS-ESI *m/z:* 295.9 $[M+H]^+$.

**Reference example 15: Fragment BB-15**

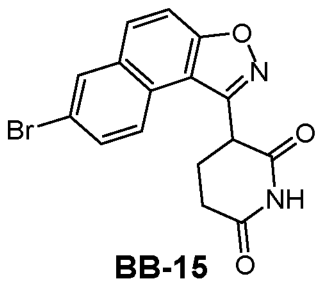

**BB-15**

Synthetic route:

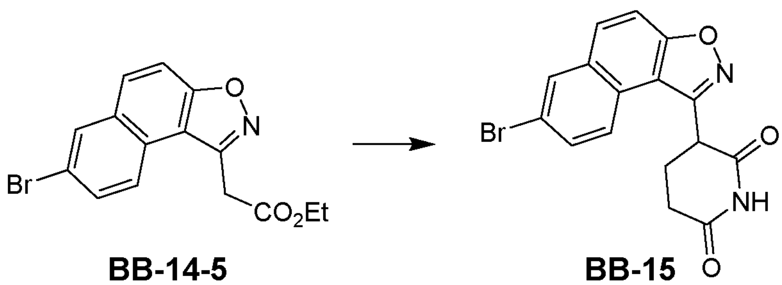

**BB-14-5** **BB-15**

Synthesis of compound **BB-15**

Compound **BB-14-5** (3 g, 8.98 mmol) and acrylamide (701.92 mg, 9.88 mmol) were dissolved in tetrahydrofuran (70 mL) at room temperature under nitrogen atmosphere. The mixture was cooled to 0°C, and potassium tert-butoxide (1.21 g, 10.77 mmol) was added thereto in batches. The reaction mixture was warmed to room temperature, and stirred and reacted for 1 hour. After the reaction was completed, the reaction mixture was poured into 1 N hydrochloric acid (20 mL), and extracted with ethyl acetate (40 mL × 3). The organic phases were combined, washed with saturated brine (50 mL), dried over anhydrous sodium sulfate, and filtered. The filtrate was concentrated under reduced pressure to remove the solvent. The resulting residue was added with methyl tert-butyl ether (20 mL), stirred at room temperature for 10 minutes, and filtered. The filter cake was rinsed with methyl tert-butyl ether (5 mL × 2), collected, and dried under vacuum to obtain compound **BB-15.** MS-ESI *m/z:* 359.0 $[M+H]^+$, 361.0 $[M+H+2]^+$. $^1H$ NMR (400 MHz, DMSO_$d_6$) δ: 11.14 (s, 1H), 8.47 (d, *J*=2.0 Hz, 1H), 8.24-8.17 (m, 2H), 8.01 (d, *J*=9.2 Hz, 1H), 7.85 (dd, *J*=2.0 Hz, 8.8 Hz, 1H), 5.06 (dd, *J*=4.8 Hz, 11.6 Hz, 1H), 2.89-2.78 (m, 1H), 2.70-2.56 (m, 2H), 2.44-2.34 (m, 1H).

**Reference example 16: Fragment BB-16**

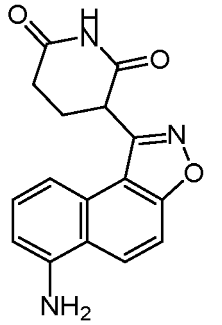

**BB-16**

Synthetic route:

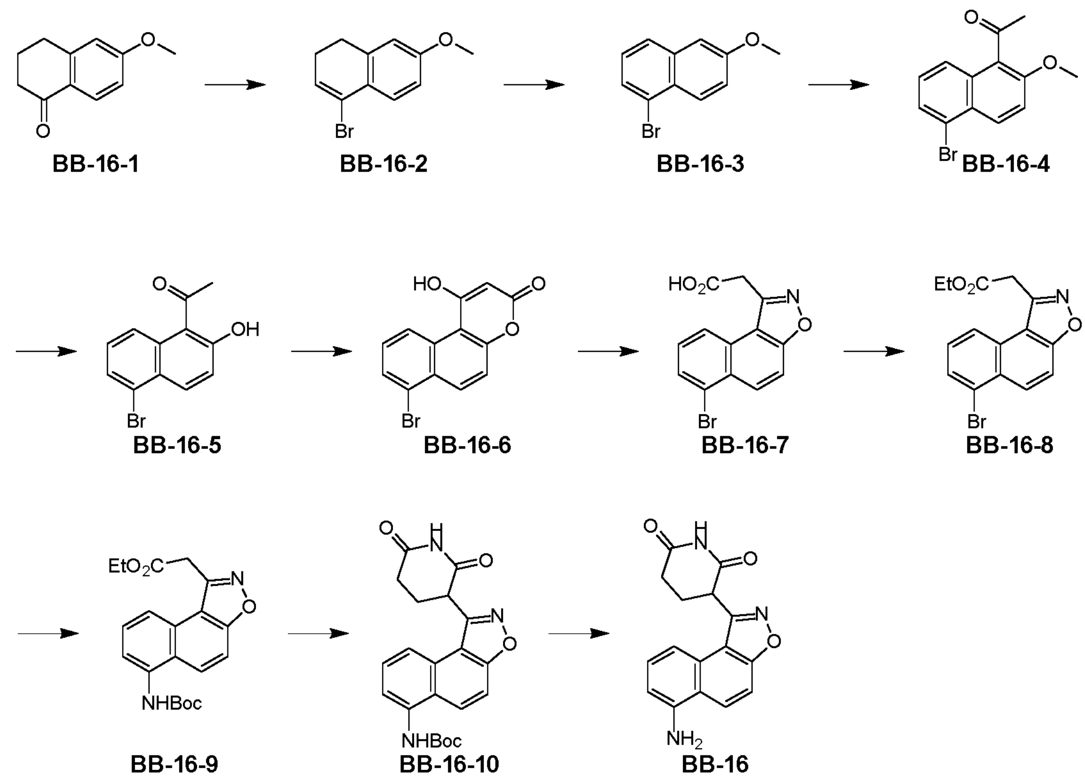

BB-16-1 BB-16-2 BB-16-3 BB-16-4

BB-16-5 BB-16-6 BB-16-7 BB-16-8

BB-16-9 BB-16-10 BB-16

Step **1:** Synthesis of compound **BB-16-2**

Triphenyl phosphite (193.69 g, 624.25 mmol) was dissolved in dichloromethane (1 L) at room temperature under nitrogen atmosphere. The mixture was cooled to -70°C, and liquid bromine (108.83 g, 681.00 mmol, 35.11 mL) was added dropwise thereto. After the dropwise addition was completed, triethylamine (74.65 g, 737.75 mmol, 102.69 mL) and compound **BB-16-1** (100, 567.50 mmol) dissolved in dichloromethane (500 mL) were sequentially added dropwise thereto. After the dropwise addition was completed, the reaction mixture was slowly warmed to room temperature, and stirred and reacted for 15 hours. After the reaction was completed, the reaction mixture was poured into a saturated aqueous solution of sodium sulfite (700 mL), stirred for 10 minutes, and extracted with dichloromethane (800 mL). The organic phases were washed with saturated brine (800 mL), dried over anhydrous sodium sulfate, and filtered.

The filtrate was concentrated under reduced pressure to remove the solvent. The resulting residue was separated by column chromatography (eluent: petroleum ether/ethyl acetate = 20/1 to 7/1, v/v) to obtain compound **BB-16-2.** $^1$H NMR (400 MHz, DMSO_*d$_6$*) δ: 7.33 (d, *J*=8.0 Hz, 1H), 6.84-6.75 (m, 2H), 6.33 (t, *J*=4.8 Hz, 1H), 3.75 (s, 3H), 2.76 (t, *J*=8.0 Hz, 2H), 2.32-2.25 (m, 2H).

Step **2:** Synthesis of compound **BB-16-3**

Compound **BB-16-2** (39.5 g, 165.20 mmol) was dissolved in toluene (500 mL) at room temperature under nitrogen atmosphere. The mixture was cooled to 0°C, and 2,3-dichloro-5,6-dicyano-p-benzoquinone (41.25 g, 181.72 mmol) was slowly added thereto in batches. The reaction mixture was slowly warmed to room temperature, and stirred and reacted for 12 hours. After the reaction was completed, the mixture was cooled to 0 to 10°C. A saturated aqueous solution of sodium sulfite (1 L) and an aqueous solution of 1 N sodium hydroxide (1 L) were added dropwise thereto. The mixture was filtered. The filter cake was rinsed with ethyl acetate (300 mL × 3), and discarded. The filtrate was extracted with ethyl acetate (500 mL × 3). The organic phases were combined, washed with saturated brine (500 mL × 2), dried over anhydrous sodium sulfate, and filtered. The filtrate was concentrated under reduced pressure to remove the solvent. The resulting residue was separated by column chromatography (eluent: petroleum ether) to obtain compound **BB-16-3.** MS-ESI *m/z*: 237.1 [M+H]$^+$, 239.1 [M+H+2]$^+$. $^1$H NMR (400 MHz, $CDCl_3$) δ: 8.05 (d, *J*=9.2 Hz, 1H), 7.60 (d, *J*=8.0 Hz, 1H), 7.53 (d, *J*=7.2 Hz, 1H), 7.22-7.13 (m, 2H), 7.04 (s, 1H), 3.85 (s, 3H).

Step **3:** Synthesis of compound **BB-16-4**

Acetic anhydride (21.53 g, 210.89 mmol, 19.75 mL) was dissolved in dichloromethane (400 mL) at room temperature under nitrogen atmosphere. The mixture was cooled to -60°C, and a solution of boron trifluoride diethyl etherate (63.68 g, 210.89 mmol, 55.38 mL, purity of 47%) was added dropwise thereto. The mixture was stirred at -60°C for 10 minutes. Compound **BB-16-3** (25, 105.44 mmol) dissolved in dichloromethane (250 mL) was added dropwise thereto. The reaction mixture was slowly warmed to room temperature, and stirred and reacted for 12 hours. After the reaction was completed, the reaction mixture was added with ice water (200 mL), and extracted with dichloromethane (100 mL × 3). The organic phases were combined, washed with saturated brine (100 mL), dried over anhydrous sodium sulfate, and filtered. The filtrate was concentrated under reduced pressure to remove the solvent. The resulting residue was separated by column chromatography (eluent: petroleum ether/ethyl acetate = 100/1 to 20/1, v/v) to obtain compound **BB-16-4.** MS-ESI *m/z:* 279.0 [M+H]$^+$, 281.0 [M+H+2]$^+$. $^1$H NMR (400 MHz, $CDCl_3$) δ: 8.34 (d, *J*=9.2 Hz, 1H), 7.72 (d, *J*=8.4 Hz, 1H), 7.67 (dd, *J*=0.8 Hz, 7.2 Hz, 1H), 7.38 (d, *J*=9.2 Hz, 1H), 7.31 (dd, *J*=7.6 Hz, 8.8 Hz, 1H), 4.01 (s, 3H), 2.65 (s, 3H).

Step **4:** Synthesis of compound **BB-16-5**

Compound **BB-16-4** (18.8 g, 67.35 mmol) was dissolved in dichloromethane (200 mL) at room temperature under nitrogen atmosphere. The mixture was cooled to 0°C, and boron tribromide (20.25 g, 80.82 mmol, 7.79 mL) was added dropwise thereto. The mixture was stirred and reacted at 0°C for 1 hour. After the reaction was completed, the reaction mixture was poured into ice water (300 mL), and extracted with dichloromethane (100 mL × 3). The organic phases were combined, washed with saturated brine (100 mL × 3), dried over anhydrous sodium sulfate, and filtered. The filtrate was concentrated under reduced pressure to remove the solvent to obtain compound **BB-16-5.** $^1$H NMR (400 MHz, $CDCl_3$) δ: 13.05 (s, 1H), 8.41 (d, *J*=9.2 Hz, 1H), 8.04 (d, *J*=8.8 Hz, 1H), 7.70 (dd, *J*=0.8 Hz, 7.6 Hz, 1H), 7.40 (dd, *J*=7.6 Hz, 8.4 Hz, 1H), 7.25 (d, *J*=9.6 Hz, 1H), 2.85 (s, 3H).

Step **5:** Synthesis of compound **BB-16-6**

Compound **BB-16-5** (13 g, 49.04 mmol) and dimethyl carbonate (17.67 g, 196.15 mmol, 16.51 mL) were dissolved in tetrahydrofuran (130 mL) at room temperature under nitrogen atmosphere. The mixture was cooled to 0 to 10°C, and potassium tert-butoxide (33.02 g, 294.23 mmol) was added thereto in batches. The reaction mixture was heated to 70°C, and stirred and reacted for 12 hours. After the reaction was completed, the mixture was cooled to room temperature, and concentrated under reduced pressure to remove the solvent. The residue was added with ice water (200 mL), and extracted with methyl tert-butyl ether (70 mL × 2). The pH of the aqueous phase was adjusted to 2 with 6 N hydrochloric acid, resulting in the precipitation of a large amount of solid. The mixture was filtered. The filter cake was washed with water (30 mL×2), collected, and dried under vacuum to obtain compound **BB-16-6.** MS-ESI*m/z*: 291.0 [M+H]$^+$, 293.0 [M+H+2]$^+$. $^1$H NMR (400 MHz, DMSO_*d$_6$*) δ: 13.28 (br s, 1H), 9.38 (t, *J*=7.2 Hz, 1H), 8.43 (dd, *J*=6.8 Hz, 9.2 Hz, 1H), 7.96 (t, *J*=7.2 Hz, 1H), 7.71-7.65 (m, 1H), 7.63-7.56 (m, 1H), 5.92-5.87 (m, 1H).

Step **6:** Synthesis of compound **BB-16-7**

Compound **BB-16-6** (22 g, 75.58 mmol), sodium acetate (43.40 g, 529.03 mmol), and hydroxylamine hydrochloride (36.76 g, 529.03 mmol) were dissolved in ethanol (400 mL) at room temperature. The reaction mixture was heated to 80°C, and stirred and reacted for 12 hours. After the reaction was completed, the mixture was cooled to room temperature, and concentrated under reduced pressure to remove the solvent. The resulting residue was added with water (100 mL) and methyl tert-butyl ether (50 mL), stirred at room temperature for 0.5 hours, and filtered. The filter cake was rinsed with methyl tert-butyl ether (20 mL × 3), collected, and dried under vacuum to obtain compound **BB-16-7.** MS-ESI *m*/*z*: 306.0 $[M+H]^+$, 308.0 $[M+H+2]^+$. $^1H$ NMR (400 MHz, DMSO_$d_6$) δ: 8.40 (d, *J*=9.2 Hz, 1H), 8.27 (d, *J*=8.4 Hz, 1H), 8.05 (d, *J*=9.2 Hz, 1H), 7.96 (d, *J*=7.6 Hz, 1H), 7.62 (t, *J*=8.0 Hz, 1H), 4.25 (s, 2H).

Step **7:** Synthesis of compound **BB-16-8**

Compound **BB-16-7** (15 g, 49.00 mmol) was dissolved in ethanol (300 mL) at room temperature, then concentrated sulfuric acid (1.29 g, 12.87 mmol, 0.7 mL, purity of 98%) was added thereto. The reaction mixture was heated to 80°C, and stirred and reacted for 12 hours. The mixture was cooled to room temperature. Additional concentrated sulfuric acid (4 mL, purity of 98%) was added thereto. The reaction mixture was heated to 80°C, and stirred and reacted for 3 hours. After the reaction was completed, the mixture was cooled to room temperature, and concentrated under reduced pressure to remove the solvent. The resulting residue was added with a saturated solution of sodium bicarbonate at 0 to 10°C (100 mL), and extracted with ethyl acetate (70 mL × 3). The organic phases were combined, washed with saturated brine (70 mL × 2), dried over anhydrous sodium sulfate, and filtered. The filtrate was concentrated under reduced pressure to remove the solvent to obtain compound **BB-16-8.** MS-ESI *m/z:* 334.0 $[M+H]^+$, 336.0 $[M+H+2]^+$. $^1H$ NMR (400 MHz, $CDCl_3$) δ: 8.50 (d, *J*=9.6 Hz, 1H), 8.10 (d, *J*=8.4 Hz, 1H), 7.88 (dd, *J*=0.8 Hz, 7.6 Hz, 1H), 7.81 (d, *J*=9.2 Hz, 1H), 7.52 (t, *J*=8.0 Hz, 1H), 4.34 (s, 2H), 4.22 (q, *J*=7.2 Hz, 2H), 1.21 (t, *J*=7.2 Hz, 3H).

Step **8:** Synthesis of compound **BB-16-9**

Compound **BB-16-8** (9.5 g, 28.43 mmol), tris(dibenzylideneacetone)dipalladium (1.30 g, 1.42 mmol), 2-di-tert-butylphosphino-2',4',6'-triisopropyl biphenyl (1.21 g, 2.84 mmol), potassium phosphate (24.14 g, 113.72 mmol), and tert-butyl carbamate (3.66 g, 31.27 mmol) were sequentially added to a mixed solvent of toluene (90 mL) and water (30 mL) at room temperature under nitrogen atmosphere. The reaction mixture was heated to 100°C, and stirred and reacted for 12 hours. After the reaction was completed, the mixture was cooled to room temperature, added with water (200 mL), and extracted with ethyl acetate (150 mL × 3). The organic phases were combined, washed with saturated brine (200 mL × 2), dried over anhydrous sodium sulfate, and filtered. The filtrate was concentrated under reduced pressure to remove the solvent. The resulting residue was separated by column chromatography (eluent: petroleum ether/ethyl acetate = 6/1 to 2/1, v/v) to obtain compound **BB-16-9.** MS-ESI *m/z:* 371.2 $[M+H]^+$. $^1H$ NMR (400 MHz, DMSO_$d_6$) δ: 8.48 (s, 1H), 8.28 (d, *J*=9.2 Hz, 1H), 7.95-7.87 (m, 2H), 7.75-7.60 (m, 2H), 4.50 (s, 2H), 4.14 (q, *J*=7.2 Hz, 2H), 1.50 (s, 9H), 1.16 (t, *J*=7.2 Hz, 3H).

Step **9:** Synthesis of compound **BB-16-10**

Compound **BB-16-9** (3.0 g, 8.10 mmol) was dissolved in tetrahydrofuran (25 mL) at room temperature under nitrogen atmosphere, then acrylamide (690.82 mg, 9.72 mmol) and potassium tert-butoxide (1.36 g, 12.15 mmol) were added thereto. The reaction mixture was stirred and reacted at room temperature for 1 hour. After the reaction was completed, the reaction mixture was poured into 1 N hydrochloric acid (30 mL), and extracted with ethyl acetate (45 mL × 3). The organic phases were combined, washed with saturated brine (30 mL × 2), dried over anhydrous sodium sulfate, and filtered. The filtrate was concentrated under reduced pressure to remove the solvent. The resulting residue was separated by column chromatography (eluent: petroleum ether/ethyl acetate = 5/1 to 1/1, v/v) to obtain compound **BB-16-10.** MS-ESI *m*/*z:* 396.2 $[M+H]^+$. $^1H$ NMR (400 MHz, DMSO_$d_6$) δ: 11.14 (s, 1H), 9.47 (s, 1H), 8.28 (d, *J*=9.6 Hz, 1H), 8.04 (br d, *J*=6.8 Hz, 1H), 7.93 (d, *J*=9.2 Hz, 1H), 7.75-7.59 (m, 2H), 5.07 (dd, *J*=4.6 Hz, 11.4 Hz, 1H), 2.93-2.77 (m, 1H), 2.71-2.56 (m, 2H), 2.43-2.31 (m, 1H), 1.50 (s, 9H).

Step **10:** Synthesis of hydrochloride of compound **BB-16**

Compound **BB-16-10** (1.3 g, 3.29 mmol) was dissolved in a solution of hydrochloric acid in ethyl acetate (4 M, 20 mL) at room temperature. The reaction mixture was stirred and reacted at room temperature for 12 hours. After the reaction was completed, the mixture was filtered. The filter cake was rinsed with ethyl acetate (30 mL), collected, and dried under vacuum to obtain the hydrochloride of compound **BB-16.** MS-ESI *m/z:* 296.1 $[M+H]^+$. $^1H$ NMR (400 MHz,

DMSO_$d_6$) δ: 11.14 (s, 1H), 8.34 (d, *J*=9.6 Hz, 1H), 7.96 (d, *J*=9.2 Hz, 1H), 7.85 (t, *J*=8.0 Hz, 1H), 7.61 (t, *J*=7.8 Hz, 1H), 7.33 (d, *J*=7.6 Hz, 1H), 5.03 (dd, *J*=4.6 Hz, 11.4 Hz, 1H), 2.90-2.76 (m, 1H), 2.70-2.55 (m, 2H), 2.42-2.30 (m, 1H).

**Reference example 17: Fragment BB-17**

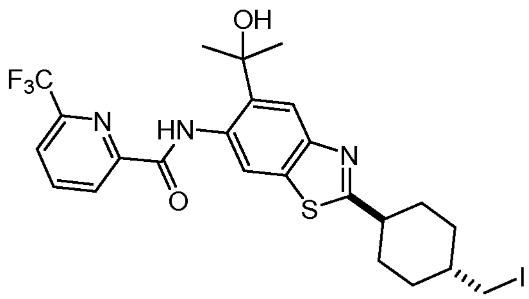

**BB-17**

Synthetic route:

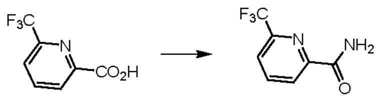

**BB-17-1** **BB-17-2**

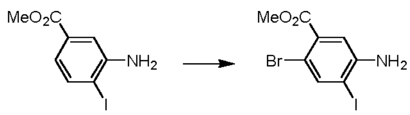

**BB-17-3** **BB-17-4**

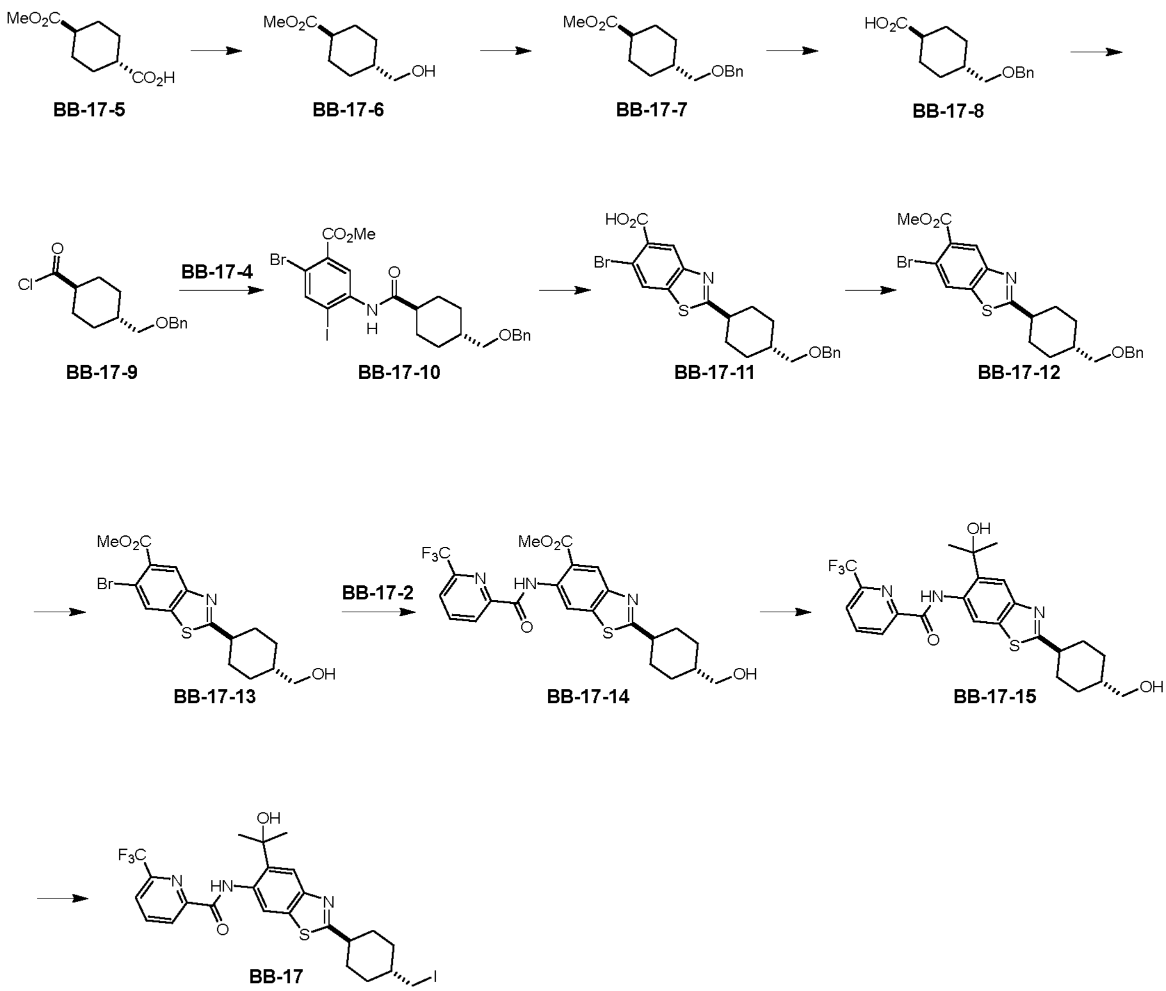


BB-17-5 BB-17-6 BB-17-7 BB-17-8

BB-17-9 BB-17-10 BB-17-11 BB-17-12

BB-17-13 BB-17-14 BB-17-15

BB-17

Step **1:** Synthesis of compound **BB-17-2**

N, N-dimethylformamide (422.74 μL) was added to a solution of compound **BB-17-1** (21 g 109.89 mmol) in dichloromethane (300 mL) at room temperature under nitrogen atmosphere. The reaction mixture was cooled to 0°C, and oxalyl chloride (27.89 g, 219.77 mmol, 19.24 mL) was added dropwise thereto. After the dropwise addition was completed, the mixture was slowly warmed to room temperature, and stirred and reacted for 1 hour. After the reaction was completed, the reaction mixture was directly concentrated under reduced pressure to obtain a residue, which was used in the next step directly. The crude product was dissolved in tetrahydrofuran (100 mL). The mixture was cooled to 0°C, ammonia water (169.08 mL, purity of 25%) was added thereto. The reaction mixture was stirred and reacted at room temperature for 1 hour. After the reaction was completed, the reaction mixture was concentrated under reduced pressure to remove tetrahydrofuran. The resulting residue was filtered. The filter cake was collected, and dried under vacuum to obtain compound **BB-17-2.** MS-ESI *m/z:* 191.0 $[M+H]^+$. $^1H$ NMR (400 MHz, DMSO_$d_6$) δ: 8.33-8.25 (m, 2H), 8.09 (dd, *J*=2.4, 6.4 Hz, 1H), 8.04 (s, 1H), 7.87 (s, 1H).

Step **2:** Synthesis of compound **BB-17-4**

Compound **BB-17-3** (25 g, 90.23 mmol) was dissolved in N, N-dimethylformamide (125 mL) at room temperature under nitrogen atmosphere. The reaction system was cooled to 0°C, then N-bromosuccinimide (17.67 g, 99.26 mmol) was added thereto in batches. The reaction mixture was stirred and reacted at 0°C for 2 hours. After the reaction was completed, the reaction mixture was poured into water (200 L), resulting in the precipitation of solid. The mixture was filtered. The filter cake was rinsed with water (100 mL×3), collected, and dried under vacuum to obtain compound **BB-17-4.** MS-ESI *m/z*: 355.8 $[M+H]^+$, 357.8 $[M+H+2]^+$. $^1H$ NMR (400 MHz, DMSO_$d_6$) δ: 7.84 (s, 1H), 7.13 (s, 1H), 5.66 (s, 2H), 3.81 (s, 3H).

Step **3:** Synthesis of compound **BB-17-6**

Compound **BB-17-5** (10 g, 53.70 mmol) was dissolved in dichloromethane (50 mL) at room temperature under nitrogen atmosphere. The mixture was cooled to -10°C, and a solution of borane in tetrahydrofuran (1 M, 59.07 mL) was added dropwise thereto. The reaction mixture was stirred and reacted at 0°C for 1 hour. After the reaction was completed, the mixture was added with methanol (10 mL) and stirred at room temperature for 30 minutes, then 2 M hydrochloric acid (200 mL) was added thereto. The mixture was stirred at room temperature for 30 minutes, extracted with ethyl acetate (200 mL × 2). The organic phases were combined, washed with saturated brine (100 mL × 2), dried over anhydrous sodium sulfate, and filtered. The filtrate was concentrated under reduced pressure to obtain a residue. The resulting residue was separated by column chromatography (eluent: petroleum ether/ethyl acetate = 1/0 to 7/3, v/v) to obtain compound **BB-17-6.** $^1$H NMR (400 MHz, $CDCl_3$) δ: 3.67 (s, 3H), 3.47 (d, *J*=6.4 Hz, 2H), 2.32-2.20 (m, 1H), 2.05-1.98 (m, 2H), 1.92-1.83 (m, 2H), 1.56-1.38 (m, 3H), 1.07-0.93 (m, 2H).

Step **4:** Synthesis of compound **BB-17-7**

Compound **BB-17-6** (8.2 g, 47.61 mmol) was dissolved in tetrahydrofuran (80 mL) at room temperature under nitrogen atmosphere, then potassium hydroxide (4.01 g, 71.42 mmol), benzyl bromide (10.59 g, 61.90 mmol, 7.35 mL), tetrabutylamine iodide (3.52 g, 9.52 mmol), and potassium iodide (1.58 g, 9.52 mmol) were added thereto. The reaction mixture was stirred and reacted at room temperature for 12 hours. After the reaction was completed, the mixture was filtered. The filter cake was rinsed with tetrahydrofuran (50 mL × 3). The filtrate was collected and concentrated under reduced pressure to obtain a residue. The resulting residue was separated by column chromatography (eluent: petroleum ether/ethyl acetate = 1/0 to 19/1, v/v) to obtain compound **BB-17-7.** $^1$H NMR (400 MHz, $CDCl_3$) δ: 7.39-7.27 (m, 5H), 4.50 (s, 2H), 3.29 (d, *J*=6.0 Hz, 2H), 2.32-2.19 (m, 1H), 2.08-1.97 (m, 2H), 1.96-1.86 (m, 2H), 1.72-1.58 (m, 1H), 1.53-1.38 (m, 2H), 1.09-0.95 (m, 2H).

Step **5:** Synthesis of compound **BB-17-8**

Compound **BB-17-7** (5.9, 22.49 mmol) was dissolved in tetrahydrofuran (50 mL) and methanol (10 mL) at room temperature, then an aqueous solution (10 mL) of lithium hydroxide monohydrate (4.72 g, 112.45 mmol) was added thereto. The reaction mixture was stirred and reacted at room temperature for 12 hours. After the reaction was completed, the reaction mixture was concentrated under reduced pressure to remove the solvent, added with water (200 mL), and extracted with petroleum ether (200 mL). The organic phase was discarded. The aqueous phase was collected, added with 6 N hydrochloric acid to adjust the pH 5 to 6, and extracted with dichloromethane (100 mL × 3). The organic phases were combined, washed with saturated brine (100 mL), dried over anhydrous sodium sulfate, and filtered. The filtrate was concentrated under reduced pressure to obtain compound **BB-17-8.** MS-ESI *m/z:* 266.2 $[M+18]^+$. $^1$H NMR (400 MHz, $CDCl_3$) δ: 11.27 (s, 1H), 7.40-7.27 (m, 5H), 4.51 (s, 2H), 3.30 (d, *J*=6.4 Hz, 2H), 2.35-2.23 (m, 1H), 2.11-2.00 (m, 2H), 1.98-1.87 (m, 2H), 1.75-1.56 (m, 1H), 1.53-1.39 (m, 2H), 1.11-0.91 (m, 2H).

Step **6:** Synthesis of compound **BB-17-9**

Compound **BB-17-8** (5 g, 20.14 mmol) was dissolved in dichloromethane (50 mL) at room temperature under nitrogen atmosphere. The mixture was cooled to 0°C, and N, N-dimethylformamide (147.18 mg, 2.01 mmol, 154.92 μL) and oxalyl chloride (3.83 g, 30.20 mmol, 2.64 mL) were sequentially added thereto. The reaction mixture was stirred and reacted at 0°C for 1 hour. After the reaction was completed, the reaction mixture was directly concentrated under reduced pressure to remove the solvent to obtain compound **BB-17-9,** which was used in the next step directly.

Step 7: Synthesis of compound **BB-17-10**

Compound **BB-17-4** (6.5 g, 18.26 mmol) was dissolved in dichloromethane (100 mL) at room temperature under nitrogen atmosphere. The mixture was cooled to 0°C, and then triethylamine (5.54 g, 54.78 mmol, 7.62 mL) and a solution of compound **BB-17-9** (5.36 g, 20.09 mmol) in dichloromethane (100 mL) were added thereto. The reaction mixture was returned to room temperature, and stirred and reacted for 12 hours. After the reaction was completed, the mixture was added with water (100 mL), and extracted with dichloromethane (100 mL × 2). The organic phases were combined, dried over anhydrous sodium sulfate, and filtered. The filtrate was concentrated under reduced pressure to obtain a residue. The residue was added with ethyl acetate (50 mL), stirred at room temperature for 30 minutes, and filtered. The solid was collected, and dried under vacuum to obtain compound **BB-17-10.** MS-ESI *m/z:* 585.8 $[M+H]^+$, 587.8 $[M+H+2]^+$. $^1$H NMR (400 MHz, $CDCl_3$) δ: 8.76 (s, 1H), 8.08 (s, 1H), 7.52 (s, 1H), 7.40-7.27 (m, 5H), 4.52 (s, 2H), 3.92 (s, 3H), 3.33 (d, *J*=6.4 Hz, 2H), 2.35-2.24 (m, 1H), 2.16-2.06 (m, 2H), 2.05-1.93 (m, 2H), 1.78-1.68 (m, 1H), 1.65-1.51

(m, 2H), 1.20-1.03 (m, 2H).

Step **8:** Synthesis of compound **BB-17-11**

Compound **BB-17-10** (7.5 g, 12.79 mmol) was dissolved in N, N-dimethylformamide (80 mL) at room temperature under nitrogen atmosphere, then cuprous iodide (487.29 mg, 2.56 mmol) and sodium sulfide nonahydrate (18.44 g, 76.76 mmol) were added thereto. The reaction mixture was heated to 80°C, and stirred and reacted for 6 hours. After the LCMS tracked the complete consumption of raw materials, the reaction mixture was cooled to room temperature, and trifluoroacetic acid (20.42 g, 179.10 mmol, 13.26 mL) was added thereto. The reaction mixture was stirred and reacted at room temperature for 16 hours. Additional trifluoroacetic acid (7 mL) was added thereto, and the reaction mixture was further stirred and reacted for 2 hours. After the reaction was completed, the reaction mixture was poured into water (300 mL), diluted with ethyl acetate (200 mL), and filtered. The filter cake was rinsed with ethyl acetate (100 mL × 2). The filtrate was collected and the phases were separated. The aqueous phase was extracted with ethyl acetate (200 mL × 2). The organic phases were combined, washed with 10% brine (300 mL × 2), dried over anhydrous sodium sulfate, and filtered. The filtrate was concentrated under reduced pressure to obtain compound **BB-17-11.** MS-ESI *m/z:* 459.9 $[M+H]^+$, 461.9 $[M+H+2]^+$.

Step **9:** Synthesis of compound **BB-17-12**

Compound **BB-17-11** (5.89 g, 12.79 mmol) was dissolved in N, N-dimethylformamide (70 mL) at room temperature, then potassium carbonate (3.54 g, 25.59 mmol) and methyl iodide (3.63 g, 25.59 mmol, 1.59 mL) were added thereto. The reaction mixture was stirred and reacted at room temperature for 12 hours. Additional potassium carbonate (3.54 g, 25.59 mmol) and methyl iodide (1 mL) were added thereto. The reaction mixture was further stirred and reacted for 1 hour. After the reaction was completed, the reaction mixture was poured into water (200 mL) and extracted with ethyl acetate (200 mL × 3). The organic phases were combined, washed sequentially with 10% brine (200 mL × 2) and saturated brine (100 mL), dried over anhydrous sodium sulfate, and filtered. The filtrate was concentrated under reduced pressure to obtain a residue. The resulting residue was separated by column chromatography (eluent: petroleum ether/ethyl acetate = 1/0 to 4/1, v/v) to obtain compound **BB-17-12.** MS-ESI *m/z:* 473.9 $[M+H]^+$, 475.9 $[M+H+2]^+$. $^1$H NMR (400 MHz, $CDCl_3$) δ: 8.41 (s, 1H), 8.14 (s, 1H), 7.41-7.27 (m, 5H), 4.53 (s, 2H), 3.97 (s, 3H), 3.36 (d, *J*=6.4 Hz, 2H), 3.12-3.00 (m, 1H), 2.36-2.23 (m, 2H), 2.06-1.99 (m, 2H), 1.84-1.74 (m, 1H), 1.73-1.61 (m, 2H), 1.26-1.13 (m, 2H).

Step **10:** Synthesis of compound **BB-17-13**

Compound **BB-17-12** (4.08 g, 8.60 mmol) was dissolved in dichloromethane (20 mL) at room temperature under nitrogen atmosphere, then a solution of boron trichloride (1 M, 100 mL) in dichloromethane was added thereto. The reaction mixture was stirred and reacted at room temperature for 1 hour. After the reaction was completed, the reaction mixture was poured into a saturated aqueous solution of sodium bicarbonate (100 mL), and extracted with dichloromethane (50 mL × 3). The organic phases were combined, washed with saturated brine (100 mL), dried over anhydrous sodium sulfate, and filtered. The filtrate was concentrated under reduced pressure to obtain compound **BB-17-13.** MS-ESI *m/z:* 383.9 $[M+H]^+$, 386.0 $[M+H+2]^+$. $^1$H NMR (400 MHz, $CDCl_3$) δ: 8.40 (s, 1H), 8.14 (s, 1H), 3.97 (s, 3H), 3.54 (d, *J*=6.0 Hz, 2H), 3.10-3.00 (m, 1H), 2.36-2.23 (m, 2H), 2.07-1.95 (m, 2H), 1.75-1.55 (m, 4H), 1.28-1.13 (m, 2H).

Step **11:** Synthesis of compound **BB-17-14**

Compound **BB-17-13** (3.4 g, 8.85 mmol) was dissolved in dioxane (70 mL) at room temperature under nitrogen atmosphere, and then compound **BB-17-2** (1.85 g, 9.37 mmol), 4,5-bisdiphenylphosphino-9,9-dimethylxanthene (1.02 g, 1.77 mmol), tris(dibenzylideneacetone)dipalladium (810.18 mg, 884.75 μmol), and cesium carbonate (8.65 g, 26.54 mmol) were added thereto. The reaction mixture was heated to 80°C, and stirred and reacted for 12 hours. After the reaction was completed, the reaction mixture was cooled to room temperature, and filtered. The filter cake was rinsed with tetrahydrofuran (100 mL × 3). The filtrate was collected, and concentrated under reduced pressure to obtain a residue. The residue was separated by column chromatography (eluent: petroleum ether/ethyl acetate = 1/0 to 11/9, v/v) to obtain compound **BB-17-14.** MS-ESI *m/z:* 494.0 $[M+H]^+$. $^1$H NMR (400 MHz, DMSO_$d_6$) δ: 12.79 (s, 1H), 9.41 (s, 1H), 8.50 (s, 1H), 8.45 (d, *J*=7.6 Hz 1H), 8.39 (t, *J*=7.8 Hz, 1H), 8.21 (d, *J*=7.2 Hz, 1H), 4.45 (t, *J*=5.4 Hz, 1H), 3.97 (s, 3H), 3.27 (t, *J*=5.8 Hz, 2H), 3.11-3.00 (m, 1H), 2.23-2.12 (m, 2H), 1.93-1.82 (m, 2H), 1.62-1.50 (m, 2H), 1.49-1.36 (m, 1H), 1.17-1.03 (m, 2H).

Step **12:** Synthesis of compound **BB-17-15**

Compound **BB-17-14** (2 g, 4.05 mmol) was dissolved in tetrahydrofuran (40 mL) at room temperature under nitrogen atmosphere. The mixture was cooled to 0°C, and then a solution of methylmagnesium bromide (3 M, 8.11 mL) in tetrahydrofuran was added dropwise thereto. The reaction mixture was stirred and reacted at 0°C for 2 hours. An additional solution of methylmagnesium bromide (3 M, 1.35 mL) in tetrahydrofuran was added. The mixture was further stirred and reacted for 1 hour. After the reaction was completed, the reaction mixture was poured into saturated ammonium chloride (100 mL) and extracted with ethyl acetate (50 mL × 3). The organic phases were combined, dried over anhydrous sodium sulfate, and filtered. The filtrate was concentrated under reduced pressure to obtain a residue. The residue was separated by column chromatography (eluent: petroleum ether/ethyl acetate = 1/0 to 1/1, v/v) to obtain compound **BB-17-15.** MS-ESI *m/z*: 494.0 $[M+H]^+$. $^1H$ NMR (400 MHz, DMSO_$d_6$) δ: 12.55 (s, 1H), 9.06 (s, 1H), 8.47 (d, *J*=8.0 Hz, 1H), 8.38 (t, *J*=7.8 Hz, 1H), 8.18 (dd, *J*=0.8, 7.6 Hz, 1H), 7.89 (s, 1H), 6.07 (s, 1H), 4.45 (t, *J*=5.2 Hz, 1H), 3.27 (t, *J*=5.8 Hz, 2H), 3.08-2.98 (m, 1H), 2.20-2.13 (m, 2H), 1.90-1.83 (m, 2H), 1.63 (s, 6H), 1.59-1.50 (m, 2H), 1.49-1.38 (m, 1H), 1.15-1.06 (m, 2H).

Step **13:** Synthesis of compound **BB-17**

Compound **BB-17-15** (1.63 g, 3.30 mmol) was dissolved in dichloromethane (40 mL) at room temperature under nitrogen atmosphere, then triphenylphosphine (1.04 g, 3.96 mmol) and imidazole (337.25 mg, 4.95 mmol) were added thereto. The mixture was cooled to 0 °C, and iodine (1.09 g, 4.29 mmol) was added thereto. The reaction mixture was stirred and reacted at room temperature for 12 hours. After the reaction was completed, the reaction mixture was poured into saturated sodium sulfite (200 mL), and extracted with dichloromethane (100 mL ×3). The organic phases were combined, dried over anhydrous sodium sulfate, and filtered. The filtrate was concentrated under reduced pressure to obtain a residue. The residue was first separated by column chromatography (eluent: petroleum ether/ethyl acetate = 1/0 to 7/3, v/v), and further separated twice by preparative high performance liquid chromatography (chromatographic column: Phenomenex luna C18 (250 * 70 mm, 15 μm); mobile phase: water (0.2% formic acid)-acetonitrile; B%: 60%-90%, 30 minutes) and by (chromatogram: Phenomenex Luna C18 75 * 30 mm*3 μm; mobile phase: water (0.2% formic acid)-acetonitrile; B%: 45%-85%, 8 minutes) to obtain compound **BB-17.** MS-ESI *m/z:* 603.9 $[M+H]^+$. $^1H$ NMR (400 MHz, DMSO_$d_6$) δ: 12.55 (s, 1H), 9.07 (s, 1H), 8.47 (d, *J*=7.6 Hz, 1H), 8.38 (t, *J*=8.0 Hz, 1H), 8.18 (d, *J*=8.0 Hz, 1H), 7.89 (s, 1H), 6.07 (s, 1H), 3.27 (d, *J*=6.4 Hz, 2H), 3.08-2.97 (m, 1H), 2.22-2.13 (m, 2H), 2.02-1.92 (m, 2H), 1.71-1.56 (m, 8H), 1.55-1.42 (m, 1H), 1.28-1.10 (m, 2H).

**Reference example 18: Fragment BB-18**

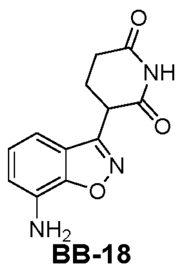

**BB-18**

Synthetic route:

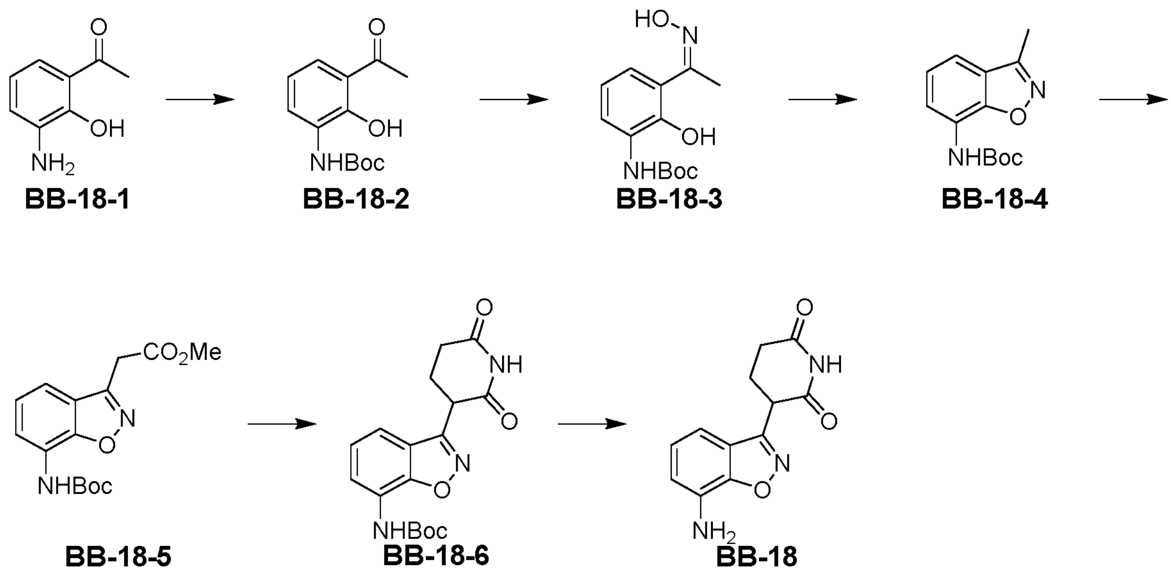

BB-18-1 BB-18-2 BB-18-3 BB-18-4

BB-18-5 BB-18-6 BB-18

Step **1:** Synthesis of compound **BB-18-2**

Compound **BB-18-1** (200 g, 1.32 mol) was dissolved in ethanol (1.5 L) at room temperature under nitrogen atmosphere, then di-tert-butyl dicarbonate (346.51 g, 1.59 mol) was added thereto in batches. The reaction mixture was heated to 60°C, and stirred and reacted for 16 hours. After the reaction was completed, the reaction mixture was cooled to room temperature, and concentrated under reduced pressure to remove the solvent. The residue was added with isopropanol (500 mL), cooled to 0°C, stirred for 1 hour, and filtered. The filter cake was rinsed with isopropanol (200 mL). The filtrate was collected and concentrated under reduced pressure to obtain compound **BB-18-2.** MS-ESI *m/z:* 196.1 $[M+H-56]^+$.

Step **2:** Synthesis of compound **BB-18-3**

Compound **BB-18-2** (200 g, 795.93 mmol), potassium acetate (97.94 g, 1.19 mol), and hydroxylamine hydrochloride (60.84 g, 875.53 mmol) were dissolved in methanol (1.2 L) at room temperature under nitrogen atmosphere. The reaction mixture was stirred at room temperature for 16 hours, then heated to 50°C and reacted for 1 hour. Additional hydroxylamine hydrochloride (11.06 g, 159.19 mmol) was added thereto, and the mixture was further reacted for 1 hour. After the reaction was completed, the reaction mixture was cooled to room temperature, added with water (1.5 L) and methyl tert-butyl ether (1.5 L). The mixture was stirred for 5 minutes and the phases were separated. The aqueous phase was extracted with methyl tert-butyl ether (500 mL × 2). The organic phases were combined, washed with 10% brine (500 mL× 2), dried over anhydrous sodium sulfate, and filtered. The filtrate was concentrated under reduced pressure to remove the solvent to obtain compound **BB-18-3.** MS-ESI *m/z*: 211.1 $[M+H-56]^+$. $^1H$ NMR (400 MHz, DMSO_$d_6$) δ: 12.27 (s, 1H), 11.67 (s, 1H), 7.82 (s, 1H), 7.69 (d, *J*=7.2 Hz, 1H), 7.23 (d, *J*=8.0 Hz, 1H), 6.86 (t, *J*=8.0 Hz, 1H), 2.27 (s, 3H), 1.46 (s, 9H).

Step **3:** Synthesis of compound **BB-18-4**

Compound **BB-18-3** (100 g, 375.53 mmol) and triethylamine (49.40 g, 488.19 mmol) were dissolved in tetrahydrofuran (1 L) at room temperature under nitrogen atmosphere, and N, N'-carbonyldiimidazole (66.98 g, 413.08 mmol) was slowly added thereto. The reaction mixture was heated to 70°C, and stirred and reacted for 1 hour. After the reaction was completed, the reaction mixture was cooled to room temperature, added with water (1 L), and extracted with a mixed solvent of methyl tert-butyl ether and petroleum ether (1/1, v/v, 500 mL × 2). The organic phases were washed with 10% brine (500 mL × 2), dried over anhydrous sodium sulfate, and filtered. The filtrate was concentrated under reduced pressure to remove the solvent to obtain compound **BB-18-4.** MS-ESI *m/z:* 249.1 $[M+H]^+$. $^1H$ NMR (400 MHz, $CDCl_3$) δ: 8.16 (s, 1H), 7.30 (s, 1H), 7.00 (s, 1H), 2.59 (s, 3H), 1.57 (s, 9H).

Step **4:** Synthesis of compound **BB-18-5**

A solution of lithium diisopropylamide (2 M, 741.11 mL) in tetrahydrofuran was cooled to -68°C at room temperature under nitrogen atmosphere. Dimethyl carbonate (36.72 g, 407.61 mmol, 34.31 mL) and a solution of compound **BB-18-4** (92 g, 370.55 mmol) in tetrahydrofuran (920 mL) were added dropwise thereto, the duration of which is about 30 minutes. The mixture was stirred and reacted for 30 minutes, with temperature controlled at -50°C to -68°C. After the reaction was completed, the reaction mixture was slowly poured into saturated ammonium chloride (1.5 L), and extracted with ethyl acetate (500 mL × 3). The organic phase was washed with saturated brine (500 mL), dried over anhydrous sodium sulfate, and filtered. The filtrate was concentrated under reduced pressure to remove the solvent to obtain compound **BB-18-5.** MS-ESI *m/z:* 307.1 $[M+H]^+$.

Step **5:** Synthesis of compound **BB-18-6**

Compound **BB-18-5** (10 g, 32.65 mmol) and acrylamide (2.78 g, 39.18 mmol) were dissolved in tetrahydrofuran (100 mL) at room temperature under nitrogen atmosphere. The mixture was cooled to 0 to 5°C, and a solution of potassium tert-butoxide in tetrahydrofuran (1 M, 48.97 mL) was added dropwise thereto. After the dropwise addition was completed, the mixture was stirred at the same temperature for 2 hours. After the reaction was completed, the reaction mixture was poured into 1 M hydrochloric acid (150 mL), and extracted with ethyl acetate (150 mL × 2). The organic phases were combined, washed with saturated brine (150 mL), dried over anhydrous sodium sulfate, and filtered. The filtrate was concentrated under reduced pressure to obtain a crude product. The crude product was added with methyl tert-butyl ether (150 mL). The mixture was stirred at room temperature for 30 minutes, and filtered. The filter cake was rinsed with methyl tert-butyl ether (50 mL), collected, and dried under vacuum to obtain compound **BB-18-6.** $^1H$ NMR (400 MHz, DMSO_$d_6$) δ: 11.10 (s, 1H), 9.55 (s, 1H), 7.69 (d, *J*=7.6 Hz, 1H), 7.54 (d, *J*=7.6 Hz, 1H), 7.30 (t, *J*=8.0 Hz, 1H), 4.60 (dd, *J*=4.8, 12.0 Hz, 1H), 2.84-2.72 (m, 1H), 2.66-2.57 (m, 1H), 2.57-2.43 (m, 1H), 2.25-2.15 (m, 1H), 1.49 (s, 9H).

Step **6:** Synthesis of hydrochloride of compound **BB-18**

Compound **BB-18-6** (9.4 g, 27.22 mmol) was dissolved in ethyl acetate (50 mL) at room temperature, then a solution of hydrochloric acid in ethyl acetate (4 M, 150 mL) was added thereto. The reaction mixture was stirred and reacted at room temperature for 12 hours. After the reaction was completed, the mixture was filtered. The filter cake was rinsed with ethyl acetate (20 mL), and collected to obtain the hydrochloride of compound **BB-18.** $^1H$ NMR (400 MHz, DMSO_$d_6$) δ: 11.09 (s, 1H), 8.32 (s, 2H), 7.31 (d, *J*=7.6 Hz, 1H), 7.26-7.16 (m, 2H), 4.59 (dd, *J*=4.8, 12.0 Hz, 1H), 2.83-2.71 (m, 1H), 2.65-2.56 (m, 1H), 2.55-2.42 (m, 1H), 2.25-2.15 (m, 1H).

**Example 1**

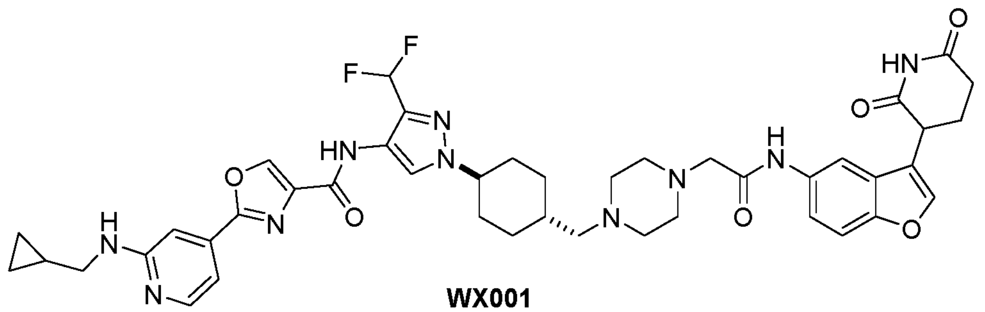

**WX001**

Synthetic route:

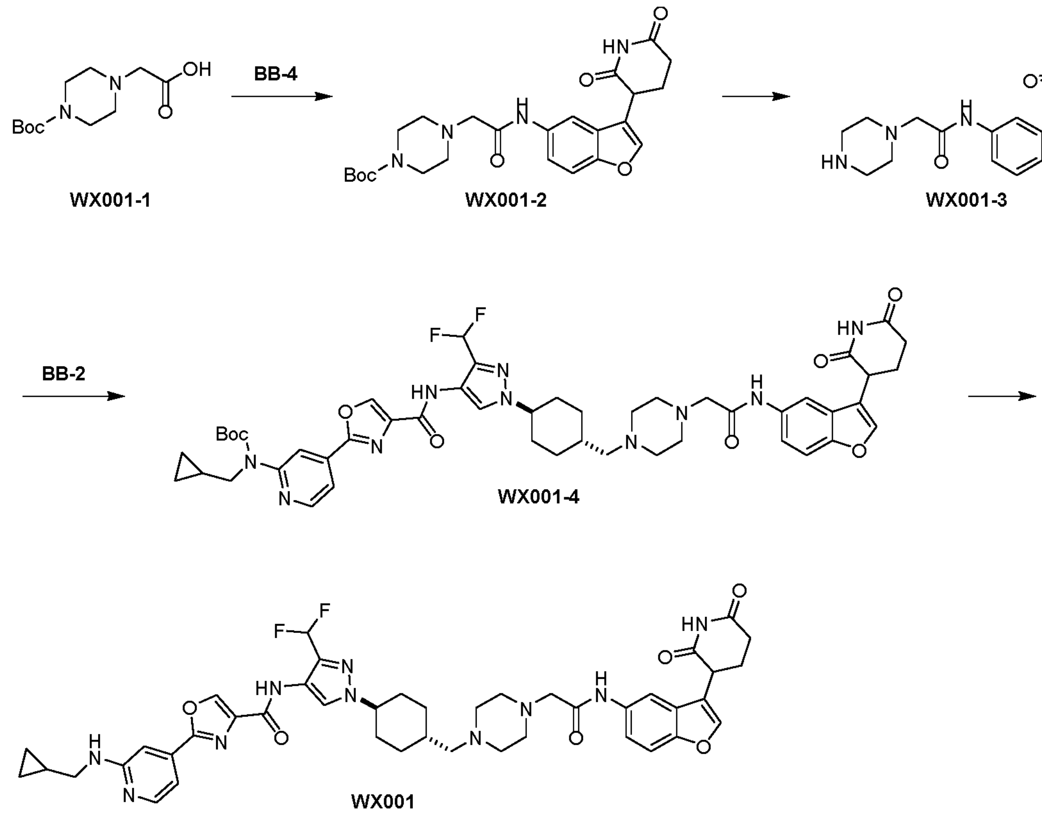


WX001-1 WX001-2 WX001-3

WX001-4

WX001

Step **1:** Synthesis of compound **WX001-2**

Compound **WX001-1** (612.43 mg, 1.85 mmol, purity of 84.92%) and triethylamine (720.96 mg, 7.12 mmol) were dissolved in N, N-dimethylformamide (5 mL) at room temperature, and 2-(7-azabenzotriazol)-N, N, N', N'-tetramethyluronium hexafluorophosphate (880.46 mg, 2.32 mmol) was added thereto. The reaction mixture was stirred and reacted at room temperature for 1 hour, and the hydrochloride of compound **BB-4** (0.5 g, 1.78 mmol) was added thereto. The reaction mixture was further stirred and reacted for 4 hours. After the reaction was completed, methyl tert-butyl ether (20 mL) was added thereto. The mixture was stirred for 5 minutes, and filtered. The filter cake was rinsed with methyl tert-butyl ether (5mL ) and discard. The filtrate was collected. The filtrate was added with water (50 mL) and dichloromethane (100 mL), then the phases were separated. The organic phases were combined, dried over anhydrous sodium sulfate, and filtered. The filtrate was concentrated under reduced pressure to remove the solvent to obtain compound **WX001-2,** which was used in the next step directly. MS-ESI *m/z:* 471.3 $[M+H]^+$.

Step **2:** Synthesis of hydrochloride of compound **WX001-3**

Compound **WX001-2** (0.8 g, 1.70 mmol) was dissolved in dichloromethane (5 mL) at room temperature, then hydrochloric acid/ethyl acetate solution (4 M, 20 mL) was added thereto. The reaction mixture was stirred and reacted at room temperature for 2 hours. After the reaction was completed, the reacted mixture was filtered. The filter cake was rinsed with ethyl acetate (20 mL), and collected to obtain the hydrochloride of compound **WX001-3.** MS-ESI *m/z*: 371.1 $[M+H]^+$. $^1H$ NMR (400 MHz, $D_2O$) $\delta$: 7.78 (s, 1H), 7.64 (d, *J*=2.0 Hz, 1H), 7.57 (d, *J*=8.8 Hz, 1H), 7.30 (dd, *J*=2.0, 8.8 Hz, 1H), 4.21 (dd, *J*=5.2, 12.4 Hz, 1H), 4.06 (s, 2H), 3.62-3.56 (m, 4H), 3.55-3.50 (m, 4H), 2.90-2.75 (m, 2H), 2.51-2.38 (m, 1H), 2.33-2.24 (m, 1H).

Step **3:** Synthesis of compound **WX001-4**

Compound **BB-2** (0.1 g, 171.05 $\mu$mol) and the hydrochloride of compound **WX001-3** (69.60 mg, 171.05 $\mu$mol) were dissolved in tetrahydrofuran (4 mL) and N, N-dimethylformamide (0.8 mL) at room temperature under nitrogen atmosphere, and potassium acetate (50.36 mg, 513.16 $\mu$mol) and acetic acid (5.14 mg, 85.53 $\mu$mol) were added thereto. The reaction mixture was stirred and reacted at room temperature for 2 hours. Sodium triacetoxyborohydride (145.01 mg, 684.21 $\mu$mol) was added thereto. The mixture was further stirred and reacted for 10 hours. After the reaction was completed, the reaction mixture was added with a saturated solution of ammonium chloride (10 mL), and extracted with

ethyl acetate (20 mL × 3). The organic phases were combined, washed with 10% brine (30 mL × 2), dried over anhydrous sodium sulfate, and filtered. The filtrate was concentrated under reduced pressure to remove the solvent to obtain compound **WX001-4.** MS-ESI *m/z:* 939.5 $[M+H]^+$.

Step **4:** Synthesis of hydrochloride of compound **WX001**

Compound **WX001-4** (160 mg, 170.39 μmol) was dissolved in dichloromethane (5 mL) and trifluoroacetic acid (5 mL) at room temperature under nitrogen atmosphere. The reaction mixture was stirred and reacted at room temperature for 2 hours. After the reaction was completed, the reaction mixture was concentrated under reduced pressure to remove the solvent. The resulting residue was separated by preparative high performance liquid chromatography (chromatographic column: Phenomenex Luna C18 80 × 40 mm × 3 μm; mobile phase: water (0.04% hydrochloric acid)-acetonitrile; acetonitrile%: 13% to 33%, 7 minutes) to obtain the hydrochloride of the target compound **WX001.** MS-ESI *m/z:* 839.5 $[M+H]^+$. $^1H$ NMR (400 MHz, DMSO_$d_6$) δ: 10.94 (s, 1H), 9.98 (s, 1H), 9.14 (s, 1H), 8.27-8.15 (m, 1H), 8.06 (d, *J*=5.2 Hz, 1H), 7.93 (s, 2H), 7.69 (s, 1H), 7.57 (d, *J*=8.8 Hz, 1H), 7.48 (d, *J*=8.8 Hz, 1H), 7.37-7.03 (m, 2H), 4.37-4.17 (m, 3H), 4.13 (dd, *J*=4.4, 12.0 Hz, 2H), 3.41-3.32 (m, 5H), 3.15-3.03 (m, 2H), 2.86-2.73 (m, 2H), 2.69-2.55 (m, 2H), 2.35-2.17 (m, 2H), 2.16-1.99 (m, 5H), 1.98-1.88 (m, 1H), 1.87-1.67 (m, 3H), 1.55 (d, *J*=7.2 Hz, 1H), 1.27-1.07 (m, 3H), 0.55 (d, *J*=7.2 Hz, 2H), 0.34 (d, *J*=4.0 Hz, 2H).

**Example 2**

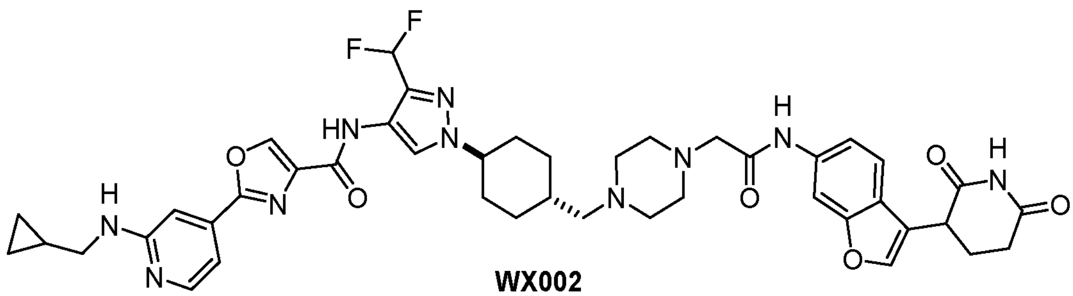

**WX002**

Synthetic route:

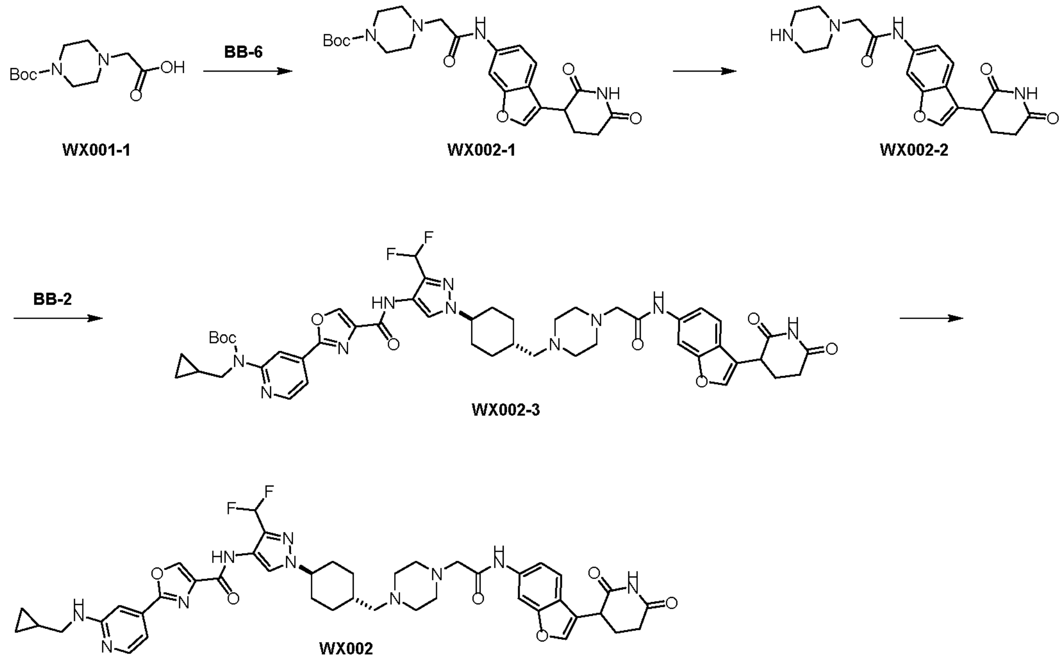


WX001-1 WX002-1 WX002-2

WX002-3

WX002

Step **1:** Synthesis of compound **WX002-1**

Compound **WX001-1** (0.5 g, 1.78 mmol) and triethylamine (632.58 mg, 6.25 mmol) were dissolved in N, N-dimethylformamide (10 mL) at room temperature, and 2-(7-azabenzotriazol)-N, N, N', N'-tetramethyluronium hexafluorophosphate (812.61 mg, 2.14 mmol) was added thereto. The reaction mixture was stirred and reacted for 1 hour. The hydrochloride of compound **BB-6** (522.08 mg, 1.33 mmol) was added thereto. The reaction mixture was further stirred and reacted for 19 hours. After the reaction was completed, the reaction mixture was filtered. The filter cake was washed with N, N-dimethylformamide (5 mL), again washed with tert-butyl methyl ether (20 mL) again, collected, and dried under vacuum to obtain compound **WX002-1.** MS-ESI *m/z:* 471.3 $[M+H]^+$. $^1H$ NMR (400 MHz, DMSO_$d_6$) δ: 10.88 (s, 1H), 9.93 (s, 1H), 8.05 (d, *J*=1.2 Hz, 1H), 7.83 (s, 1H), 7.49 (d, *J*=8.4 Hz, 1H), 7.38 (dd, *J*=1.2, 8.4 Hz, 1H), 4.10 (dd, *J*=4.8, 12.0 Hz, 1H), 3.43-3.36 (m, 4H), 3.35-3.33 (m, 2H), 3.22-3.16 (m, 2H), 2.79-2.64 (m, 2H), 2.63-2.52 (m, 2H), 2.37-2.24 (m, 1H), 2.16-2.06 (m, 1H), 1.40 (s, 9H).

Step 2: Synthesis of hydrochloride of compound **WX002-2**

Compound **WX002-1** (0.8 g, 1.70 mmol) was dissolved in ethyl acetate (10 mL) at room temperature, then hydrochloric acid/ethyl acetate (4 M, 40 mL) was added thereto. The reaction mixture was stirred and reacted at room temperature for 15 hours. After the reaction was completed, the reaction mixture was filtered. The filter cake was washed with ethyl acetate (5 mL), collected, and dried under vacuum to obtain the hydrochloride of compound **WX002-2.** MS-ESI *m/z:* 371.3 $[M+H]^+$. $^1H$ NMR (400 MHz, $D_2O$) δ: 7.75 (d, *J*=1.6 Hz, 1H), 7.66 (s, 1H), 7.43 (d, *J*=8.4 Hz, 1H), 7.19 (dd, *J*=1.2, 8.4 Hz, 1H), 4.31 (s, 2H), 4.10-4.03 (m, 1H), 3.80-3.72 (m, 4H), 3.71-3.63 (m, 4H), 2.78-2.61 (m, 2H), 2.33-2.20 (m, 1H), 2.18-2.09 (m, 1H).

Step **3:** Synthesis of compound **WX002-3**

Compound **BB-2** (0.1 g, 171.05 μmol) and the hydrochloride of compound **WX002-2** (69.60 mg, 171.05 μmol) were dissolved in tetrahydrofuran (4 mL) and N, N-dimethylformamide (0.8 mL) at room temperature under nitrogen atmosphere. Potassium acetate (50.36 mg, 513.16 μmol) and acetic acid (5.14 mg, 85.53 μmol, 4.89 μL) were added thereto. The reaction mixture was stirred and reacted at room temperature for 2 hours. Sodium triacetoxyborohydride (145.01 mg, 684.21 μmol) was added thereto. The reaction mixture was further stirred and reacted for 10 hours. After the reaction was completed, the reaction mixture was added with a saturated solution of ammonium chloride (10 mL), and extracted with ethyl acetate (20 mL × 3). The organic phases were combined, washed with 10% brine (30 mL × 2), dried over anhydrous sodium sulfate, and filtered. The filtrate was concentrated under reduced pressure to remove the solvent to obtain compound **WX002-3.** MS-ESI *m/z:* 939.5 $[M+H]^+$.

Step **4:** Synthesis of hydrochloride of compound **WX002**

Compound **WX002-3** (160 mg, 170.39 μmol) was dissolved in dichloromethane (5 mL) and trifluoroacetic acid (5 mL) at room temperature under nitrogen atmosphere. The reaction mixture was stirred and reacted at room temperature for 2 hours. After the reaction was completed, the reaction mixture was concentrated under reduced pressure to remove the solvent. The resulting residue was separated by preparative high performance liquid chromatography (chromatographic column: Phenomenex Luna C18 80 × 40 mm × 3 μm; mobile phase: water (0.04% hydrochloric acid) -acetonitrile; acetonitrile%: 13% to 33%, 7 minutes) to obtain the hydrochloride of the target compound **WX002.** MS-ESI *m/z:* 839.5 $[M+H]^+$. $^1H$ NMR (400 MHz, DMSO_$d_6$) δ: 10.91 (s, 1H), 9.94 (s, 1H), 9.13 (s, 1H), 8.19 (s, 1H), 8.10-8.04 (m, 2H), 7.88 (s, 1H), 7.66 (s, 1H), 7.55 (d, *J*=8.6 Hz, 1H), 7.38 (dd, *J*=1.4, 8.4 Hz, 1H), 7.34-7.04 (m, 2H), 4.31-4.22 (m, 1H), 4.12 (dd, *J*=4.6, 11.8 H, 2H), 3.50-3.25 (m, 7H), 3.08 (s, 2H), 2.80-2.65 (m, 2H), 2.62-2.53 (m, 2H), 2.38-2.25 (m, 2H), 2.15-2.01 (m, 5H), 1.98-1.88 (m, 1H), 1.87-1.77 (m, 2H), 1.54 (d, *J*=6.8 Hz, 1H), 1.27-1.10 (m, 3H), 0.60-0.53 (m, 2H), 0.36-0.30 (m, 2H).

**Example 4**

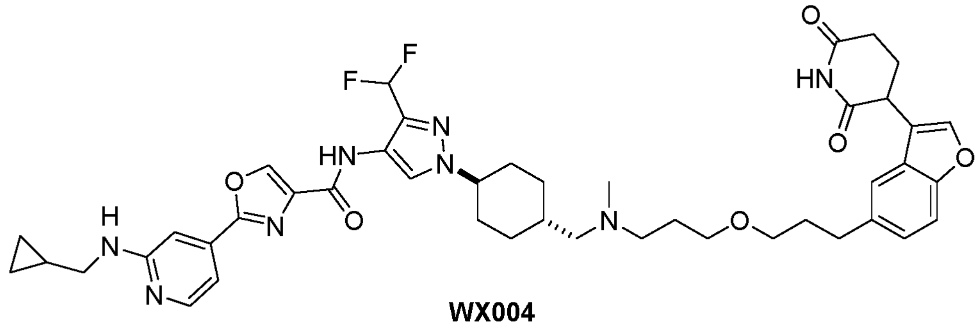

**WX004**

Synthetic route:

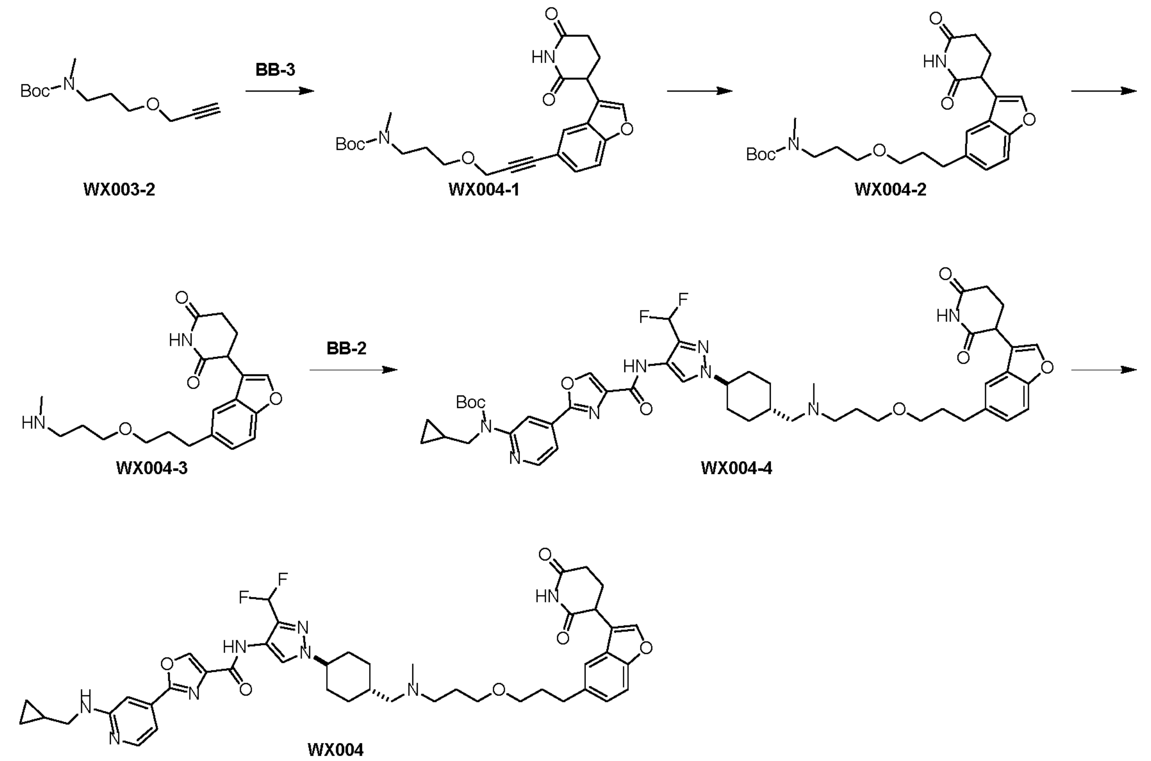


WX003-2 WX004-1 WX004-2 WX004-3 WX004-4 WX004

Step **1:** Synthesis of compound **WX004-1**

Compound **WX003-2** (553.26 mg, 2.43 mmol) was added to N, N-dimethylformamide (10 mL) at room temperature under nitrogen atmosphere. Compound **BB-3** (0.5 g, 1.62 mmol), cesium carbonate (2.11 g, 6.49 mmol), cuprous iodide (61.81 mg, 324.54 μmol), and bis(triphenylphosphine)palladium(II) chloride (227.80 mg, 324.54 μmol) were sequentially added thereto. The reaction mixture was heated to 80 °C, and stirred and reacted for 5 hours. After the reaction was completed, the reaction mixture was poured into 1 mol/L hydrochloric acid (12 mL), added with water (15 mL), and extracted with ethyl acetate (15 mL × 3). The organic phases were combined, washed with saturated brine (25 mL × 2) , dried over anhydrous sodium sulfate, and filtered. The filtrate was concentrated under reduced pressure to remove the solvent. The resulting residue was separated by column chromatography (eluent: petroleum ether/ethyl acetate = 5/1 to 1/1, v/v) to obtain compound **WX004-1.** MS-ESI *m/z:* 399.2 $[M-55]^+$. $^1H$ NMR (400 MHz, DMSO_$d_6$) δ: 10.90 (s, 1H), 7.97 (s, 1H), 7.74 (s, 1H), 7.59 (d, *J*=8.4 Hz, 1H), 7.38 (dd, *J*=1.2, 8.4 Hz, 1H), 4.37 (s, 2H), 4.16 (dd, *J*=4.8, 12.0 Hz, 1H), 3.51 (t, *J*=6.4 Hz, 2H), 3.22 (t, *J*=7.0 Hz, 2H), 2.77 (s, 3H), 2.74-2.66 (m, 1H), 2.61-2.54 (m, 1H), 2.41-2.28 (m, 1H), 2.15-2.05 (m, 1H), 1.78-1.69 (m, 2H), 1.38 (s, 9H).

Step **2:** Synthesis of compound **WX004-2**

Compound **WX004-1** (0.6 g, 1.32 mmol) was dissolved in tetrahydrofuran (20 mL) at room temperature, then wet palladium on carbon (0.1 g, 1.32 mmol, purity of 10%) and palladium hydroxide/carbon (0.1 g, 1.32 mmol, purity of 10%) were added thereto. The reaction mixture was stirred and reacted at room temperature under hydrogen (15 psi) atmosphere for 12 hours. After the reaction was completed, the reaction mixture was filtered. The filter cake was rinsed with ethyl acetate (30 mL). The filtrate was collected and concentrated under reduced pressure to remove the solvent to obtain compound **WX004-2.** MS-ESI *m/z:* 403.2 $[M-55]^+$.

Step **3:** Synthesis of hydrochloride of compound **WX004-3**

Compound **WX004-2** (0.1 g, 218.08 μmol ) was dissolved in hydrochloric acid/ethyl acetate (4 mol/L, 4 mL) at room temperature. The reaction mixture was stirred and reacted at room temperature for 1 hour. After the reaction was completed, the reaction mixture was filtered. The filter cake was rinsed with ethyl acetate (15 mL), and collected to obtain the hydrochloride of compound **WX004-3.** MS-ESI *m/z*: 359.2 $[M+H]^+$. $^1H$ NMR (400 MHz, DMSO_$d_6$) δ: 10.89 (s, 1H), 8.73 (s, 1H), 7.86 (s, 1H), 7.47 (d, *J*=8.4 Hz, 1H), 7.39 (s, 1H), 7.15 (dd, *J*=1.6, 8.4 Hz, 1H), 4.12 (dd, *J*=4.8, 12.0 Hz, 1H), 3.43 (t, *J*=6.0 Hz, 2H), 3.38 (t, *J*=6.6 Hz, 2H), 3.34 (s, 3H), 2.95-2.88 (m, 2H), 2.81-2.74 (m, 1H), 2.73-2.67 (m, 2H),

2.62-2.54 (m, 1H), 2.38-2.27 (m, 1H), 2.14-2.05 (m, 1H), 1.88-1.78 (m, 4H).

Step **4:** Synthesis of compound **WX004-4**

Compound **BB-2** (0.1 g, 171.05 μmol) and the hydrochloride of compound **WX004-3** (67.55 mg, 171.05 μmol) were dissolved in tetrahydrofuran (4 mL) and N, N-dimethylformamide (0.8 mL) at room temperature under nitrogen atmosphere, and potassium acetate (50.36 mg, 513.16 μmol) and acetic acid (5.14 mg, 85.53 μmol) were added thereto. The reaction mixture was stirred and reacted at room temperature for 2 hours. Sodium triacetoxyborohydride (145.01 mg, 684.21 μmol) was added thereto. The mixture was further stirred and reacted for 10 hours. After the reaction was completed, the reaction mixture was added with saturated ammonium chloride solution (10 mL), and extracted with ethyl acetate (3 × 30 mL). The organic phases were combined, washed with saturated brine (2 × 30 mL), dried over anhydrous sodium sulfate, and filtered. The filtrate was concentrated under reduced pressure to remove the solvent to obtain compound **WX004-4.** MS-ESI *m*/*z:* 927.6 $[M+H]^+$.

Step **5:** Synthesis of hydrochloride of compound **WX004**

Compound **WX004-4** (150 mg, 161.80 μmol) was dissolved in dichloromethane (5 mL) and trifluoroacetic acid (5 mL) at room temperature under nitrogen atmosphere. The reaction mixture was stirred and reacted at room temperature for 2 hours. After the reaction was completed, the reaction mixture was concentrated under reduced pressure to remove the solvent. The resulting residue was separated by preparative high performance liquid chromatography (chromatographic column: Phenomenex Luna C18 80 × 40 mm × 3 μm; mobile phase: water (0.04% hydrochloric acid)-acetonitrile; acetonitrile%: 17% to 37%, 7 minutes) to obtain the hydrochloride of the target compound **WX004.** MS-ESI *m*/*z:* 827.5 $[M+H]^+$. $^1H$ NMR (400 MHz, DMSO_$d_6$) δ: 10.90 (s, 1H), 10.12 (s, 1H), 9.95 (s, 1H), 9.15 (s, 1H), 8.18 (d, *J*=28.8 Hz, 1H), 8.17-8.03 (m, 2H), 7.86 (s, 1H), 7.69 (s, 1H), 7.47 (d, *J*=8.4 Hz, 1H), 7.40 (s, 1H), 7.34-7.04 (m, 3H), 4.13 (dd, *J*=5.0, 12.2 Hz, 2H), 3.44 (t, *J*=5.8 Hz, 2H), 3.41-3.33 (m, 4H), 3.24-3.10 (m, 1H), 3.09-2.97 (m, 2H), 2.96-2.87 (m, 1H), 2.81-2.65 (m, 6H), 2.62-2.55 (m, 1H), 2.39-2.27 (m, 2H), 2.15-2.03 (m, 4H), 2.02-1.89 (m, 3H), 1.87-1.80 (m, 4H), 1.27-1.07 (m, 3H), 0.61-0.51 (m, 2H), 0.39-0.30 (m, 2H).

**Example** 7

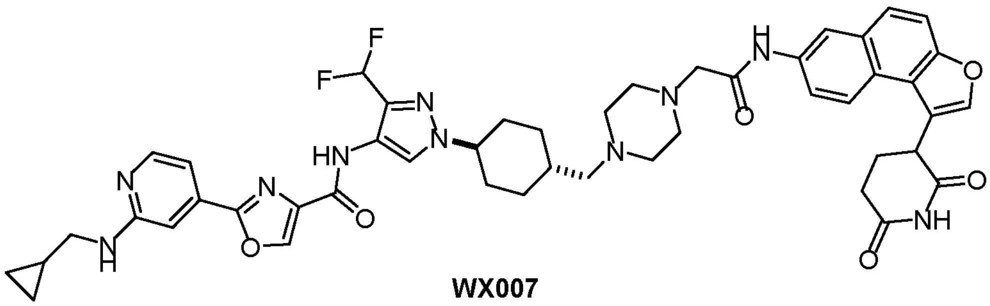

**WX007**

Synthetic route:

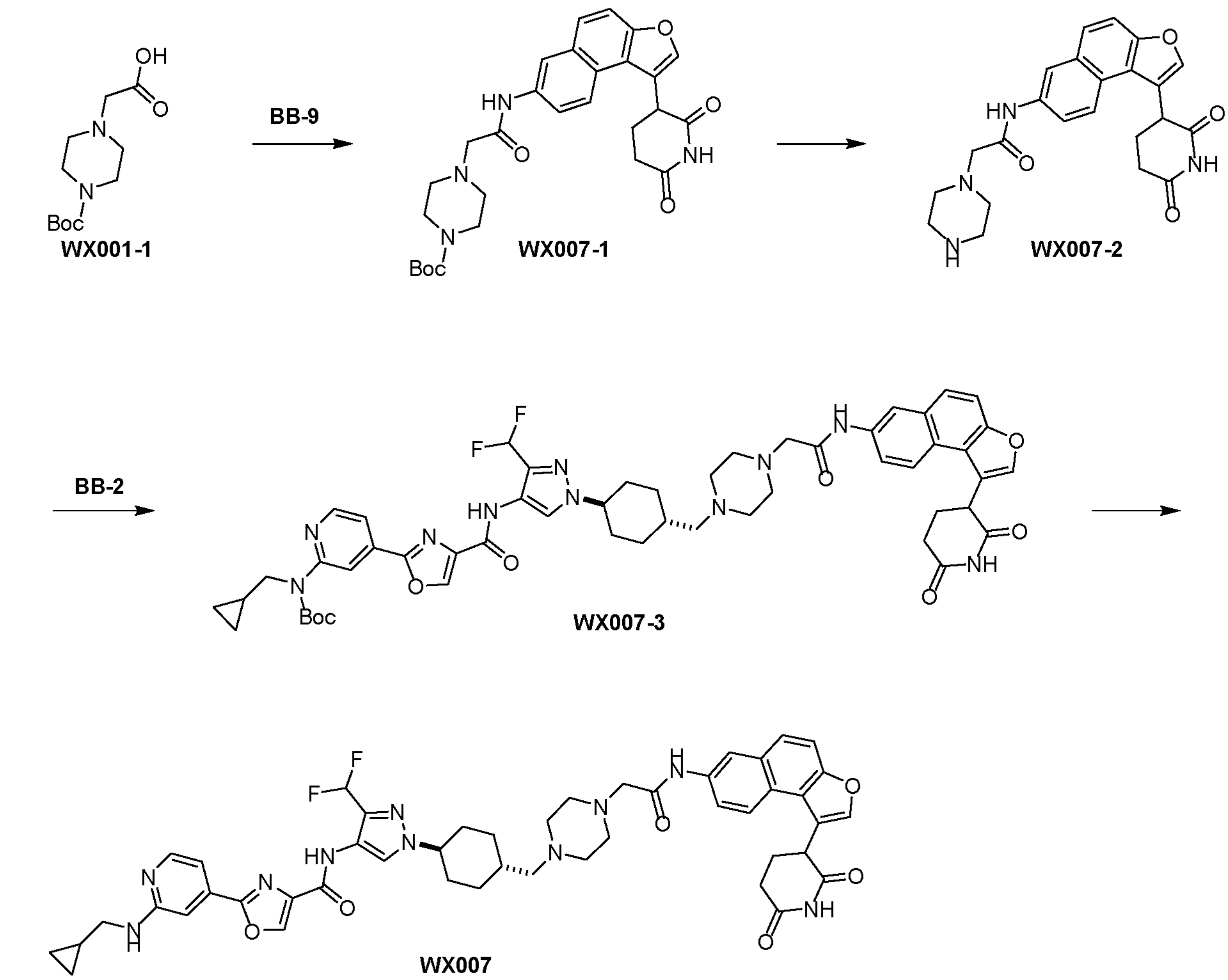


WX001-1 WX007-1 WX007-2 WX007-3 WX007

Step **1:** Synthesis of compound **WX007-1**

Compound **WX001-1** (387.74 mg, 1.59 mmol) was dissolved in N, N-dimethylformamide (10 mL) at room temperature, then 2-(7-azabenzotriazol)-N, N, N', N'-tetramethyluronium hexafluorophosphate (482.81 mg, 1.27 mmol), N, N-diisopropylethylamine (410.27 mg, 3.17 mmol), and the hydrochloride of compound **BB-9** (350 mg, 1.06 mmol) were sequentially added thereto. The reaction mixture was stirred and reacted at room temperature for 12 hours. After the reaction was completed, the reaction mixture was poured into water (50 mL), and extracted with ethyl acetate (10 mL × 3). The organic phases were combined, washed with saturated brine (10 mL), dried over anhydrous sodium sulfate, and filtered. The filtrate was concentrated under reduced pressure to remove the solvent. The resulting residue was separated by column chromatography (eluent: petroleum ether/ethyl acetate = 5/1 to 1/1, v/v) to obtain compound **WX007-1.** MS-ESI *m/z:* 521.1 $[M+H]^+$. $^1H$ NMR (400 MHz, $CDCl_3$) δ: 9.25 (s, 1H), 8.28 (s, 1H), 8.10 (s, 1H), 7.95 (d, *J*=8.8 Hz, 1H), 7.77-7.70 (m, 2H), 7.69-7.64 (m, 2H), 4.48 (dd, *J*=5.0, 8.2 Hz, 1H), 3.61-3.51 (m, 4H), 3.24 (s, 2H), 2.90-2.76 (m, 2H), 2.70-2.59 (m, 4H), 2.56-2.46 (m, 2H), 1.49 (s, 9H).

Step **2:** Synthesis of hydrochloride of compound **WX007-2**

Compound **WX007-1** (100 mg, 180.19 μmol, purity of 93.8%) was dissolved in hydrochloric acid/dioxane (4 mol/L, 7.5 mL). The reaction mixture was stirred and reacted at room temperature for 12 hours. After the reaction was completed, the reaction mixture was concentrated under reduced pressure to remove the solvent. The resulting residue was added with ethyl acetate (20 mL), then concentrated under reduced pressure to remove the solvent to obtain the hydrochloride of compound **WX007-2.** MS-ESI *m/z:* 421.1 $[M+H]^+$.

Step **3:** Synthesis of compound **WX007-3**

Compound **BB-2** (77 mg, 131.71 μmol) and the hydrochloride of compound **WX007-2** (60.18 mg, 131.71 μmol)

were dissolved in tetrahydrofuran (5 mL) and N, N-dimethylformamide (1 mL) at room temperature, and then potassium acetate (38.78 mg, 395.13 μmol) and acetic acid (3.95 mg, 65.86 μmol) were added thereto. The reaction mixture was stirred and reacted under nitrogen atmosphere for 0.5 hours. Sodium triacetoxyborohydride (83.74 mg, 395.13 μmol) was added thereto. The mixture was further stirred and reacted for 0.5 hours. After the reaction was completed, the reaction mixture was added with a saturated solution of ammonium chloride (20 mL), and extracted with ethyl acetate (20 mL × 3). The organic phases were combined, washed with saturated brine (20 mL), dried over anhydrous sodium sulfate, and filtered. The filtrate was concentrated under reduced pressure to remove the solvent to obtain compound **WX007-3.** MS-ESI *m/z:* 889.5 [M+ H-100]$^+$.

Step **4:** Synthesis of compound **WX007**

Compound **WX007-3** (130 mg, 131.44 μmol) was dissolved in dichloromethane (3 mL) at room temperature and trifluoroacetic acid (3 mL) was added thereto. The reaction mixture was heated to 40°C and stirred and reacted for 1 hour. After the reaction was completed, the mixture was concentrated under reduced pressure to remove the solvent. The resulting residue was separated by preparative high performance liquid chromatography (chromatographic column: Boston Green ODS 150 * 30 mm * 5 μm; mobile phase: water (0.04% hydrochloric acid)-acetonitrile; acetonitrile%: 19%-34%, 10 minutes), and again separated by preparative high performance liquid chromatography (chromatographic column: Welch Xtimate C18 150 * 25 mm * 5 μm; mobile phase: water (10 mM ammonium bicarbonate)-acetonitrile; acetonitrile%: 45% to 75%, 11 minutes) to obtain the target compound **WX007.** MS-ESI *m/z:* 890.8 [M+H]$^+$. $^1$H NMR (400 MHz, DMSO_$d_6$) δ: 10.94 (s, 1H), 9.92 (s, 1H), 9.72 (s, 1H), 8.88 (s, 1H), 8.36 (d, *J*=2.0 Hz, 1H), 8.17-8.08 (m, 3H), 7.97 (s, 1H), 7.79-7.70 (m, 3H), 7.15-6.96 (m, 4H), 4.63 (dd, *J*=4.2, 11.8 Hz, 1H), 4.24-4.10 (m, 1H), 3.18-3.14 (m, 3H), 2.94-2.81 (m, 1H), 2.68-2.63 (m, 1H), 2.61-2.54 (m, 4H), 2.30-2.23 (m, 2H), 2.17-2.12 (m, 2H), 2.08-1.95 (m, 4H), 1.93-1.85 (m, 2H), 1.78-1.70 (m, 2H), 1.63-1.55 (m, 1H), 1.22 (s, 3H), 1.09-1.00 (m, 2H), 0.84 (t, *J*=6.4 Hz, 1H), 0.48-0.40 (m, 2H), 0.24-0.17 (m, 2H).

**Example 8**

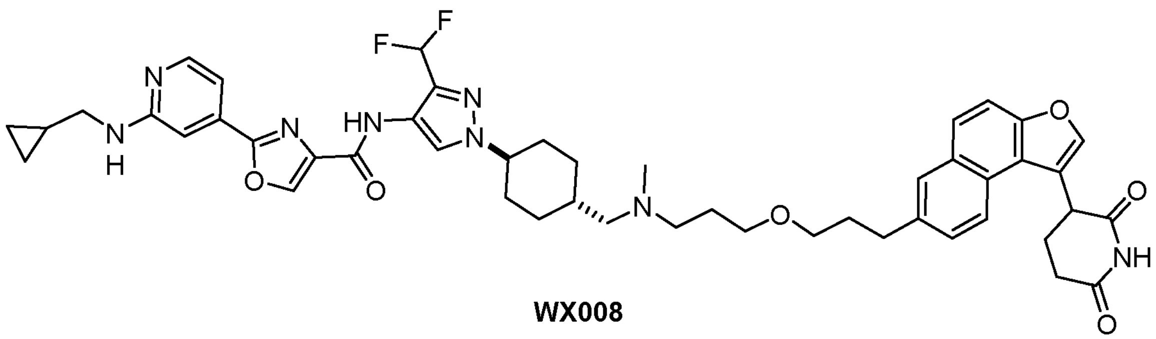

**WX008**

Synthetic route:

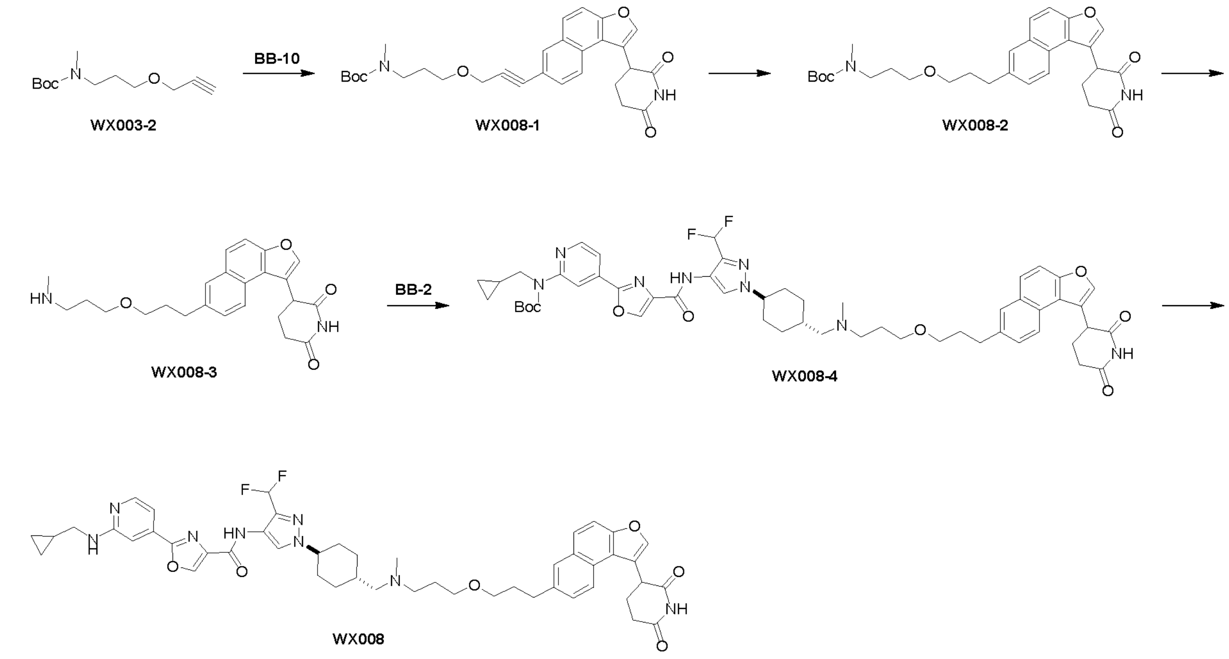


WX003-2 WX008-1 WX008-2 WX008-3 WX008-4 WX008

Step **1:** Synthesis of compound **WX008-1**

Compound **BB-10** (300 mg, 837.55 μmol) was dissolved in N, N-dimethylformamide (10 mL) at room temperature under nitrogen atmosphere, then compound WX003-2 (285.56 mg, 1.26 mmol), cesium carbonate (818.68 mg, 2.51 mmol), cuprous iodide (31.90 mg, 167.51 μmol), and bis(triphenylphosphine)palladium(II) chloride (117.58 mg, 167.51 μmol) were sequentially added thereto. The reaction mixture was heated to 80 °C, and stirred and reacted for 5 hours. After the reaction was completed, the reaction mixture was cooled to room temperature, poured into a saturated solution of ammonium chloride (100 mL), and extracted with ethyl acetate (10 mL × 3). The organic phases were combined, washed with saturated brine (10 mL), dried over anhydrous sodium sulfate, and filtered. The filtrate was concentrated under reduced pressure to remove the solvent. The resulting residue was separated by column chromatography (eluent: petroleum ether/ethyl acetate = 5/1 to 1/1, v/v) to obtain compound **WX008-1.** MS-ESI *m/z:* 405.2 $[M+H-100]^+$. $^1H$ NMR (400 MHz, $CDCl_3$) δ: 8.15-8.01 (m, 2H), 7.92 (d, *J*=8.4 Hz, 1H), 7.73 (d, *J*=9.2 Hz, 1H), 7.69 (d, *J*=7.6 Hz, 2H), 7.61 (dd, *J*=1.6, 8.8 Hz, 1H), 4.50 (dd, *J*=5.2, 8.8 Hz, 1H), 4.43 (s, 2H), 3.65 (t, *J*=6.2 Hz, 2H), 3.35 (t, *J*=6.6 Hz, 2H), 2.89 (s, 3H), 2.85-2.78 (m, 2H), 2.58-2.41 (m, 2H), 1.94-1.83 (m, 2H), 1.47 (s, 9H).

Step **2:** Synthesis of compound **WX008-2**

Compound **WX008-1** (160 mg, 317.10 μmol) was dissolved in tetrahydrofuran (10 mL) at room temperature, then wet palladium on carbon (0.2 g, 317.10 μmol, purity of 10%) and palladium hydroxide/carbon (0.2 g, 317.10 μmol, purity of 20%) were sequentially added thereto. The reaction mixture was stirred and reacted at room temperature under hydrogen (15 psi) atmosphere for 2 hours. After the reaction was completed, the mixture was filtered. The filtrate was concentrated under reduced pressure to remove the solvent to obtain compound **WX008-2.** MS-ESI *m/z:* 409.1 $[M+H-100]^+$. $^1H$ NMR (400 MHz, $CDCl_3$) δ: 8.09 (s, 1H), 7.92 (d, *J*=8.4 Hz, 1H), 7.78 (s, 1H), 7.72 (d, *J*=9.2 Hz, 1H), 7.67-7.63 (m, 2H), 7.45 (d, *J*=1.4, 8.6 Hz, 1H), 4.52 (dd, *J*=5.6, 8.0 Hz, 1H), 3.45 (q, *J*=6.6 Hz, 4H), 3.36-3.28 (m, 2H), 2.92-2.85 (m, 5H), 2.82-2.76 (m, 2H), 2.55-2.46 (m, 2H), 2.04-1.95 (m, 2H), 1.86-1.78 (m, 2H), 1.46 (s, 9H).

Step **3:** Synthesis of hydrochloride of compound **WX008-3**

Compound **WX008-2** (100 mg, 196.62 μmol) was dissolved in ethyl acetate (5 mL) at room temperature, then a solution of hydrochloric acid in ethyl acetate (4 M, 5 mL) was added thereto. The reaction mixture was stirred and reacted at room temperature for 12 hours. After the reaction was completed, the mixture was concentrated under reduced pressure to remove the solvent. The resulting residue was added with ethyl acetate (20 mL), then concentrated under reduced pressure to remove the solvent to obtain hydrochloride of compound **WX008-3.** MS-ESI *m/z:* 409.2 $[M+H]^+$.

Step **4:** Synthesis of compound **WX008-4**

Compound **BB-2** (91 mg, 147.64 μmol) was dissolved in tetrahydrofuran (5 mL) and N, N-dimethylformamide (1 mL) at room temperature, then the hydrochloride of compound **WX008-3** (65.69 mg, 147.64 μmol), potassium acetate (43.47 mg, 442.93 μmol) and acetic acid (8.87 mg, 8.44 μL, 147.64 μmol) were added thereto. The reaction mixture was stirred and reacted for 0.5 hours. Sodium triacetoxyborohydride (93.87 mg, 442.93 μmol) was added thereto. The mixture was further stirred and reacted for 1 hour. After the reaction was completed, the reaction mixture was added with a saturated solution of ammonium chloride (20 mL), and extracted with ethyl acetate (20 mL × 3). The organic phases were combined, washed with saturated brine (20 mL), dried over anhydrous sodium sulfate, and filtered. The filtrate was concentrated under reduced pressure to remove the solvent. The resulting residue was separated by column chromatography (eluent: dichloromethane/methanol = 1/0 to 20/1, v/v) to obtain compound **WX008-4.** MS-ESI *m/z:* 977.6 $[M+H]^+$.

Step **5:** Synthesis of hydrochloride of compound **WX008**

Compound **WX008-4** (110 mg, 100.36 μmol, purity of 89.15%) was dissolved in dichloromethane (3 mL) at room temperature, then trifluoroacetic acid (3 mL) was added thereto. The reaction mixture was heated to 40°C, and stirred and reacted for 1 hour. After the reaction was completed, the mixture was concentrated under reduced pressure to remove the solvent. The resulting residue was separated by preparative high performance liquid chromatography (chromatographic column: Boston Green ODS 150 * 30 mm * 5 μm; mobile phase: water (0.04% hydrochloric acid)-acetonitrile; acetonitrile%: 25% to 45%, 10 minutes) to obtain the hydrochloride of the target compound **WX008.** MS-ESI *m/z:* 877.5 $[M+H]^+$. $^1H$ NMR (400 MHz, DMSO_$d_6$) δ: 10.96 (s, 1H), 9.89 (s, 1H), 9.54 (s, 1H), 9.07 (s, 1H), 8.17 (s, 1H), 8.07 (d, *J*=6.0 Hz, 2H), 7.98 (s, 1H), 7.84 (s, 1H), 7.77 (dd, *J*=8.8, 16.0 Hz, 2H), 7.57 (s, 1H), 7.46 (d, *J*=8.4 Hz, 1H), 7.35-7.00 (m, 2H), 4.64 (dd, *J*=3.8, 11.8 Hz, 1H), 4.23 (t, *J*=11.8 Hz, 1H), 3.31-3.24 (m, 2H), 3.20-3.11 (m, 2H), 3.10-2.97 (m, 3H), 2.96-2.86 (m, 2H), 2.82 (t, *J*=7.4 Hz, 2H), 2.76 (d, *J*=4.0 Hz, 3H), 2.67-2.59 (m, 1H), 2.42-2.31 (m, 1H), 2.30-2.21 (m, 1H), 2.10-1.74 (m, 12H), 1.27-1.10 (m, 4H), 0.61-0.51 (m, 2H), 0.36-0.27 (m, 2H).

**Example 10**

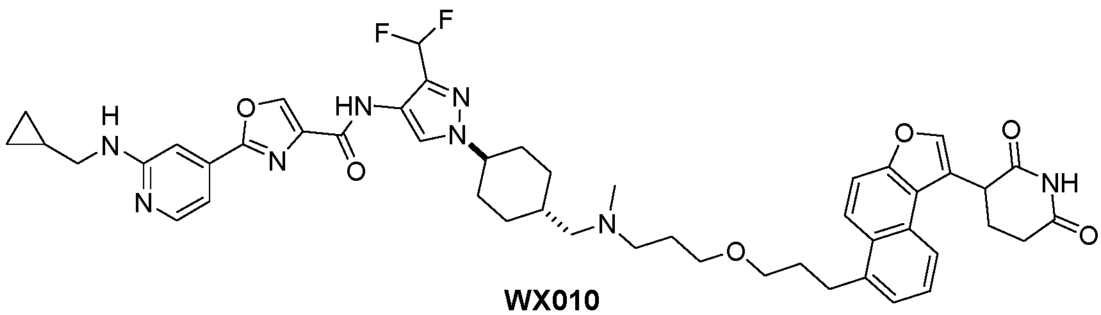

**WX010**

Synthetic route:

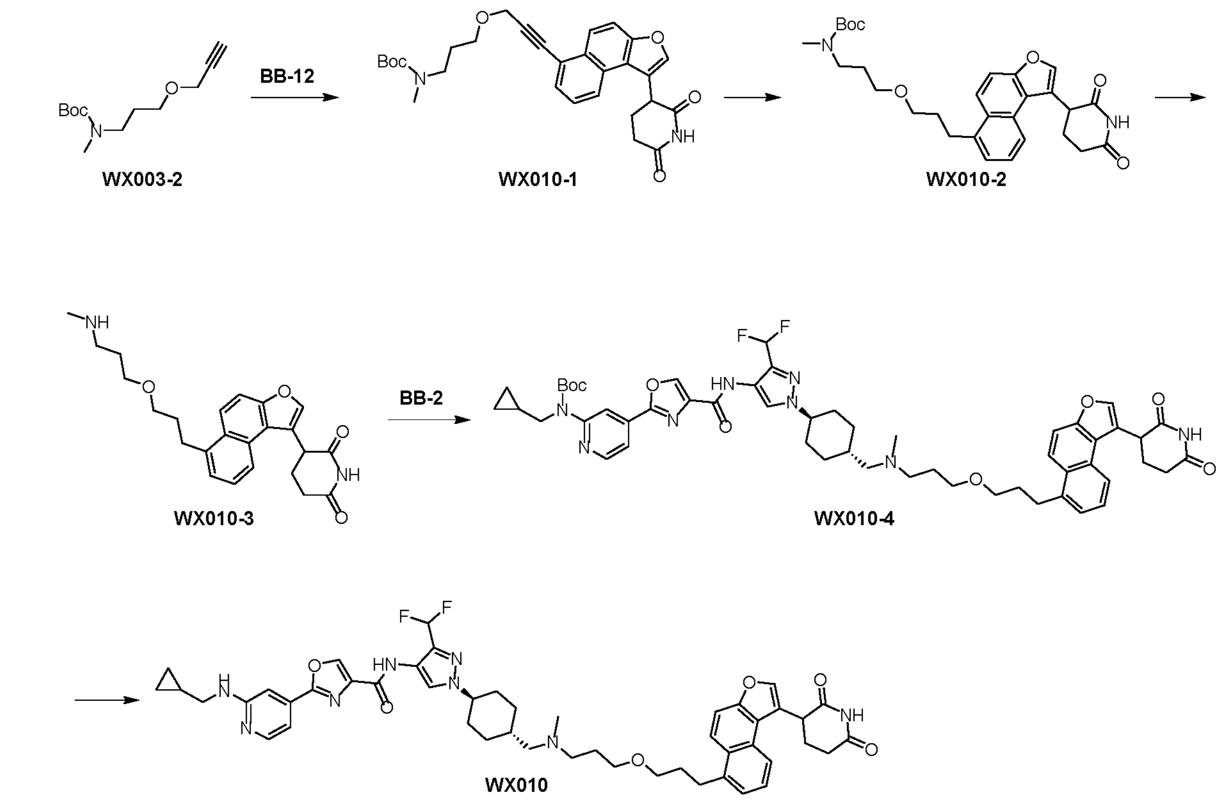


WX003-2 WX010-1 WX010-2

WX010-3 WX010-4

WX010

Step **1:** Synthesis of compound **WX010-1**

Compound **BB-12** (0.1 g, 279.18 μmol), compound **WX003-2** (126.92 mg, 558.37 μmol), cuprous iodide (10.63 mg, 55.84 μmol), bis(triphenylphosphine)palladium(II) dichloride (39.19 mg, 55.84 μmol), and N, N-diisopropylethylamine (72.17 mg, 558.37 μmol) were dissolved in dimethyl sulfoxide (2 mL) at room temperature under nitrogen atmosphere. The reaction mixture was heated to 85°C, and stirred and reacted for 2.5 hours. After the reaction was completed, the reaction mixture was cooled to room temperature, added with water (2 mL), and extracted with dichloromethane (5 mL × 2). The organic phases were combined, washed with saturated brine (5 mL), dried over anhydrous sodium sulfate, and filtered. The filtrate was concentrated under reduced pressure. The resulting residue was purified by a chromatography plate (eluent: petroleum ether/ethyl acetate = 1/1, v/v) to obtain compound **WX010-1.** MS-ESI *m/z:* 405.2 $[M+H-100]^+$. $^1H$ NMR (400 MHz, $CDCl_3$) δ: 8.35 (d, *J*=9.2 Hz, 1H), 8.12 (s, 1H), 7.99 (d, *J*=8.4 Hz, 1H), 7.76 (d, *J*=9.2 Hz, 1H), 7.71 (d, J=6.8 Hz, 1H), 7.69 (s, 1H), 7.53 (t, *J*=8.0 Hz, 1H), 4.54 (s, 2H), 4.50 (dd, *J*=5.4, 9.0 Hz, 1H), 3.70 (t, *J*=6.4 Hz, 2H), 3.36 (t, *J*=6.4 Hz, 2H), 2.89 (s, 3H), 2.85-2.77 (m, 2H), 2.57-2.42 (m, 2H), 1.96-1.86 (m, 2H), 1.46 (s, 9H).

Step **2:** Synthesis of compound **WX010-2**

Compound **WX010-1** (60 mg, 118.91 μmol) was dissolved in tetrahydrofuran (5 mL) at room temperature, then wet palladium on carbon (10 mg, purity of 10%) and palladium hydroxide/carbon (10 mg, purity of 20%) were sequentially added thereto. The reaction mixture was stirred and reacted at room temperature under hydrogen (15 psi) atmosphere for 15 hours. After the reaction was completed, the mixture was filtered. The filter cake was rinsed with tetrahydrofuran (10 mL × 2). The filtrate was collected and concentrated under reduced pressure to obtain compound **WX010-2.** MS-ESI *m/z:* 409.2 $[M+H-100]^+$.

Step **3:** Synthesis of trifluoroacetate of compound **WX010-3**

Compound **WX010-2** (60 mg, 117.97 μmol) was dissolved in dichloromethane (1 mL) at room temperature and trifluoroacetic acid (184.80 mg, 1.62 mmol) was added thereto. The reaction mixture was stirred and reacted at room temperature for 2 hours. After the reaction was completed, the mixture was concentrated under reduced pressure to remove the solvent to obtain the trifluoroacetate of compound **WX010-3.** MS-ESI *m/z:* 409.2 $[M+H]^+$.

Step **4:** Synthesis of compound **WX010-4**

Compound **BB-2** (50 mg, 85.53 μmol), the trifluoroacetate of compound **WX010-3** (53.63 mg, 102.63 μmol), and potassium acetate (25.18 mg, 256.58 μmol) were dissolved in a mixed solvent of N, N-dimethylformamide (1 mL) and glacial acetic acid (0.1 mL) at room temperature under nitrogen atmosphere. The reaction mixture was stirred and reacted at room temperature for 2 hours. Sodium triacetoxyborohydride (54.38 mg, 256.58 μmol) was added thereto. The reaction mixture was further stirred and reacted for 15 hours. After the reaction was completed, the reaction mixture was added with a saturated solution of ammonium chloride (8 mL), and extracted with ethyl acetate (10 mL × 2). The organic phases were combined, washed with saturated brine (10 mL), dried over anhydrous sodium sulfate, and filtered. The filtrate was concentrated under reduced pressure to obtain compound **WX010-4.** MS-ESI *m/z:* 977.6 $[M+H]^+$.

Step **5:** Synthesis of trifluoroacetate of compound **WX010**

Compound **WX010-4** (0.1 g, 102.34 μmol) was dissolved in dichloromethane (2 mL) at room temperature and trifluoroacetic acid (3.08 g, 27.01 mmol) was added dropwise thereto. The reaction mixture was stirred and reacted at room temperature for 4 hours. After the reaction was completed, the mixture was concentrated under reduced pressure to remove the solvent. The resulting crude product was separated by preparative high performance liquid chromatography (chromatographic column: Phenomenex Luna C18 80 * 30 mm * 3 μm; mobile phase: water (0.1% trifluoroacetic acid)-acetonitrile; acetonitrile%: 20% to 50%, 8 minutes) to obtain the trifluoroacetate of the target compound **WX010.** MS-ESI *m/z:* 877.6 $[M+H]^+$. $^1H$ NMR (400 MHz, MeOD_$d_4$) δ: 8.81 (s, 1H), 8.16 (d, *J*=1.6 Hz, 1H), 8.12 (d, *J*=9.6 Hz, 1H), 8.06 (d, *J*=8.4 Hz, 1H), 7.99 (d, *J*=6.4 Hz, 1H), 7.84 (s, 1H), 7.74 (d, *J*=9.6 Hz, 1H), 7.70 (s, 1H), 7.53 (t, *J*=7.8 Hz, 1H), 7.45 (d, *J*=6.4 Hz, 1H), 7.41 (d, *J*=6.8 Hz, 1H), 6.93 (t, *J*=54.4 Hz 1H), 4.65 (dd, *J*=5.0 Hz, 10.2 Hz, 1H), 4.11-4.02 (m, 1H), 3.65-3.53 (m, 4H), 3.31-3.20 (m, 4H), 3.18-3.07 (m, 2H), 3.01-2.88 (m, 2H),2.87 (s, 3H), 2.79-2.71 (m, 1H), 2.55-2.40 (m, 2H), 2.17-1.75 (m, 12H), 1.29-1.17 (m, 3H), 0.75-0.68 (m, 2H), 0.45-0.38 (m, 2H).

**Example 11**

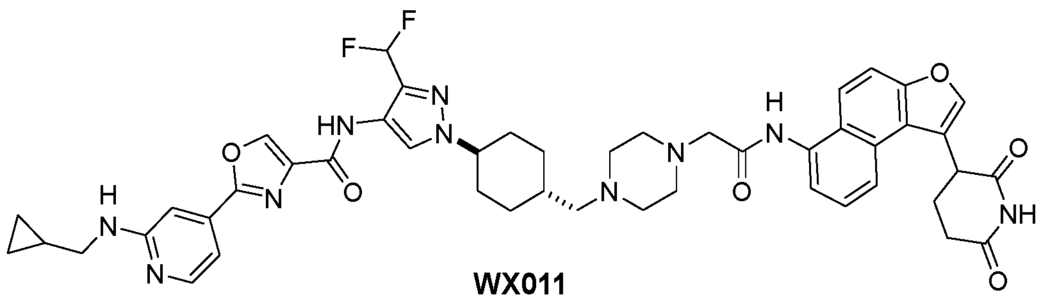

**WX011**

Synthetic route:

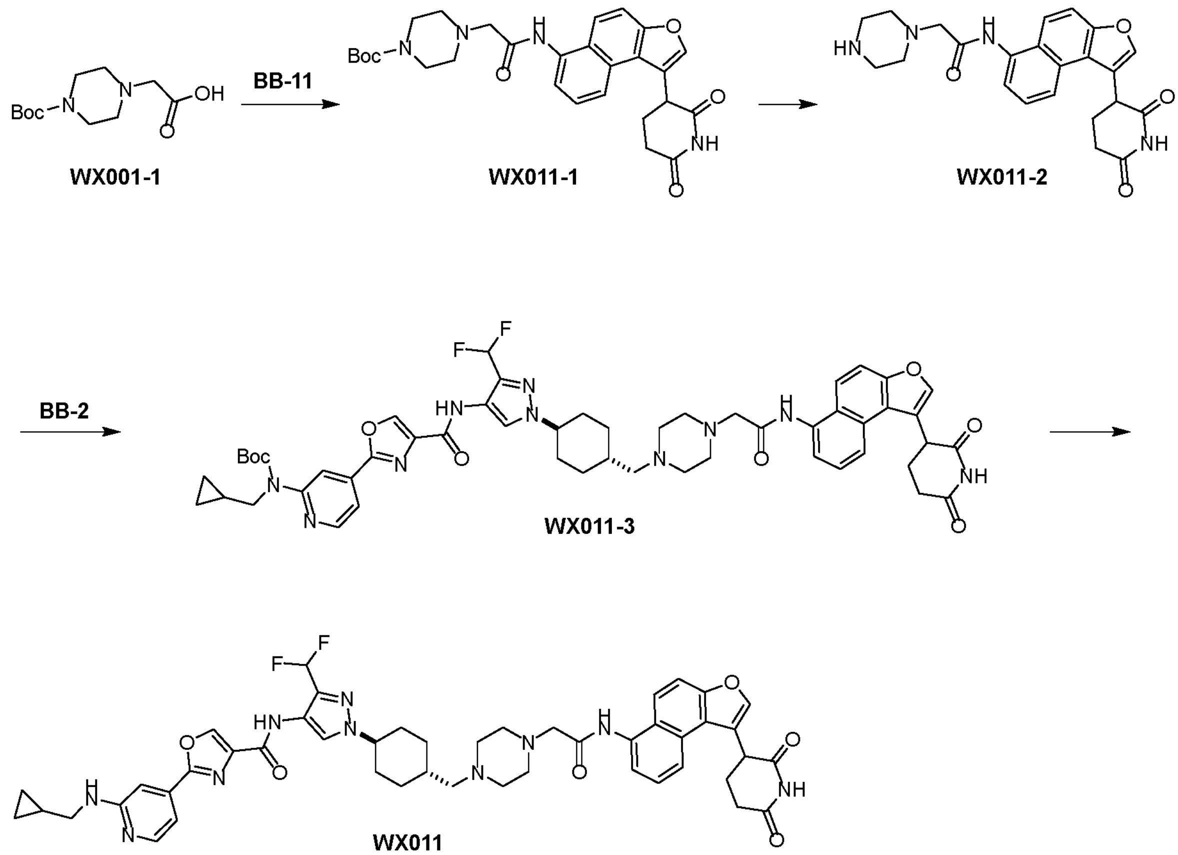

WX001-1 WX011-1 WX011-2

WX011-3

WX011

Step **1:** Synthesis of compound **WX011-1**

Compound **WX001-1** (102.70 mg, 365.82 μmol) was dissolved in: N, N-dimethylformamide (2 mL) at room temperature under nitrogen atmosphere, and 2-(7-azabenzotriazol)-N, N, N, N-tetramethyluronium hexafluorophosphate (164.39 mg, 432.33 μmol) and N, N-diisopropylethylamine (214.91 mg, 1.66 mmol) were added thereto. The reaction mixture was stirred and reacted at room temperature for 0.5 hours. The hydrochloride of compound **BB-11** (0.11 g, 332.56 μmol) was added thereto. The reaction mixture was stirred and reacted at room temperature for 15 hours. After the reaction was completed, the reaction mixture was added with water (5 mL) and extracted with ethyl acetate (2 mL × 2). The organic phases were combined, washed with saturated brine (5 mL), dried over anhydrous sodium sulfate, and filtered. The filtrate was concentrated under reduced pressure to remove the solvent. The resulting residue was purified by a chromatography plate (developing solvent: ethyl acetate) to obtain compound **WX011-1.** MS-ESI *m/z:* 521.2 $[M+H]^+$.

Step **2:** Synthesis of hydrochloride of compound **WX011-2**

Compound **WX011-1** (0.12 g, 230.51 μmol) was dissolved in ethyl acetate (1 mL) at room temperature, and a solution of hydrochloric acid in ethyl acetate (4 M, 6 mL) was added thereto. The reaction mixture was stirred at room temperature for 1 hour. After the reaction was completed, the mixture was concentrated under reduced pressure to remove the solvent to obtain the hydrochloride of compound **WX011-2.** MS-ESI *m/z:* 421.2 $[M+H]^+$.

Step **3:** Synthesis of compound **WX011-3**

Compound **BB-2** (50 mg, 85.53 μmol), the hydrochloride of the compound **WX011-2** (42.99 mg, 94.08 μmol), and potassium acetate (25.18 mg, 256.58 μmol) were dissolved in a mixed solvent of N, N-dimethylformamide (1 mL) and glacial acetic acid (0.1 mL) at room temperature under nitrogen atmosphere. The reaction mixture was stirred and reacted at room temperature for 2 hours. Sodium triacetoxyborohydride (54.38 mg, 256.58 μmol) was added thereto. The reaction mixture was stirred and reacted at room temperature for 15 hours. After the reaction was completed, the mixture was added with a saturated solution of ammonium chloride (10 mL), and extracted with ethyl acetate (10 mL × 2). The organic phases were combined, washed with saturated brine (10 mL), dried over anhydrous sodium sulfate, and

filtered. The filtrate was concentrated under reduced pressure to obtain compound **WX011-3.** MS-ESI *m/z:* 467.3 [(M-56)/2+H]$^+$.

Step **4:** Synthesis of trifluoroacetate of compound **WX011**

Compound **WX011-3** (80 mg, 80.88 μmol) was dissolved in dichloromethane (1 mL) at room temperature and trifluoroacetic acid (308.00 mg, 2.70 mmol, 0.2 ml) was added thereto. The reaction mixture was stirred and reacted at room temperature for 5 hours. After the reaction was completed, the mixture was concentrated under reduced pressure to remove the solvent. The resulting residue was separated by preparative high performance liquid chromatography (chromatographic column: Phenomenex Luna C18 100 * 40 mm * 5 μm; mobile phase: water (0.1% trifluoroacetic acid)-acetonitrile; acetonitrile%: 25% to 70%, 8 minutes) to obtain the trifluoroacetate of the target compound **WX011.** MS-ESI *m/z:* 889.5 [M+H]$^+$. $^1$H NMR (400 MHz, MeOD_$d_4$) δ: 8.79 (s, 1H), 8.26 (s, 1H), 8.13 (d, *J*=7.6 Hz, 1H), 7.97 (d, *J*=6.8 Hz, 1H), 7.93 (d, *J*=9.2 Hz, 1H), 7.88 (s, 1H), 7.77 (d, *J*=9.6 Hz, 1H), 7.70 (s, 1H), 7.67-7.60 (m, 2H), 7.44 (dd, *J*=0.8 Hz, 6.8 Hz, 1H), 7.09-6.79 (m, 1H), 4.67 (dd, *J*=5.4 Hz, 10.6 Hz, 1H), 4.31-4.19 (m, 1H), 3.60 (s, 2H), 3.53-3.38 (m, 3H), 3.28 (s, 2H), 3.24-3.18 (m, 2H), 3.16-3.00 (m, 4H), 2.96-2.85 (m, 1H), 2.81-2.72 (m, 1H), 2.56-2.41 (m, 2H), 2.23 (d, *J*=11.6 Hz, 2H), 2.10-1.65 (m, 6H), 1.43-1.27 (m, 2H), 1.26-1.17 (m, 1H), 0.74-0.67 (m, 2H), 0.43-0.37 (m, 2H).

**Example 13**

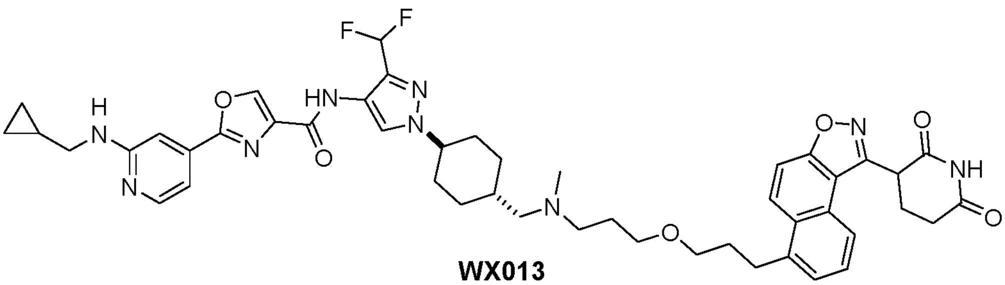

**WX013**

Synthetic route:

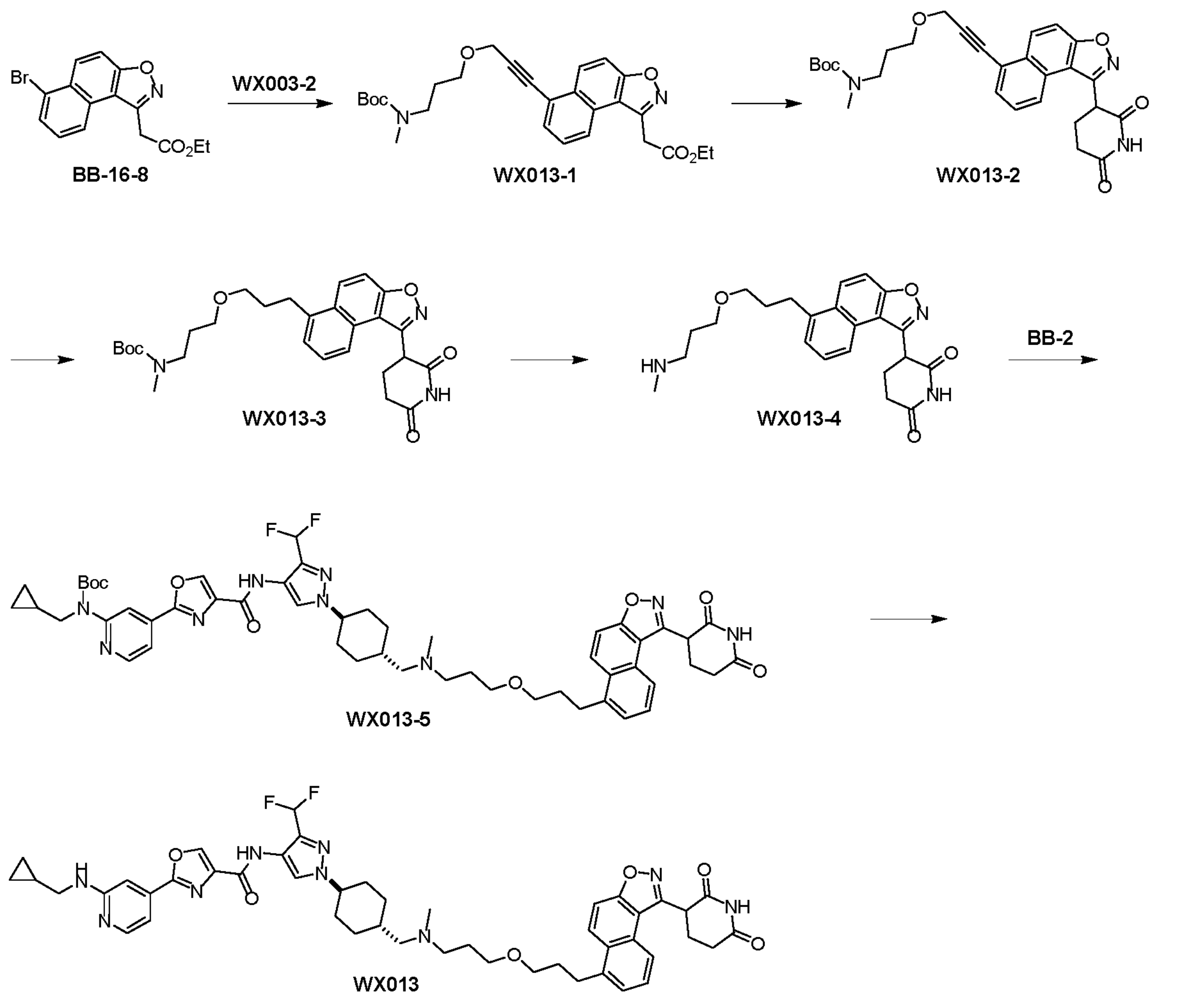


BB-16-8 WX013-1 WX013-2 WX013-3 WX013-4 WX013-5 WX013

Step **1:** Synthesis of compound **WX013-1**

Compound **BB-16-8** (1 g, 2.99 mmol) and compound **WX003-2** (816.24 mg, 3.59 mmol) were dissolved in dimethyl sulfoxide (10 mL) at room temperature under nitrogen atmosphere. Bis(triphenylphosphine)palladium(II) chloride (420.09 mg, 598.51 μmol), cuprous iodide (113.99 mg, 598.51 μmol), and N, N-diisopropylethylamine (773.51 mg, 5.99 mmol) were added thereto. The reaction mixture was heated to 85°C, and stirred and reacted for 2 hours. After the reaction was completed, the mixture was cooled to room temperature, added with water (30 mL), and extracted with ethyl acetate (3 × 20 mL). The organic phases were combined, washed with 10% brine (3 × 40 mL), dried over anhydrous sodium sulfate, and filtered. The filtrate was concentrated under reduced pressure to remove the solvent. The resulting residue was separated by column chromatography (eluent: petroleum ether/ethyl acetate = 1/0 to 4/1, v/v) to obtain compound **WX013-1.** MS-ESI *m/z:* 381.2 $[M+H-100]^+$. $^1H$ NMR (400 MHz, $CDCl_3$) δ: 8.54 (d, *J*=9.2 Hz, 1H), 8.11 (d, *J*=8.4 Hz, 1H), 7.79 (d, *J*=9.2 Hz, 1H), 7.75 (dd, *J*=1.0 Hz, 7.4 Hz, 1H), 7.62 (d, *J*=8.0 Hz, 1H), 4.54 (s, 2H), 4.34 (s, 2H), 4.22 (q, *J*=7.2 Hz, 2H), 3.70 (t, *J*=6.4 Hz, 2H), 3.36 (t, *J*=6.6 Hz, 2H), 2.89 (s, 3H), 1.95-1.86 (m, 2H), 1.46 (s, 9H), 1.20 (t, *J*=7.0 Hz, 3H).

Step **2:** Synthesis of compound **WX013-2**

Compound **WX013-1** (0.8 g, 1.66 mmol) and acrylamide (130.16 mg, 1.83 mmol) were dissolved in tetrahydrofuran (10 mL) at room temperature under nitrogen atmosphere. The mixture was cooled to 0°C, and potassium tert-butoxide (224.16 mg, 2.00 mmol) was added thereto. The reaction mixture was stirred and reacted at room temperature for 1 hour. After the reaction was completed, the reaction mixture was added to 1 N hydrochloric acid to adjust the pH to 3 to 4, added with water (10 mL), and extracted with ethyl acetate (3 × 20 mL). The organic phases were combined, dried over anhydrous sodium sulfate, and filtered. The filtrate was concentrated under reduced pressure to remove the solvent. The resulting residue was separated by column chromatography (eluent: petroleum ether/ethyl acetate = 3/1 to 1/1, v/v) to obtain compound **WX013-2.** MS-ESI m/z: 406.2 $[M+H-100]^+$. $^1H$ NMR (400 MHz, $CDCl_3$) δ: 8.59 (d, *J*=9.2

Hz, 1H), 8.07 (s, 1H), 8.04 (d, *J*=8.4 Hz, 1H), 7.82 (d, *J*=9.2 Hz, 1H), 7.77 (dd, *J*=0.8, 7.2 Hz, 1H), 7.65 (t, *J*=7.8 Hz, 1H), 4.74 (dd, *J*=5.0 Hz, 8.2 Hz, 1H), 4.54 (s, 2H), 3.70 (t, *J*=6.2 Hz, 2H), 3.37 (t, *J*=6.2 Hz, 2H), 2.99-2.91 (m, 1H), 2.90 (s, 3H), 2.84-2.68 (m, 2H), 2.64-2.53 (m, 1H), 1.96-1.86 (m, 2H), 1.46 (s, 9H).

Step **3:** Synthesis of compound **WX013-3**

Wet palladium on carbon (20 mg, purity of 10%) and palladium hydroxide/carbon (20 mg, purity of 20%) were dissolved in tetrahydrofuran (3 mL) at room temperature. Compound WX013-2 (150 mg, 296.70 μmol) and triethylamine (30.02 mg, 296.70 μmol) were added thereto. The reaction mixture was stirred and reacted at room temperature under hydrogen (15 psi) atmosphere for 12 hours. After the reaction was completed, the mixture was filtered through diatomite. The filter cake was rinsed with ethyl acetate (20 mL). The filtrate was concentrated under reduced pressure to obtain compound **WX013-3.** MS-ESI m/z: 410.2 [M+H-100]$^+$. 1H NMR (400 MHz, $CDCl_3$) δ: 8.31 (d, *J*=9.2 Hz, 1H), 8.12 (s, 1H), 7.91 (d, *J*=8.4 Hz, 1H), 7.75 (d, *J*=9.6 Hz, 1H), 7.62 (t, *J*=7.6 Hz, 1H), 7.45 (d, *J*=6.8 Hz, 1H), 4.77 (dd, *J*=5.4 Hz, 7.8 Hz, 1H), 3.52-3.45 (m, 4H), 3.38-3.30 (m, 2H), 3.24 (t, *J*=7.6 Hz, 2H), 2.91-2.85 (m, 4H), 2.82-2.68 (m, 2H), 2.62-2.52 (m, 1H), 2.05-1.98 (m, 2H), 1.90-1.81 (m, 2H), 1.47 (s, 9H).

Step **4:** Synthesis of trifluoroacetate of compound **WX013-4**

Compound **WX013-3** (120 mg, 235.48 μmol) was dissolved in dichloromethane (3 mL) and trifluoroacetic acid (0.5 mL) at room temperature. The reaction mixture was stirred and reacted at room temperature for 1 hour. After the reaction was completed, the mixture was concentrated under reduced pressure to remove the solvent to obtain the trifluoroacetate of compound **WX013-4.** MS-ESI *m/z:* 410.3 [M+H]$^+$.

Step **5:** Synthesis of compound **WX013-5**

Compound **BB-2** (0.1 g, 171.05 μmol) and the trifluoroacetate of compound **WX013-4** (107.45 mg, 205.26 μmol) were dissolved in N, N-dimethylformamide (3 mL) at room temperature under nitrogen atmosphere. Potassium acetate (50.36 mg, 513.15 μmol) and acetic acid (5.14 mg, 85.53 μmol) were added thereto. The reaction mixture was stirred and reacted at room temperature for 2 hours. Sodium triacetoxyborohydride (145.01 mg, 684.20 μmol) was added thereto. The reaction mixture was further stirred and reacted for 1 hour. After the reaction was completed, the mixture was added with 1 N hydrochloric acid solution (1 mL), and concentrated under reduced pressure to remove the solvent. The resulting residue was separated by preparative high performance liquid chromatography (chromatographic column: Phenomenex Luna C18 80 * 40 mm * 3 μm; mobile phase: water (0.04% hydrochloric acid)-acetonitrile; acetonitrile%; 40% to 60%, 7 minutes) to obtain compound **WX013-5.** MS-ESI *m/z:* 978.5 [M+H]$^+$. $^1$H NMR (400 MHz, $CDCl_3$) δ: 9.06 (s, 1H), 8.40 (d, *J*=5.6 Hz, 2H), 8.41 (s, 1H), 8.33 (s, 1H), 8.29 (t, *J*=4.8 Hz, 2H), 7.92 (d, *J*=8.4 Hz, 1H), 7.78 (d, *J*=9.2 Hz, 1H), 7.65-7.57 (m, 2H), 7.44 (d, *J*=7.2 Hz, 1H), 7.00-6.65 (m, 1H), 4.82-4.73 (m, 1H), 4.09-4.00 (m, 1H), 3.94 (d, *J*=6.8 Hz, 2H), 3.52 (t, *J*=5.6 Hz, 4H), 3.30-3.16 (m, 3H), 2.96-2.85 (m, 2H), 2.81 (s, 3H), 2.78-2.70 (m, 2H), 2.62-2.50 (m, 1H), 2.26-2.12 (m, 4H), 1.93-1.75 (m, 5H), 1.73-1.62 (m, 4H), 1.58 (s, 9H), 1.26-1.15 (m, 3H), 0.47-0.40 (m, 2H), 0.31-0.25 (m, 2H).

Step **6:** Synthesis of hydrochloride of compound **WX013**

Compound **WX013-5** (50 mg, 51.12 μmol) was dissolved in dichloromethane (2 mL) and trifluoroacetic acid (2 mL) at room temperature under nitrogen atmosphere. The reaction mixture was stirred and reacted at room temperature for 1.5 hours. After the reaction was completed, the mixture was concentrated under reduced pressure to remove the solvent. The resulting residue was separated by preparative high performance liquid chromatography (chromatographic column: Phenomenex Luna C18 80 * 40 mm * 3 μm; mobile phase: water (0.04% hydrochloric acid)-acetonitrile; acetonitrile%: 14% to 34%, 7 minutes) to obtain the hydrochloride of the target compound **WX013.** MS-ESI m/z: 878.4 [M+H]$^+$. $^1$H NMR (400 MHz, MeOD_$d_4$) δ: 8.80 (s, 1H), 8.40 (d, *J*=9.2 Hz, 1H), 8.17 (s, 1H), 8.11 (d, *J*=8.4 Hz, 1H), 7.97 (d, *J*=6.8 Hz, 1H), 7.81 (d, *J*=9.6 Hz, 1H), 7.72 (d, *J*=1.2 Hz, 1H), 7.64 (t, *J*=7.8 Hz, 1H), 7.49 (d, *J*=6.8 Hz, 1H), 7.45 (dd, *J*=1.4 Hz, 6.6 Hz, 1H), 6.91 (t, *J*=54.6 Hz, 1H), 4.97 (dd, *J*=4.8 Hz, 10.0 Hz, 1H), 4.19-4.07 (m, 1H), 3.62-3.55 (m, 4H), 3.27-3.08 (m, 4H), 3.04-2.94 (m, 1H), 2.89 (s, 3H), 2.87-2.59 (m, 4H), 2.54-2.45 (m, 1H), 2.17-1.77 (m, 13H), 1.30-1.15 (m, 3H), 0.74-0.68 (m, 2H), 0.44-0.38 (m, 2H).

**Example 14**

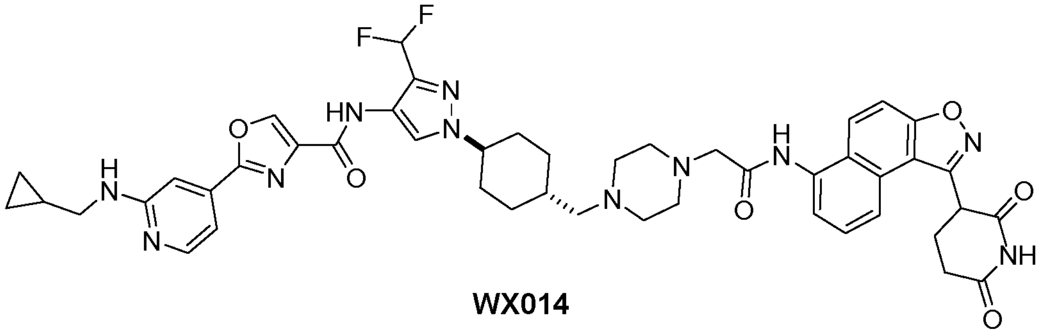

WX014

Synthetic route:

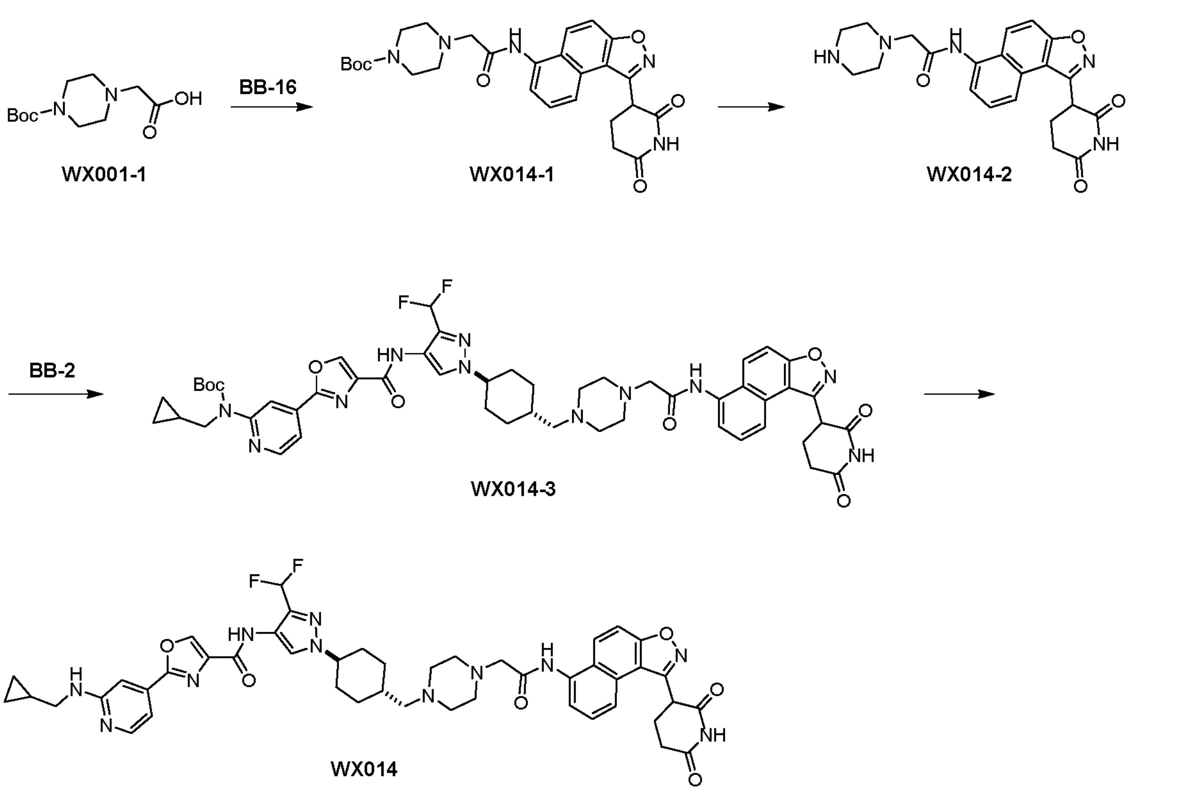


WX001-1 WX014-1 WX014-2 WX014-3 WX014

Step **1:** Synthesis of compound **WX014-1**

Compound **WX001-1** (93.09 mg, 331.57 μmol) was dissolved in N, N-dimethylformamide (2 mL) at room temperature under nitrogen atmosphere. N, N-Diisopropylethylamine (194.78 mg, 1.51 mmol) and 2-(7-azabenzotriazol)-N, N, N', N'-tetramethyluronium hexafluorophosphate (171.92 mg, 452.14 μmol) were added thereto. The reaction mixture was stirred and reacted at room temperature for 0.5 hours. The hydrochloride of compound **BB-16** (0.1 g, 301.43 μmol) was added thereto. The reaction mixture was stirred and reacted at room temperature for 11.5 hours. After the reaction was completed, the mixture was added with water (20 mL), and extracted with ethyl acetate (3 × 20 mL). The organic phases were combined, washed with 10% brine (2×30 mL), dried over anhydrous sodium sulfate, and filtered. The filtrate was concentrated under reduced pressure to remove the solvent. The resulting residue was separated by column chromatography (eluent: petroleum ether/ethyl acetate = 3/1 to 1/1, v/v) to obtain compound **WX014-1.** MS-ESI *m/z:* 522.3 $[M+H]^+$.

Step **2:** Synthesis of hydrochloride of compound **WX014-2**

Compound **WX014-1** (0.1 g, 191.73 μmol) was dissolved in dichloromethane (1 mL) at room temperature, and

a solution of hydrochloric acid in ethyl acetate (4 M, 2 mL) was added thereto. The reaction mixture was stirred and reacted at room temperature for 12 hours. After the reaction was completed, the mixture was concentrated under reduced pressure to remove the solvent to obtain the hydrochloride of compound **WX014-2.** MS-ESI *m/z:* 422.2 $[M+H]^+$. $^1H$ NMR (400 MHz, DMSO_$d_6$) δ: 11.15 (s, 1H), 10.75 (s, 1H), 9.58 (s, 1H), 8.36 (d, *J*=9.2 Hz, 1H), 8.16 (d, *J*=7.2 Hz, 1H), 8.00 (d, *J*=9.6 Hz, 1H), 7.76 (t, *J*=7.8 Hz, 1H), 7.71 (d, *J*=6.8 Hz, 1H), 5.10 (dd, *J*=4.6, 11.4 Hz, 1H), 4.35-4.10 (m, 2H), 3.41-3.34 (m, 6H), 2.92-2.80 (m, 2H), 2.70-2.66 (m, 1H), 2.65-2.61 (m, 1H), 2.60-2.56 (m, 1H), 2.44-2.36 (m, 1H).

Step **3:** Synthesis of compound **WX014-3**

Compound **BB-2** (0.1 g, 171.05 μmol) and the hydrochloride of compound **WX014-2** (78.33 mg, 171.05 μmol) were dissolved in tetrahydrofuran (2 mL) and N, N-dimethylformamide (0.4 mL) at room temperature under nitrogen atmosphere, and potassium acetate (50.36 mg, 513.16 μmol) and acetic acid (5.14 mg, 85.53 μmol) were added thereto. The reaction mixture was stirred and reacted at room temperature for 2 hours. Sodium triacetoxyborohydride (145.01 mg, 684.21 μmol) was added thereto. The mixture was further stirred and reacted for 10 hours. After the reaction was completed, the mixture was added with a saturated solution of ammonium chloride (20 mL), and extracted with ethyl acetate (3 × 20 mL). The organic phases were combined, washed with 10% brine (2 × 30 mL), dried over anhydrous sodium sulfate, and filtered. The filtrate was concentrated under reduced pressure to remove the solvent to obtain compound **WX014-3.** MS-ESI *m/z:* 990.6 $[M+H]^+$.

Step **4:** Synthesis of hydrochloride of compound **WX014**

Compound **WX014-3** (0.2 g, 202.01 μmol) was dissolved in dichloromethane (7 mL) and trifluoroacetic acid (7 mL) at room temperature under nitrogen atmosphere. The reaction mixture was stirred and reacted at room temperature for 2 hours. After the reaction was completed, the mixture was concentrated under reduced pressure to remove the solvent. The resulting residue was separated by preparative high performance liquid chromatography (chromatographic column: Phenomenex Luna C18 80 * 40 mm * 3 μm; mobile phase: water (0.04% hydrochloric acid)-acetonitrile; acetonitrile%: 12% to 32%, 7 minutes) to obtain the hydrochloride of the target compound **WX014.** MS-ESI *m/z*: 890.5 $[M+H]^+$. $^1H$ NMR (400 MHz, MeOD_$d_4$) δ: 8.79 (s, 1H), 8.29 (d, *J*=9.6 Hz, 1H), 8.26 (s, 1H), 8.19 (dd, *J*=1.2 Hz, 6.8 Hz, 1H), 7.97 (d, *J*=6.8 Hz, 1H), 7.86 (d, *J*=9.2 Hz, 1H), 7.79-7.69 (m, 3H), 7.45 (dd, *J*=1.2 Hz, 6.8 Hz, 1H), 6.94 (t, *J*=54.6 Hz, 1H), 5.00 (dd, J=5.0 Hz, 10.2 Hz, 1H), 4.30-4.23 (m, 1H), 4.21 (s, 2H), 3.86-3.54 (m, 8H), 3.20 (d, *J*=6.4 Hz, 2H), 2.94-2.63 (m, 4H), 2.58-2.47 (m, 1H), 2.22 (d, *J*=11.6 Hz, 2H), 2.11 (d, *J*=12.0 Hz, 2H), 2.05-1.88 (m, 3H), 1.42-1.30 (m, 2H), 1.27-1.16 (m, 1H), 0.75-0.68 (m, 2H), 0.45-0.38 (m, 2H).

**Example 15**

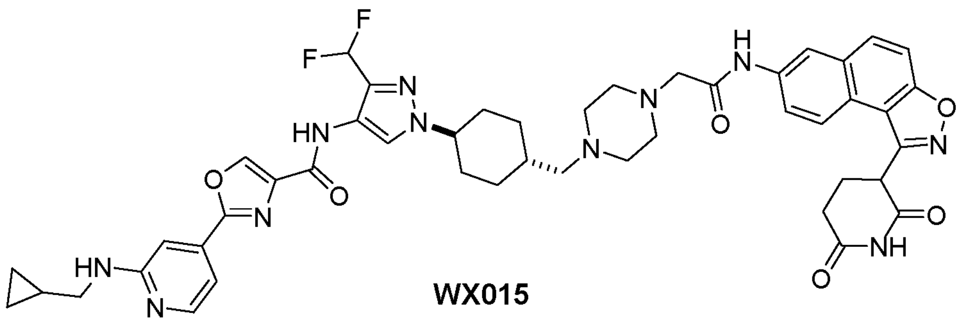

**WX015**

Synthetic route:

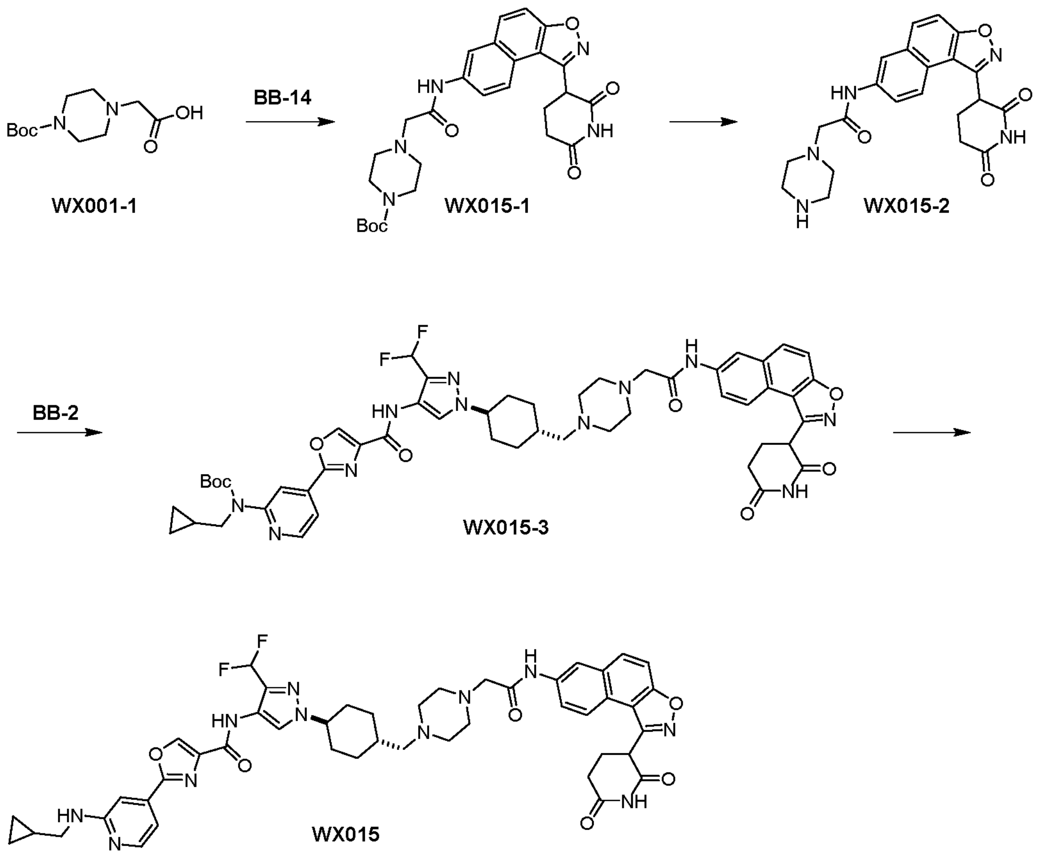


WX001-1 WX015-1 WX015-2

WX015-3

WX015

Step **1:** Synthesis of compound **WX015-1**

Compound **WX001-1** (93.09 mg, 331.57 μmol) was dissolved in N, N-dimethylformamide (2 mL) at room temperature under nitrogen atmosphere. N, N-diisopropylethylamine (194.78 mg, 1.51 mmol) and 2-(7-azabenzotriazol)-N, N, N', N'-tetramethyluronium hexafluorophosphate (171.92 mg, 452.14 μmol) were added thereto. The reaction mixture was stirred and reacted at room temperature for 0.5 hours. The hydrochloride of compound **BB-14** (0.1 g, 301.43 μmol) was added thereto. The reaction mixture was further reacted at room temperature for 1.5 hours. After the reaction was completed, the mixture was added with water (20 mL), and extracted with ethyl acetate (4 × 20 mL). The organic phases were combined, washed with 10% brine (2 × 30 mL), dried over anhydrous sodium sulfate, and filtered. The filtrate was concentrated under reduced pressure to remove the solvent to obtain compound **WX015-1.** MS-ESI *m/z:* 522.3 $[M+H]^+$.

Step **2:** Synthesis of hydrochloride of compound **WX015-2**

Compound **WX015-1** (155 mg, 297.18 μmol) was dissolved in dichloromethane (3 mL) at room temperature. A solution of hydrochloric acid in ethyl acetate (4 M, 5 mL) was added thereto. The reaction mixture was stirred and reacted at room temperature for 12 hours, then heated to 40°C, and stirred and reacted for 2 hours. After the reaction was completed, the mixture was concentrated under reduced pressure to remove the solvent to obtain the hydrochloride of compound **WX015-2.** MS-ESI *m/z:* 422.2 $[M+H]^+$.

Step **3:** Synthesis of compound **WX015-3**

Compound **BB-2** (0.1 g, 171.05 μmol) and the hydrochloride of compound **WX015-2** (78.33 mg, 171.05 μmol) were dissolved in N, N-dimethylformamide (3 mL) at room temperature under nitrogen atmosphere. Potassium acetate (50.36 mg, 513.15 μmol) and acetic acid (5.14 mg, 85.53 μmol) were added thereto. The reaction mixture was stirred and reacted at room temperature for 2 hours. Sodium triacetoxyborohydride (145.01 mg, 684.20 μmol) was added thereto. The reaction mixture was further reacted for 10 hours. After the reaction was completed, the mixture was added with a saturated solution of ammonium chloride (20 mL), and extracted with ethyl acetate (4 × 20 mL). The organic phases were combined, washed with 10% brine (2 × 30 mL), dried over anhydrous sodium sulfate, and filtered. The filtrate was concentrated under reduced pressure to remove the solvent to obtain compound **WX015-3.** MS-ESI *m/z:*

990.6 [M+H]+.

Step **4:** Synthesis of hydrochloride of compound **WX015**

Compound **WX015-3** (160 mg, 161.61 μmol) was dissolved in dichloromethane (6 mL) and trifluoroacetic acid (6 mL) at room temperature. The reaction mixture was stirred and reacted at room temperature for 2 hours. After the reaction was completed, the mixture was concentrated under reduced pressure to remove the solvent. The resulting residue was separated by preparative high performance liquid chromatography (chromatographic column: Phenomenex Luna C18 80 * 40 mm * 3 μm; mobile phase: water (0.04% hydrochloric acid)-acetonitrile; acetonitrile%: 14% to 34%, 7 minutes) to obtain the hydrochloric acid of the target compound **WX015.** MS-ESI *m/z:* 890.4 [M+H]+. $^1$H NMR (400 MHz, MeOD_$d_4$) δ: 8.79 (s, 1H), 8.42 (d, *J*=2.0 Hz, 1H), 8.26 (s, 1H), 8.19 (d, *J*=8.8 Hz, 1H), 8.01 (d, *J*=9.2 Hz, 1H), 7.96 (d, J=6.8 Hz, 1H), 7.88 (dd, *J*=2.0 Hz, 8.8 Hz, 1H), 7.77 (d, *J*=9.2 Hz, 1H), 7.69 (s, 1H), 7.43 (dd, *J*=1.6 Hz, 6.8 Hz, 1H), 6.93 (t, *J*=54.4 Hz, 1H), 4.95 (dd, J=5.0 Hz, 10.2 Hz, 1H), 4.32-4.19 (m, 1H), 4.05 (s, 2H), 3.88-3.49 (m, 8H), 3.29 (s, 1H), 3.21 (d, *J*=6.4 Hz, 2H), 2.95-2.74 (m, 2H), 2.73-2.61 (m, 1H), 2.58-2.45 (m, 1H), 2.22 (d, *J*=10.4 Hz, 2H), 2.12 (d, *J*=12.8 Hz, 2H), 2.07-1.70 (m, 4H), 1.45-1.30 (m, 2H), 1.29-1.17 (m, 1H), 0.75-0.67 (m, 2H), 0.44-0.37 (m, 2H).

**Example 16**

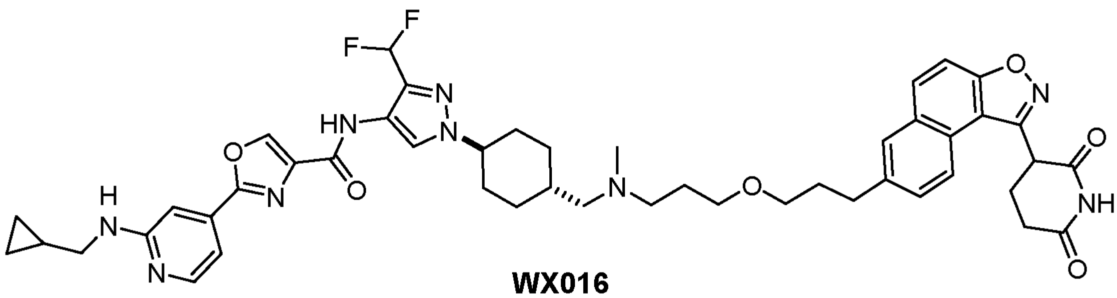

**WX016**

Synthetic route:

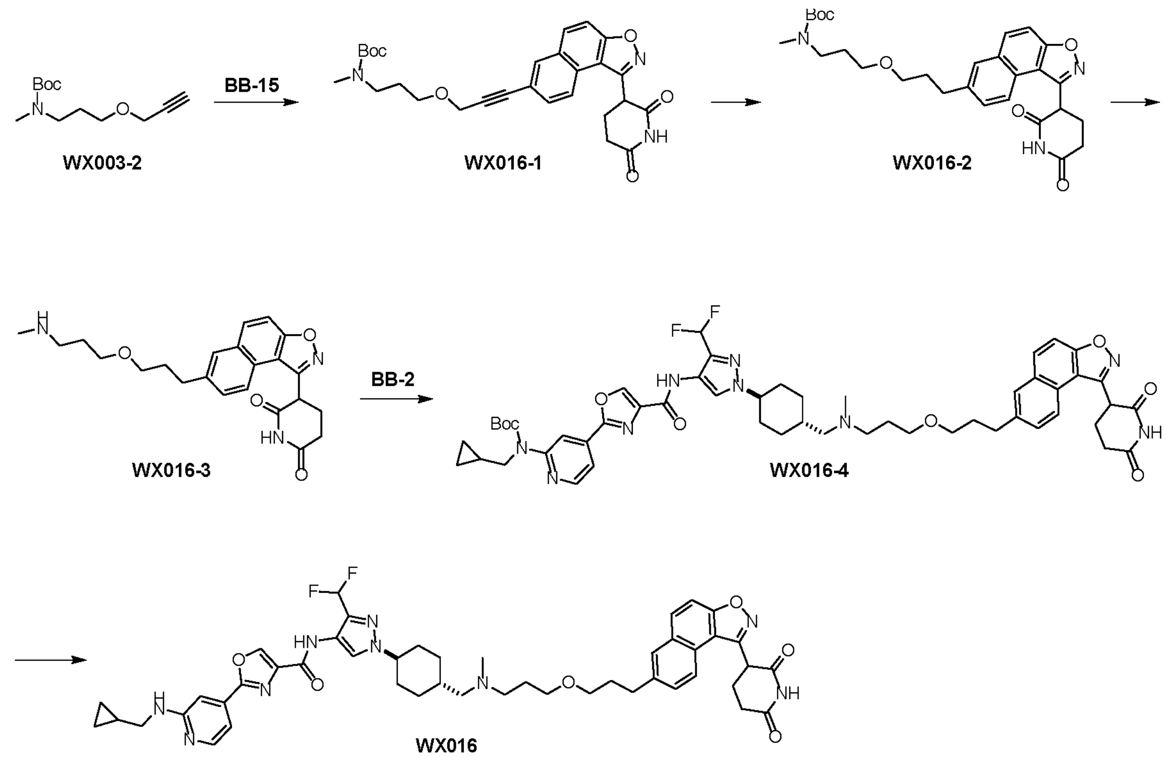

WX003-2 WX016-1 WX016-2 WX016-3 WX016-4 WX016

Step **1:** Synthesis of compound **WX016-1**

Compound **BB-15** (0.2 g, 556.83 μmol) and compound **WX003-2** (164.54 mg, 723.88 μmol) were dissolved in dimethyl sulfoxide (5 mL) at room temperature under nitrogen atmosphere. Bis(triphenylphosphine)palladium(II) chloride (78.17 mg, 111.37 μmol), cuprous iodide (21.21 mg, 111.37 μmol), and N, N-diisopropylethylamine (143.93 mg, 1.11 mmol) were added thereto. The reaction mixture was heated to 85°C, and stirred and reacted for 2 hours. After the reaction was completed, the mixture was cooled to room temperature, added with water (30 mL), and extracted with ethyl acetate (3 × 30 mL). The organic phases were combined, washed with 10% brine (3 × 40 mL), dried over anhydrous sodium sulfate, and filtered. The filtrate was concentrated under reduced pressure to remove the solvent. The resulting residue was separated by column chromatography (eluent: petroleum ether/ethyl acetate = 3/1 to 1/1, v/v) to obtain compound **WX016-1.** MS-ESI *m/z:* 406.2 [M+H-100]$^+$. $^1$H NMR (400 MHz, $CDCl_3$) δ: 8.18 (s, 1H), 8.13 (d, *J*=1.2 Hz, 1H), 7.97 (t, *J*=9.2 Hz, 2H), 7.75 (d, *J*=8.8 Hz, 1H), 7.71 (dd, *J*=1.6 Hz, 8.4 Hz, 1H), 4.72 (dd, *J*=5.2 Hz, 8.4 Hz, 1H), 4.43 (s, 2H), 3.65 (t, *J*=6.4 Hz, 2H), 3.35 (t, *J*=6.8 Hz, 2H), 2.89 (s, 3H), 2.84-2.68 (m, 3H), 2.65-2.53 (m, 1H), 1.93-1.85 (m, 2H), 1.47 (s, 9H).

Step **2:** Synthesis of compound **WX016-2**

Wet palladium on carbon (20 mg, purity of 10%) and palladium hydroxide/carbon (20 mg, purity of 20%) were dissolved in tetrahydrofuran (3 mL) at room temperature. Compound **WX016-1** (0.1 g, 197.80 μmol) and triethylamine (20.02 mg, 197.80 μmol) were added thereto. The reaction mixture was stirred and reacted at room temperature under hydrogen (15 psi) atmosphere for 12 hours. After the reaction was completed, the mixture was filtered. The filtrate was concentrated under reduced pressure to remove the solvent. The resulting residue was purified by a chromatography plate (developing solvent: petroleum ether/ethyl acetate= 1/ 1) to obtain compound **WX016-2.** MS-ESI *m/z:* 410.2 [M+H-100]$^+$. $^1$H NMR (400 MHz, $CDCl_3$) δ: 8.20 (s, 1H), 7.96 (d, *J*=2.4 Hz, 1H), 7.94 (d, *J*=2.4 Hz, 1H), 7.82 (s, 1H), 7.70 (d, *J*=8.8 Hz, 1H), 7.55 (dd, *J*=1.6 Hz, 8.4 Hz, 1H), 4.75 (dd, *J*=4.8 Hz, 8.4 Hz, 1H), 3.51-3.41 (m, 4H), 3.36-3.26 (m, 2H), 2.96-2.88 (m, 3H), 2.87 (s, 3H), 2.83-2.66 (m, 2H), 2.63-2.51 (m, 1H), 2.04-1.94 (m, 2H), 1.87-1.77 (m, 2H), 1.46 (s, 9H).

Step **3:** Synthesis of trifluoroacetate of compound **WX016-3**

Compound **WX016-2** (70 mg, 137.36 μmol) was dissolved in dichloromethane (3 mL) and trifluoroacetic acid (0.5 mL) at room temperature. The reaction mixture was stirred and reacted at room temperature for 40 minutes. After the reaction was completed, the mixture was concentrated under reduced pressure to remove the solvent to obtain the trifluoroacetate of compound **WX016-3.** MS-ESI *m/z:* 410.2 [M+H]$^+$.

Step **4:** Synthesis of compound **WX016-4**

Compound **BB-2** (70 mg, 119.74 μmol) and the trifluoroacetate of compound **WX016-3** (68.95 mg, 131.71 μmol) were dissolved in N, N-dimethylformamide (4 mL) at room temperature under nitrogen atmosphere. Potassium acetate (35.25 mg, 359.21 μmol) and acetic acid (3.60 mg, 59.87 μmol) were added thereto. The reaction mixture was stirred and reacted at room temperature for 2 hours. Sodium triacetoxyborohydride (101.51 mg, 478.95 μmol, 4 *eq*) was added thereto. The reaction mixture was further stirred and reacted for 10 hours. After the reaction was completed, the mixture was added with a saturated solution of ammonium chloride (20 mL), and extracted with ethyl acetate (2 × 20 mL). The organic phases were combined, washed with 10% brine (2 × 30 mL), dried over anhydrous sodium sulfate, and filtered. The filtrate was concentrated under reduced pressure to remove the solvent to obtain compound **WX016-4.** MS-ESI *m/z*: 978.4 $[M+H]^+$.

Step **5:** Synthesis of hydrochloride of compound **WX016**

Compound **WX016-4** (110 mg, 112.46 μmol) was dissolved in dichloromethane (4 mL) and trifluoroacetic acid (4 mL) at room temperature under nitrogen atmosphere. The reaction mixture was stirred and reacted at room temperature for 2 hours. After the reaction was completed, the mixture was concentrated under reduced pressure to remove the solvent. The resulting residue was separated by preparative high performance liquid chromatography (chromatographic column: Phenomenex Luna C18 80 × 40 mm × 3 μm; mobile phase: water (0.04% hydrochloric acid) -acetonitrile; acetonitrile%: 13% to 30%, 7 minutes) to obtain the hydrochloride of the target compound **WX016.** MS-ESI m/z: 878.5 $[M+H]^+$. $^1H$ NMR (400 MHz, MeOD_$d_4$) δ: 8.77 (s, 1H), 8.13 (d, *J*=8.0 Hz, 1H), 8.11 (s, 1H), 8.02 (d, *J*=9.2 Hz, 1H), 7.99-7.95 (m, 1H), 7.89 (s, 1H), 7.75-7.68 (m, 2H), 7.61 (d, *J*=8.0 Hz, 1H), 7.46-7.42 (m, 1H), 6.89 (t, *J*=54.4 Hz, 1H), 4.93 (dd, *J*=5.2 Hz,10.4 Hz, 1H), 4.21-4.09 (m, 1H), 3.62-3.53 (m, 3H), 3.17-3.02 (m, 2H), 2.93 (t, *J*=7.4 Hz, 3H), 2.86 (s, 3H), 2.85-2.72 (m, 2H), 2.70-2.58 (m, 1H), 2.57-2.43 (m, 1H), 2.23-1.76 (m, 13H), 1.34-1.14 (m, 4H), 0.74-0.68 (m, 2H), 0.44-0.39 (m, 2H).

**Example 17**

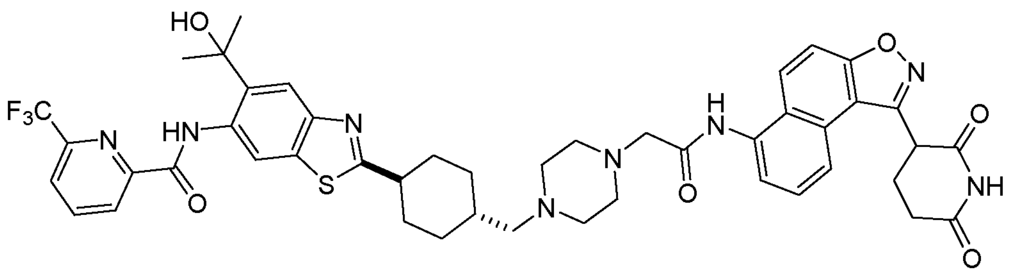

**WX017**

Synthetic route:

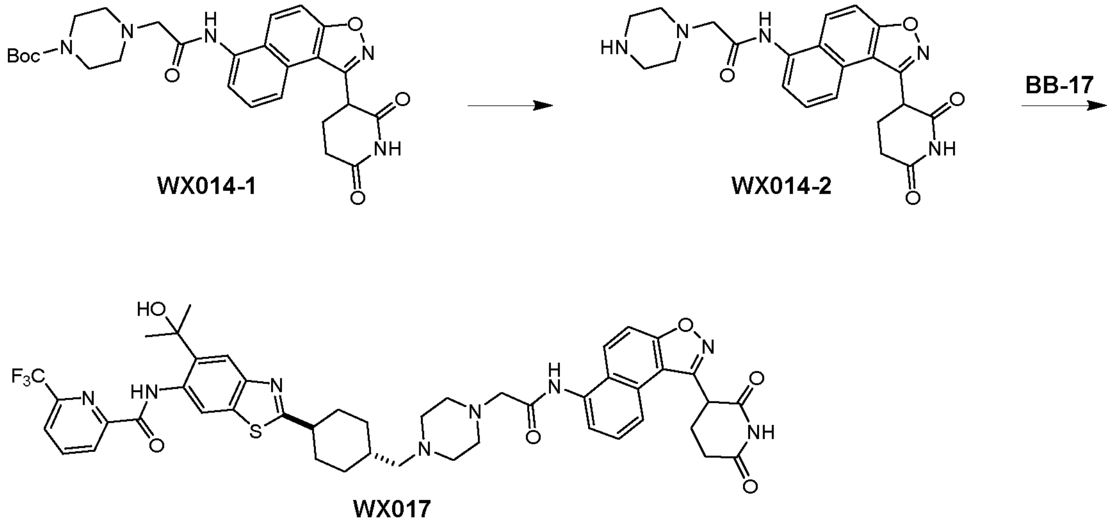


WX014-1

WX014-2

WX017

Step **1:** Synthesis of trifluoroacetate of compound **WX014-2**

Compound **WX014-1** (100 mg, 191.73 μmol) was dissolved in dichloromethane (1 mL) at room temperature under nitrogen atmosphere, then trifluoroacetic acid (308.00 mg, 2.70 mmol, 0.2 mL) was added thereto. The reaction mixture was stirred and reacted at room temperature for 12 hours. After the reaction was completed, the reaction mixture was concentrated under reduced pressure to obtain the trifluoroacetate of compound **WX014-2.** MS-ESI *m/z:* 422.1 $[M+H]^+$.

Step **2:** Synthesis of formate of compound **WX017**

The trifluoroacetate of compound **WX014-2** (100 mg, 186.75 μmol) was dissolved in acetonitrile (2 mL) at room temperature under nitrogen atmosphere, then N, N-diisopropylethylamine (120.68 mg, 933.76 μmol, 162.64 μL) and compound **BB-17** (112 mg, 167.01 μmol, purity of 89.98%) were added thereto. The reaction mixture was heated to 90°C, and stirred and reacted for 12 hours. After the reaction was completed, the reaction mixture was concentrated under reduced pressure to remove the solvent. The residue was separated by preparative high performance liquid chromatography (chromatographic column: Phenomenex Luna C18 75 * 30 mm * 3 μm; mobile phase: water (0.2% formic acid)-acetonitrile; acetonitrile%: 30% to 60%, 8 minutes) to obtain the formate of the target compound **WX017.** MS-ESI *m/z:* 897.0 $[M+H]^+$. $^1H$ NMR (400 MHz, DMSO_$d_6$) δ: 12.55 (s, 1H), 11.14 (s, 1H), 10.13 (s, 1H), 9.07 (s, 1H), 8.46 (t, *J*=7.6 Hz, 1H), 8.38 (t, *J*=8.0 Hz, 1H), 8.23-8.16 (m, 2H), 8.15-8.08 (m, 1H), 8.01 (d, *J*=9.2 Hz, 1H), 7.89 (s, 1H), 7.79 (d, *J*=7.6 Hz, 1H), 7.72 (t, *J*=8.0 Hz, 1H), 6.08 (s, 1H), 5.09 (dd, *J*=4.8 Hz, 11.6 Hz, 1H), 3.30-3.23 (m, 2H), 3.13-3.02 (m, 1H), 2.92-2.79 (m, 1H), 2.78-2.51 (m, 10H), 2.45-2.34 (m, 2H), 2.32-2.25 (m, 1H), 2.19 (d, *J*=10.8 Hz, 2H), 1.94 (d, *J*=10.8 Hz, 2H), 1.70-1.55 (m, 9H), 1.18-1.02 (m, 2H).

**Example 18**

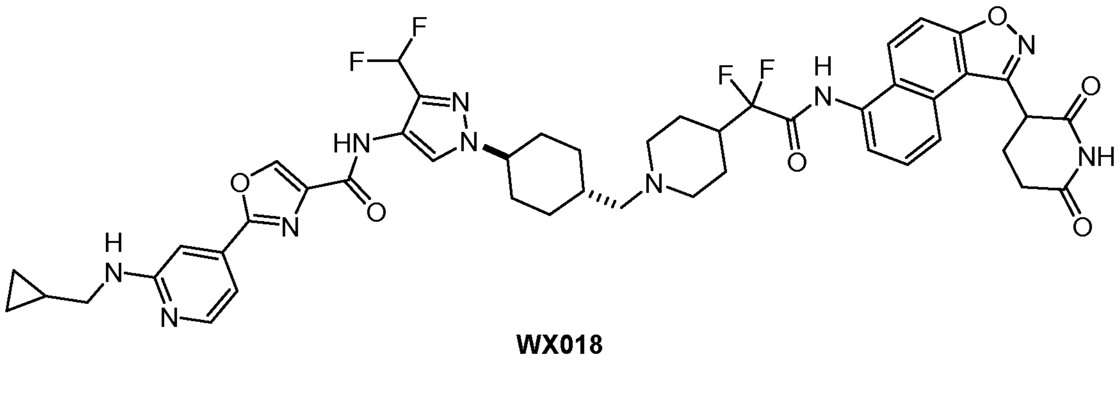

**WX018**

Synthetic route:

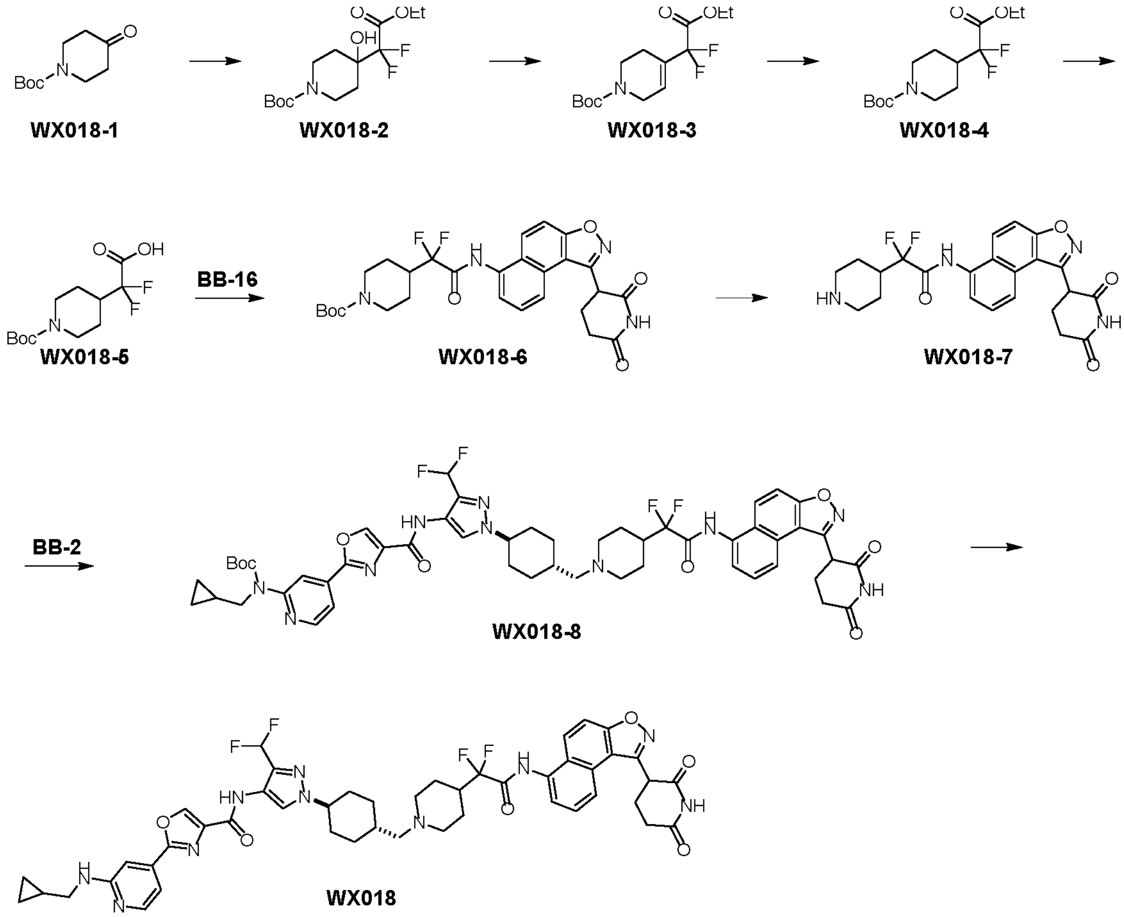


WX018-1 WX018-2 WX018-3 WX018-4 WX018-5 WX018-6 WX018-7 WX018-8 WX018

Step **1:** Synthesis of compound **WX018-2**

Titanocene dichloride (1.30 g, 5.02 mmol) and zinc powder (13.31 g, 203.55 mmol) were added to a dried reaction flask at room temperature under nitrogen atmosphere. Tetrahydrofuran (100 mL) was added for dissolution. A solution of ethyl bromodifluoroacetate (20.37 g, 100.38 mmol, 12.90 mL) in tetrahydrofuran (20 mL) was added dropwise thereto (first adding 1/10 of the solution, after the reaction was initiated (with a noticeable increase in temperature), then adding the remaining solution dropwise). After the dropwise addition was completed, the reaction mixture was further stirred for 0.5 hours, and filtered. The filtrate was added to a solution of compound **WX018-1** (10 g, 50.19 mmol) in tetrahydrofuran (50 mL), and stirred and reacted for 12 hours. After the reaction was completed, the mixture was added with 1 M hydrochloric acid to adjust the pH to 3, then added with water (100 mL), and extracted with ethyl acetate (100 mL × 3). The organic phase was washed with saturated brine (50 mL × 3), dried over anhydrous sodium sulfate, and filtered. The filtrate was concentrated under reduced pressure. The resulting residue was subjected to column chromatography (eluent: petroleum ether/ethyl acetate = 0/1 to 1/0, v/v) to obtain compound **WX018-2.** MS-ESI m/z: 268.0 $[M+H-56]^+$. $^1H$ NMR (400 MHz, $CDCl_3$) δ: 4.37 (q, *J*=7.2 Hz, 2H), 4.20-3.92 (m, 2H), 3.17-2.94 (m, 2H), 2.33 (s, 1H), 1.87-1.74 (m, 2H), 1.72-1.65 (m, 2H), 1.47 (s, 9H), 1.38 (t, *J*=7.2 Hz, 3H).

Step **2:** Synthesis of compound **WX018-3**

Compound **WX018-2** (1 g, 3.09 mmol), thionyl chloride (2.21 g, 18.56 mmol), and pyridine (1.47 g, 18.56 mmol) were added to dioxane (15 mL) at room temperature under nitrogen atmosphere. The reaction mixture was stirred at room temperature for 1.5 hours, then 4-dimethylaminopyridine (37.78 mg, 309.28 μmol) was added thereto. The reaction mixture was further stirred and reacted for 12 hours. After the reaction was completed, the reaction mixture was added with water (20 mL), and extracted with ethyl acetate (30 mL × 3). The organic phase was washed with saturated brine

(20 mL × 3), dried over anhydrous sodium sulfate, and filtered. The filtrate was concentrated under reduced pressure to remove the solvent. The resulting residue was subjected to column chromatography (eluent: petroleum ether/ethyl acetate = 20/1 to 9/1, v/v) to obtain compound **WX018-3.** MS-ESI *m/z:* 206.2 $[M+H-100]^+$. $^1H$ NMR (400 MHz, $CDCl_3$) δ: 6.18 (s, 1H), 4.34 (q, *J*=7.0 Hz, 2H), 4.02 (d, *J*=2.0 Hz, 2H), 3.54 (t, *J*=5.6 Hz, 2H), 2.25 (s, 2H), 1.48 (s, 9H), 1.36 (t, *J*=7.2 Hz, 3H)

Step **3:** Synthesis of compound **WX018-4**

Wet palladium hydroxide (0.5 g, 712.07 μmol, purity of 20 % ) was added to a hydrogenation flask at room temperature under argon atmosphere, then methanol (20 mL) and compound **WX018-3** (1.5 g, 4.91 mmol) were added thereto. The atmosphere was replaced with hydrogen three times, and the reaction mixture was stirred and reacted at room temperature and at 40 psi for 12 hours. After the reaction was completed, the mixture was filtered. The filter cake was rinsed with methanol (50 mL × 4). The filtrate was collected and concentrated under reduced pressure to remove the remaining solvent. The resulting residue was subjected to column chromatography (eluent: petroleum ether/ethyl acetate = 20/1 to 10/1, v/v) to obtain compound **WX018-4.** MS-ESI *m/z*: 252.2 $[M+H-56]^+$. $^1H$ NMR (400 MHz, $CDCl_3$) δ: 4.35 (q, *J*=7.0 Hz, 2H), 4.30-4.15 (m, 2H), 2.68 (t, *J*=12.8 Hz, 2H), 2.32-2.14 (m, 1H), 1.73 (d, *J*=12.8 Hz, 2H), 1.49 (d, *J*=4.8 Hz, 1H), 1.47 (s, 9H), 1.43 (d, *J*=4.4 Hz, 1H), 1.37 (t, *J*=7.0 Hz, 3H).

Step **4:** Synthesis of compound **WX018-5**

Compound **WX018-4** (0.26 g, 845.99 μmol) was dissolved in a mixed solvent of ethanol (5 mL) and water (2 mL) at room temperature under nitrogen atmosphere, then lithium hydroxide monohydrate (71.00 mg, 1.69 mmol) was added thereto. The reaction mixture was stirred and reacted at room temperature for 12 hours. After the reaction was completed, the mixture was concentrated under reduced pressure. The residue was added with 1 M hydrochloric acid to adjust the pH to 2 to 3, resulting in the precipitation of solid. The mixture was filtered. The filter cake was collected, and dried under vacuum to obtain compound **WX018-5.** MS-ESI *m/z:* 224.0 $[M+H-56]^+$. $^1H$ NMR (400 MHz, DMSO_$d_6$) δ: 4.05-3.94 (m, 2H), 2.80-2.63 (m, 2H), 2.37-2.21 (m, 1H), 1.71-1.61 (m, 2H), 1.39 (s, 9H), 1.27-1.12 (m, 2H).

Step **5:** Synthesis of compound **WX018-6**

Compound **WX018-5** (101.02 mg, 361.71 μmol) was dissolved in N, N-dimethylformamide (3 mL) at room temperature under nitrogen atmosphere, and 2-(7-azabenzotriazol)-N, N, N', N'-tetramethyluronium hexafluorophosphate (229.22 mg, 602.86 μmol) and N, N-diisopropylethylamine (233.74 mg, 1.81 mmol, 315.01 μL) were added thereto. The reaction mixture was stirred for 0.5 hours. The hydrochloride of compound **BB-16** (0.1 g, 301.43 μmol) was added thereto. The reaction mixture was further stirred and reacted at room temperature for 12 hours. After the reaction was completed, the reaction mixture was added with water (20 mL), resulting in the precipitation of solid. The mixture was filtered. The solid was collected, and dried under vacuum to obtain compound **WX018-6.** MS-ESI *m/z*: 457.1 $[M+H-100]^+$. $^1H$ NMR (400 MHz, DMSO_$d_6$) δ: 11.15 (s, 1H), 11.02 (s, 1H), 8.31-8.18 (m, 1H), 8.05 (q, *J*=9.2 Hz, 2H), 7.83-7.71 (m, 1H), 7.58 (d, *J*=7.6 Hz, 1H), 5.18-5.04 (m, 1H), 4.15-4.04 (m, 2H), 2.90-2.80 (m, 2H), 2.74-2.61 (m, 4H), 2.44-2.34 (m, 2H), 1.42 (s, 9H), 1.18-1.10 (m, 3H).

Step **6:** Synthesis of hydrochloride of compound **WX018-7**

Compound **WX018-6** (110 mg, 197.64 μmol) was dissolved in ethyl acetate (2 mL) at room temperature. A solution of hydrochloric acid in ethyl acetate (3 mL, 4 M) was added thereto. The reaction mixture was stirred and reacted for 2 hours. After the reaction was completed, the mixture was filtered. The filter cake was rinsed with ethyl acetate (5 mL× 3), collected, and dried under vacuum to obtain the hydrochloride of compound **WX018-7.** MS-ESI *m/z:* 457.2 $[M+H]^+$.

Step **7:** Synthesis of compound **WX018-8**

The hydrochloride of compound **WX018-7** (97 mg, 196.79 μmol) was dissolved in tetrahydrofuran (5 mL) and N, N-dimethylformamide (1 mL) at room temperature under nitrogen atmosphere. Compound **BB-2** (115.05 mg, 196.79 μmol), potassium acetate (57.94 mg, 590.38 μmol), and glacial acetic acid (5.91 mg, 98.40 μmol, 5.63 μL) were added thereto. The reaction mixture was stirred for 2 hours, then sodium triacetoxyborohydride (166.83 mg, 787.17 μmol) was added thereto. The reaction mixture was further stirred and reacted for 1 hour. After the reaction was completed, the reaction mixture was added with a saturated aqueous solution of ammonium chloride (20 mL), and extracted with ethyl acetate (30 mL × 3). The organic phase was washed with saturated brine (30 mL × 2), dried over anhydrous sodium

sulfate, and filtered. The filtrate was concentrated under reduced pressure to remove the solvent to obtain compound **WX018-8.** MS-ESI *m/z*: 1025.4 $[M+H]^+$.

Step **8:** Synthesis of hydrochloride of compound **WX018**

Compound **WX018-8** (0.2 g, 195.11 μmol) was dissolved in ethyl acetate (2 mL) at room temperature. A solution of hydrochloric acid in ethyl acetate (4.98 mL, 4 M) was added thereto. The reaction mixture was stirred and reacted for 2 hours. After the reaction was completed, the mixture was filtered. The filter cake was rinsed with ethyl acetate (5 mL×3), and collected. The filter cake was separated by preparative high performance liquid chromatography (chromatographic column: Phenomenex Luna 80 * 30 mm * 3 μm; mobile phase: water (0.04% hydrochloric acid)-acetonitrile; acetonitrile%: 10% to 30%, 8 minutes) to obtain the hydrochloride of the target compound **WX018.** MS-ESI *m/z:* 925.5 $[M+H]^+$. $^1H$ NMR (400 MHz, MeOD_*d4*) δ: 8.80 (s, 1H), 8.30-8.23 (m, 2H), 8.15 (d, *J*=9.2 Hz, 1H), 7.97 (d, *J*=6.8 Hz, 1H), 7.89 (d, *J*=9.6 Hz, 1H), 7.78 (t, *J*=8.0 Hz, 1H), 7.72-7.68 (m, 1H), 7.68-7.61 (m, 1H), 7.45 (dd, *J*=1.6 Hz, 6.8 Hz, 1H), 6.94 (t, *J*=54.6 Hz, 1H), 5.01 (dd, *J*=5.0 Hz, 10.2 Hz, 1H), 4.32-4.19 (m, 1H), 3.79 (d, *J*=12.0 Hz, 2H), 3.30-3.27 (m, 1H), 3.24-3.00 (m, 4H), 2.97-2.63 (m, 4H), 2.59-2.46 (m, 1H), 2.34-2.15 (m, 4H), 2.14-1.90 (m, 7H), 1.42-1.27 (m, 3H), 1.26-1.17 (m, 1H), 0.75-0.66 (m, 2H), 0.45-0.36 (m, 2H).

**Example 19**

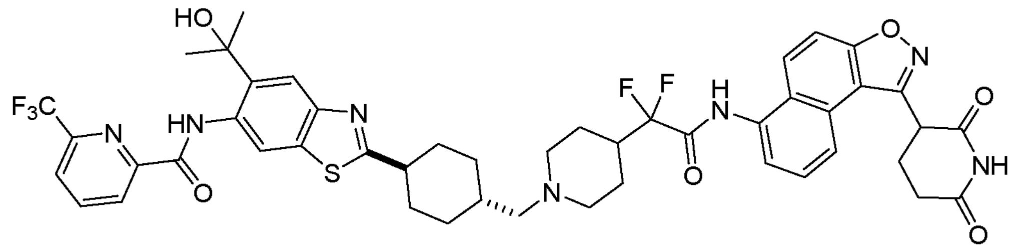

**WX019**

Synthetic route:

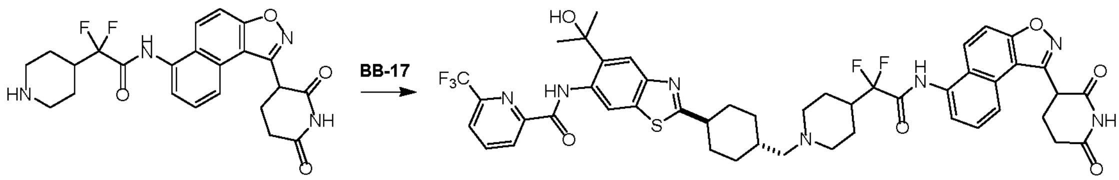


WX018-7

WX019

Synthesis of formate of compound **WX019**

Compound **BB-17** (0.1 g, 165.72 μmol) and the trifluoroacetate of compound **WX018**-7 (0.15 g, 262.94 μmol) were dissolved in acetonitrile (3 mL) at room temperature under nitrogen atmosphere, and N, N-diisopropylethylamine (214.18 mg, 1.66 mmol, 288.65 μL) was added thereto. The reaction mixture was heated to 90°C, and stirred and reacted for 14 hours. After the reaction was completed, the reaction mixture was concentrated under reduced pressure to remove the solvent. The resulting residue was separated by preparative high performance liquid chromatography (chromatographic column: Phenomenex Luna C18 200 * 40 mm * 10 μm; mobile phase: water (0.2% formic acid)-acetonitrile; acetonitrile%: 20% to 50%, 8 minutes) to obtain the formate of the target compound **WX019.** MS-ESI *m/z*: 932.3 $[M+H]^+$. $^1H$ NMR (400 MHz, DMSO_$d_6$) δ: 12.55 (s, 1H), 11.15 (s, 1H), 10.99 (s, 1H), 9.07 (s, 1H), 8.47 (d, J=8.0 Hz, 1H), 8.38 (t, *J*=7.8 Hz, 1H), 8.27-8.22 (m, 1H), 8.19 (d, *J*=8.8 Hz, 1H), 8.06 (q, *J*=9.6 Hz, 2H), 7.89 (s, 1H), 7.77 (t, *J*=8.0 Hz, 1H), 7.58 (d, *J*=7.2 Hz, 1H), 6.07 (s, 1H), 5.11 (dd, *J*=4.2 Hz, 11.4 Hz, 1H), 3.13-2.96 (m, 3H), 2.92-2.80 (m, 1H), 2.71-2.57 (m, 2H), 2.48-2.24 (m, 3H), 2.22-2.12 (m, 4H), 1.99-1.88 (m, 4H), 1.86-1.77 (m, 2H), 1.63 (s, 6H), 1.62-1.51 (m, 4H), 1.18-1.01 (m, 2H).

**Example 20**

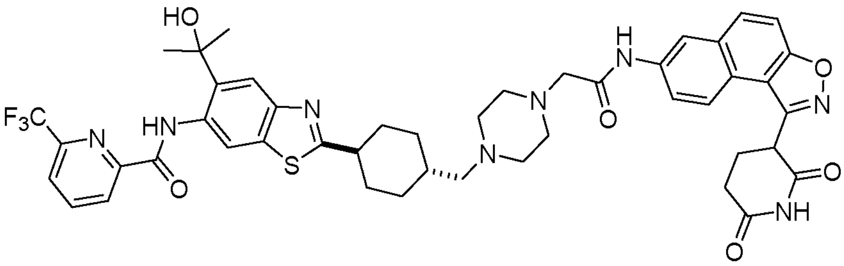

**WX020**

Synthetic route:

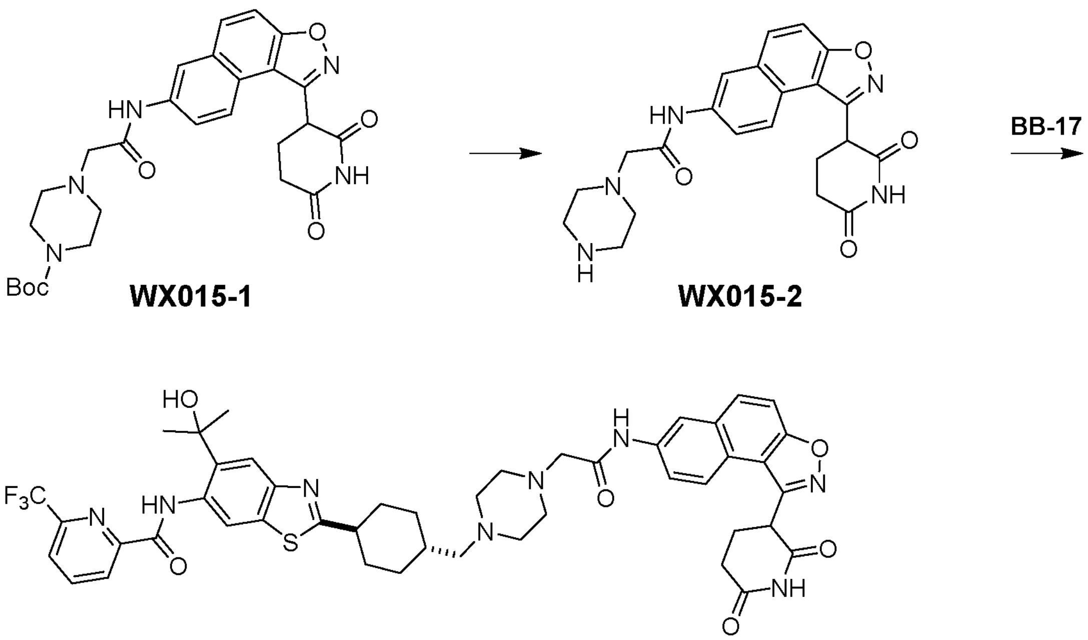


**WX015-1**

**WX015-2**

**WX020**

Step **1:** Synthesis of trifluoroacetate of compound **WX015-2**

Compound WX015-1 (0.1 g, 191.73 μmol) was dissolved in dichloromethane (3 mL) at room temperature under nitrogen atmosphere, and trifluoroacetic acid (459.10 mg, 4.03 mmol, 298.11 μL) was added thereto. The reaction mixture was stirred and reacted at room temperature for 4 hours. After the reaction was completed, the reaction mixture was concentrated under reduced pressure to obtain the trifluoroacetate of compound WX015-2. MS-ESI m/z: 422.1 $[M+H]^+$.

Step **2:** Synthesis of formate of compound **WX020**

Compound **BB-17** (0.075 g, 124.29 μmol) and the trifluoroacetate of compound **WX015-2** (0.125 g, 185.93 μmol) were dissolved in acetonitrile (3 mL) at room temperature under nitrogen atmosphere, and N, N-diisopropylethylamine (160.63 mg, 1.24 mmol, 216.49 μL) was added thereto. The reaction mixture was heated to 90°C, and stirred and reacted for 36 hours. After the reaction was completed, the reaction mixture was concentrated under reduced pressure to remove the solvent. The resulting residue was separated by preparative high performance liquid chromatography (chromatographic column: Phenomenex Luna C18 75 * 30 mm * 3 μm; mobile phase: water (0.2% formic

acid)-acetonitrile; acetonitrile%: 10% to 45%, 8 minutes) to obtain the formate of the target compound **WX020.** MS-ESI *m*/*z:* 897.4 [M+H]$^+$. $^1$H NMR (400 MHz, DMSO_$d_6$) δ: 12.54 (s, 1H), 11.13 (s, 1H), 10.01 (s, 1H), 9.06 (s, 1H), 8.50 (d, *J*=2.0 Hz, 1H), 8.46 (d, *J*=7.6 Hz, 1H), 8.38 (t, *J*=7.8 Hz, 1H), 8.18 (d, J=7.6 Hz, 2H), 8.12 (d, *J*=9.2 Hz, 1H), 7.95-7.86 (m, 3H), 6.07 (s, 1H), 5.03 (dd, *J*=4.6 Hz, 11.4 Hz, 1H), 3.20 (s, 2H), 3.12-2.99 (m, 1H), 2.91-2.76 (m, 2H), 2.70-2.62 (m, 3H), 2.58-2.53 (m, 4H), 2.43-2.31 (m, 3H), 2.22-2.12 (m, 4H), 1.97-1.85 (m, 2H), 1.72-1.49 (m, 10H), 1.16-0.99 (m, 2H).

**Example 21**

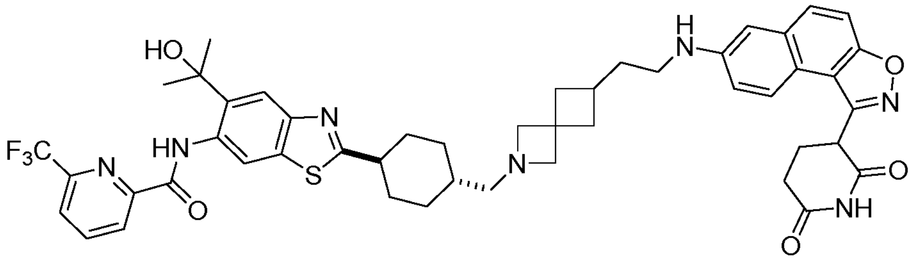

**WX021**

Synthetic route:

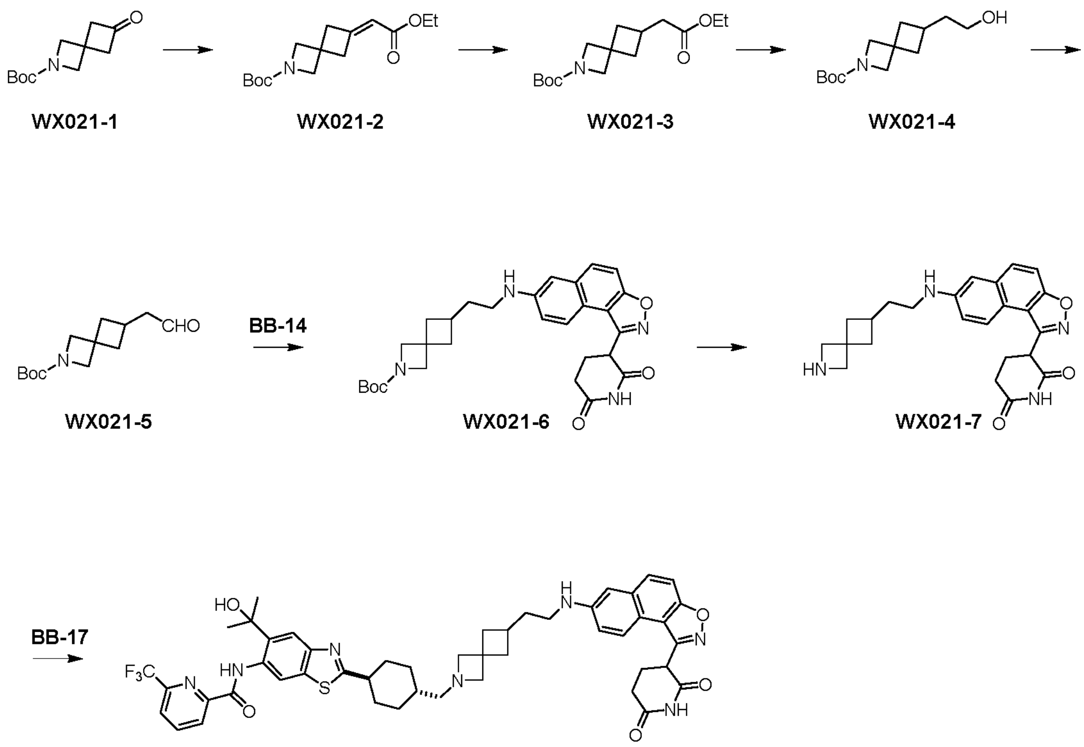


WX021-1 WX021-2 WX021-3 WX021-4

WX021-5 WX021-6 WX021-7

WX021

Step **1:** Synthesis of compound **WX021-2**

Compound **WX021-1** (30 g, 142.01 mmol) and (ethoxycarbonylmethylene)triphenylphosphorane (54.42 g, 156.21 mmol) were dissolved in toluene (450 mL) at room temperature under nitrogen atmosphere. The reaction mixture was heated to 80°C, and stirred and reacted for 12 hours. After the reaction was completed, the reaction mixture was cooled to room temperature and washed with water (3 × 200 mL). The organic phase was dried over anhydrous sodium sulfate, and filtered. The filtrate was concentrated under reduced pressure to obtain a yellow residue. The residue was

separated by column chromatography (eluent: petroleum ether/ethyl acetate = 1/0 to 4/1, v/v) to obtain compound **WX021-2.** MS-ESI *m/z*: 226.2 $[M+H-56]^+$. $^1H$ NMR (400 MHz, $CDCl_3$) δ: 5.67-5.63 (m, 1H), 4.14 (q, *J*=7.2 Hz, 2H), 4.05-3.87 (m, 4H), 3.29 (d, *J*=2.4 Hz, 2H), 3.00 (s, 2H), 1.43 (s, 9H), 1.27 (t, *J*=7.2 Hz, 3H).

Step **2:** Synthesis of compound **WX021-3**

Compound **WX021-2** (37 g, 131.51 mmol) was dissolved in ethanol (400 mL) at room temperature under nitrogen atmosphere, then palladium on carbon (3.8 g, purity of 10%) was added thereto. The atmosphere was replaced with hydrogen three times, and the reaction mixture was stirred and reacted at room temperature and at 25 psi for 12 hours. After the reaction was completed, the mixture was filtered. The filter cake was rinsed with ethyl acetate (700 mL). The filtrate was concentrated under reduced pressure to obtain compound **WX021-3.** MS-ESI *m/z*: 228.2 $[M+H-56]^+$. $^1H$ NMR (400 MHz, $CDCl_3$) δ: 4.11 (q, *J*=7.0 Hz, 2H), 3.93 (s, 2H), 3.80 (s, 2H), 2.60-2.45 (m, 1H), 2.40-2.29 (m, 4H), 1.91-1.82 (m, 2H), 1.43 (s, 9H), 1.24 (t, *J*=7.0 Hz, 3H).

Step **3:** Synthesis of compound **WX021-4**

Lithium borohydride (2.59 g, 118.90 mmol) was dissolved in tetrahydrofuran (100 mL) at room temperature under nitrogen atmosphere. A solution of compound **WX021-3** (10 g, 35.29 mmol) in tetrahydrofuran (20 mL) was added thereto. The reaction mixture was heated to 30 °C, and methanol (20 mL) was slowly added dropwise thereto. After the dropwise addition was completed, the reaction mixture was further stirred and reacted for 12 hours. After the reaction was completed, the reaction mixture was poured into water (300 L), and extracted with ethyl acetate (3×300 mL). The organic phase was washed with saturated brine (400 mL), dried over anhydrous sodium sulfate, and filtered. The filtrate was concentrated under reduced pressure to obtain compound **WX021-4.** $^1H$ NMR (400 MHz, $CDCl_3$) δ: 3.92 (s, 2H), 3.80 (s, 2H), 3.57 (t, *J*=6.6 Hz, 2H), 2.33-2.18 (m, 3H), 1.84-1.75 (m, 2H), 1.63 (q, *J*=6.8 Hz, 2H), 1.43 (s, 9H).

Step **4:** Synthesis of compound **WX021-5**

Compound **WX021-4** (5.3 g, 21.96 mmol) was dissolved in dichloromethane (70 mL) at room temperature under nitrogen atmosphere, then Dess-Martin periodinane (12.11 g, 28.55 mmol) was added thereto. The reaction mixture was stirred and reacted for 1.5 hours. After the reaction was completed, the reaction mixture was concentrated under reduced pressure. The residue was separated by column chromatography (eluent: petroleum ether/ethyl acetate = 1/0 to 4/1, v/v) to obtain compound **WX021-5.** $^1H$ NMR (400 MHz, $CDCl_3$) δ: 9.69 (s, 1H), 3.95 (s, 2H), 3.81 (s, 2H), 2.67-2.51 (m, 3H), 2.45-2.32 (m, 2H), 1.92-1.81 (m, 2H), 1.42 (s, 9H).

Step **5:** Synthesis of compound **WX021-6**

Compound **WX021-5** (0.5 g, 2.09 mmol) and the hydrochloride of compound **BB-14** (0.6 g, 1.81 mmol) were dissolved in tetrahydrofuran (15 mL) at room temperature under nitrogen atmosphere, and potassium acetate (709.99 mg, 7.23 mmol) and acetic acid (217.22 mg, 3.62 mmol) were added thereto. The reaction mixture was stirred for 2 hours. Sodium triacetoxyborohydride (1.53 g, 7.23 mmol) was added thereto. The reaction mixture was further stirred for 1 hour. After the reaction was completed, the reaction mixture was added with water (20 mL) and extracted with ethyl acetate (20 mL × 4). The organic phases were combined, washed with saturated brine (80 mL × 3), dried over anhydrous sodium sulfate, and filtered,. The filtrate was concentrated under reduced pressure to remove the solvent. The resulting residue was separated by column chromatography (eluent: petroleum ether/ethyl acetate = 2/1 to 1/1, v/v) to obtain compound **WX021-6**. MS-ESI *m/z*: 419.2 $[M+H-100]^+$. $^1H$ NMR (400 MHz, $CDCl_3$) δ: 8.18 (s, 1H), 7.80 (d, *J*=3.2 Hz, 1H), 7.77 (d, *J*=3.6 Hz, 1H), 7.61 (d, *J*=9.2 Hz, 1H), 7.03 (dd, *J*=2.4 Hz, 8.8 Hz, 1H), 7.00 (d, *J*=2.4 Hz, 1H), 4.69 (dd, *J*=5.2 Hz, 8.0 Hz, 1H), 3.95 (s, 2H), 3.83 (s, 2H), 3.16 (t, *J*=7.0 Hz, 2H), 2.94-2.84 (m, 1H), 2.80-2.63 (m, 2H), 2.58-2.48 (m, 1H), 2.40-2.25 (m, 3H), 1.91-1.83 (m, 2H), 1.77 (q, *J*=7.0 Hz, 2H), 1.44 (s, 9H).

Step **6:** Synthesis of trifluoroacetate of compound **WX021-7**

Compound **WX021-6** (0.18 g, 347.09 μmol) was dissolved in dichloromethane (6 mL) at room temperature under nitrogen atmosphere, and trifluoroacetic acid (893.20 mg, 7.83 mmol, 0.58 mL) was added thereto. The reaction mixture was stirred and reacted at room temperature for 3 hours. After the reaction was completed, the reaction mixture was concentrated under reduced pressure to remove the solvent to obtain the trifluoroacetate of compound **WX021-7.** MS-ESI *m/z:* 419.1 $[M+H]^+$.

Step **7:** Synthesis of compound **WX021**

Compound **BB-17** (0.1 g, 165.72 μmol) and the trifluoroacetate of compound **WX021**-7 (125 mg, 174.84 μmol) was dissolved in acetonitrile (3 mL) at room temperature under nitrogen atmosphere, and N, N-diisopropylethylamine (257.01 mg, 1.99 mmol, 346.38 μL) was added thereto. The reaction mixture was heated to 90°C, and stirred and reacted for 30 hours. After the reaction was completed, the reaction mixture was concentrated under reduced pressure to remove the solvent. The resulting residue was first separated by preparative high performance liquid chromatography (chromatographic column: Waters Xbridge BEH C18 100*30 mm*10 μm; mobile phase: water (10 mM ammonium bicarbonate)-acetonitrile; acetonitrile%: 50% to 70%, 8 minutes), then separated by thin-layer chromatography (developing solvent: dichloromethane/methanol = 5/1, v/v) to obtain the target compound **WX021.** MS-ESI *m/z*: 894.4 $[M+H]^+$. $^1H$ NMR (400 MHz, DMSO_$d_6$) δ: 12.54 (s, 1H), 11.11 (s, 1H), 9.06 (s, 1H), 8.46 (d, *J*=7.6 Hz, 1H), 8.38 (t, *J*=7.8 Hz, 1H), 8.19 (d, *J*=8.0 Hz, 1H), 7.94-7.86 (m, 3H), 7.71 (d, *J*=9.2 Hz, 1H), 7.12 (dd, *J*=2.2 Hz, 9.0 Hz, 1H), 6.99 (d, *J*=2.4 Hz, 1H), 6.08 (s, 1H), 5.92 (t, *J*=5.4 Hz, 1H), 4.93 (dd, *J*=4.8 Hz, 11.2 Hz, 1H), 3.31-3.21 (m, 4H), 3.07-3.00 (m, 3H), 2.87-2.78 (m, 1H), 2.69-2.52 (m, 3H), 2.42-2.18 (m, 5H), 2.16-2.11 (m, 2H), 1.88-1.81 (m, 3H), 1.73-1.66 (m, 2H), 1.63 (s, 6H), 1.59-1.41 (m, 3H), 1.29-1.20 (m, 1H), 1.18-1.03 (m, 2H).

Example 23

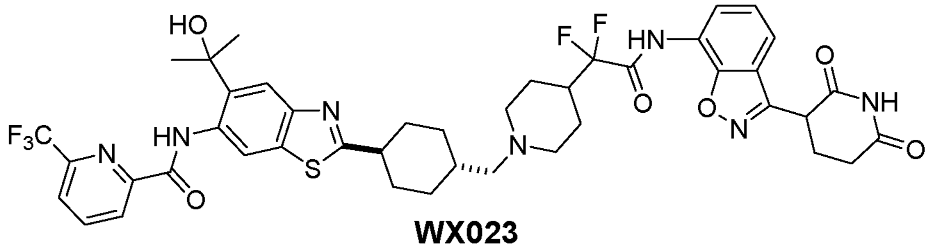

**WX023**

Synthetic route:

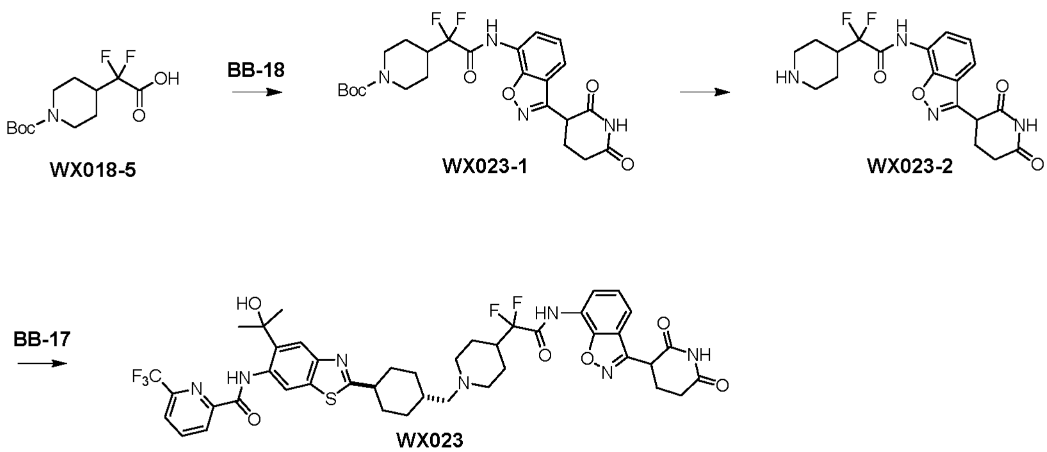


Step **1**: Synthesis of compound **WX023-1**

Compound **WX018-5** (600 mg, 2.15 mmol) was dissolved in N, N-dimethylformamide (6 mL) at room temperature under nitrogen atmosphere, then N, N-diisopropylethylamine (1.11 g, 8.59 mmol, 1.50 mL) and 2-(7-azabenzotriazol)-N, N, N', N'-tetramethyluronium hexafluorophosphate (1.09 g, 2.86 mmol) were added thereto. The reaction mixture was stirred for 0.5 hours. The hydrochloride of compound **BB-18** (403.46 mg, 1.43 mmol) was added thereto. The reaction mixture was further stirred for 12 hours. After the reaction was completed, the reaction mixture was added with ethyl acetate (30 mL) and 10% brine (15 mL). The organic phase was separated out. The aqueous phase was extracted with ethyl acetate (20 mL). The organic phases were combined, washed with saturated brine (20 mL), dried over anhydrous

sodium sulfate, and filtered. The filtrate was concentrated under reduced pressure to obtain a crude product. The crude product was separated by column chromatography (eluent: petroleum ether/ethyl acetate = 1/0 to 0/1, v/v) to obtain compound **WX023-1.** MS-ESI *m*/*z*: 451.2 [M+H-56]$^+$. $^1$H NMR (400 MHz, DMSO_$d_6$) δ: 11.13 (s, 1H), 10.99 (s, 1H), 7.80 (d, *J*=7.6 Hz, 1H), 7.62 (d, *J*=7.6 Hz, 1H), 7.42 (t, *J*=7.8 Hz, 1H), 4.65 (dd, *J*=5.2 Hz, 12.0 Hz, 1H), 4.13-4.02 (m, 2H), 2.84-2.75 (m, 3H), 2.68-2.54 (m, 2H), 2.28-2.18 (m, 1H), 1.89-1.75 (m, 2H), 1.41 (s, 9H), 1.38-1.22 (m, 3H).

Step **2:** Synthesis of trifluoroacetate of compound **WX023-2**

Compound **WX023-1** (600 mg, 1.18 mmol) was dissolved in dichloromethane (5 mL) at room temperature under nitrogen atmosphere, and trifluoroacetic acid (1.85 g, 16.21 mmol, 1.20 mL) was added thereto. The reaction mixture was stirred and reacted at room temperature for 2 hours. After the reaction was completed, the reaction mixture was concentrated under reduced pressure to obtain the trifluoroacetate of compound **WX023-2**. MS-ESI *m*/*z:* 407.1 [M+H]$^+$.

Step **3:** Synthesis of formate of compound **WX023**

Compound **BB-17** (150 mg, 248.58 μmol) was added to acetonitrile (2 mL) at room temperature under nitrogen atmosphere, and N, N-diisopropylethylamine (149.01 mg, 1.15 mmol) was added thereto. The mixture was stirred and reacted for 10 minutes, then the trifluoroacetate of compound **WX023-2** (200 mg, 384.32 μmol) was added thereto. The reaction mixture was heated to 85°C, and stirred and reacted for 6 hours. After the reaction was completed, the reaction mixture was concentrated under reduced pressure to obtain a crude product. The crude product was separated by preparative high performance liquid chromatography (chromatographic column: Phenomenex Luna C18 75 * 30 mm * 3 μm; mobile phase: water (0.2% formic acid)-acetonitrile; acetonitrile%: 20% to 50%, 8 minutes) to obtain the formate of the target compound **WX023.** MS-ESI *m*/*z:* 882.9 [M+H]$^+$. $^1$H NMR (400 MHz, DMSO_$d_6$) δ: 12.56 (s, 1H), 11.13 (s, 1H), 11.00 (s, 1H), 9.08 (s, 1H), 8.47 (d, *J*=8.0 Hz, 1H), 8.39 (t, *J*=7.8 Hz, 1H), 8.19 (d, *J*=8.0 Hz, 1H), 8.14 (s, 1H), 7.90 (s, 1H), 7.80 (d, *J*=8.0 Hz, 1H), 7.63 (d, *J*=7.6 Hz, 1H), 7.42 (t, *J*=7.8 Hz, 1H), 6.08 (s, 1H), 4.65 (dd, *J*=5.2 Hz, 12.0 Hz, 1H), 3.19-3.00 (m, 3H), 2.87-2.72 (m, 1H), 2.68-2.53 (m, 2H), 2.41-2.28 (m, 3H), 2.27-2.07 (m, 5H), 1.93 (d, *J*=11.6 Hz, 2H), 1.84 (d, *J*=11.6 Hz, 2H), 1.76-1.51 (m, 11H), 1.18-1.03 (m, 2H).

**Example 24**

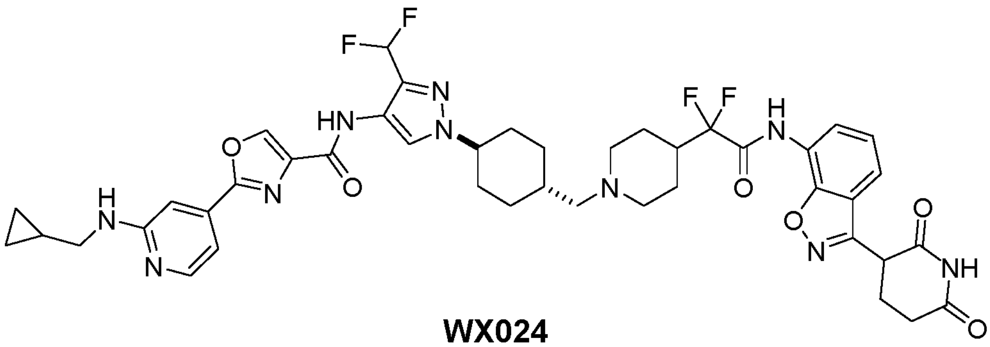

**WX024**

Synthetic route:

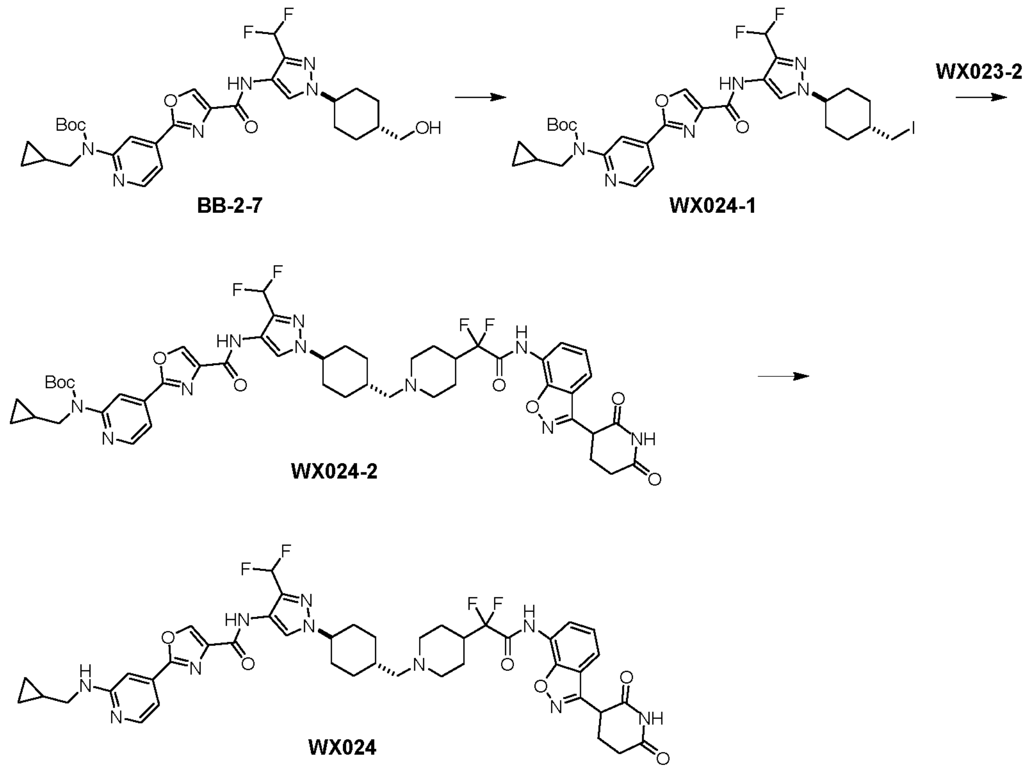


BB-2-7

WX024-1

WX024-2

WX024

Step **1:** Synthesis of compound **WX024-1**

Compound **BB-2-7** (2 g, 3.41 mmol) was dissolved in dichloromethane (40 mL) at room temperature under nitrogen atmosphere, then triphenylphosphine (1.07 g, 4.09 mmol) and imidazole (348.15 mg, 5.11 mmol) were added thereto. The mixture was cooled in an ice-water bath to 0°C, and iodine (1.12 g, 4.43 mmol) was added thereto. The reaction mixture was stirred and reacted at room temperature for 12 hours. After the reaction was completed, the reaction mixture was poured into a saturated solution of sodium sulfite (50 mL), and extracted with dichloromethane (80 mL × 3). The organic phases were combined, washed with saturated brine (50 mL × 2), dried over anhydrous sodium sulfate, and filtered. The filtrate was concentrated under reduced pressure to remove the solvent. The resulting residue was separated by column chromatography (eluent: petroleum ether/ethyl acetate = 5/1 to 3/1, v/v) to obtain compound **WX024-1.** MS-ESI *m/z*: 697.0 $[M+H]^+$. $^1H$ NMR (400 MHz, $CDCl_3$) δ: 9.07 (s, 1H), 8.52 (d, *J*=4.8 Hz, 1H), 8.37 (s, 1H), 8.33 (s, 1H), 8.32 (s, 1H), 7.64 (dd, *J*=1.2 Hz, 5.2 Hz, 1H), 6.83 (t, *J*=54.8 Hz, 1H), 4.16-4.03 (m, 1H), 3.95 (d, *J*=7.2 Hz, 2H), 3.17 (d, *J*=6.0 Hz, 2H), 2.28-2.18 (m, 2H), 2.15-2.07 (m, 2H), 1.93-1.80 (m, 2H), 1.60-1.54 (m, 10H), 1.32-1.17 (m, 3H), 0.48-0.40 (m, 2H), 0.31-0.25 (m, 2H).

Step **2:** Synthesis of compound **WX024-2**

Compound **WX024-1** (196.30 mg, 281.83 μmol) was added to acetonitrile (2 mL) at room temperature under nitrogen atmosphere, and N, N-diisopropylethylamine (218.54 mg, 1.69 mmol) was added thereto. The mixture was stirred and reacted for 10 minutes, then the trifluoroacetate of compound **WX023-2** (220 mg, 422.75 μmol) was added thereto. The reaction mixture was heated to 85°C, and stirred and reacted for 6 hours. After the reaction was completed, the reaction mixture was cooled to room temperature, added with ethyl acetate (20 mL) and water (10 mL) to separate the organic phase. The aqueous phase was extracted with ethyl acetate (15 mL × 2). The organic phases were combined, dried over anhydrous sodium sulfate, and filtered, The filtrate was concentrated under reduced pressure to obtain compound **WX024-2.** MS-ESI *m/z*: 975.3 $[M+H]^+$.

Step 3: Synthesis of formate of compound **WX024**

Compound **WX024-2** (270 mg, 276.92 μmol) was dissolved in dichloromethane (5 mL) at room temperature under nitrogen atmosphere, and trifluoroacetic acid (770.00 mg, 6.75 mmol, 0.5 mL) was added thereto. The reaction mixture was stirred and reacted for 5 hours. After the reaction was completed, the reaction mixture was concentrated under reduced pressure to obtain a crude product. The crude product was separated by preparative high performance

liquid chromatography (chromatographic column: Phenomenex Luna C18 75 * 30 mm * 3 μm; mobile phase: water (0.2% formic acid)-acetonitrile; acetonitrile%: 1% to 30%, 8 minutes) to obtain the formate of the target compound **WX024.** MS-ESI *m/z*: 875.5 $[M+H]^+$ $^1H$ NMR (400 MHz, DMSO_$d_6$) δ: 11.12 (s, 1H), 10.97 (s, 1H), 9.69 (s, 1H), 8.92 (s, 1H), 8.20-8.12 (m, 2H), 7.79 (d, *J*=8.0 Hz, 1H), 7.61 (d,*J*=7.6 Hz, 1H), 7.41 (t, *J*=7.8 Hz, 1H), 7.32-6.99 (m, 4H), 4.64 (dd, *J*=4.8 Hz, 12.0 Hz, 1H), 4.27-4.14 (m, 1H), 3.18 (t, *J*=6.2 Hz, 2H), 3.01 (d, *J*=10.8 Hz, 2H), 2.86-2.73 (m, 1H), 2.66-2.52 (m, 2H), 2.36-2.14 (m, 4H), 2.10-1.87 (m, 6H), 1.85-1.70 (m, 4H), 1.68-1.49 (m, 3H), 1.15-0.99 (m, 3H), 0.50-0.42 (m, 2H), 0.26-0.18 (m, 2H).

**Example 25**

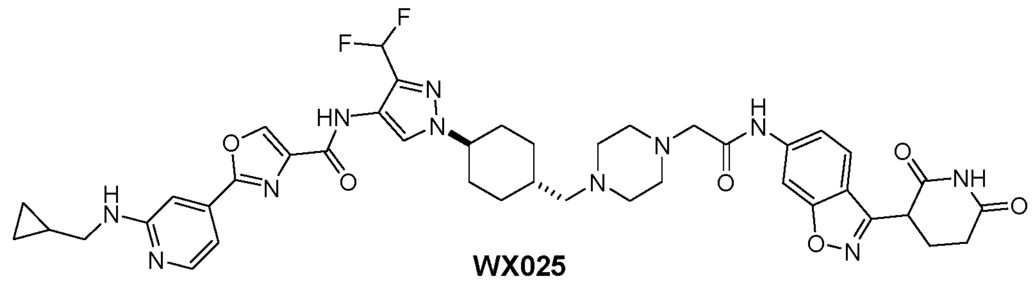

**WX025**

Synthetic route:

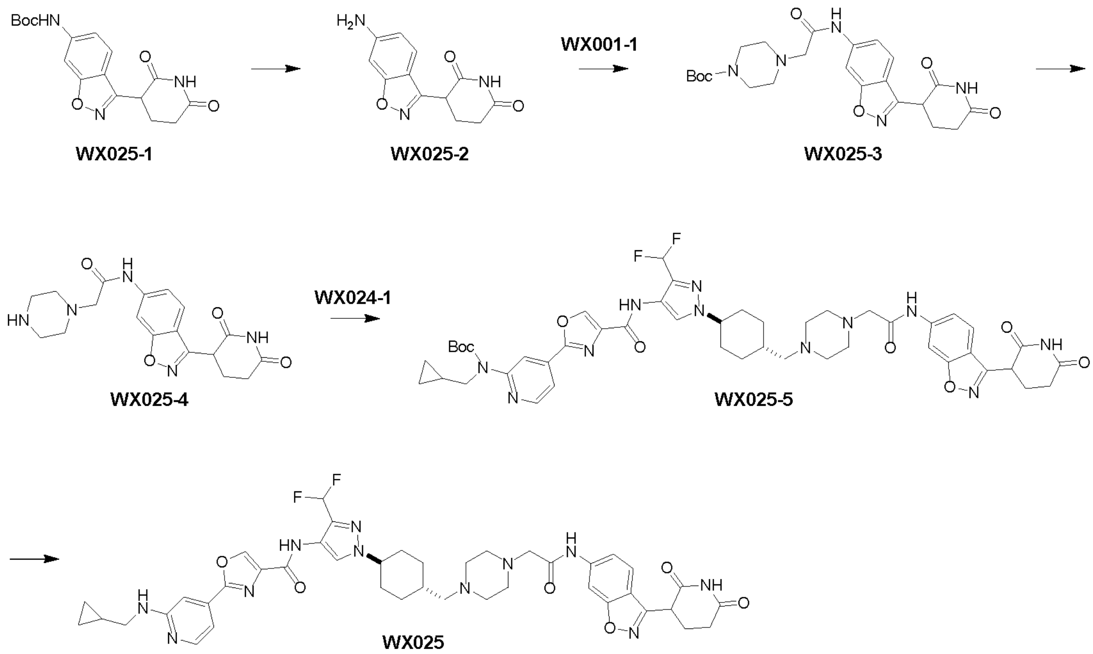


WX025-1 WX025-2 WX025-3 WX025-4 WX025-5 WX025

Step **1:** Synthesis of hydrochloride of compound **WX025-2**

Compound **WX025-1** (700 mg, 2.03 mmol) was added to a solution of hydrochloric acid in ethyl acetate (4 M, 10 mL) at room temperature under nitrogen atmosphere. The mixture was stirred and reacted for 1 hour. After the reaction was completed, the reaction mixture was concentrated under reduced pressure to obtain the hydrochloride of the compound **WX025-2.** MS-ESI *m/z:* 246.2 $[M+H]^+$. $^1H$ NMR (400 MHz, DMSO_$d_6$) δ: 11.04 (s, 1H), 7.47 (d, *J*=8.4 Hz, 1H), 6.77 (s, 1H), 6.72 (dd, *J*=1.4 Hz, 8.6 Hz, 1H), 4.41 (dd, *J*=5.0 Hz, 11.4 Hz, 1H), 2.79-2.68 (m, 1H), 2.62-2.54 (m, 1H), 2.47-2.35 (m, 1H), 2.21-2.10 (m, 1H).

Step **2:** Synthesis of compound **WX025-3**

Compound **WX001-1** (728.45 mg, 2.98 mmol) was dissolved in N, N-dimethylformamide (15 mL), then 2-(7-azabenzotriazol)-N, N, N', N'-tetramethyluronium hexafluorophosphate (1.89 g, 4.97 mmol) and N, N-diisopropylethylamine (1.93 g, 14.91 mmol, 2.60 mL) were added thereto, stirred and reacted for 30 minutes, then the hydrochloride of compound **WX025-2** (0.7 g, 2.48 mmol) was added thereto. The reaction mixture was stirred and reacted for another 4.5 hours. After the reaction was completed, the reaction mixture was added with water (10 mL) and ethyl acetate (30 mL), stirred at room temperature for 15 minutes, and filtered. The filter cake was collected. The filtrate was extracted with ethyl acetate (30 mL × 3). The organic phases were combined, washed with saturated brine (30 mL), dried over anhydrous sodium sulfate, and filtered. The filtrate was concentrated under reduced pressure to obtain a crude product. The crude product was separated by column chromatography (eluent: petroleum ether/ethyl acetate = 1/0 to 0/1, v/v) . The crude product obtained was added with ethyl acetate (10 mL), stirred at room temperature for 1 hour, and filtered. The filter cake was collected, and dried under vacuum to obtain compound **WX025-3.** MS-ESI mlz: 472.3 $[M+H]^+$. $^1H$ NMR (400 MHz, DMSO_$d_6$) δ : 11.09 (s, 1H), 10.26 (s, 1H), 8.23 (s, 1H), 7.77 (d, *J*=8.4 Hz, 1H), 7.51 (d, *J*=8.8 Hz, 1H), 4.55 (dd, *J*=4.6 Hz, 11.8 Hz, 1H), 3.43-3.35 (m, 4H), 3.24 (s, 2H), 2.82-2.71 (m, 1H), 2.64-2.56 (m, 1H), 2.49-2.42 (m, 5H), 2.27-2.12 (m, 1H), 1.40 (s, 9H).

Step 3: Synthesis of trifluoroacetate of compound **WX025-4**

Compound **WX025-3** (400 mg, 848.35 μmol) was dissolved in dichloromethane (4 mL) at room temperature under nitrogen atmosphere, and trifluoroacetic acid (1.23 g, 10.80 mmol, 0.8 mL) was added thereto. The reaction mixture was stirred and reacted for 1 hour. After the reaction was completed, the reaction mixture was concentrated under reduced pressure to obtain the trifluoroacetate of compound **WX025-4.** MS-ESI *m*/*z*: 372.3 $[M+H]^+$.

Step **4:** Synthesis of compound **WX025-5**

The trifluoroacetate of compound **WX025-4** (252.98 mg, 521.16 μmol) was added to acetonitrile (7 mL) at room temperature under nitrogen atmosphere, and N, N-diisopropylethylamine (367.39 mg, 2.84 mmol) was added thereto. The reaction mixture was stirred for 10 minutes, then compound **WX024-1** (330 mg, 473.78 μmol) was added thereto. The reaction mixture was heated to 85°C, and stirred and reacted for 6 hours. After the reaction was completed, the reaction mixture was cooled to room temperature, then poured into a saturated solution of ammonium chloride (10 mL), and extracted with ethyl acetate (10 mL × 3). The organic phases were combined, washed with saturated brine (20 mL), dried over anhydrous sodium sulfate, and filtered. The filtrate was concentrated under reduced pressure to obtain a crude product. The crude product was purified by column chromatography (eluent: petroleum ether/ethyl acetate = 1/0 to 5/1, v/v) to obtain compound **WX025-5**. MS-ESI *m*/*z*: 940.6 $[M+H]^+$.

Step 5: Synthesis of hydrochloride of compound **WX025**

Compound **WX025-5** (300 mg, 319.15 μmol) was dissolved in ethyl acetate (1 mL) at room temperature under nitrogen atmosphere. A solution of hydrochloric acid in ethyl acetate (4 M, 3.00 mL) was added thereto. The reaction mixture was stirred and reacted for 2 hours. After the reaction was completed, the reaction mixture was filtered, and rinsed with ethyl acetate (2 mL × 2). The filter cake was collected to obtain a crude product. The crude product was added with a mixed solution of acetonitrile (1 mL) and water (1 mL). The mixture was stirred at room temperature for 0.5 hours, and filtered. The filter cake was collected, and dried under vacuum to obtain the hydrochloride of the target compound **WX025.** MS-ESI *m*/*z*: 840.5 $[M+H]^+$. $^1H$ NMR (400 MHz, DMSO_$d_6$) δ: 11.11 (s, 1H), 9.91 (s, 1H), 9.13 (s, 1H), 8.23 (d, *J*=1.2 Hz, 1H), 8.20 (s, 1H), 8.09 (d, *J*=6.8 Hz, 1H), 7.82 (d, *J*=8.4 Hz, 1H), 7.64 (s, 1H), 7.51 (dd, *J*=1.2 Hz, 8.8 Hz, 1H), 7.36-7.05 (m, 2H), 4.58 (dd, *J*=4.8 Hz, 12.0 Hz, 1H), 4.34-4.22 (m, 1H), 3.99-3.84 (m, 2H), 3.78-3.70 (m 2H), 3.47-3.37 (m, 4H), 3.36-3.28 (m, 4H), 3.12-2.98 (m, 2H), 2.87-2.73 (m, 1H), 2.66-2.58 (m, 1H), 2.26-2.17 (m, 1H), 2.13-2.01 (m, 4H), 1.98-1.88 (m, 1H), 1.87-1.75 (m, 2H), 1.32-1.09 (m, 3H), 0.61-0.54 (m, 2H), 0.37-0.31 (m, 2H).

**Example 26**

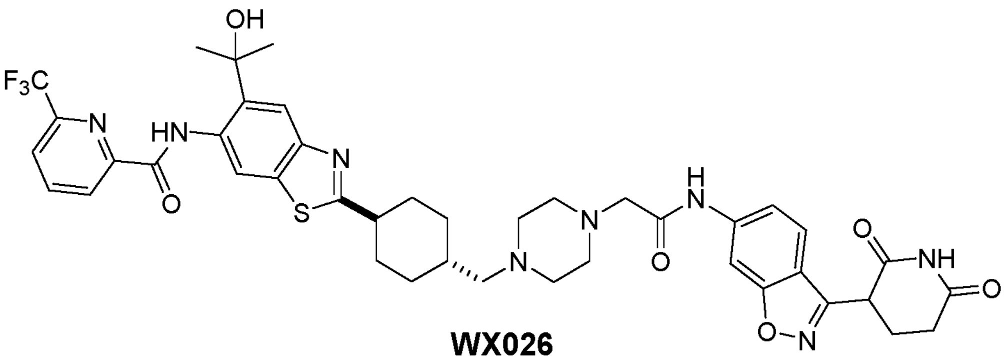

**WX026**

Synthetic route:

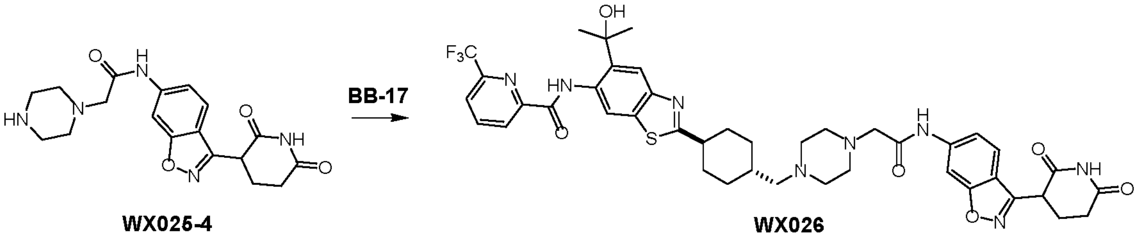


**WX025-4** **WX026**

Synthesis of formate of compound **WX026**

The trifluoroacetate of compound **WX025-4** (125.49 mg, 258.52 μmol) was added to acetonitrile (2 mL) at room temperature under nitrogen atmosphere, and N, N-diisopropylethylamine (133.64 mg, 1.03 mmol) was added thereto. The reaction mixture was stirred and reacted for 10 minutes, then compound **BB-17** (104.00 mg, 172.35 μmol) was added thereto. The reaction mixture was heated to 85°C, and stirred and reacted for 6 hours. After the reaction was completed, the reaction mixture was concentrated under reduced pressure to obtain a crude product. The crude product was separated by preparative high performance liquid chromatography (chromatographic column: Phenomenex Luna C18 75 * 30 mm * 3 μm; mobile phase: water (0.2% formic acid)-acetonitrile; acetonitrile%: 30% to 70%, 8 minutes) to obtain the formate of the target compound **WX026.** MS-ESI *m/z*: 847.0 $[M+H]^+$. $^1H$ NMR (400 MHz, DMSO_$d_6$) δ: 12.55 (s, 1H), 11.09 (s, 1H), 10.16 (s, 1H), 9.07 (s, 1H), 8.47 (d, *J*=7.6 Hz, 1H), 8.39 (t, *J*=7.8 Hz, 1H), 8.23 (s, 1H), 8.19 (dd, *J*=0.6 Hz, 7.8 Hz, 1H), 7.90 (s, 1H), 7.78 (d, *J*=8.8 Hz, 1H), 7.49 (dd, *J*=1.4 Hz, 8.6 Hz, 1H), 6.08 (s, 1H), 4.55 (dd, *J*=5.0 Hz, 11.8 Hz, 1H), 3.30 (s, 2H), 3.22 (s, 2H), 3.11-3.01 (m, 1H), 2.82- 2.72 (m, 1H), 2.66-2.55 (m, 5H), 2.47-2.41 (m, 2H), 2.28-2.13 (m, 5H), 1.98-1.87 (m, 2H), 1.72-1.50 (m, 10H), 1.17-1.00 (m, 2H).

**Example 28**

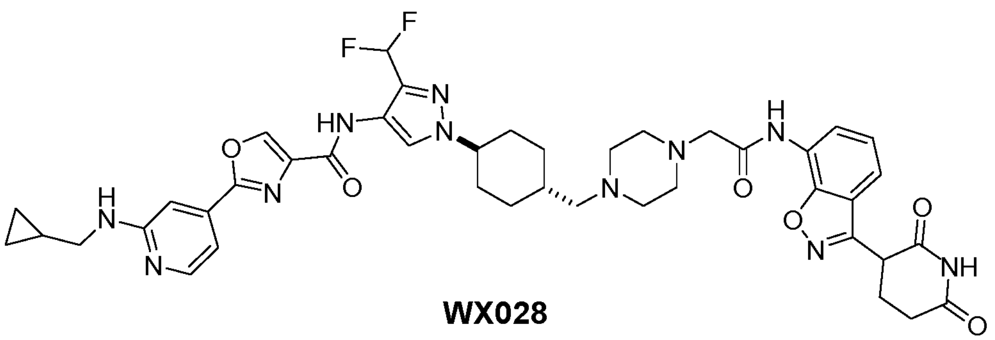

**WX028**

Synthetic route:

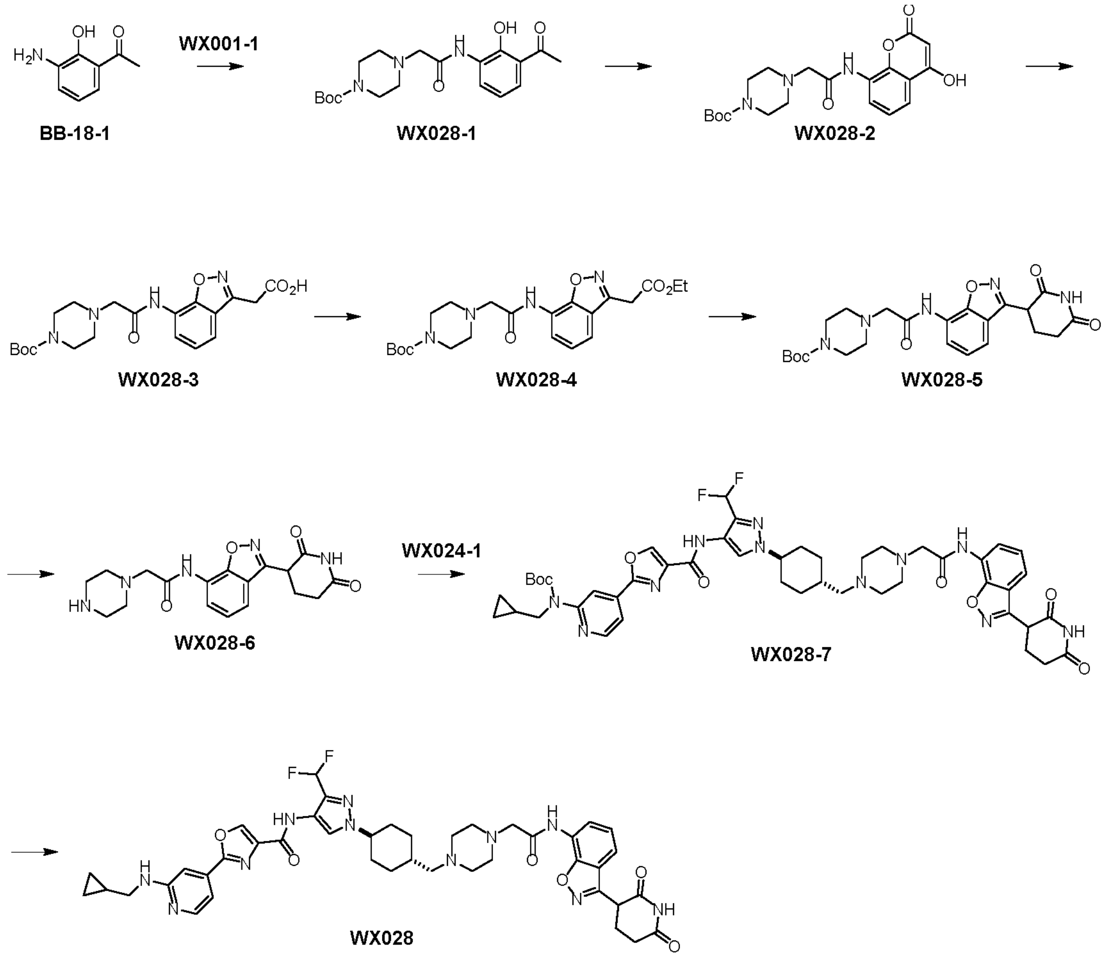


**BB-18-1**, **WX028-1**, **WX028-2**, **WX028-3**, **WX028-4**, **WX028-5**, **WX028-6**, **WX028-7**, **WX028**

Step **1:** Synthesis of compound **WX028-1**

Compound **WX001-1** (9.7 g, 39.69 mmol) was dissolved in N, N-dimethylformamide (50 mL) at room temperature under nitrogen atmosphere, then N, N-diisopropylethylamine (8.55 g, 66.15 mmol) and 2-(7-azabenzotriazol)-N, N', N'-tetramethyluronium hexafluorophosphate (16.35 g, 43.00 mmol) were sequentially added thereto. The reaction mixture was stirred and reacted at room temperature for 0.5 hours, then compound **BB-18-1** (5 g, 33.08 mmol) was added thereto. The reaction mixture was further stirred and reacted at room temperature for 2 hours. After the reaction was completed, the reaction mixture was added with water (200 mL) and extracted with ethyl acetate (50 mL × 3). The organic phases were combined, washed with saturated brine (100 mL), dried over anhydrous sodium sulfate, and filtered. The filtrate was concentrated under reduced pressure to remove the solvent. The resulting residue was separated by column chromatography (eluent: petroleum ether/ethyl acetate = 2/1, v/v) to obtain compound **WX028-1.** MS-ESI *m/z*: 378.3 $[M+H]^+$.

Step **2**: Synthesis of compound **WX028-2**

Compound **WX028-1** (4 g, 10.60 mmol) and dimethyl carbonate (3.82 g, 42.39 mmol, 3.57 mL) were dissolved in tetrahydrofuran (50 mL) at room temperature under nitrogen atmosphere, and potassium tert-butoxide (7.14 g, 63.59 mmol) was added thereto. The reaction mixture was heated to 90°C, and stirred and reacted for 4 hours. After the reaction was completed, the mixture was concentrated under reduced pressure to remove the solvent, added with ice water (50 mL) for dissolution, and extracted with methyl tert-butyl ether (50 mL × 2). The aqueous phase was collected, added with 6 M hydrochloric acid to adjust the pH to 7. The aqueous phase was concentrated under reduced pressure to obtain compound **WX028-2.** MS-ESI *m/z*: 404.2 $[M+H]^+$. $^1H$ NMR (400 MHz, $D_2O$) $\delta$: 7.78 (d, *J*=7.6 Hz, 1H), 7.60 (d,

*J*=7.6 Hz, 1H), 7.19 (t, *J*=7.8 Hz, 1H), 5.17 (s, 1H), 3.58-3.45 (m, 4H), 3.27 (s, 2H), 2.62-2.50 (m, 4H), 1.42 (s, 9H).

Step **3:** Synthesis of compound **WX028-3**

Compound **WX028-2** (4.2 g, 10.41 mmol) was dissolved in ethanol (80 mL) at room temperature, then sodium acetate (3.42 g, 41.64 mmol) and hydroxylamine hydrochloride (2.53 g, 36.44 mmol) were added thereto. The reaction mixture was heated to 80°C, and stirred and reacted for 5 hours. After the reaction was completed, the reaction mixture was cooled to room temperature, and filtered. The filter cake was rinsed with dichloromethane (50 mL × 2). The filtrate was concentrated to remove most of the solvent, added with water (100 mL), and extracted with dichloromethane (50 mL × 3). The organic phases were combined, washed with saturated brine (100 mL), dried over anhydrous sodium sulfate, and filtered. The filtrate was concentrated under reduced pressure to obtain compound **WX028-3.** MS-ESI *m*/*z*: 419.1 $[M+H]^+$.

Step **4:** Synthesis of compound **WX028-4**

Compound **WX028-3** (2.5 g, 5.97 mmol) was dissolved in dichloromethane (40 mL) at 30°C, 4-dimethylaminopyridine (1.09 g, 8.96 mmol) and di-tert-butyl carbonate (2 g, 9.16 mmol, 2.11 mL) were added thereto. After the reaction mixture was stirred and reacted at 30°C for 1.5 hours, anhydrous ethanol (2.75 g, 59.75 mmol) was added thereto. The mixture was further reacted for 1 hour. After the reaction was completed, the reaction mixture was poured into water (50 mL), and the phases were separated. The aqueous was again extracted with dichloromethane (30 mL). The organic phases were combined, dried over anhydrous sodium sulfate, and filtered. The filtrate was concentrated under reduced pressure to obtain a crude product. The crude product was purified by column chromatography (eluent: ethyl acetate/petroleum ether = 1/5) to obtain compound **WX028-4.** MS-ESI *m*/*z*: 447.3 $[M+H]^+$. $^1H$ NMR (400 MHz, $CDCl_3$) δ: 9.79 (s, 1H), 8.46 (d, *J*=7.6 Hz, 1H), 7.41 (d, *J*=7.6 Hz, 1H), 7.32 (t, *J*=8.0 Hz, 1H), 4.22 (q, *J*=7.0 Hz, 2H), 4.04 (s, 2H), 3.63-3.54 (m, 4H), 3.26 (s, 2H), 2.69-2.58 (m, 4H), 1.48 (s, 9H), 1.26 (t, *J*=7.2 Hz, 3H).

Step **5:** Synthesis of compound **WX028-5**

Compound **WX028-4** (0.92 g, 2.06 mmol) and acrylamide (175.75 mg, 2.47 mmol) were dissolved in tetrahydrofuran (20 mL) at room temperature under nitrogen atmosphere. The mixture was cooled to 0°C, and a solution of potassium tert-butoxide in tetrahydrofuran (1 M, 3.09 mL) was added dropwise thereto. The reaction mixture was stirred and reacted at 0°C for 1.5 hours. After the reaction was completed, the reaction mixture was added with 0.05 M hydrochloric acid (70 mL), and extracted with ethyl acetate (40 mL × 2). The organic phases were combined, washed with saturated brine (40 mL), dried over anhydrous sodium sulfate, and filtered. The filtrate was concentrated under reduced pressure to obtain a crude product. The crude product was purified by column chromatography (eluent: petroleum ether/ethyl acetate = 5/1 to 1/1) to obtain compound **WX028-5.** MS-ESI *m*/*z*: 472.2 $[M+H]^+$. $^1H$ NMR (400 MHz, $CDCl_3$) δ: 9.83 (s, 1H), 8.49 (d, *J*=6.4 Hz, 1H), 8.10 (s, 1H), 7.42 (d, *J*=7.6 Hz, 1H), 7.35 (t, *J*=7.8 Hz, 1H), 4.34 (dd, *J*=5.0 Hz, 9.0 Hz, 1H), 3.66-3.52 (m, 4H), 3.27 (s, 2H), 3.06-2.95 (m, 1H), 2.83-2.73 (m, 1H), 2.71-2.56 (m, 5H), 2.53-2.43 (m, 1H), 1.49 (s, 9H).

Step **6:** Synthesis of trifluoroacetate of compound **WX028-6**

Compound **WX028-5** (3 g, 6.36 mmol) was dissolved in dichloromethane (40 mL) at room temperature, and trifluoroacetic acid (9.24 g, 81.04 mmol) was added dropwise thereto. The reaction mixture was stirred at room temperature for 4 hours. After the reaction was completed, the mixture was concentrated under reduced pressure to remove the solvent to obtain the trifluoroacetate of compound **WX028-6**. MS-ESI *m*/*z*: 372.0 $[M+H]^+$.

Step **7:** Synthesis of compound **WX028-7**

Compound **WX024-1** (0.2 g, 287.14 μmol) was dissolved in acetonitrile (4 mL) at room temperature under nitrogen atmosphere. The trifluoroacetate of compound **WX028-6** (225.35 mg, 315.85 μmol) and N, N-diisopropylethylamine (259.78 mg, 2.01 mmol) were added thereto. The reaction mixture was heated to 90°C, and stirred and reacted for 12 hours. After the reaction was completed, the reaction mixture was cooled to room temperature, poured into water (30 mL), and extracted with ethyl acetate (50 mL × 3). The organic phase was washed with saturated brine (50 mL×3), dried over anhydrous sodium sulfate, and filtered. The filtrate was concentrated under reduced pressure to remove the remaining solvent. The resulting residue was added with methyl tert-butyl ether (3 mL), stirred at room temperature for 0.5 hours, and filtered. The filter cake was rinsed with methyl tert-butyl ether (3 mL × 2), collected, and dried under vacuum to obtain compound **WX028-7**. MS-ESI *m*/*z:* 940.5 $[M+H]^+$

Step **8**: Synthesis of hydrochloride of compound **WX028**

Compound **WX028-7** (0.21 g, 223.40 μmol) was dissolved in ethyl acetate (2 mL) at room temperature, and hydrochloric acid/ethyl acetate (5 mL, 4 M) was added thereto. The reaction mixture was stirred and reacted at room temperature for 1 hour. After the reaction was completed, the reaction mixture was filtered. The filter cake was rinsed with ethyl acetate (3 mL × 3), and collected. The filter cake was separated by preparative high performance liquid chromatography (chromatographic column: Phenomenex Luna 80 * 30 mm * 3 μm; mobile phase: water (0.04% hydrochloric acid))-acetonitrile; acetonitrile%: 1% to 30%, 8 minutes) to obtain the hydrochloride of the target compound **WX028.** MS-ESI *m/z*: 840.4 $[M+H]^+$. $^1H$ NMR (400 MHz, MeOD_$d_4$) δ: 8.80 (s, 1H), 8.26 (s, 1H), 8.13 (d, *J*=7.6 Hz, 1H), 7.97 (d, *J*=7.2 Hz, 1H), 7.71 (d, *J*=0.8 Hz, 1H), 7.63 (d, *J*=8.0 Hz, 1H), 7.45 (dd, J=1.4 Hz, 7.0 Hz, 1H), 7.39 (t, *J*=7.8 Hz, 1H), 6.93 (t, *J*=54.4 Hz, 1H), 4.56 (dd, *J*=5.0, 11.0 Hz, 1H), 4.32-4.20 (m, 1H), 4.09-3.97 (m, 2H), 3.87-3.41 (m, 8H), 3.29 (s, 2H), 3.25-3.16 (m, 2H), 2.86-2.77 (m, 2H), 2.65-2.50 (m, 1H), 2.43-2.34 (m, 1H), 2.23 (d, *J*=12.0 Hz, 2H), 2.11 (d, *J*=13.2 Hz, 2H), 2.06-1.88 (m, 3H), 1.45-1.30 (m, 2H), 1.28-1.14 (m, 1H), 0.77-0.64 (m, 2H), 0.46-0.35 (m, 2H).

**Example 29**

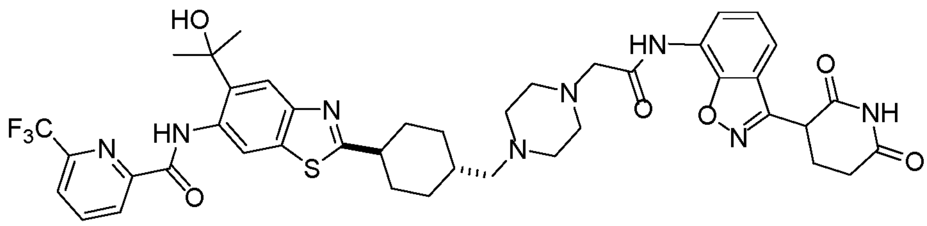

**WX029**

Synthetic route:

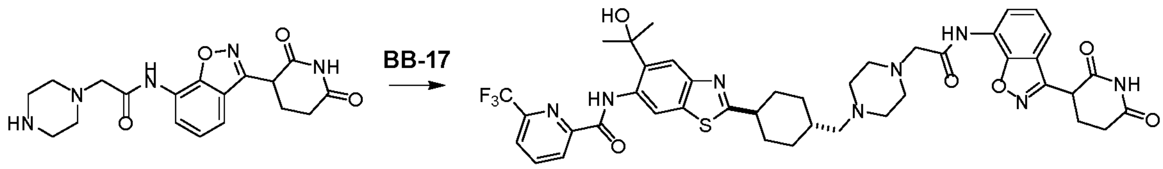


**WX028-6** **WX029**

Step **1:** Synthesis of formate of compound **WX029**

The trifluoroacetate of compound **WX028-6** (170 mg, 238.28 μmol) was dissolved in acetonitrile (2 mL) at room temperature under nitrogen atmosphere, then N, N-diisopropylethylamine (153.97 mg, 1.19 mmol, 207.51 μL) and compound **BB-17** (143.78 mg, 238.28μmol) were added thereto. The reaction mixture was heated to 90°C, and stirred and reacted for 12 hours. After the reaction was completed, the reaction mixture was concentrated under reduced pressure to remove the solvent. The resulting residue was separated by preparative high performance liquid chromatography (chromatographic column: Phenomenex Luna C18 75 * 30 mm * 3 μm; mobile phase: water (0.2% formic acid)-acetonitrile; acetonitrile%: 20% to 50%, 8 minutes) to obtain the formate of the target compound **WX029.** MS-ESI *m/z*: 847.3 $[M+H]^+$. $^1H$ NMR (400 MHz, DMSO_$d_6$) δ: 12.55 (s, 1H), 11.12 (s, 1H), 10.02 (s, 1H), 9.07 (s, 1H), 8.46 (*J*=7.6 Hz, 1H), 8.38 (t, *J*=8.0 Hz, 1H), 8.18 (dd, *J*=0.6, 7.8 Hz, 1H), 8.15 (s, 1H), 8.10 (d, *J*=7.6 Hz, 1H), 7.89 (s, 1H), 7.59 (dd, *J*=0.8 Hz, 8.0 Hz, 1H), 7.36 (t, *J*=7.8 Hz, 1H), 6.07 (s, 1H), 4.63 (dd, *J*=4.8 Hz, 12.0 Hz, 1H), 3.25 (s, 2H), 3.11-2.99 (m, 1H), 2.85-2.72 (m, 1H), 2.70-2.51 (m, 8H), 2.48-2.43 (m, 2H), 2.28-2.13 (m, 5H), 1.93 (d, *J*=11.2 Hz, 2H), 1.71-1.52 (m, 9H), 1.17-0.99 (m, 2H).

**Example 30 and example 31**

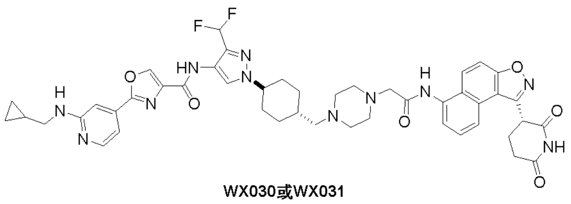

WX030或WX031

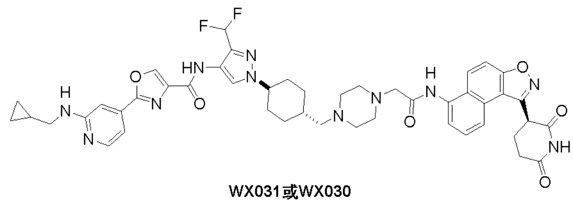

WX031或WX030

Synthetic route:

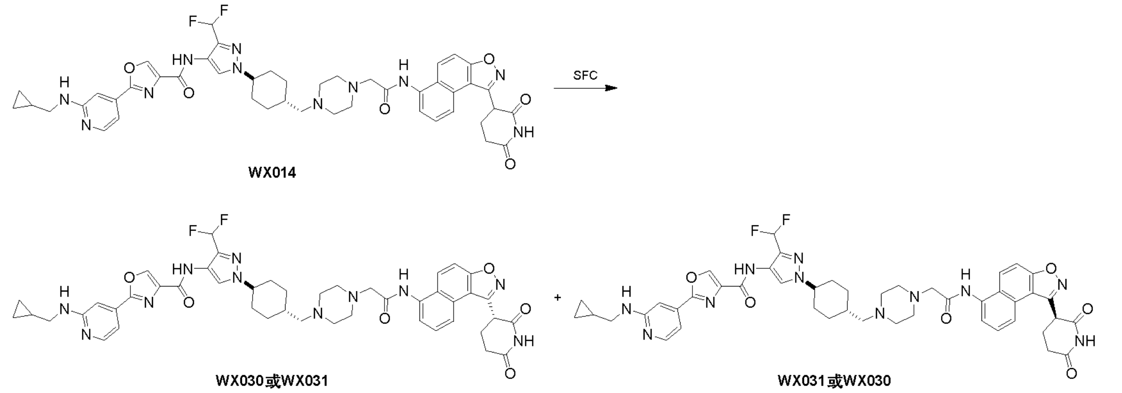


WX014

WX030或WX031 + WX031或WX030

Synthesis of hydrochloride of compound **WX030** or hydrochloride of compound **WX031**

The hydrochloride of compound **WX014** (480 mg, 518.13 μmol) was separated by supercritical fluid chromatography (separation conditions, chromatographic column: REGIS(S,S)WHELK-O1(250 mm * 25 mm,10 μm); mobile phase: A:$CO_2$; B: EtOH/ACN (0.1% IPAm, v/v); B%: 65%-65%, 15 minutes) and the samples with a retention time of 1.576 minutes were collected and then separated by preparative high performance liquid chromatography (chromatographic column: Phenomenex Luna 80 * 30 mm * 3 μm; mobile phase: water (0.04% hydrochloric acid) -acetonitrile; acetonitrile%: 1%-30%, 8 minutes) to obtain the hydrochloride of the target compound **WX030** (ee%: 99.12%). The samples with a retention time of 2.770 minutes were collected, then separated by preparative high performance liquid chromatography (chromatographic column: Phenomenex Luna 80 * 30 mm * 3 μm; mobile phase: water (0.04% hydrochloric acid))-acetonitrile; acetonitrile %: 1% to 30%, 8 minutes) to obtain the hydrochloride of the target compound **WX031** (ee%: 96.36%). The SFC analysis method corresponding to the retention time as described above: chromatographic column: (S,S)-WHELK-O1, 50 × 4.6 mm I.D., 3.5 μm; mobile phase: A: $CO_2$; B: EtOH: ACN = 1:1 (0.1% IPAm, v/v), 4 min.

Synthesis of hydrochloride of compound **WX030:** $^1$H NMR (400 MHz, MeOD_*$d_4$*) δ: 11.15 (s, 1H), 10.62 (s, 1H), 9.88 (s, 1H), 9.11 (s, 1H), 8.34 (d, *J*=9.2 Hz, 1H), 8.20 (s, 1H), 8.18-8.12 (m, 1H), 8.08 (d, *J*=6.4 Hz, 1H), 8.00 (d, *J*=9.6 Hz, 1H), 7.80-7.70 (m, 2H), 7.66-7.54 (m, 1H), 7.36-7.03 (m, 2H), 5.10 (dd, *J*=4.8 Hz, 11.2 Hz, 1H), 4.38-4.20 (m, 2H), 4.18-3.27 (m, 12H), 3.18-2.98 (m, 2H), 2.93-2.80 (m, 1H), 2.72-2.54 (m, 2H), 2.45-2.35 (m, 1H), 2.17-1.99 (m, 4H), 1.97-1.66 (m, 3H), 1.33-1.06 (m, 3H), 0.63-0.48 (m, 2H), 0.38-0.27 (m, 2H).

Synthesis of hydrochloride of compound **WX031:** $^1$H NMR (400 MHz, MeOD_*$d_4$*) δ: 11.15 (s, 1H), 10.44 (s, 1H), 9.86 (s, 1H), 9.09 (s, 1H), 8.31 (d, *J*=9.2 Hz, 1H), 8.20 (s, 1H), 8.18-8.12 (m, 1H), 8.09 (d, *J*=6.4 Hz, 1H), 8.00 (d, *J*=9.2 Hz, 1H), 7.79-7.69 (m, 2H), 7.61-7.47 (m, 1H), 7.34-7.02 (m, 2H), 5.10 (dd, *J*=4.8 Hz, 11.2 Hz, 1H), 4.40-4.21 (m, 2H), 4.18-3.27 (m, 12H), 3.15-2.95 (m, 2H), 2.94-2.79 (m, 1H), 2.71-2.58 (m, 2H), 2.45-2.35 (m, 1H), 2.13-1.62 (m, 7H), 1.32-1.06 (m, 3H), 0.61-0.50 (m, 2H), 0.38-0.24 (m, 2H).

**Bioassay**

**Experimental example 1: Evaluation of target protein degradation effect in K562 IRAK4-HiBiT cells**

**Experimental objective:** The experiment aims to evaluate the degradation effect of the test compounds on the target protein IRAK4 in K562 IRAK4-HiBiT cells.

**Experimental materials:**

1. Cells and culture medium

Cells: K562 IRAK4-HiBiT cells

Culture medium: RPMI 1640 + 10% fetal bovine serum + 2 mM glutamine + 1 mM sodium pyruvate + penicillin/streptomycin.

Positive control: 1000 nM; negative control: 0.1% DMSO.

Table **1** reagents and consumables.

| Reagents and consumables | Manufacturers | Cat. No. |
|---|---|---|
| RPMI 1640 | Gibco | Cat # 31800 |
| Fetal bovine serum (FBS) | Biosera | #FB-1058/500 |
| Penicillin/streptomycin | Biosera | #LM-A4118 |
| DPBS | Invitrogen | #14190 |
| 0.25% Trypsin/EDTA solution | Invitrogen | #25200 |
| Glutamine | Invitrogen | Cat # 35050061 |
| Sodium pyruvate | Invitrogen | Cat# 11360070 |
| Nano-Glo ® HiBiT assay kit | Promega | promega# N3040 |
| 384-well white plate, flat-bottom | Corning | Cat # 3570 |
| 384_LDV compound plate | Labcyte | Cat# LP-0200 |

Table **2** Instruments

| Instrument name | Manufacturers | Instrument type |
|---|---|---|
| ECHO liquid handler | Labcyte | Echo 550 |
| Bravo automated liquid handling workstation | Agilent | 16050-101 |
| Envision plate reader | Perkin Elmer | 2104 |
| Autosampler | Thermofisher | Multidrop Combi |
| Cell counter | Thermo | Countess II FL |

**Experimental scheme:**

**Day 1**

1. Preparation of compounds

(1). The powder of the compound to be tested was dissolved in DMSO to a storage concentration of 10 mM. A total of 9 μL of the 10 mM of the compound to be tested was manually pipetted into the 1st and 13th columns of the LDV plate using a pipettor;
(2). A volume of 6 μL of DMSO was added to columns 2-12 and 14-24 using a Multidrop Combi;
(3). A 3-fold dilution of the test compounds (3 μL + 6 μL) was performed using Bravo, for columns 1-11 and 13-23;
(4). According to the plate layout, 25 nL of compound solution (columns 1-24 of the LDV plate) was transferred to the experimental plate using Echo;
(5) 25 nL of the 1 mM positive control solution was transferred to the assay plate as a 100% degradation control (i.e. LC, HPE), and 25 nL of DMSO was transferred to the assay plate as a 0% control (i.e. HC, ZPE).

2. Cell plating

(1) The culture medium was discarded, and the cells were washed once with DPBS, digested with trypsin, counted, and prepared into a cell suspension of $2\times10^{-5}$ cells/mL.
(2) Using the MultiDrop Combi at a medium speed, 25 μL/well of the cell suspension was added to the experimental plate containing the test compounds;
(3) The experimental plate containing cells was returned to the incubator and cultured for 16 to 18 hours at 37°C and 5% $CO_2$.

**Day 2**

(1). Using the MultiDrop Combi at a high speed, 25 μL/well of the assay reagent (NanoGlo lytic solution + substrate + LgBit protein) was added to the assay plate, followed by shaking for 10 minutes;
(2) The plate was centrifuged at 2000 rpm for 1 minute to remove any bubbles;
(3) Readings were obtained by using an Envision, US Luminescence reader.

3. Data analysis

To calculate the degradation rate (DR) of the test compounds, the following formula is used: IR (%) = (RLU vehicle control - RLU compound) / (RLU vehicle control - RLU positive control) × 100%. The solvent control refers to the blank control. The degradation rates of compounds at different concentrations were calculated in Excel, and then XLFit software was used to plot inhibition curves and calculate relevant parameters, including minimum degradation rate, maximum degradation rate, and $DC_{50}$.
The test results are shown in Table **3.**

Table **3** Degradation effect of compounds of the present disclosure on target proteins in K562 IRAK4-HiBiT cells

| Compound No. | $DC_{50}$ (nM) | Maximum degradation rate (%) |
|---|---|---|
| Hydrochloride of **WX001** | 76.43 | 54.34 |
| Hydrochloride of **WX002** | 13.54 | 100.04 |
| Hydrochloride of **WX013** | 48.70 | 101.24 |
| Hydrochloride of **WX014** | 4.87 | 108.46 |
| Trifluoroacetate of **WX010** | 50.24 | 81.68 |
| Trifluoroacetate of **WX011** | 9.60 | 104.57 |
| **WX007** | 35.09 | 97.41 |
| Hydrochloride of **WX008** | 121.69 | 82.20 |
| Hydrochloride of **WX015** | 47.08 | 100.00 |
| Hydrochloride of **WX016** | 70.73 | 93.12 |
| Formate of **WX017** | 4.00 | 73.88 |
| Hydrochloride of **WX018** | 7.91 | 119.43 |
| Formate of **WX019** | 15.08 | 92.05 |
| Formate of **WX020** | 29.86 | 75.09 |
| Formate of **WX023** | 7.16 | 114.38 |
| Formate of **WX024** | 1.75 | 117.09 |
| Hydrochloride of **WX025** | 2.53 | 105.26 |
| Formate of **WX026** | 0.78 | 80.30 |

(continued)

| Compound No. | $DC_{50}$ (nM) | Maximum degradation rate (%) |
|---|---|---|
| Hydrochloride of **WX028** | 2.11 | 1113.90 |
| Formate of **WX029** | 4.26 | 111.86 |
| Hydrochloride of **WX030** | 4.18 | 110.35 |
| Hydrochloride of **WX031** | 2.91 | 107.77 |

Conclusion: the compounds of the present disclosure exhibit excellent target protein degradation effects in K562 IRAK4-HiBiT cells.

**Experiment example 2: Analysis of IKZF1 and IKZF3 protein expression levels in MM.1S cells using In Cell Western**

**Experimental objective:** The experiment aims to assess the degradation effect of the test compounds on IKZF 1 and IKZF3 proteins in MM.1S cells by evaluating the impact of the test compounds on the expression levels of IKZF1 and IKZF3 proteins in MM.1S cells.

**Experimental materials:**

Cell lines: MM.1S cell (sourced from ATCC; Cat. No CRL-2974)
Negative control: 0.1% DMSO.

Table **4** Reagents and consumables.

| Reagents and consumables | Supplier | Cat. No. |
|---|---|---|
| 96-well black-bottomed microplate | Jing An Biological | J09603 |
| D-PBS buffer | Bioleaper | P220610030051 |
| Tissue fixative | Meilumbio | MA0192-Jan-28G |
| TritonX-100 Solution (10% sterile) | Beyotime | ST797-500ML |
| Intercept Blocking Buffer | LI-COR | 211114 |
| Ikaros (D6N9Y) Rabbit mAb | Cell Signaling | 14859S |
| Aiolos (D1C1E) Rabbit mAb | Cell Signaling | 15103S |
| GAPDH mouse mAb | proteintech | 60004-1-Ig |
| Tween 20 | Sinopharm Chemical Reagent Co., Ltd | 30189328 |
| IRDye 800CW Goat antimouse | LI-COR | D00812-08 |
| IRDye 680RD Goat anti-Rabbit | LI-COR | D00891-05 |

Table **5** Instruments

| Instrument name | Supplier | Instrument type |
|---|---|---|
| Dual-color infrared laser imager | LI-COR | Odyssey-CLx |
| Decolorization shaker | QILNBEIER | TS-1000 |
| refrigerator | HAIER | BCD-256KT |

**Experimental scheme:**

1) Log phase MM.1S cells were plated in a 96-well plate at $1.2\times10^5$ cells per well and cultured overnight;
2) The next day, compounds were added with a starting concentration of 300 nM, diluted threefold across three replicate wells for 10 concentration gradients (including DMSO), and the cells were incubated for 24 hours in an incubator;
3) The cells were centrifuged, and the supernatant was carefully removed. 150 μL of 4% paraformaldehyde fixative was added along the wall of the wells, avoiding contact with the bottom cells, and the cells were incubated at room temperature for 20 minutes;
4) Lysis buffer was prepared by mixing 0.5 mL of 10% Triton X-100 with 49.5 mL of PBS until homogenous;
5) 200 μL of lysis buffer was added along the well walls, again avoiding the bottom cells, and incubated on a shaker at room temperature for 5 minutes;
6) The wells were washed four times;
7) 150 μL of Licor INERCEPT Blocking Buffer was added along the well walls, avoiding the bottom cells, and the cells were incubated on a shaker at room temperature for 1.5 hours;
8) Ikaros (D6N9Y) Rabbit mAb, Aiolos (D1C1E) Rabbit mAb and GAPDH mouse mAb (proteintech, 60004-1-Ig) were diluted at a 1:100 ratio using antibody dilution buffer;
9) 50 μL of mixed antibody was added to each well with three replicate wells, and the cells were incubated overnight on a shaker at 4°C;
10) PBST (PBS with 0.1% Tween 20) was prepared;
11) The primary antibodies were removed, and 200 μL of PBST was added along the well walls, avoiding the bottom cells, and the cells were incubated on a shaker at room temperature for 5 minutes;
12) The wells were washed four times;
13) Secondary antibody dilution buffer was prepared by adding Tween 20 to Licor INTERCEPT Blocking Buffer to a final concentration of 0.2%;
14) Fluorescent secondary antibodies were diluted in the dark at a 1:800 ratio. To 400 μL of secondary antibody dilution solution, 0.5 μL of each IRDye 800CW and IRDye 680CW (corresponding to the primary antibodies) was added.
15) 50 μL of the diluted fluorescent secondary antibodies was added to each well and the cells were incubated on a shaker at room temperature in the dark for 60 minutes;
16) The secondary antibodies were removed, and 200 μL of PBST was added along the well walls, avoiding the bottom cells, and the cells were incubated on a shaker at room temperature in the dark for 5 minutes;
17) The wells were washed four times; immediately after washing, the Odyssey Gel Imaging System was used for detection at dual wavelengths of 700 nm and 800 nm.

3. Data analysis

Using GraphPad Prism 6 software, inhibition rate data was entered to fit the curve and calculate the $DC_{50}$ value.

$$\text{Protein inhibition rate} = (1 - RLs/RLv) * 100\%$$

RR (Raw Ratio) = 700 nm/800 nm

RLs = RR of sample-treated cells

RLv=RR of solvent-treated cells

The test results are shown in Table **6.**

Table **6** Degradation effects of compounds of the present disclosure on IKZF1 and IKZF3 proteins in MM.1S cells

| Compound No. | Protein | $DC_{50}$ (nM) | Maximum degradation rate Dmax (%) |
|---|---|---|---|
| Hydrochloride of **WX014** | IKZF1 | 23.40 | 72.32 |
| | IKZF3 | 20.64 | 73.46 |
| Hydrochloride of **WX018** | IKZF1 | 14.50 | 88.81 |
| | IKZF3 | 8.34 | 96.49 |
| Hydrochloride of **WX030** | IKZF1 | 27.38 | 71.41 |

(continued)

| Compound No. | Protein | $DC_{50}$ (nM) | Maximum degradation rate Dmax (%) |
|---|---|---|---|
| Hydrochloride of **WX031** | IKZF1 | 20.74 | 71.89 |
| Hydrochloride of **WX008** | IKZF1 | 4.66 | 81.43 |
| Hydrochloride of **WX015** | IKZF1 | 7.70 | 81.84 |
| Hydrochloride of **WX016** | IKZF1 | 6.02 | 81.63 |
| Formate of **WX017** | IKZF1 | 15.68 | 62.94 |
| Formate of **WX020** | IKZF1 | 3.77 | 85.16 |
| **WX021** | IKZF1 | 2.66 | 87.59 |
| Formate of **WX023** | IKZF1 | 31.33 | 57.20 |
| Formate of **WX024** | IKZF1 | 35.17 | 60.75 |
| Hydrochloride of **WX025** | IKZF1 | 14.39 | 72.20 |
| Formate of **WX026** | IKZF1 | 5.64 | 84.43 |
| Hydrochloride of **WX028** | IKZF1 | 26.71 | 49.69 |
| Formate of **WX029** | IKZF1 | 38.83 | 61.76 |

**Conclusion:**

The compounds of the present disclosure exhibit excellent target protein degradation effects on IKZF 1 and IKZF3 proteins in MM.1S cells.

**Experiment example 3: Evaluation of antiproliferative effects in lymphoma cell lines OCI-LY10 and TMD-8**

**Experimental purpose:** The experiment aims to evaluate the inhibitory effects of the test compounds on cell proliferation in the diffuse large B-cell lymphoma (DLBCL) cell lines **OCI-LY10** and **TMD-8.**

**Experimental materials:**

Table **7** Cell line and culture method

| Cell line | Tumor type | Growth characteristics | Culture medium |
|---|---|---|---|
| OCI-LY10 | Lymphoma | Suspension | IMDM + 20% FBS + 55 μM β-mercaptoethanol |
| TMD-8 | Lymphoma | Suspension | MEM + 10%FBS |

Table **8** Culture media and reagents

| Culture media and reagents | Manufacturers | Cat. No. |
|---|---|---|
| Dulbecco's PBS | Hyclone | SH30256.01 |
| Fetal bovine serum (FBS) | GIBCO | 10099-141 |
| Antibiotic-antimycotic | GIBCO | 15240-062 |
| 0.25% Trypsin | GIBCO | 25200072 |
| DMSO | SIGMA | D2650 |
| β-mercaptoethanol | SIGMA | 60-24-2 |
| MEM | GIBCO | 11095-080 |

(continued)

| Culture media and reagents | Manufacturers | Cat. No. |
|---|---|---|
| IMDM | GIBCO | 12440-053 |

1. Multi-well plate

Greiner CELLSTAR® 96-well plate, flat-bottom white plate (with lid and clear bottom), #3610.

2. Reagents and instruments for cell viability assay

(1) Promega CellTiter-Glo luminescent cell viability assay kit (Promega-G7573).
(2) 2104 EnVision® plate reader, PerkinElmer.

**Experimental scheme:**

1. Cell culture

The tumor cell lines were cultured in a 37°C, 5% $CO_2$ incubator under the above culture conditions. Regular passage, and cells in logarithmic growth phase were taken for plating.

2. Cell plating

(1) Cells were stained with trypan blue and viable cells were counted.
(2) The cell concentration was adjusted to an appropriate concentration.

Table **9** Cell line and seeding density

| **Cell line** | **Seeding density (cells/well)** |
|---|---|
| OCI-LY10 | 5000 |
| TMD-8 | 5000 |

(3) 100 $\mu$L of cell suspension per well was added to the culture plate as shown above.
(4) The culture plate was cultured overnight in a 37°C, 5% $CO_2$ incubator with 100% relative humidity.

3. Preparation of compound storage plate

Preparation of stock solution storage plate with 1000 times the initial concentration of the compound: The compound was serially diluted with DMSO from the highest concentration to the lowest concentration. The solution was prepared as needed each time.

4. Preparation of 1000x compound working solution and cell treatment with compounds

(1) Preparation of the working solution with 5 times the initial concentration of the compound: For 3-fold dilution of the compound, 30 $\mu$L of the compound from a stock solution at 1000-fold concentration was taken, and 20 $\mu$L of DMSO was added to the subsequent wells. Then, 10 $\mu$L was transferred from one concentration to the next in sequence. For 5-fold dilution of the compound, 30 $\mu$L of the compound from a stock solution at 1000-fold concentration was taken, and 24 $\mu$L of DMSO was added to the subsequent wells. Then, 6 $\mu$L was transferred from one concentration to the next in sequence. 20 $\mu$L of DMSO was added to the vehicle control. The 1000-fold concentrated compound was diluted 200 times with culture medium, i.e., 1 $\mu$L of the 1000-fold diluted compound is added to 199 $\mu$L of culture medium, and the mixture was homogenized using a pipette.

(2) Compound addition: 25 μL of the 5-fold compound solution was added to the cell culture plate.
(3) The 96-well cell plate was returned to the incubator to culture OCI-LY10 (with either 3-fold or 5-fold dilution, incubated with compounds added for 5 days) and TMD-8 (with either 3-fold or 5-fold dilution, incubated with compounds added for 5 days).

5. CellTiter-Glo luminescent cell viability assay

The following steps were performed according to the instructions of Promega CellTiter-Glo luminescent cell viability assay kit (Promega-G7573).

(1) CellTiter-Glo buffer was melted and brought to room temperature.
(2) CellTiter-Glo substrate was brought to room temperature.
(3) CellTiter-Glo working solution was prepared by adding 10 mL of CellTiter-Glo buffer to a vial of CellTiter-Glo substrate to dissolve the substrate.
(4) The mixture was slowly vortexed and shaken to be fully dissolved.
(5) The cell culture plate was taken out and left for 30 minutes to equilibrate to room temperature.
(6) 60 μL of CellTiter-Glo working solution was added per well (equal to half the volume of cell culture medium per well). The cell plate was wrapped in aluminum foil to be protected from light.
(7) The culture plate was shaken on an orbital shaker for 2 minutes to induce cell lysis.
(8) The culture plate was left at room temperature for 10 minutes to stabilize the luminescent signal.
(9) The luminescent signal was detected on a 2104 EnVision plate reader.

6. Data analysis

The inhibition rate (IR) of the test compound was calculated using the following formula: IR (%) = (1-RLU compound/RLU vehicle control) * 100%. The inhibition rates of compounds at different concentrations were calculated in Excel, and then GraphPad Prism software was used to plot inhibition curves and calculate relevant parameters, including minimum inhibition rate, maximum inhibition rate, and $IC_{50}$.
The test results are shown in Table **10.**

Table **10** Inhibitory effect of compounds of the present disclosure on cell proliferation in OCI-LY10 and TMD-8 cell lines

| **Compound No.** | OCI-LY10 | | TMD-8 | |
|---|---|---|---|---|
| | $IC_{50}$ (nM) | Maximum inhibition rate (%) | $IC_{50}$ (nM) | Maximum inhibition rate (%) |
| Hydrochloride of **WX002** | 192.20 | 63.27 | 92.0 | 83.31 |
| Hydrochloride of **WX014** | 13.66 | 94.54 | 10.34 | 89.86 |
| Trifluoroacetate of **WX011** | 17.02 | 93.42 | 17.03 | 94.65 |
| Hydrochloride of **WX018** | 6.99 | 99.92 | 12.85 | 99.53 |
| Hydrochloride of **WX028** | 85.25 | 99.01 | / | / |
| Hydrochloride of **WX025** | 24.39 | 97.39 | / | / |
| Formate of **WX020** | 24.97 | 94.96 | / | / |
| Formate of **WX026** | / | / | 8.65 | 99.92 |
| "/" indicates not tested. | | | | |

**Conclusion:** The compounds of the present disclosure exhibit excellent inhibitory effect on cell proliferation in both lymphoma cell lines OCI-LY10 and TMD-8.

**Experiment example 4: Evaluation of antiproliferative effects in lymphoma cell line SU-DHL-2**

**Experimental purpose:** The experiment aims to evaluate the inhibitory effects of the test compounds on cell proliferation in the lymphoma cell line **SU-DHL-2.**

Table **11** Cell line and culture method

| Cell line | Tumor type | Growth characteristics | Culture method |
|---|---|---|---|
| SU-DHL-2 | Lympho ma | Suspension | RPMI1640 + 10% FBS (EXCELL) + 1% penicillin/streptomycin |

Table **12** Culture media and reagents

| Culture media and reagents | Manufacturers | Cat. No. |
|---|---|---|
| RPMI 1640 | GIBCO | 22400-089 |
| Dulbecco's PBS | Thermo | SH30028.02B |
| Fetal bovine serum (FBS) | Hyclone | 11H233 |
| Antibiotic-antimycotic | GIBCO | 15240-062 |
| DMSO | SIGMA | D2650 |

1. Multi-well plate

Greiner CELLSTAR 384-well plate, flat-bottom black plate (with lid), #781090.

2. Reagents and instruments for cell viability assay

(1) Promega CellTiter-Glo luminescent cell viability assay kit (Promega-G7573).
(2) 2104 EnVision® plate reader, PerkinElmer.

**Experimental scheme:**

1. Cell culture

The tumor cell lines were cultured in a 37°C, 5% $CO_2$ incubator under the above culture conditions. Regular passage, and cells in logarithmic growth phase were taken for plating.

2. Cell plating

(1) Cells were stained with trypan blue and viable cells were counted.
(2) The cell concentration was adjusted to an appropriate concentration.

Table **13** Cell line and seeding density

| **Cell line** | **Seeding density (cells/well)** |
|---|---|
| SU-DHL-2 | 1500 |

(3) 50 μL of cell suspension per well was added to the culture plate as shown in the figure above.
(4) The culture plate was cultured overnight in a 37°C, 5% $CO_2$ incubator with 100% relative humidity.

3. Preparation of compound storage plate

Compounds were added using the Echo655 instrument. The volume of compound added was 50 nL, with a

final DMSO concentration of 0.1%. The culture plate was centrifuged at 1000 rpm for 1 minute, then incubated for 4 days in an incubator at 37°C, 5% CO2, and with 100% relative humidity.

4. CellTiter-Glo luminescent cell viability assay

The following steps were performed according to the instructions of Promega CellTiter-Glo luminescent cell viability assay kit (Promega-G7573).

(1) CellTiter-Glo buffer was melted and brought to room temperature.
(2) CellTiter-Glo substrate was brought to room temperature.
(3) CellTiter-Glo working solution was prepared by adding CellTiter-Glo buffer to a vial of CellTiter-Glo substrate to dissolve the substrate;
(4) The mixture was slowly vortexed and shaken to be fully dissolved.
(5) The cell culture plate was taken out and left for 30 minutes to equilibrate to room temperature.
(6) 25 $\mu$L of CellTiter-Glo working solution was added per well (equal to half the volume of cell culture medium per well). The cell plate was wrapped in aluminum foil to be protected from light.
(7) The culture plate was shaken on an orbital shaker for 2 minutes to induce cell lysis.
(8) The culture plate was left at room temperature for 10 minutes to stabilize the luminescent signal.
(9) The luminescent signal was detected on a 2104 EnVision plate reader.

5. Data analysis

The inhibition rate (IR) of the test compound was calculated using the following formula: IR (%) = (1-(RLU compound/RLU blank control)/(RLU vehicle control - RLU blank control) $\times$ 100%. The inhibition rates of compounds at different concentrations were calculated in Excel, and then GraphPad Prism software was used to plot inhibition curves and calculate relevant parameters, including minimum inhibition rate, maximum inhibition rate, and $IC_{50}$.

Table **14** Inhibitory effect of compounds of the present disclosure on cell proliferation in SU-DHL-2 cell line

| Compound No. | SU-DHL-2 $IC_{50}$ (nM) | Maximum inhibition rate (%) |
|---|---|---|
| Hydrochloride of **WX018** | 11.74 | 100.27 |
| Hydrochloride of **WX004** | 64.52 | 99.05 |
| Formate of **WX026** | 0.91 | 100.08 |
| Hydrochloride of **WX014** | 28.70 | 93.55 |
| Trifluoroacetate of **WX011** | 25.20 | 99.89 |

**Conclusion:**

The compounds of the present disclosure exhibit excellent inhibitory effect on cell proliferation in lymphoma cell line SU-DHL-2.

**Experimental example 5: Pharmacokinetic evaluation of compounds in rats**

**Experimental purpose:**

In this study, C57BL/6N or CD-1 male mice were selected as test animals, and LC/MS/MS method was used to quantify plasma concentrations of the test compound intravenously injected or gavage to mice at different time points, so as to evaluate the pharmacokinetic profile of the compounds of the present disclosure in mice.

**Experimental materials:**

C57BL/6N mice (male, 20 to 30 g, 7 to 10 weeks old, Beijing VitalRiver) or CD-1 mice (male, 20 to 35g, 7 to 10 weeks old, Beijing VitalRiver).

**Experimental operation A:**

The test compounds, either as clear or suspension solutions, were administered intravenously through the tail vein to C57BL/6N mice or CD-1 mice (either fasted overnight or fed) (vehicle: 10% DMSO/10% solutol/80% $H_2O$ or 5% DMSO/10% solutol/85% $H_2O$), or intragastrically administered to C57BL/6N mice or CD-1 mice (either fasted overnight or fed). For intravenous injection, blood 50 μL was collected via the cheek vein puncture at 0 h (pre-administration), 0.083, 0.25, 0.5, 1, 2, 4, 6, 8, and 24 h after administration, and placed in an anticoagulant tube added with heparin sodium. The mixture was thoroughly vortexed and mixed, and centrifuged at 6000 g for 3 minutes at 2-8°C. For oral administration, blood was collected via the cheek vein puncture at 0 h (pre-administration), 0.083, 0.25, 0.5, 1, 2, 4, 6, 8, and 24 h after administration, and placed in an anticoagulant tube added with heparin sodium. The mixture was thoroughly vortexed and mixed, and centrifuged at 6000 g for 3 minutes at 2-8°C. Plasma concentrations were determined by LC-MS/MS method, and relevant pharmacokinetic parameters were calculated using Phoenix WinNonlin8.2.0 pharmacokinetic software with non-compartmental model linear-log trapezoidal method.

**Experimental operation B:**

The test compounds, as clear solutions, were administered intravenously through the tail vein to CD-1 mice (fasted overnight) (vehicle: 10% DMSO/10% solutol/80% $H_2O$ or 5% DMSO/10% solutol/85% $H_2O$), or intragastrically administered to CD-1 mice (fasted overnight). For intravenous injection, blood was collected via the saphenous vein puncture at 0 h (pre-administration), 0.083, 0.25, 0.5, 1, 2, 4, 8, and 24 h after administration, and placed in a EDTA-K2 anticoagulant tube. The mixture was thoroughly vortexed and mixed, and centrifuged at 3200 g for 10 minutes at 4°C to obtain plasma. For oral administration, blood was collected via the saphenous vein puncture at 0 h (pre-administration), 0.083, 0.25, 0.5, 1, 2, 4, 8, and 24 h after administration, and placed in a EDTA-K2 anticoagulant tube. The mixture was thoroughly vortexed and mixed, and centrifuged at 3200 g for 10 minutes at 4°C to obtain plasma. Plasma concentrations were determined by LC-MS/MS method, and relevant pharmacokinetic parameters were calculated using Phoenix WinNonlin 6.3 pharmacokinetic software with non-compartmental model linear-log trapezoidal method.

The test results are shown in Table **15.**

Table **15** Pharmacokinetic parameters of compounds of the present disclosure in mice

| Compoun d No. | Pharmacokinetic parameters in mice | | | | | | | | |
|---|---|---|---|---|---|---|---|---|---|
| | Intravenous injection (2 mg/kg) | | | | | Intragastric administration (10 mg/kg) | | | |
| | Experim ental operatio n: | Plasma clearan ce rate (mL/mi n/kg) | Elimin ation halflife (h) | Appare nt volum e of distrib ution (L/kg) | Area under drugtime curve (0-inf,μ M.h) | Peak concentr ation (μM) | Time to peak concentr ation (h) | Area under drugtime curve (0-inf,μ M.h) | Bioavail ability F (%) |
| Hydrochl oride of **WX002** (fasted overnight ) | B | / | / | | / | 0.64 | 1.00 | 2.00 | / |
| Hydrochl oride of **WX014** (fed) | A | 7.0 | 4.22 | 1.6 | 5.40 | 0.54 | 1.00 | 4.47 | 18.90 |
| Trifluoroa cetate of **WX011** (fasted overnight ) | A | 2.76 | 9.45 | 1.8 | 15.31 | 0.45 | 1.67 | 5.00 | 6.53 |
| Hydrochl oride of **WX018** (fasted overnight ) | B | 11.8 | 5.22 | 4.39 | 3.06 | 0.09 | 6.00 | 1.01 | 6.59 |
| Formate of **WX019** (fasted overnight ) | A | / | / | / | / | 0.34 | 3.33 | 5.10 | / |
| Formate of **WX017** (fasted overnight ) | B | 12.2 | 2.32 | 2.59 | 3.17 | 0.65 | 4.00 | 5.07 | 31.9 |

"/" indicates not tested.

**Conclusion:** the compounds of the present disclosure have high plasma system exposure ($AUC_{0-inf}$) when administered orally. The compounds of the present disclosure have superior pharmacokinetic properties in rodents like mice.

**Experimental example 7: Pharmacokinetic evaluation of compounds in Beagles**

**Experimental objective:**

In this study, male beagles were selected as test animals, and LC/MS/MS method was used to quantify plasma concentrations of the test compound intravenously injected or intragastrically administered to beagles at different time points, so as to evaluate the pharmacokinetic profile of the compounds of the present disclosure in beagles.

**Experimental materials:**

Beagle (male, 7 to 10 kg, Beijing Marshall Biotechnology Co., Ltd.).

**Experimental operation:**

A clear solution of the test compound was slowly injected into beagle via the peripheral vein (fed) (vehicle: 5% DMSO/10% solutol/85% $H_2O$ or 5% DMSO/10% solutol/85% $H_2O$), or intragastrically administered to beagles (fed). For intravenous injection, 0.5 mL blood was collected via the saphenous vein puncture at 0.083, 0.25, 0.5, 1, 2, 4, 6, 8, and 24 h after administration, and placed in a EDTA-2K anticoagulant tube. The mixture was centrifuged at 3200 g for 10 minutes at 2 to 8°C, and the supernatant was separated. For oral administration, 0.5 mL blood was collected via the saphenous vein puncture at 0.083, 0.25, 0.5, 1, 2, 4, 6, 8, and 24 h after administration, and placed in a EDTA-2K anticoagulant tube. The mixture was centrifuged at 3200 g for 10 minutes at 2 to 8°C, and the supernatant was separated. Plasma drug concentrations were determined by LC-MS/MS method, and relevant pharmacokinetic parameters were calculated using Phoenix WinNonlin 6.3 pharmacokinetic software with non-compartmental model linear-log trapezoidal method.

The test results are shown in Table **16.**

Table **16** Pharmacokinetic parameters of compounds of the present disclosure in beagles

| Compound No. | Pharmacokinetic parameters of beagles | | | | | | | |
|---|---|---|---|---|---|---|---|---|
| | Intravenous injection (0.5 mg/kg) | | | | Intragastric administration (5 mg/kg) | | | |
| | Plasma clearance rate (mL/min/kg) | Elimination halflife (h) | Apparent volume of distribution (L/kg) | Area under drugtime curve (0-inf,μM .h) | Peak concentration (μM) | Time to peak concentration (h) | Area under drugtime curve (0-inf, μM.h) | Bioavailability F (%) |
| **Hydrochloride of WX014** | 21.3 | 5.03 | 5.83 | 0.44 | 0.41 | 4.00 | 3.20 | 72.6 |

**Conclusion:**

The compounds of the present disclosure have relatively high plasma system exposure ($AUC_{0-inf}$) when administered orally. The compounds of the present disclosure have superior pharmacokinetic properties in non-rodents like beagles.

**Experimental example 8: *In vivo* drug efficacy evaluation of compounds in SCID mouse xenograft model of human B-Cell lymphoma OCI-LY10 cell**

**Experimental objective:**

The study evaluates the antitumor effect of the test compounds using a SCID mouse xenograft model of human B-cell lymphoma OCI-LY10 cells.

**Experimental materials:**

1. Experimental animals: SCID mice, female, 6 to 8 weeks old, weighing 17 to 20 g. Beijing Vital River Laboratory Animal Technology Co., Ltd.
2. Cell line: The human B-cell lymphoma OCI-LY10 cell line was purchased from Nanjing Cobioer Biosciences Co. Ltd (Cat. No CBP60558).

Table **17** Reagent information

| Name | Manufacturer | Batch number | Storage condition |
|---|---|---|---|
| IMDM culture medium | GIBCO Corporation (US) | 2323369 | 4°C |
| Fetal bovine serum (FBS) | GIBCO Corporation (US) | 2305262RP | -20°C |
| Matrix glue (Matrigel) | CORNING | 2062001 | -20°C |

Table **18** Instruments

| Name | Manufacturer | Type |
|---|---|---|
| SHJ series clean workbench | Shanghai Shangjing Purifying Equipment Co., Ltd. | CA-1390-1 |
| $CO_2$ water-jacketed cell culture incubator | Thermo Scientific Forma | 3111 |
| Inverted microscope | Olympus | CKX41SF |
| Electric aspirator | Shanghai MEDICAL Instruments (GROUP) Co., Ltd. | YX930D |
| Eppendorf AG centrifuge | Eppendorf | 5811XG639987 |
| Hundredth balance scale | Shanghai Minqiao Precise Instrument Co., Ltd. | SL502N |
| Thousandth balance scale | Shanghai Jinghai Instrument Co., Ltd. | JA2003N |
| Electronic digital caliper | SHAHE | (0 to 150) mm |

**Model building:**

OCI-LY10 cells were cultured in IMDM medium containing 20% FBS and maintained in a 37°C incubator with 5% $CO_2$ and saturated humidity. Log phase OCI-LY10 cells were collected, resuspended in IMDM basic medium, and mixed 1:1 with Matrigel. The cell concentration was adjusted to $4 \times 10^7$/mL. Under sterile conditions, 0.1 mL of the cell suspension was inoculated subcutaneously into the right flank of SCID mice, with an inoculation concentration of $4\times 10^6$/0.1 mL/mouse.

**Experimental scheme:**

For the pharmacological experiment, once the tumors reached a certain size, animals with tumors that were either too large, too small, or irregularly shaped were eliminated. Animals with tumor volumes between 167.65 and 231.29 $mm^3$ were selected. Based on tumor volume, the animals were divided into groups using a random grouping method, with each group consisting of six mice, and the average tumor volume was approximately 201.15 $mm^3$. The day of grouping was designated as Day 0, and dosing began according to the body weight of the animals. The pharmacological study period lasted for 28 days, with dosing once a day and a 24-hour interval between doses. Intragastric administration was used for administration. During the experiment, the body weight and tumor size of the animals were measured twice a week. Clinical symptoms were observed and recorded daily.

The test compounds were administered at doses of 10 mg/kg, 30 mg/kg, and 100 mg/kg, with a solvent composition of 10% DMSO/10% Solutol/80% $H_2O$. The formula for calculating tumor volume (TV) was: $1/2 \times a \times b^2$, where a and b were the measured length and width of the tumor, respectively. The formula for calculating the Tumor Growth Inhibition (TGI) rate was:: TGI (%) = [(1 - (average tumor volume at the end of administration in a treatment group - average tumor volume at the beginning of administration in this treatment group)) / (average tumor volume at the end

of treatment in the vehicle control group - average tumor volume at the beginning of treatment in the vehicle control group)] × 100%. Relative Tumor Proliferation rate T/C (%): the calculation formula was as follows: T/C%=$T_{RTV}$/$C_{RTV}$ × 100% ($T_{RTV}$: average RTV of the treatment group; $C_{RTV}$: average RTV of the negative control group). According to the results of tumor measurement, the relative tumor volume (RTV) was calculated, and the calculation formula was RTV = $Vt/V_0$, where $V_0$ was the tumor volume measured at the time of group administration (i.e. Day 0), $V_T$ was the tumor volume at a certain measurement, and $T_{RTV}$ and $C_{RTV}$ were taken from the same day's data.

**Data analysis:**

In this study, experimental data were presented in Mean ± SEM.

Statistical analysis was performed using IBM SPSS Statistics software based on the RTV data at the end of the experiment. For comparisons between two groups, a T test was used. For comparisons among three or more groups, one-way ANOVA was utilized. If the variance was homogenous (no significant difference in F value), Tukey's method was applied for analysis. If the variance was heterogeneous (significant difference in F value), the Games-Howell method was used for testing. A p-value of < 0.05 was considered statistically significant.

**Experimental results:** The test results were shown in Table **19** and **20.**

Table **19** Evaluation of antitumor efficacy of compounds of the present disclosure in subcutaneous xenograft tumor model of human B-cell lymphoma OCI-LY10 cells

| Group | Dose | Tumor volume ($mm^3$)[a] (Day 0) | Tumor volume ($mm^3$)[a] (Day 28) | Relative tumor volume[a] (Day 28) | T/C (%) (Day 28) | TGI (%) (Day 28) |
|---|---|---|---|---|---|---|
| vehicle control | 0 mg/kg | 201.76 ± 7.08 | 1422.84 ± 89.14 | 7.11 ± 0.56 | -- | -- |
| Hydrochloride of **WX014** | 10 mg/kg | 202.43 ± 8.41 | 685.61 ± 75.95 | 3.37 ± 0.34 | 47.40 | 60.43 |
| Hydrochloride of **WX014** | 30 mg/kg | 199.80 ± 8.02 | 322.68 ± 20.47 | 1.64 ± 0.17 | 23.07 | 89.94 |
| Hydrochloride of **WX014** | 100 mg/kg | 199.75 ± 7.96 | 188.01 ± 35.59 | 0.91 ± 0.15 | 12.80 | 100.96 |
| **[0723]** Note: a. Average ± SEM. | | | | | | |

Table **20** *p*-Values of compounds of the present disclosure for comparison of relative tumor volumes among groups in xenograft model of human B-Cell lymphoma OCI-LY10

| Group | vehicle control | Hydrochloride of **WX014** 10 mg/kg | Hydrochloride of **WX014** 30 mg/kg | Hydrochloride of **WX014** 100 mg/kg |
|---|---|---|---|---|
| vehicle control | N/A | 0.003 | 0.001 | <0.001 |
| Hydrochloride of **WX014** 10 mg/kg | 0.003 | N/A | 0.018 | 0.003 |
| Hydrochloride of **WX014** 30 mg/kg | 0.001 | 0.018 | N/A | 0.077 |
| Hydrochloride of WX014 100 mg/kg | <0.001 | 0.003 | 0.077 | N/A |
| Note: *P* value was analyzed by IBM SPSS Statistics software. | | | | |

**Conclusion:**

The compounds of the present disclosure exhibit remarkable tumor-suppressing effects and is dose-dependent in a SCID mouse xenograft tumor model using human B-cell lymphoma OCI-LY10 cells.

**Experiment example 9: *In vivo* drug efficacy study of compounds in CB17 SCID mouse model with subcutaneous xenograft tumors of human lymphoma SU-DHL-2 cells**

**Experimental objective:**

The study evaluates the antitumor effect of the test compounds using a CB17 SCID mouse model with subcutaneous xenograft tumors of SU-DHL-2 human lymphoma cells.

**Experimental materials:**

1. Experimental animals: CB17 SCID mice, female, 6 to 8 weeks old, weighing 18 to 22 g. Beijing Vital River Laboratory Animal Technology Co., Ltd.
2. Cell line: human lymphoma SU-DHL-2 cells (Cat. No ATCC-CRL-2956).

Table **21** Reagent information

| Name | Manufacturer | Cat. No. | Batch number | Period of validity | Storage condition |
|---|---|---|---|---|---|
| 1640 culture medium | gibco | 22400089 | 2462009 | 2023-02-28 | 4°C |
| Matrix gel | Corning | 354234 | 2013002 | 2022-10-19 | -20°C |
| 100 x Double antibody (penicillin, streptomycin) | meilunbio | MA0110 | MA0110-Apr-15H | 2023-04-14 | -20°C |
| PBS | HyClone | SH30256.01 | AG29791799 | 2023-08-31 | 4°C |
| Fetal bovine serum | ExCell Bio | FND500 | 11H233 | 2023-08 | -20°C |

Table **22** Instruments

| Name | Manufacturer | Type |
|---|---|---|
| Biological safety cabinet | Suzhou Antai Air Tech Co., Ltd. | BSC-1604IIA2 |
| Vernier scale | Mitutoyo | CD-6"ASX |
| Electronic balance scale | Changzhou Tianzhiping Instrument Equipment Co., Ltd | EL-2KJ |
| Vortex mixer | HaiMen City Qilin Medical Instrument Factory | XW-80A |
| Pure water system | Millipore | MILLI-Q®Direct8 |

**Model building:**

Cell culture: Human lymphoma SU-DHL-2 cells (ATCC-CRL-2956) were cultured in suspension *in vitro*. The culture conditions were RPMI 1640 medium with 10% inactivated fetal bovine serum, 100 U/mL penicillin and 100 μg/mL streptomycin, and cultured in a 37°C, 5% $CO_2$ incubator. Routine passaging was performed twice a week. When the cell saturation reached 80%-90% and the quantity met the requirements, the cells were collected, counted, and seeded.

Tumor cell seeding and grouping: 0.2 mL ($10 \times 10^6$ cells) of SU-DHL-2 cells, in a PBS:Matrigel=1:1 mixture, were subcutaneously inoculated into the right dorsal flank of each mouse. When the average tumor volume reached approximately 139 $mm^3$, the animals were divided into groups for drug administration. The day of grouping was recorded as Day 0, and dosing began according to the body weight of the animals.

**Experimental scheme:**

The pharmacological experiment consisted of a dosing cycle of seven days, with medication administered once daily at a 24-hour interval. The test compounds were given intragastrically, with a total of three dosing cycles. During the experiment, the body weight and tumor size of the animals were measured twice a week, and clinical symptoms were observed and recorded daily.

The dosing for the test compounds were 10 mg/kg, 30 mg/kg, and 100 mg/kg, with a solvent composition of 10% DMSO/10% Solutol/80% water. The formula for calculating tumor volume (TV) was: $1/2 \times a \times b^2$, where a and b were the measured length and width of the tumor, respectively. The formula for calculating the Tumor Growth Inhibition (TGI) rate was:: TGI (%) = [(1 - (average tumor volume at the end of administration in a treatment group - average tumor volume at the beginning of administration in this treatment group)) / (average tumor volume at the end of treatment in the vehicle control group - average tumor volume at the beginning of treatment in the vehicle control group)] × 100%. Relative Tumor Proliferation rate T/C (%): the calculation formula was as follows: $T/C\%=T_{RTV}/C_{RTV} \times 100\%$ ($T_{RTV}$: average RTV of the treatment group; $C_{RTV}$: average RTV of the negative control group). According to the results of tumor measurement, the relative tumor volume (RTV) was calculated, and the calculation formula was $RTV = V_t/V_0$, where $V_0$ was the tumor volume measured at the time of group administration (i.e. Day 0), VT was the tumor volume at a certain measurement, and $T_{RTV}$ and $C_{RTV}$ were taken from the same day's data.

**Data analysis:**

Statistical analysis is based on the data of RTV at the end of the experiment and analyzed by SPSS software. For comparisons between two groups, a T test was used. For comparisons among three or more groups, one-way ANOVA was utilized. If the variance was homogenous (no significant difference in F value), Tukey's method was applied for analysis. If the variance was heterogeneous (significant difference in F value), the Games-Howell method was used for testing. Ap-value of < 0.05 was considered statistically significant.

**Experimental results:** The test results were shown in Table **23** and **24.**

Table **23** Evaluation of antitumor efficacy of compounds of the present disclosure in subcutaneous xenograft tumor model of lymphoma SU-DHL-2 cells

| Group | Dose | Tumor volume ($mm^3$)[a] (Day 0) | Tumor volume ($mm^3$)[a] (Day 21) | Relative tumor volume[a] (on day 21) | T/C (%) (Day 21) | TGI (%) (Day 21) |
|---|---|---|---|---|---|---|
| Vehicle control | 0 mg/kg | 139 ± 6 | 1883 ± 141 | 13.68 | - | - |
| Hydrochloride of **WX014** | 10 mg/kg | 139 ± 6 | 800 ± 48 | 5.79 | 42.33 | 62.13 |
| Hydrochloride of **WX014** | 30 mg/kg | 139 ± 6 | 813 ± 99 | 5.78 | 42.26 | 61.41 |
| Hydrochloride of **WX014** | 100 mg/kg | 139 ± 5 | 626 ± 51 | 4.55 | 33.23 | 72.07 |
| **[0746]** Note: a. Average ± SEM. | | | | | | |

Table **24** *p*-Values of compounds of the present disclosure for comparison of relative tumor volumes among groups in subcutaneous xenograft tumor model of lymphoma SU-DHL-2

| Group | Vehicle control | Hydrochloride of **WX014** 10 mg/kg | Hydrochloride of **WX014** 30 mg/kg | Hydrochloride of **WX014** 100 mg/kg |
|---|---|---|---|---|
| Vehicle control | N/A | 0.001 | 0.001 | <0.001 |
| Hydrochloride of **WX014** 10 mg/kg | 0.001 | N/A | 1.000 | 0.268 |

(continued)

| Group | Vehicle control | Hydrochloride of **WX014** 10 mg/kg | Hydrochloride of **WX014** 30 mg/kg | Hydrochloride of **WX014** 100 mg/kg |
|---|---|---|---|---|
| Hydrochloride of **WX014** 30 mg/kg | 0.001 | 1.000 | N/A | 0.507 |
| Hydrochloride of **WX014** | <0.001 | 0.268 | 0.507 | N/A |
| 100 mg/kg | | | | |
| Note: $p$-Values for the analysis of relative tumor volume (RTV) are derived using one-way ANOVA. In case of heterogeneity of variances, the Games-Howell test is applied for analysis. | | | | |

**Conclusion:**

The compounds of the present disclosure have remarkable tumor-suppressing effects in a CB17 SCID mouse model with subcutaneous xenograft tumors of human lymphoma SU-DHL-2 cells.

## Claims

**1.** A compound of formula (II), a stereoisomer thereof, or a pharmaceutically acceptable salt thereof,

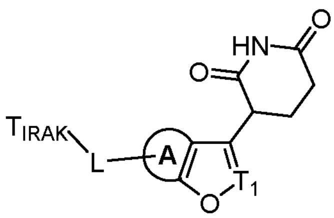

(II)

,

wherein

$T_{IRAK}$ is selected from

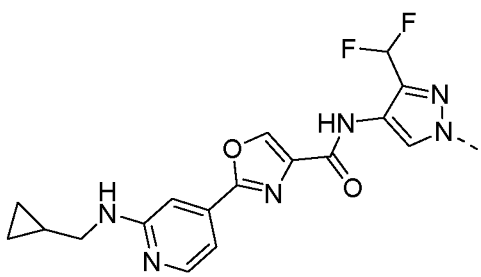

and

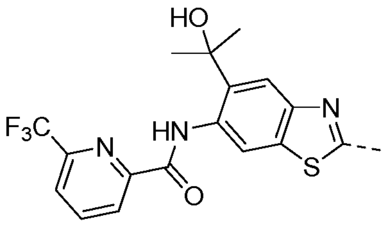

;

L is selected from $C_{2-10}$ alkylene, wherein any 2, 3 or 4 $CH_2$ on the $C_{2-10}$ alkylene are each independently substituted by Ra, and each $C_{2-10}$ alkylene is independently and optionally substituted by 1, 2, 3, 4, 5, or 6 halogens;
each Ra is independently selected from -N(R)-, -O-, -C(O)NH-, -$C_{3-6}$ cycloalkyl-, and -4-to 8-membered heterocycloalkyl-;
R is selected from H and $C_{1-4}$ alkyl;
$T_1$ is selected from CH and N;
ring A is selected from $C_{6-10}$ aryl and 5- to 10-membered heteroaryl.

2. The compound, the stereoisomer thereof, or the pharmaceutically acceptable salt thereof according to claim 1, wherein Ra is independently selected from -NH-, -N($CH_3$)-, -O-, -C(O)NH-, -cyclopropyl-, -cyclobutyl-, -cyclopentyl-, -cyclohexyl-, -piperidyl-, -piperazinyl-, -azaspiro[3.3]heptyl-, -diazspiro[3.3]heptyl-, and -azabicyclic[3.1.0]hexyl-.

3. The compound, the stereoisomer thereof, or the pharmaceutically acceptable salt thereof according to claim 2, wherein each Ra is independently selected from -NH-, -N($CH_3$)-, -O-, -C(O)NH-,

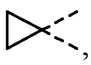, 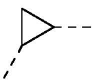, 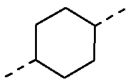, 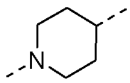, 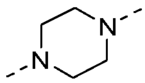, and 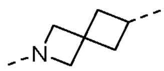.

4. The compound, the stereoisomer thereof, or the pharmaceutically acceptable salt thereof according to claim 1, wherein L is selected from $C_{4-10}$ alkylene, any 3 $CH_2$ on the $C_{4-10}$ alkylene are each independently substituted by Ra, and each $C_{4-10}$ alkylene is independently and optionally substituted by 1, 2, 3, 4, 5 or 6 halogens.

5. The compound, the stereoisomer thereof, or the pharmaceutically acceptable salt thereof according to claim 4, wherein L is selected from -$C_{3-6}$ cycloalkyl-$CH_2$-Ra-$C_{1-3}$ alkylene-Ra-$C_{0-3}$ alkylene-, -$C_{3-6}$ cycloalkyl-$C_{1-3}$ alkylene-4- to 8-membered heterocycloalkyl-$C_{1-3}$ alkylene-Ra-, and -$C_{3-6}$ cycloalkyl-$C_{1-3}$ alkylene-4- to 8-membered heterocycloalkyl-$C_{1-3}$ alkylene-, and each $C_{1-3}$ alkylene is independently and optionally substituted by 1 or 2 halogen atoms.

6. The compound, the stereoisomer thereof, or the pharmaceutically acceptable salt thereof according to claim 5, wherein L is selected from -cyclohexyl-$CH_2$-N(R)-$C_{1-3}$ alkylene-O-$C_{1-3}$ alkylene-, -cyclohexyl-$CH_2$-4- to 8-membered heterocycloalkyl-$C_{1-3}$ alkylene-N(R)-, - cyclohexyl-$CH_2$-piperidyl-$C_{1-3}$ alkylene-C(O)NH-, -cyclohexyl-$CH_2$-piperidyl-$CF_2$-C(O)NH-, -cyclohexyl-$CH_2$-piperazinyl-$C_{1-3}$ alkylene-C(O)NH-, and -cyclohexyl-$CH_2$-piperazinyl-$C_{1-3}$ alkylene-.

7. The compound, the stereoisomer thereof, or the pharmaceutically acceptable salt thereof according to claim 6, wherein L is selected from

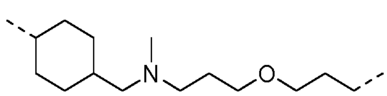,

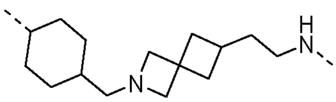, 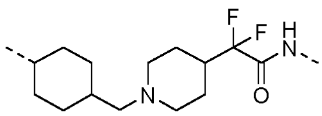, 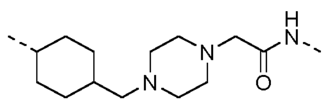,

and

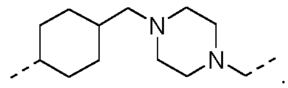

.

**8.** The compound, the stereoisomer thereof, or the pharmaceutically acceptable salt thereof according to claim 1, wherein ring A is selected from phenyl or naphthyl.

**9.** The compound, the stereoisomer thereof, or the pharmaceutically acceptable salt thereof according to claim 1, wherein the structural moiety

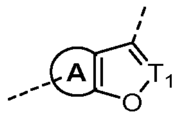

is selected from

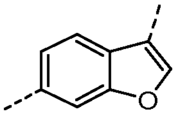 , 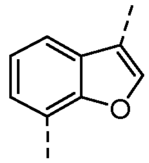 , 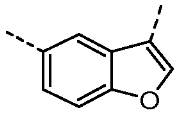 , 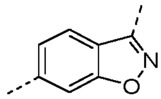 , 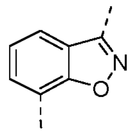 , 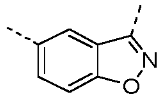 ,

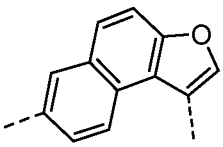 , 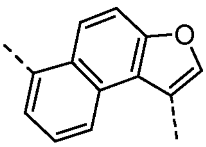 , 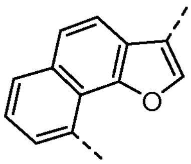 , 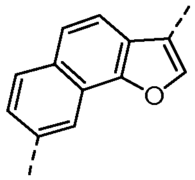 , 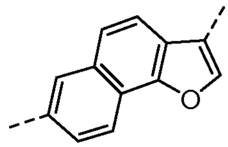 ,

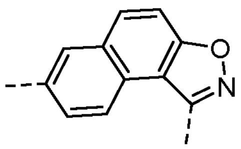 , and 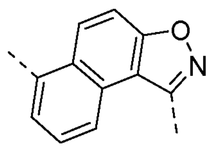 .

**10.** The compound, the stereoisomer thereof, or the pharmaceutically acceptable salt thereof according to claim 1, selected from the structures of formulas (II-1), (II-2), (II-3), and (II-4),

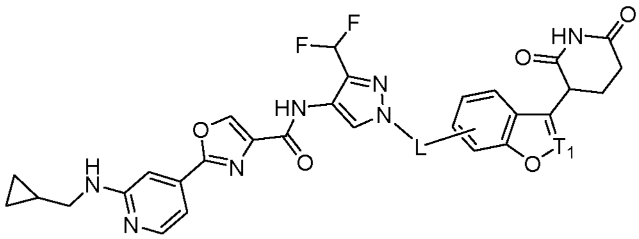

( II-1 )

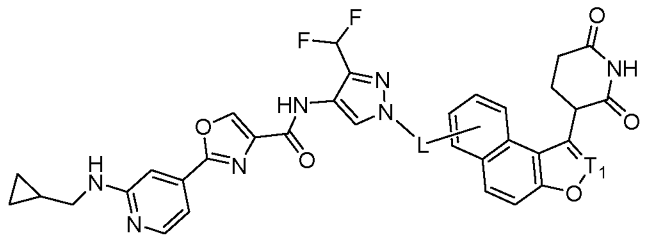

( II-2 ) ,

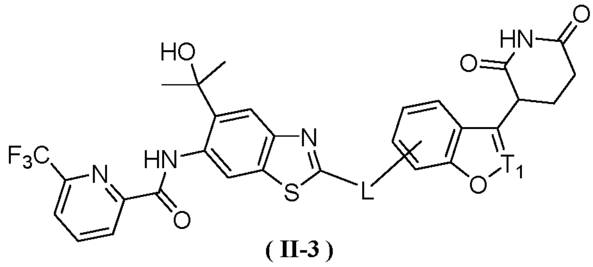

( II-3 ) ,

and

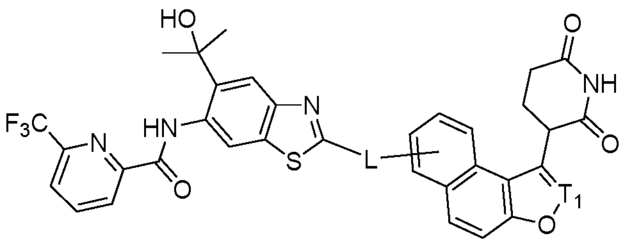

( II-4 ) ,

wherein L and $T_1$ are as defined in claim 1.

**11.** The compound, the stereoisomer thereof, or the pharmaceutically acceptable salt thereof according to claim 1, selected from the structures of formulas (II-1a), (II-2a), (II-3a), and (II-4a),

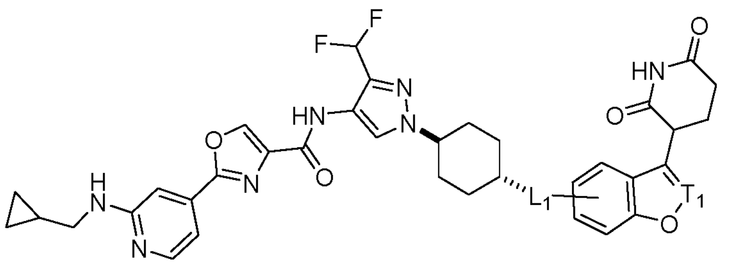

( II-1a )

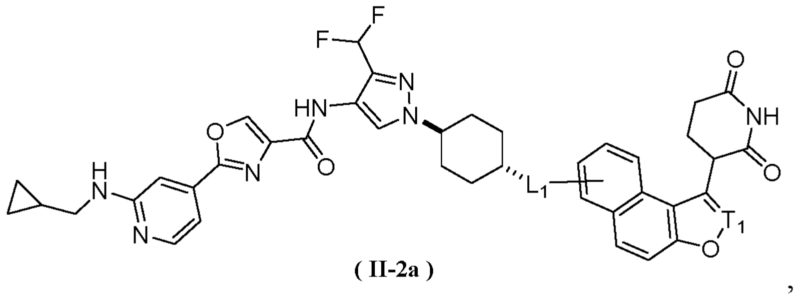

( II-2a )

,

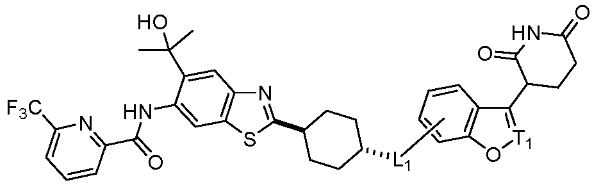

( II-3a )

, and

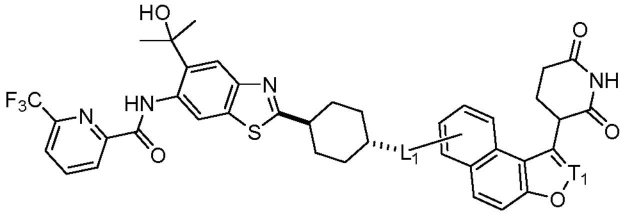

( II-4a )

,

wherein

$L_1$ is selected from -$C_{1-3}$ alkylene-Ra-$C_{1-3}$ alkylene-Ra-$C_{0-3}$ alkylene-, -$C_{1-3}$ alkylene-4- to 8-membered heterocycloalkyl-$C_{1-3}$ alkylene-Ra-, and-$C_{1-3}$ alkylene-4- to 8-membered heterocycloalkyl-$C_{1-3}$ alkylene-, and each $C_{1-3}$ alkylene is independently and optionally substituted by 1 or 2 halogen atoms;
$T_1$ and Ra are as defined in claim 1.

**12.** A compound, a stereoisomer thereof, or a pharmaceutically acceptable salt thereof as shown below,

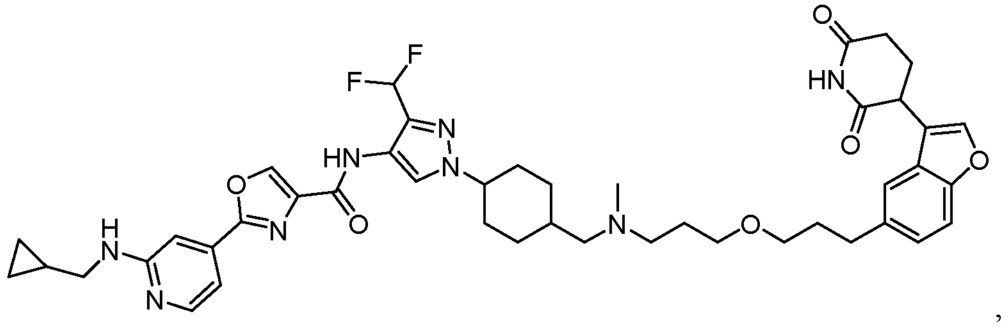

,

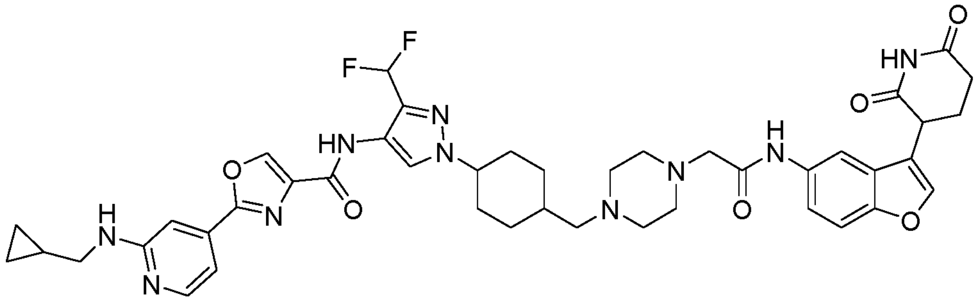

,

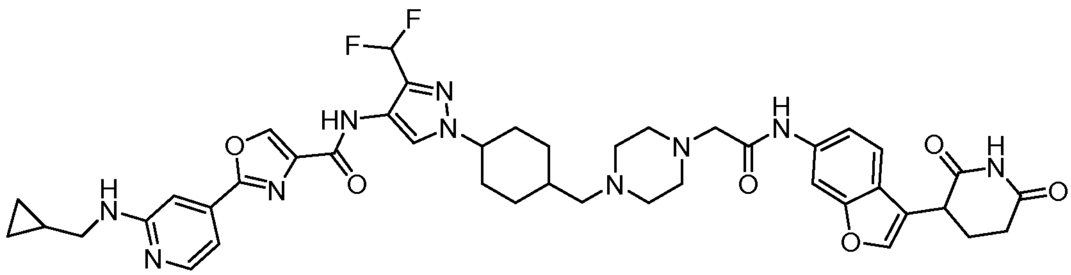

,

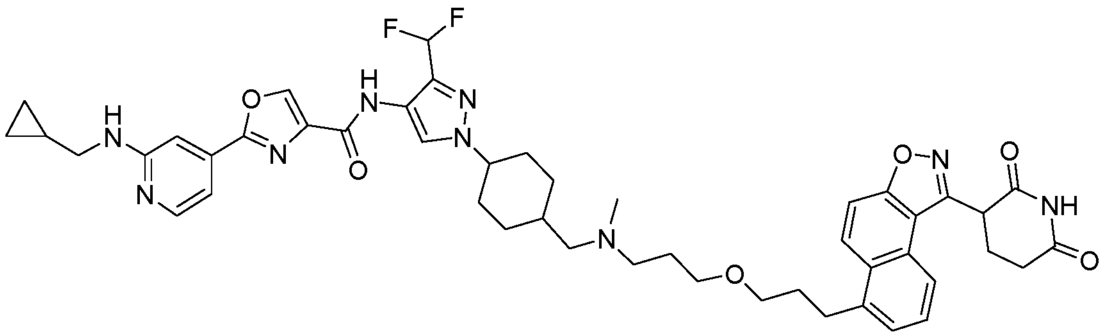

,

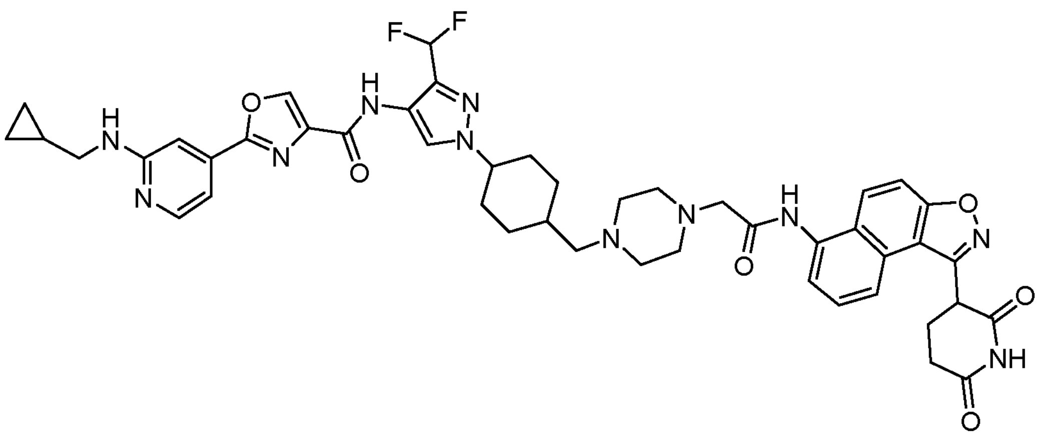

,

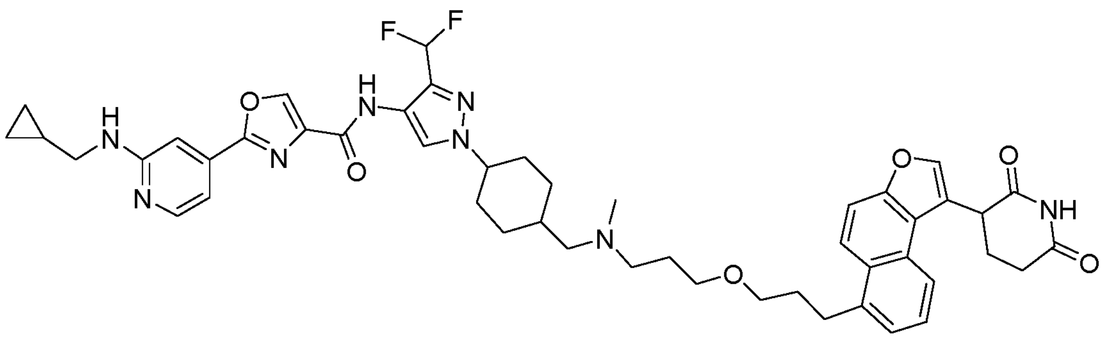

,

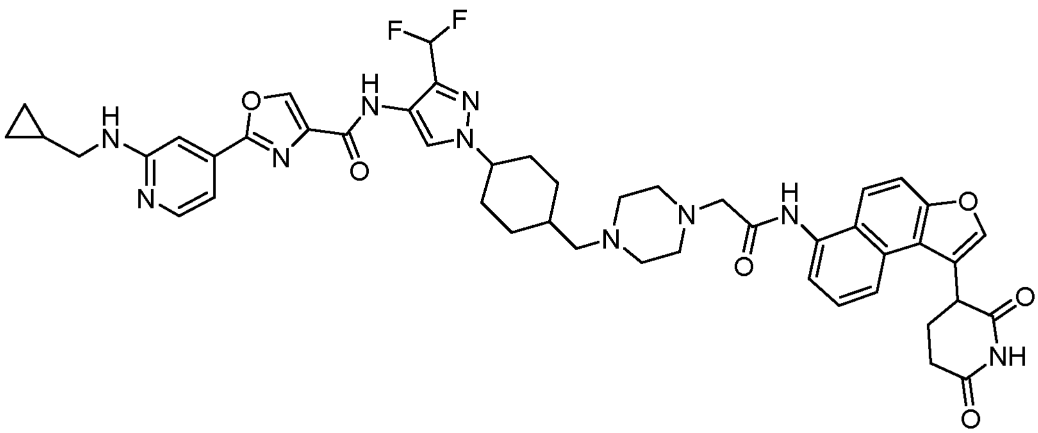

,

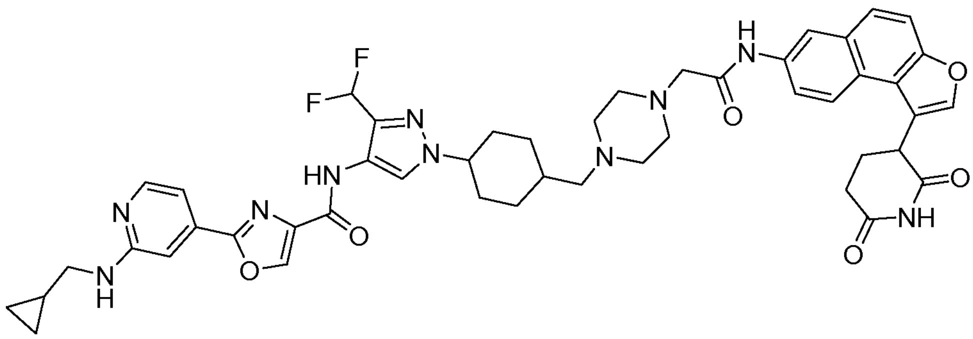

,

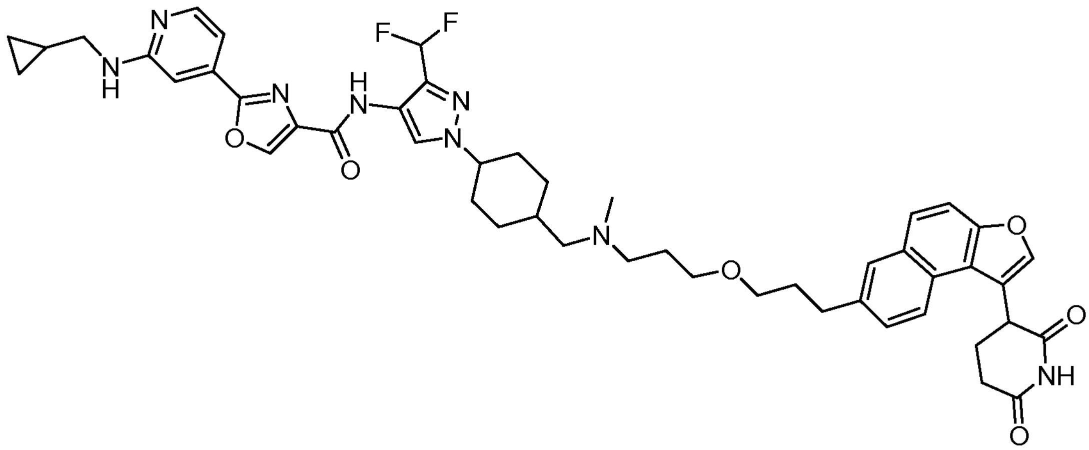

,

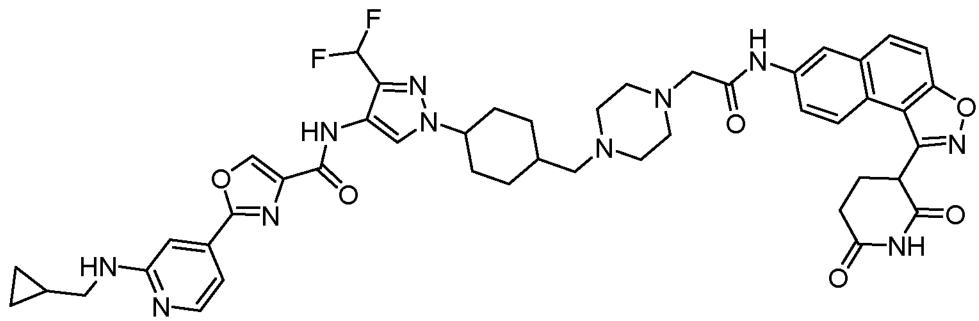

,

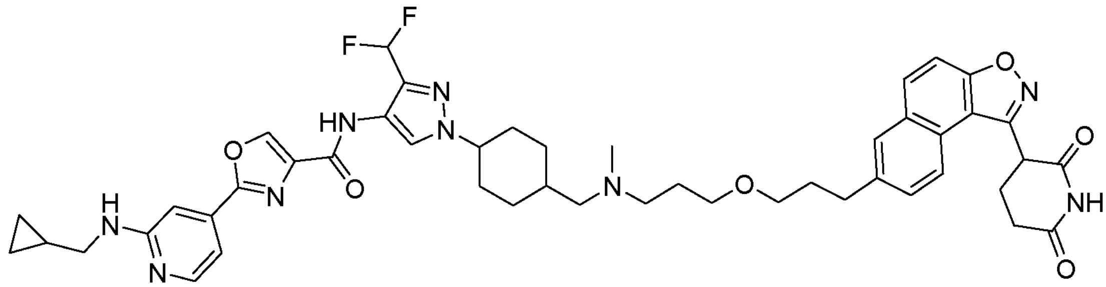

,

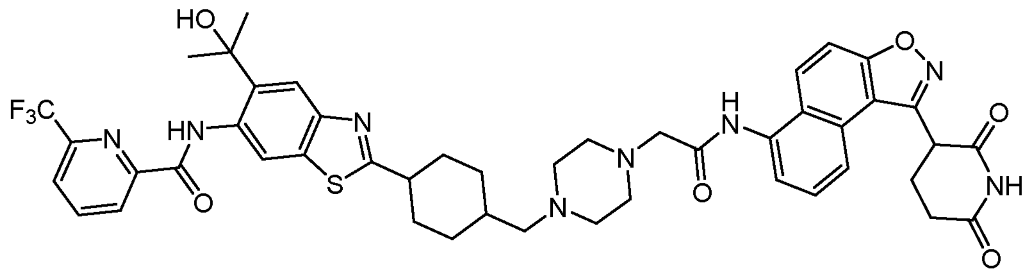

,

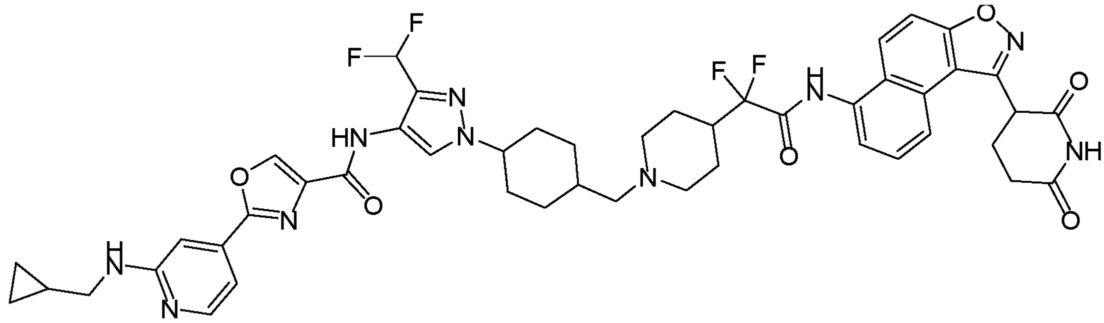

,

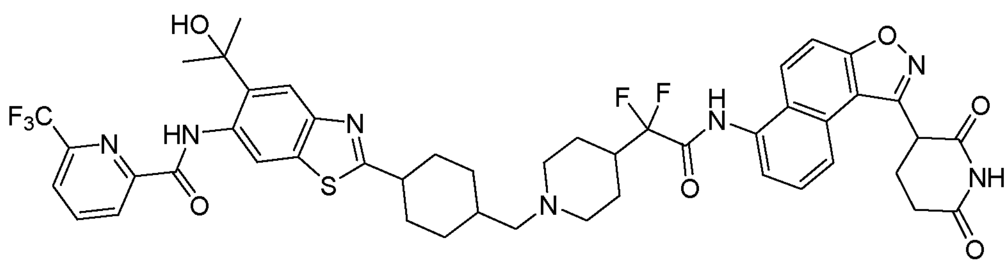

.

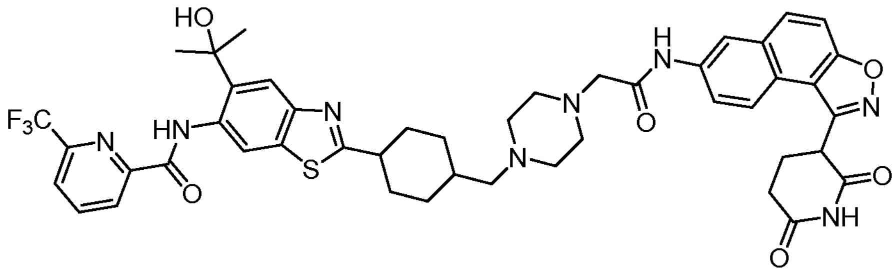

,

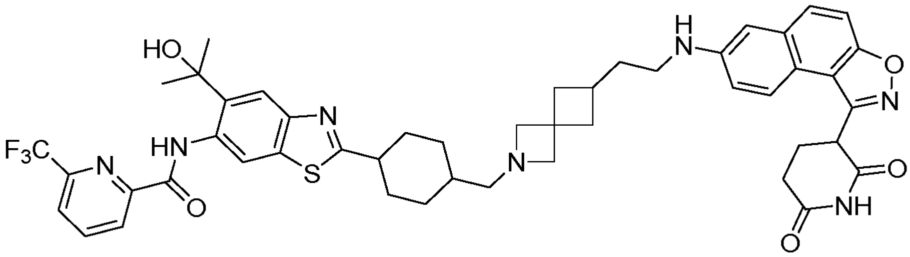

,

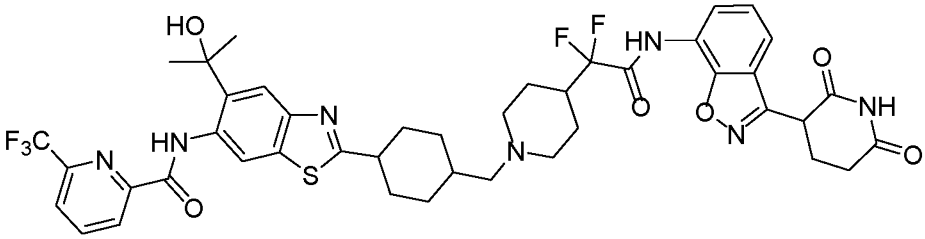

,

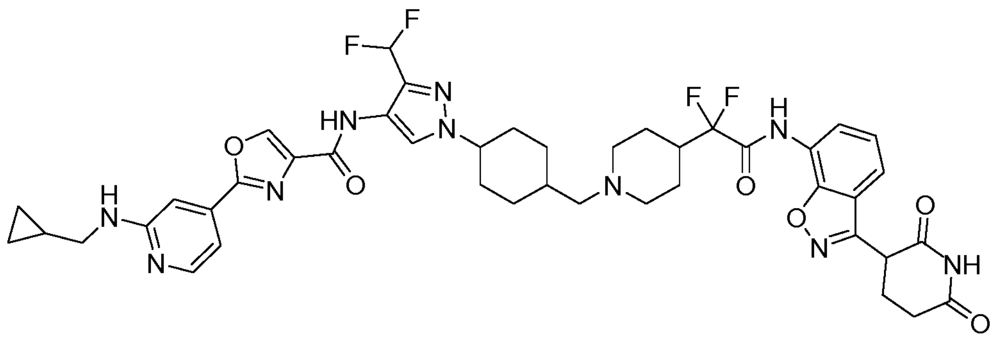

,

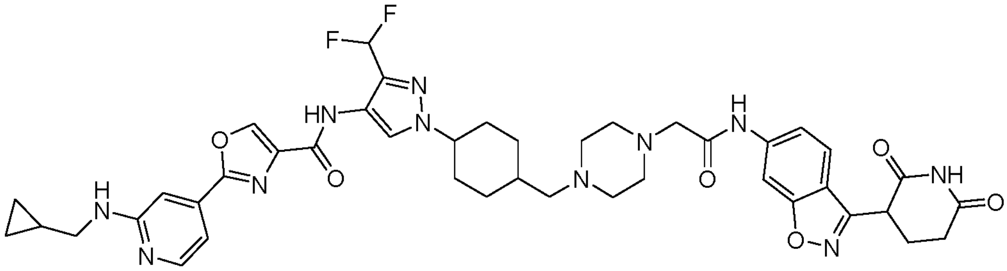

,

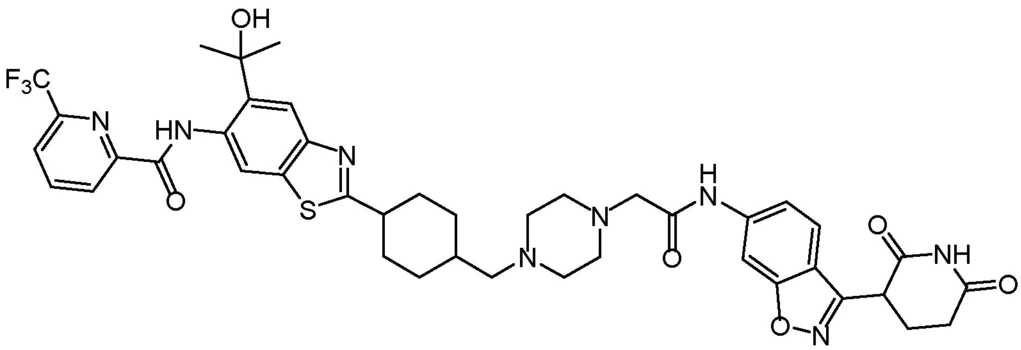

,

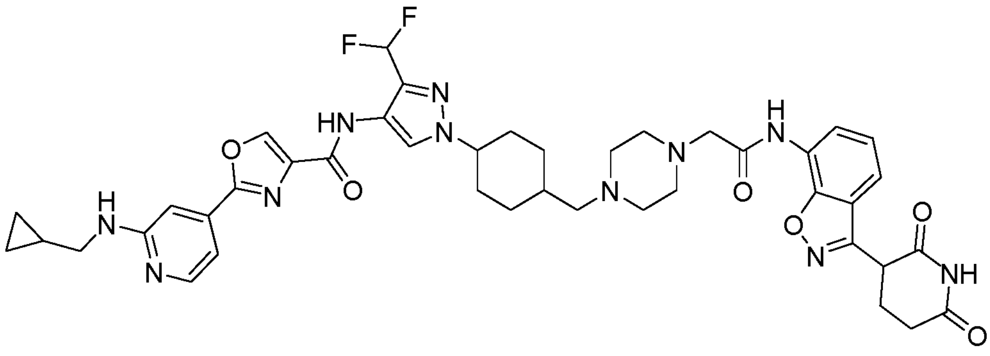

,

and

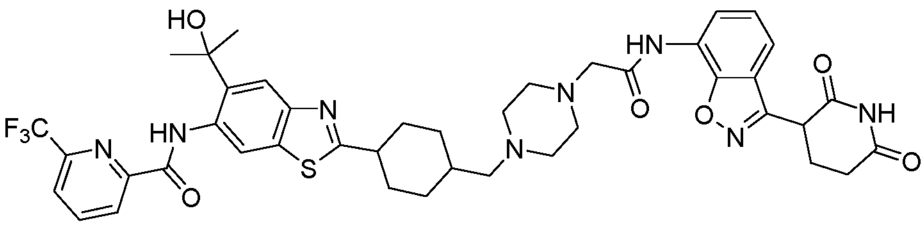

.

13. The compound, the stereoisomer thereof, or the pharmaceutically acceptable salt thereof according to claim 12, wherein the compound is selected from

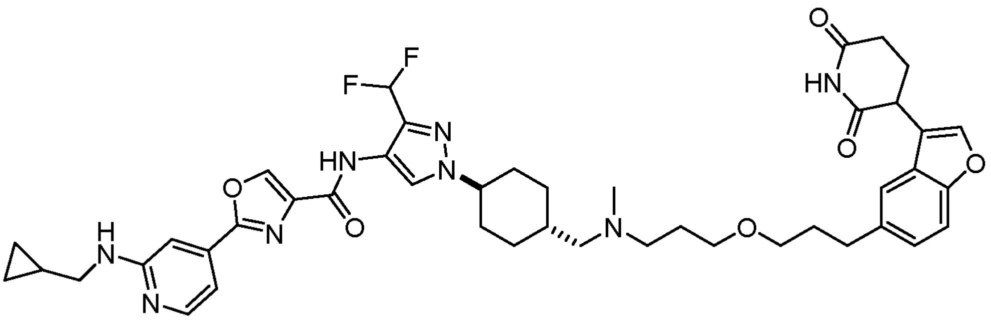

,

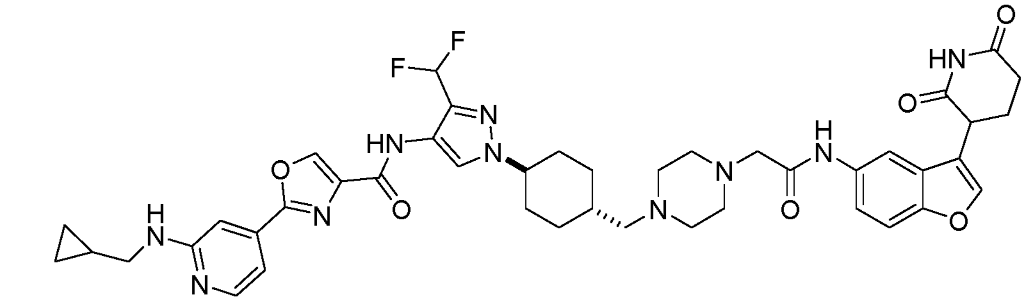

,

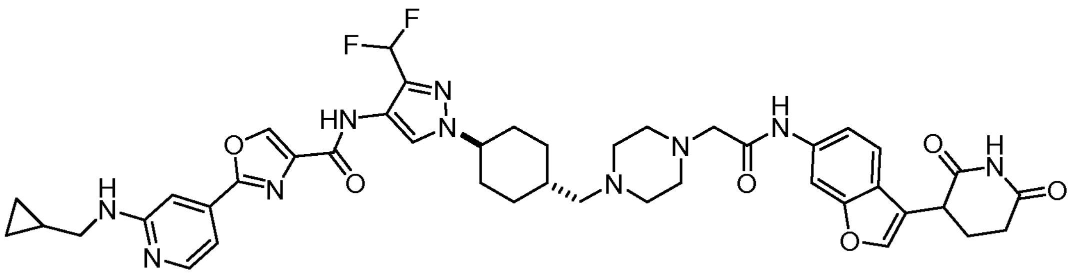

,

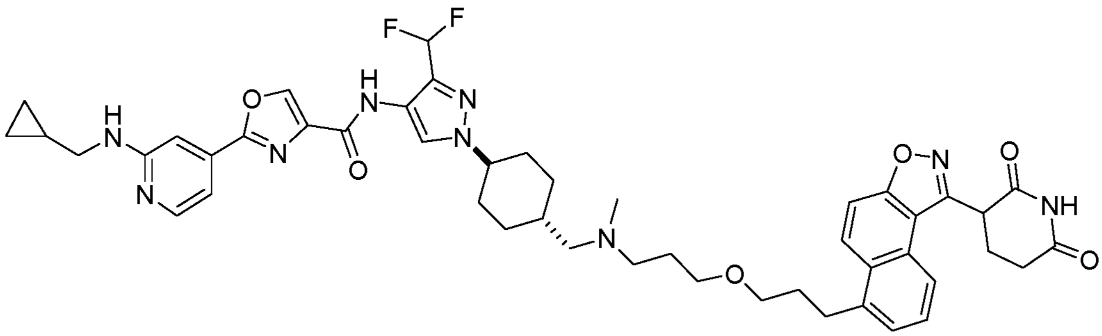

,

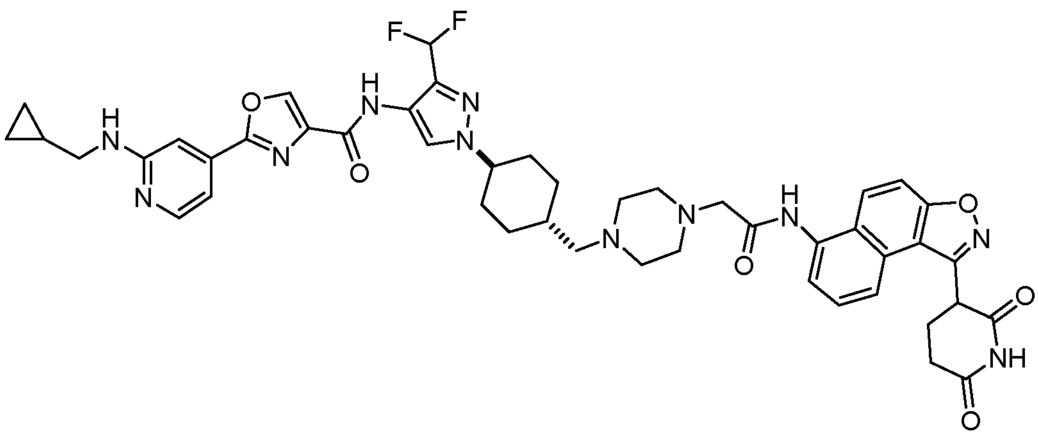

,

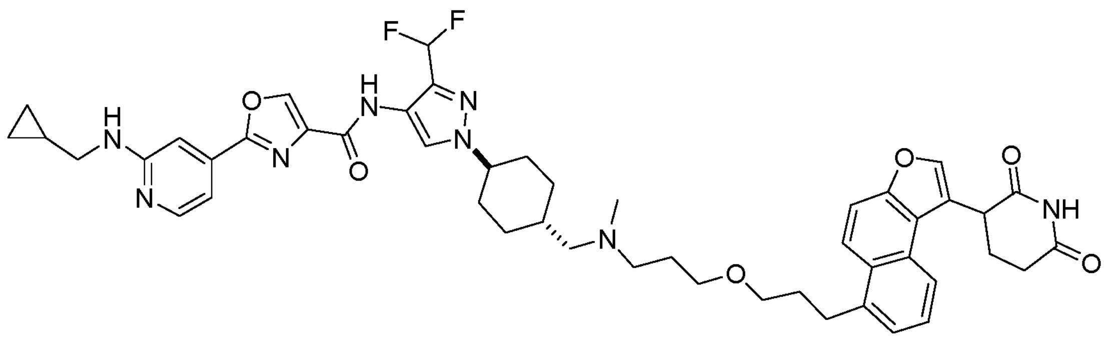

,

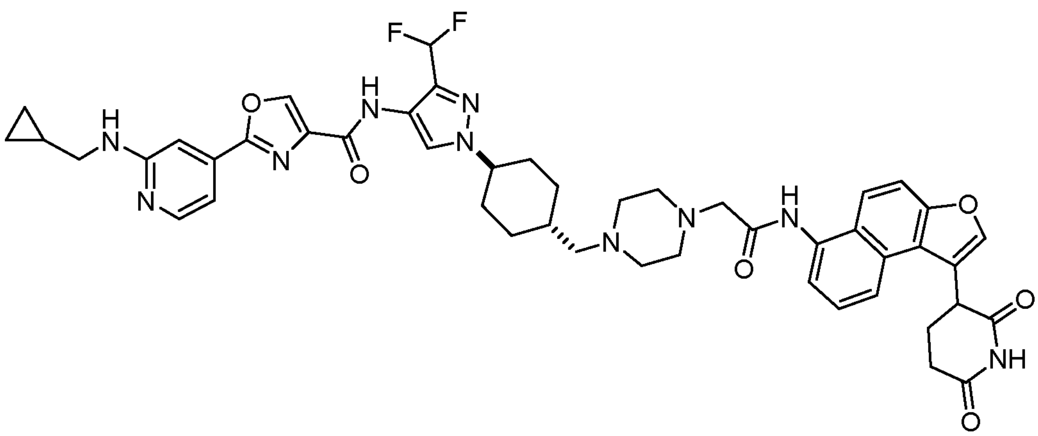

,

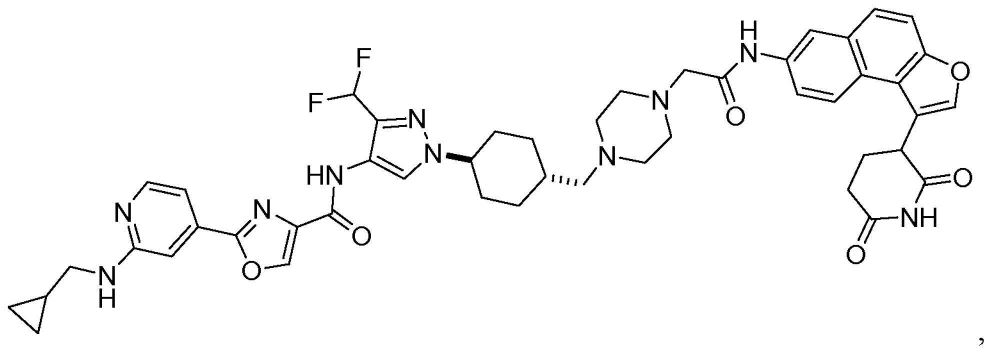

,

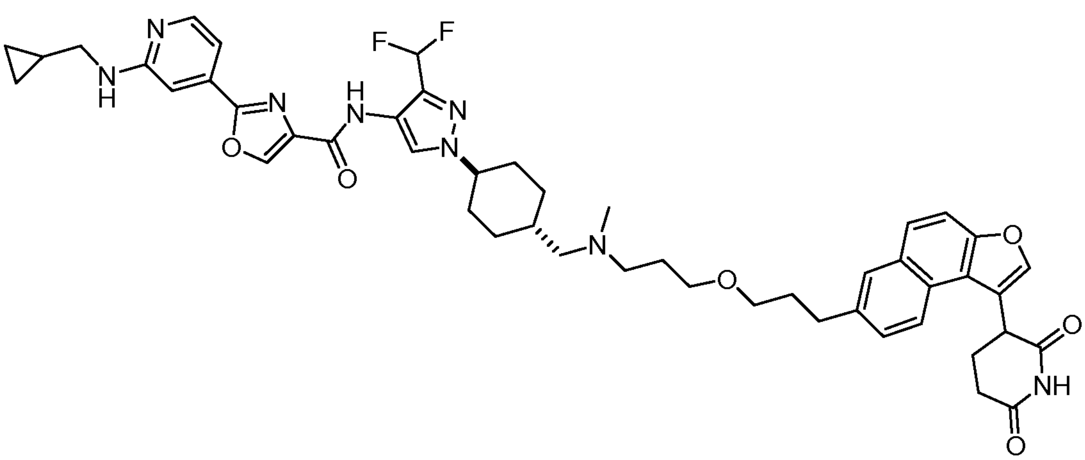

,

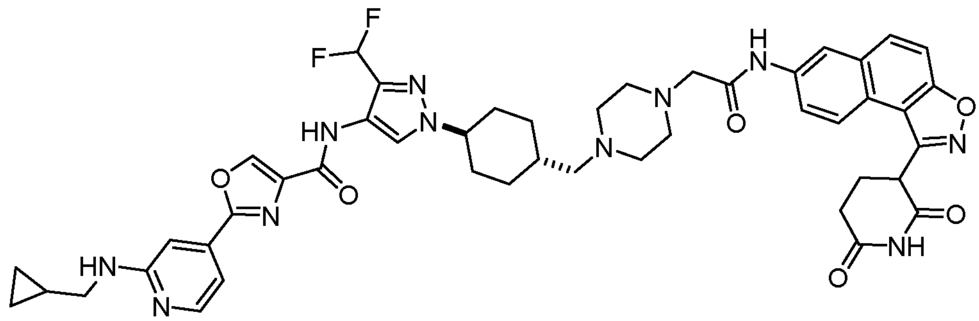

,

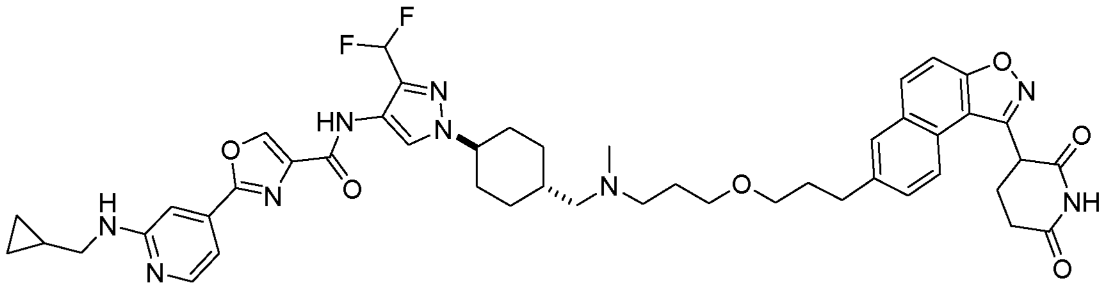

,

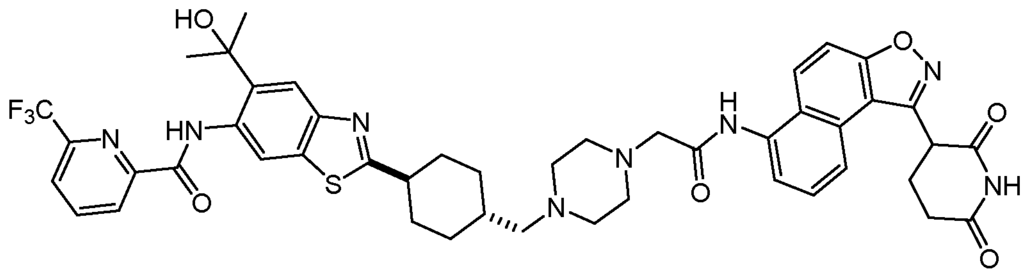

,

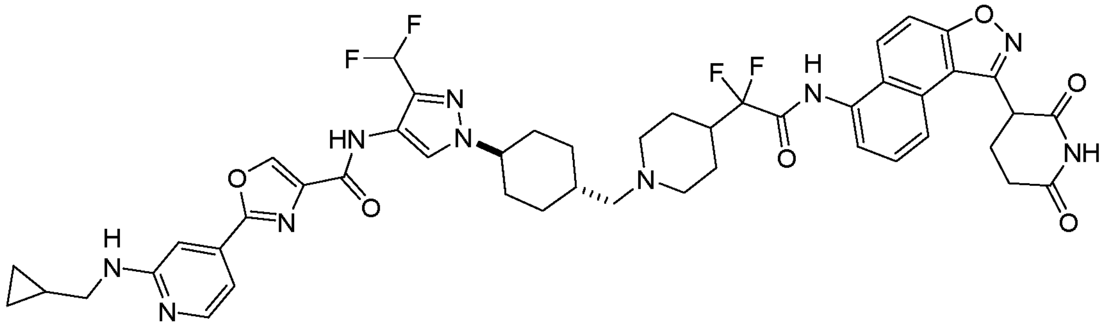

,

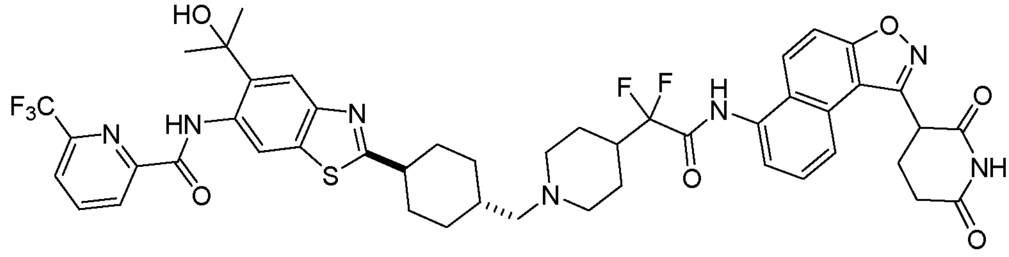

,

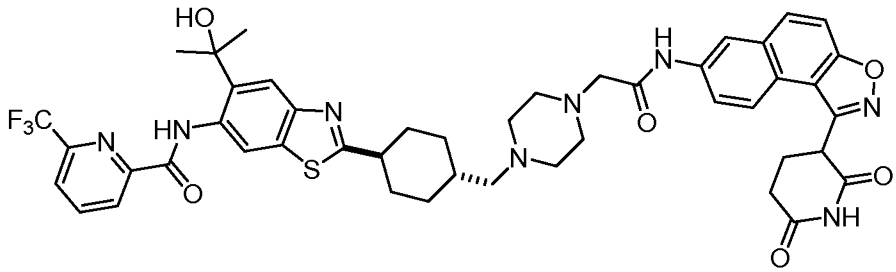

,

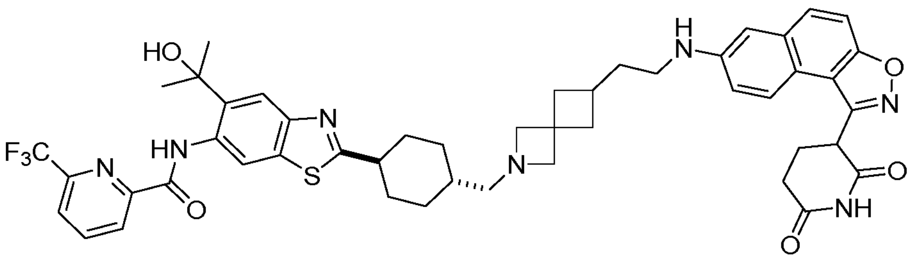

,

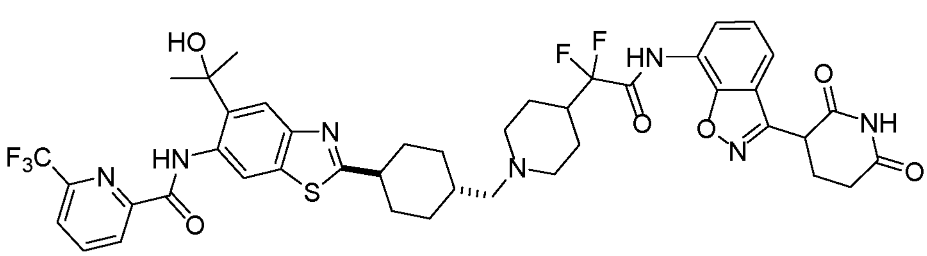

,

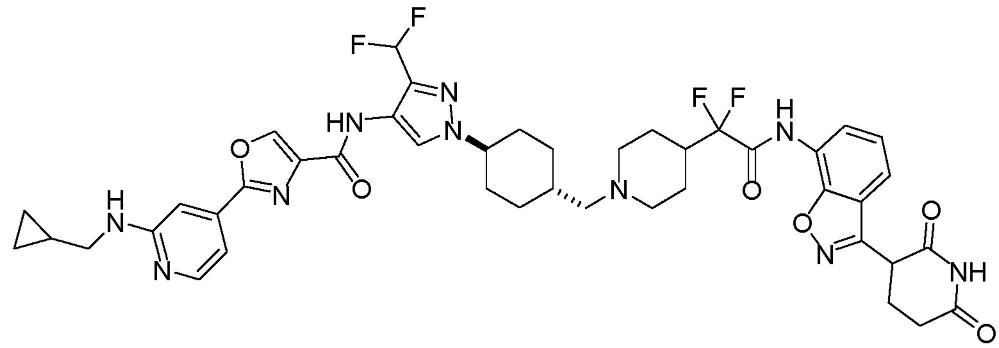

,

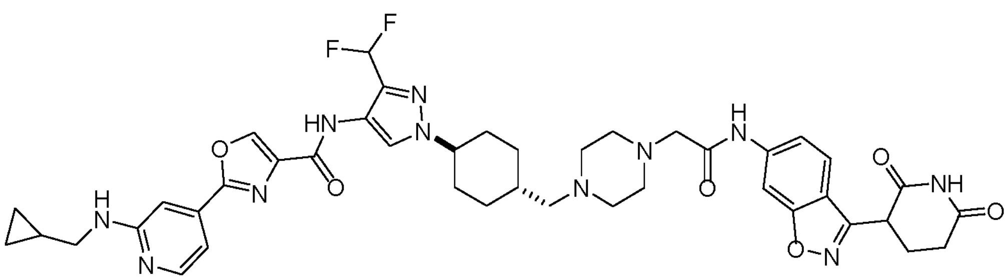

,

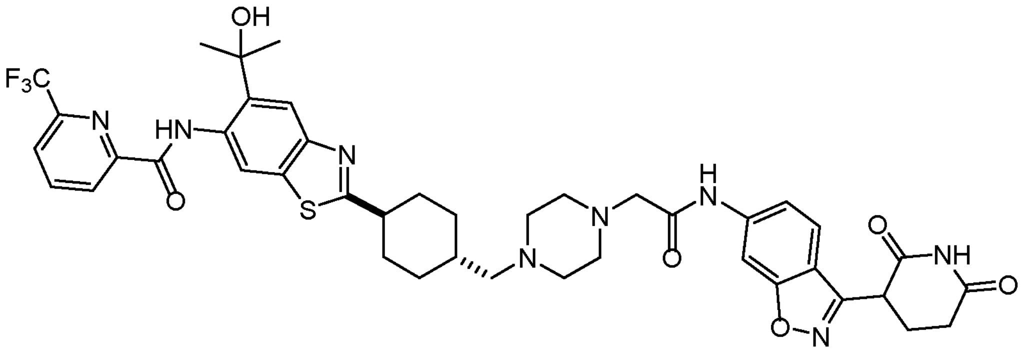

,

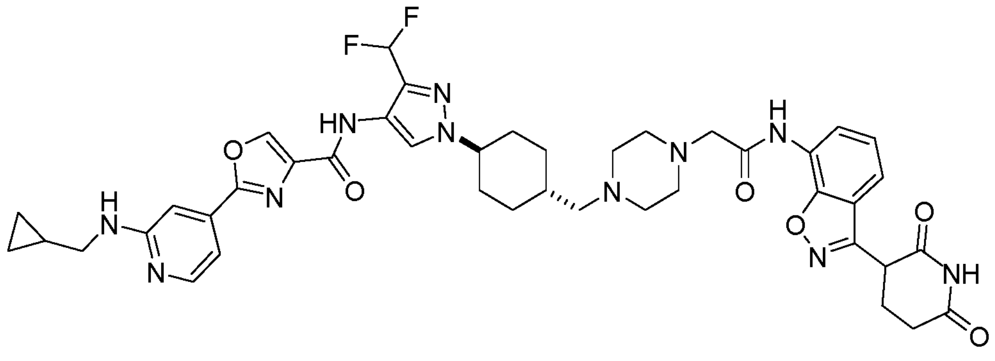

,

and

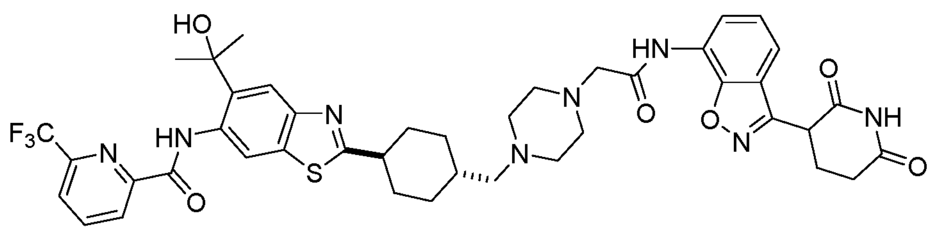

.

**14.** The compound, the stereoisomer thereof, or the pharmaceutically acceptable salt thereof according to claim 13, wherein the compound is selected from

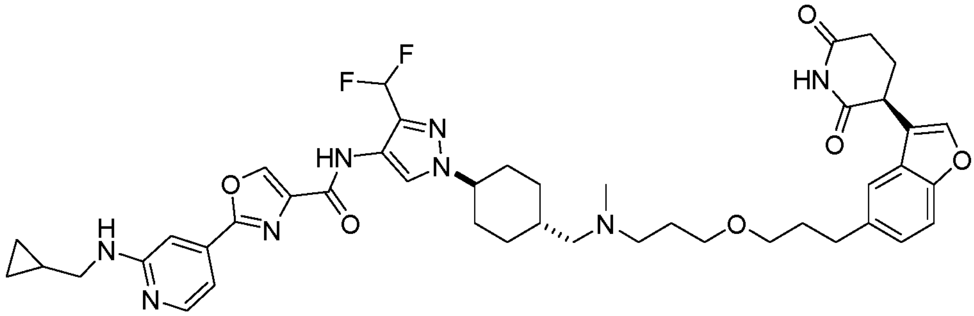

,

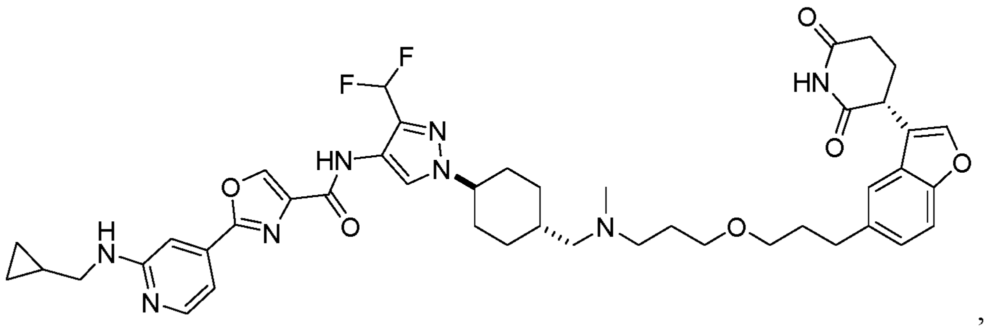

,

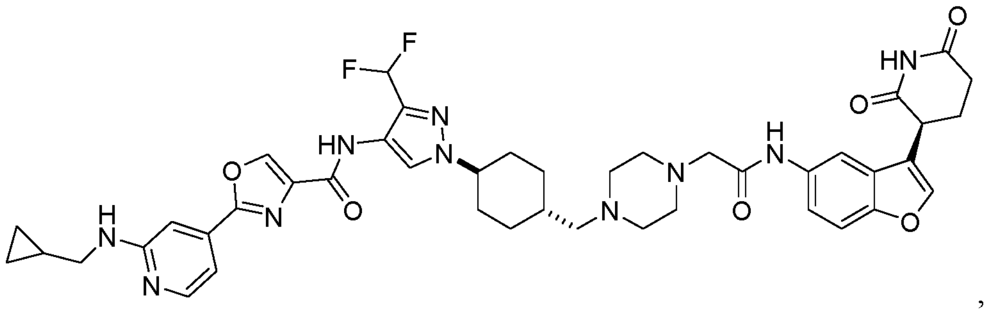

,

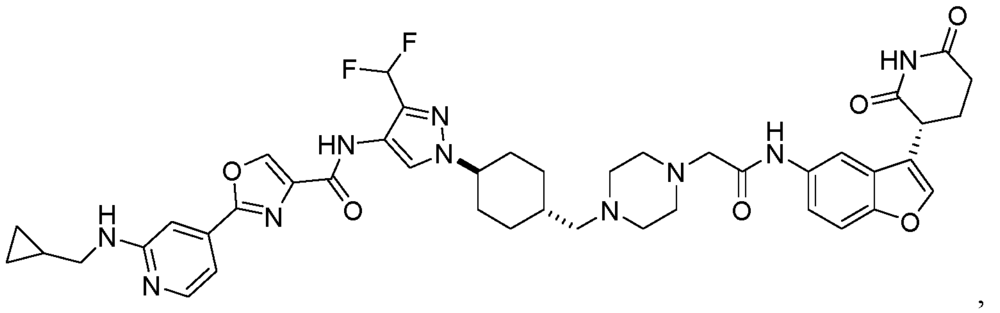

,

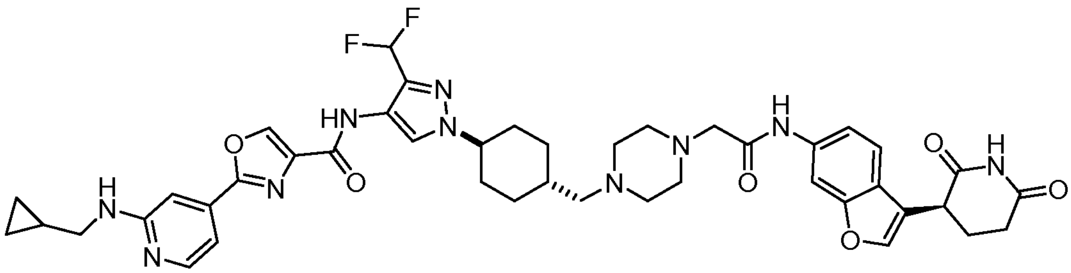

,

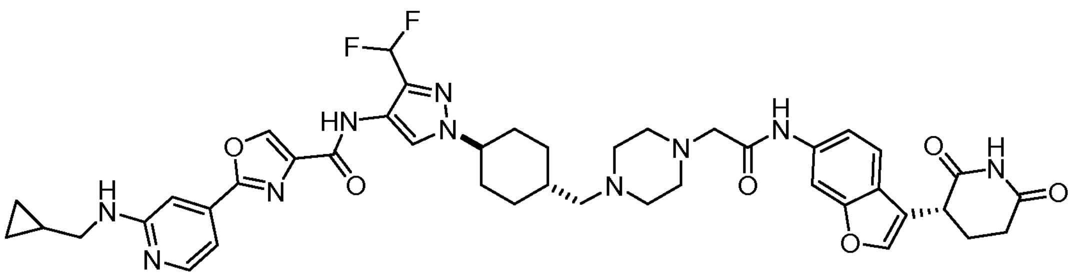

,

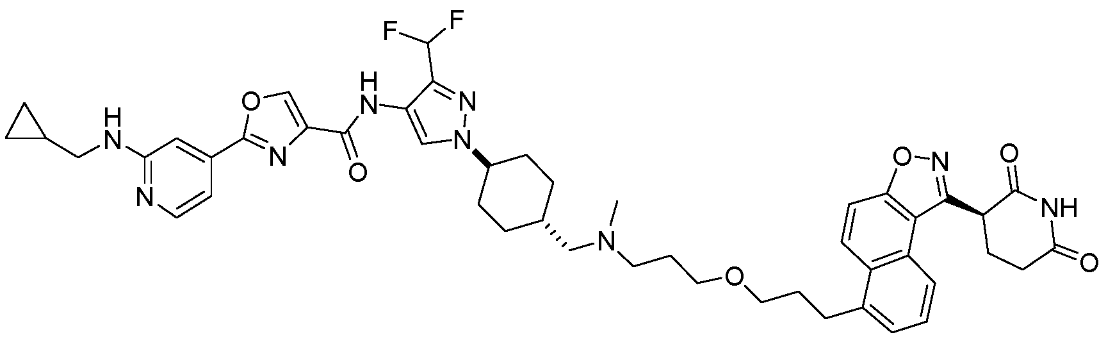

,

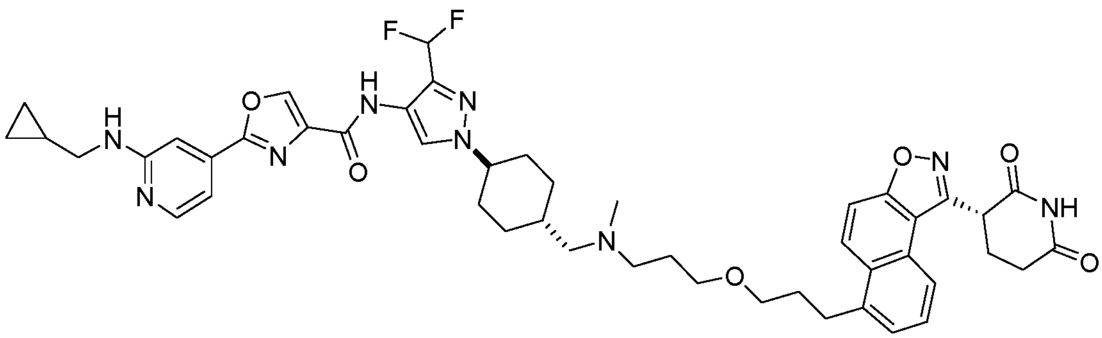

,

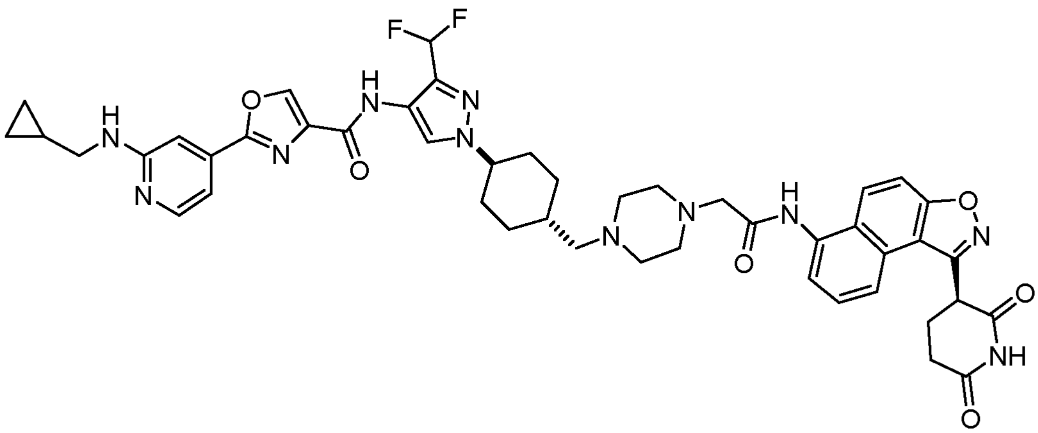

,

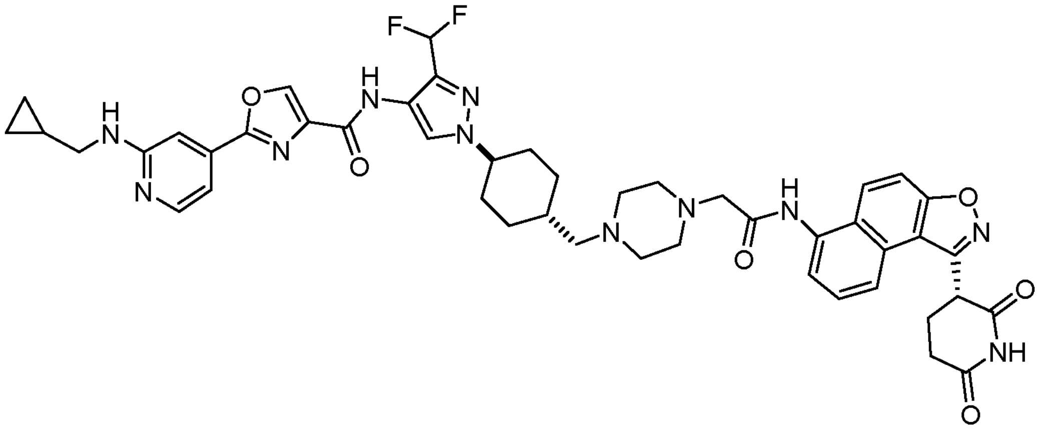

,

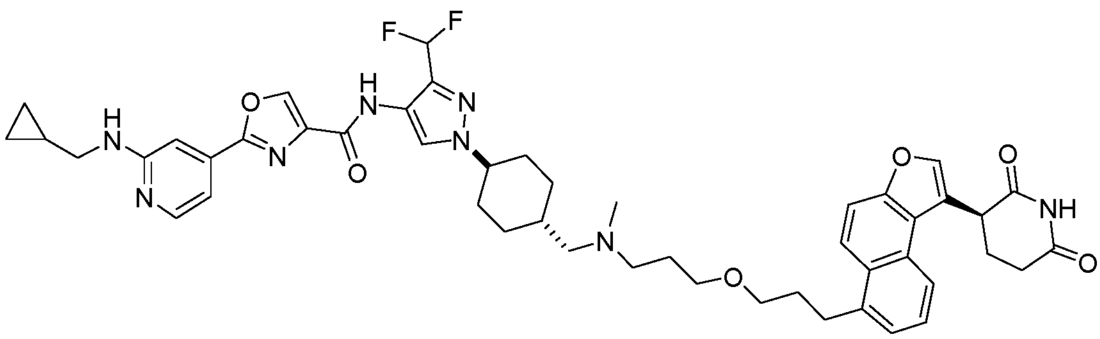

,

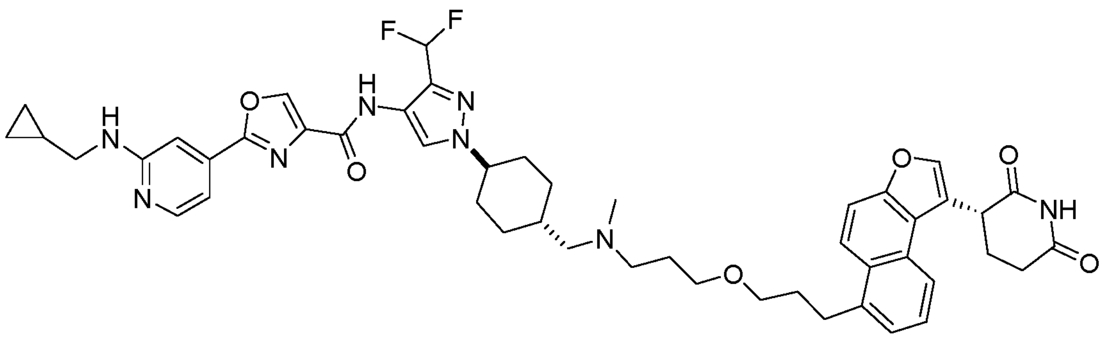

,

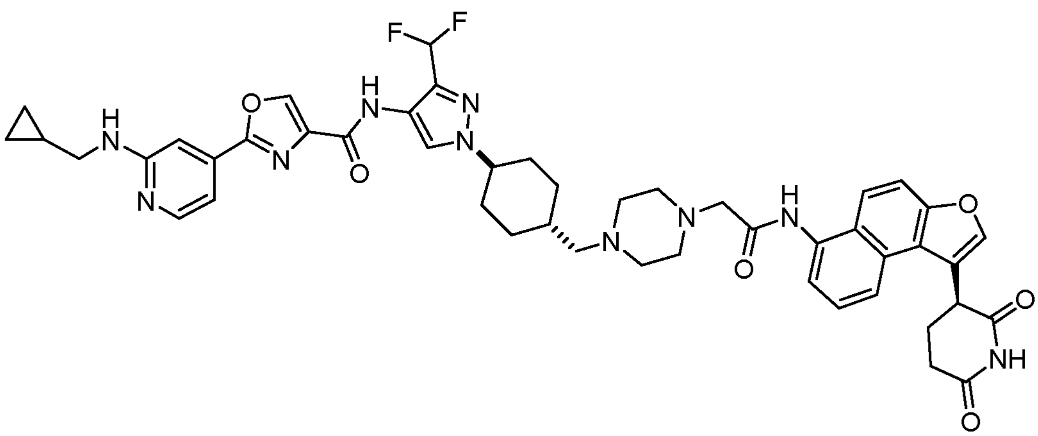

,

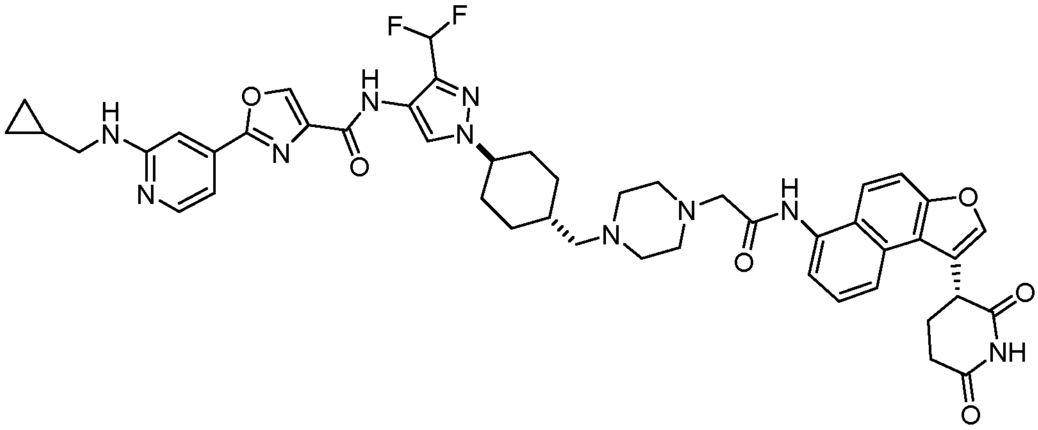

,

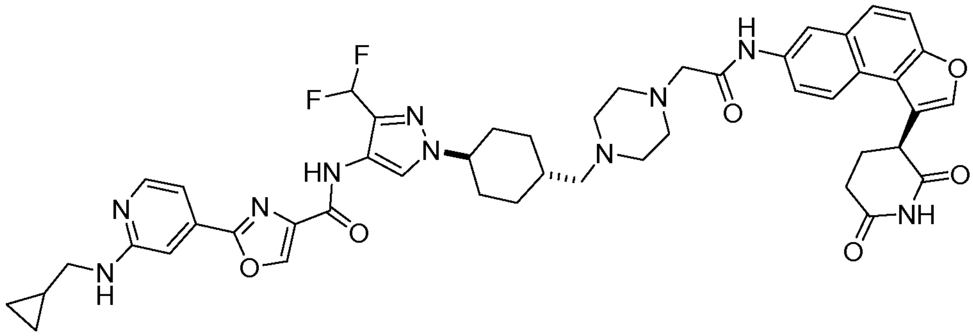

,

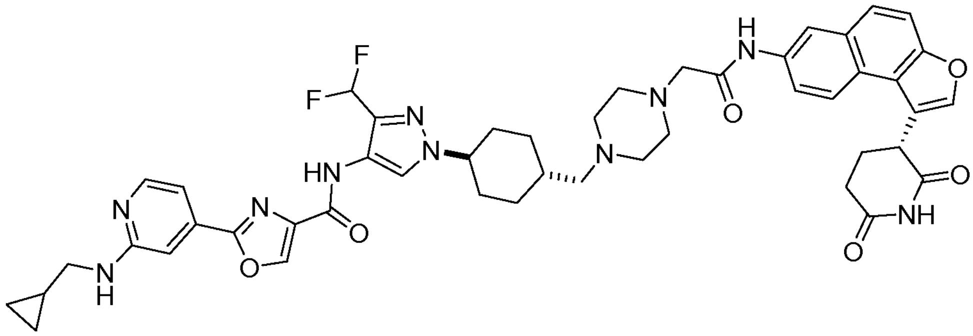

,

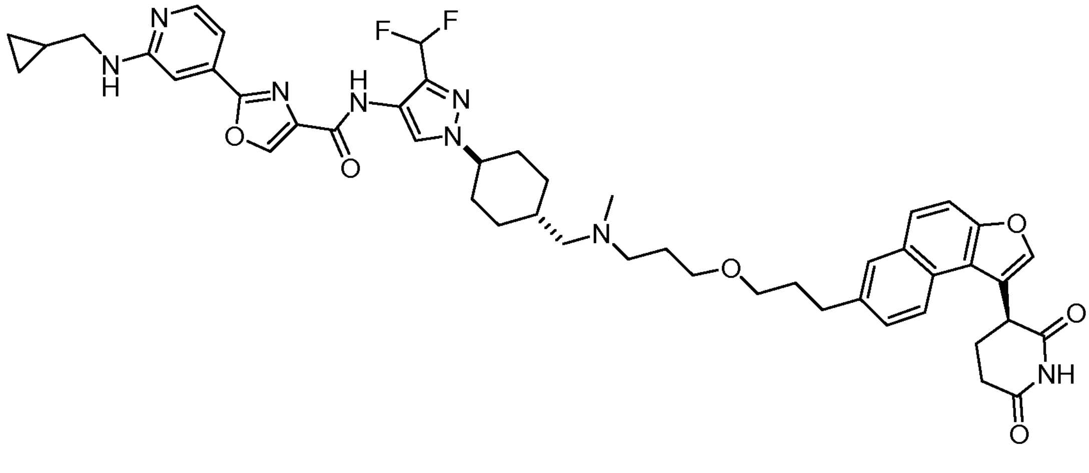

,

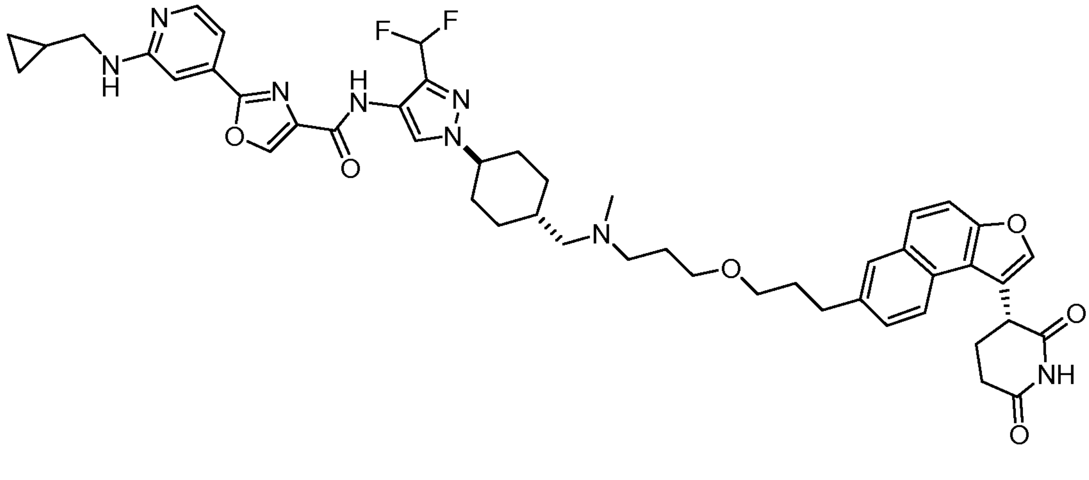

,

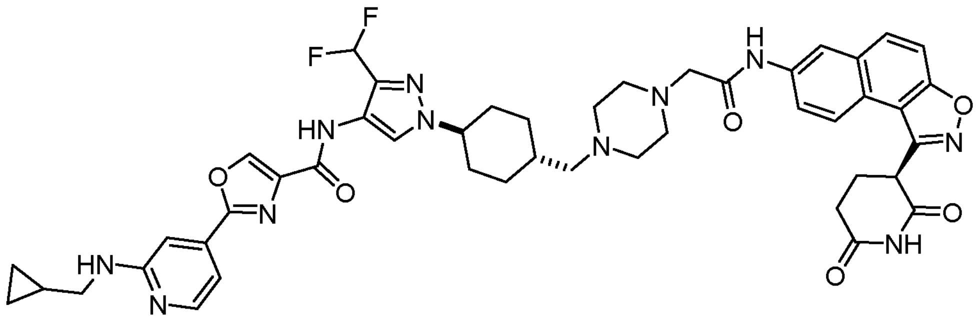

,

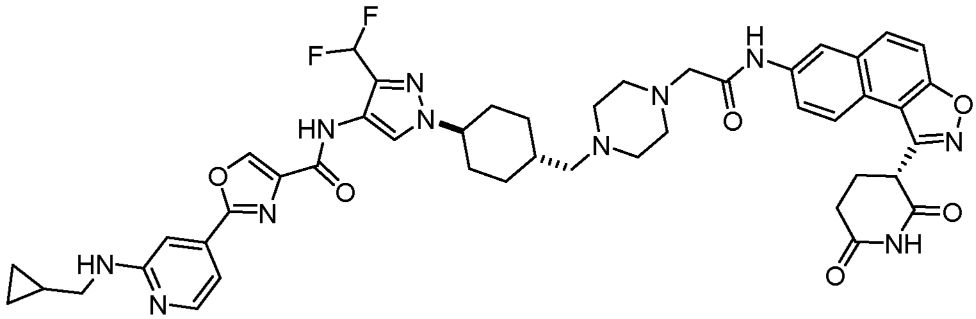

,

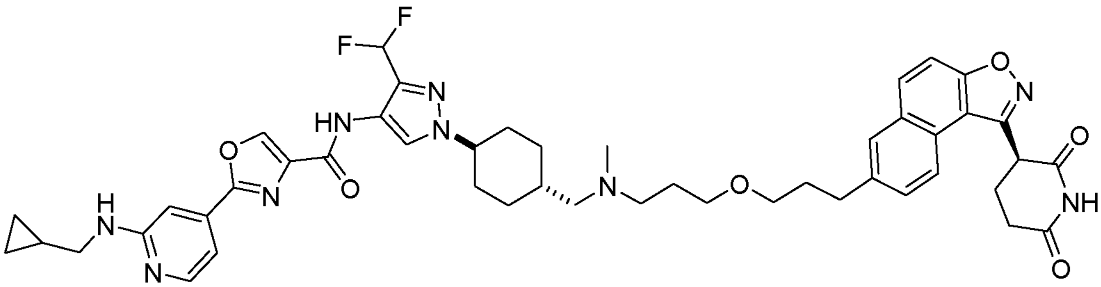

,

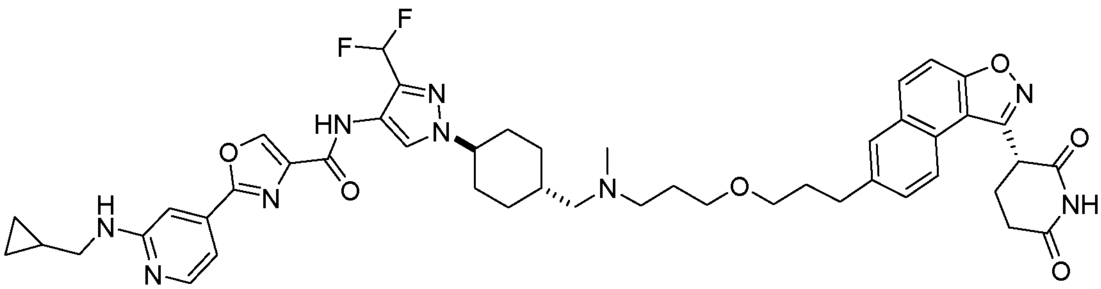

,

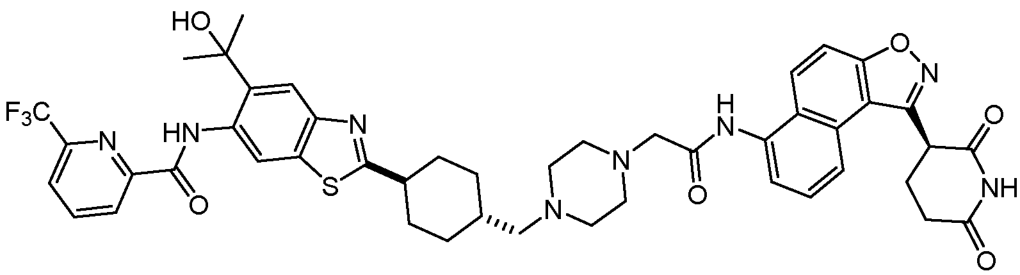

,

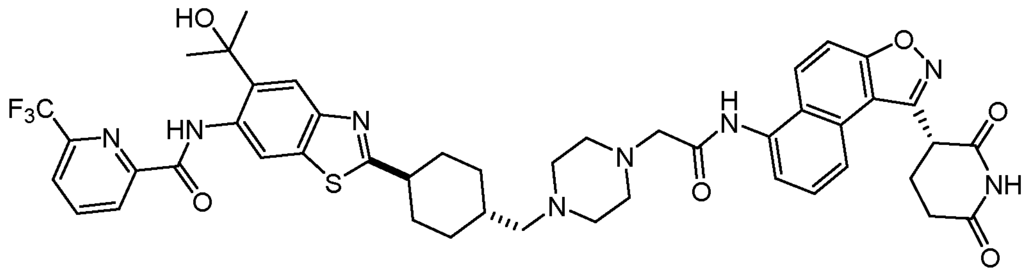,

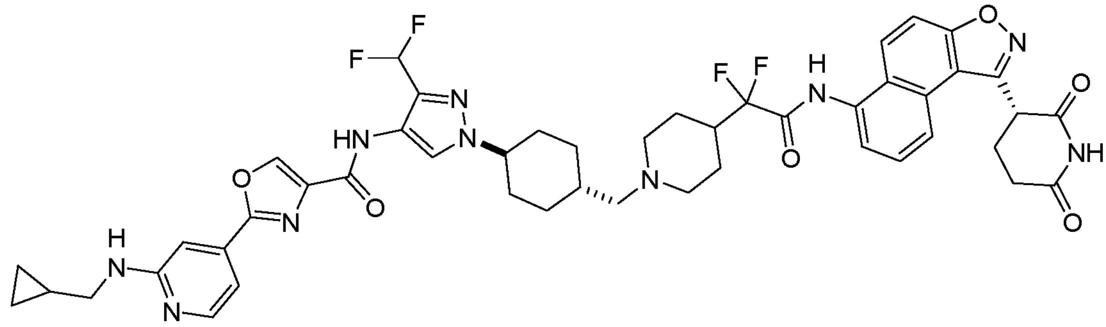,

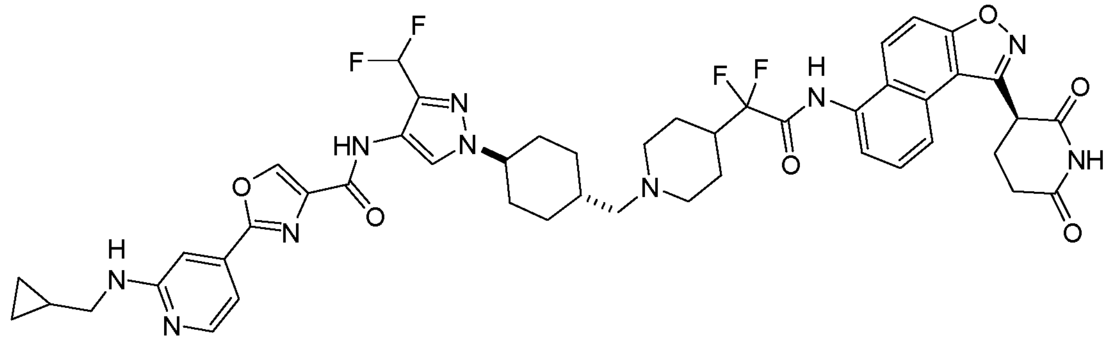,

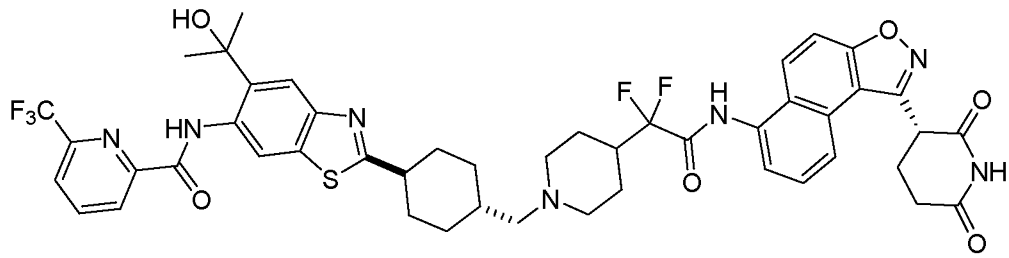,

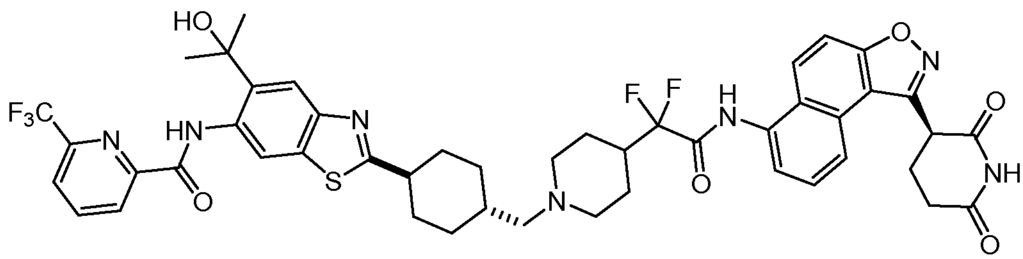,

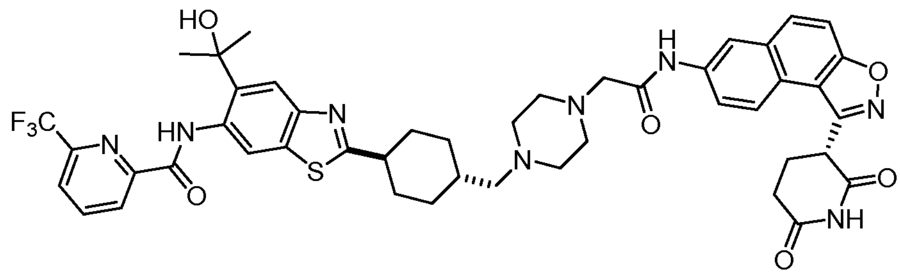

,

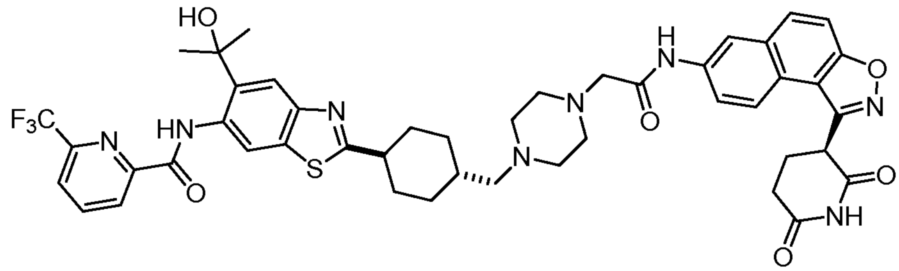

,

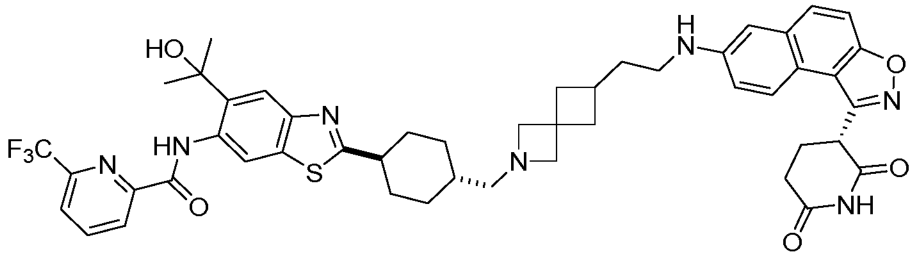

,

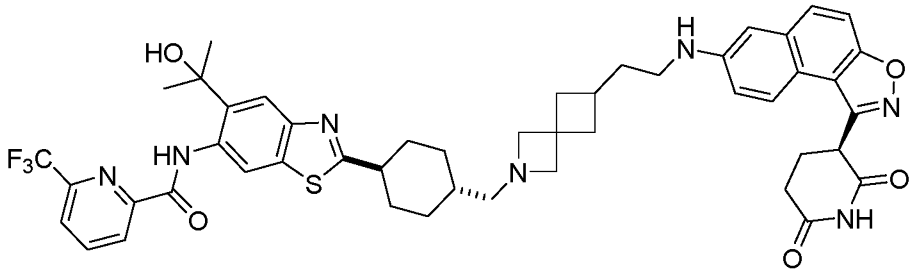

,

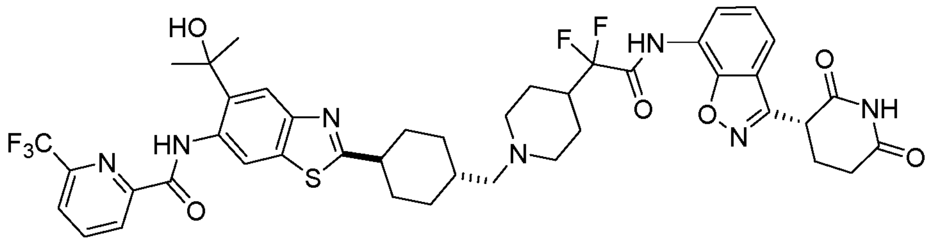

,

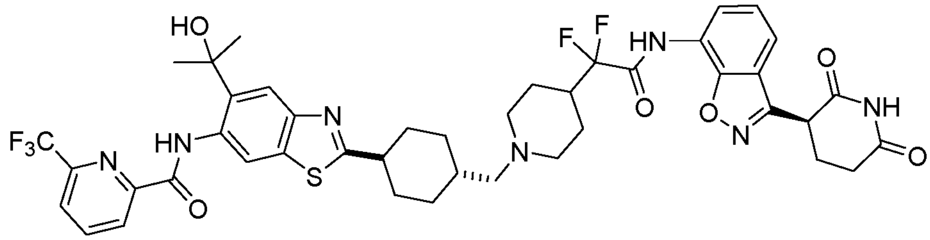

,

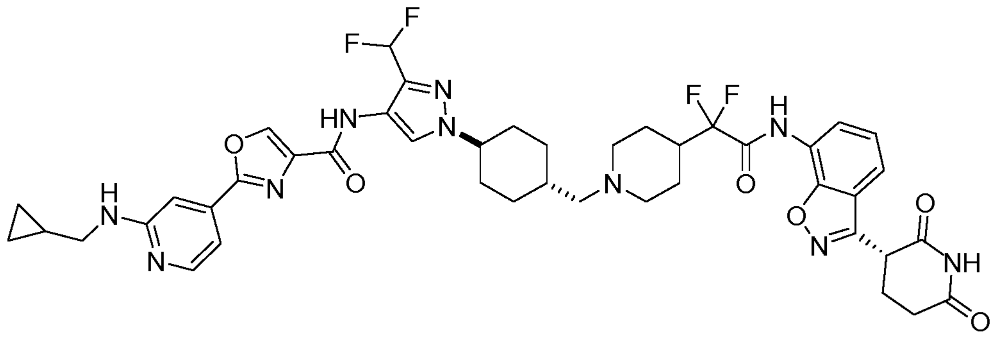

,

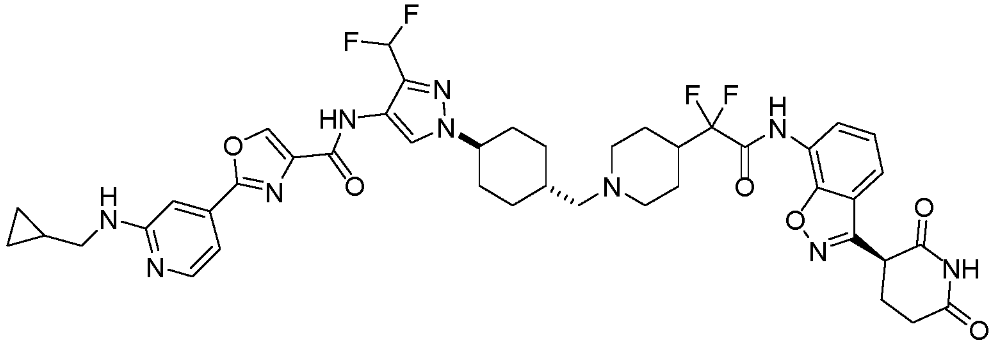

,

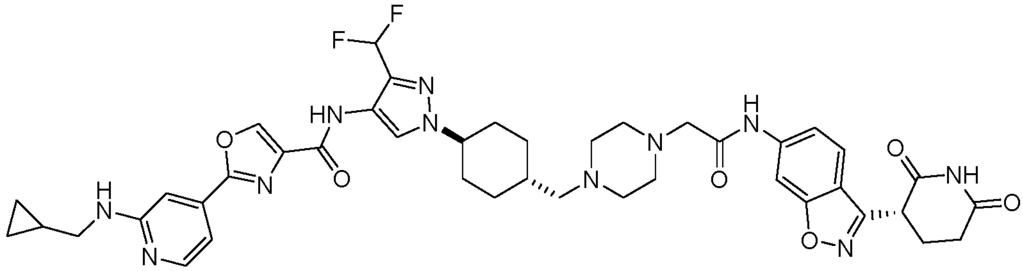

,

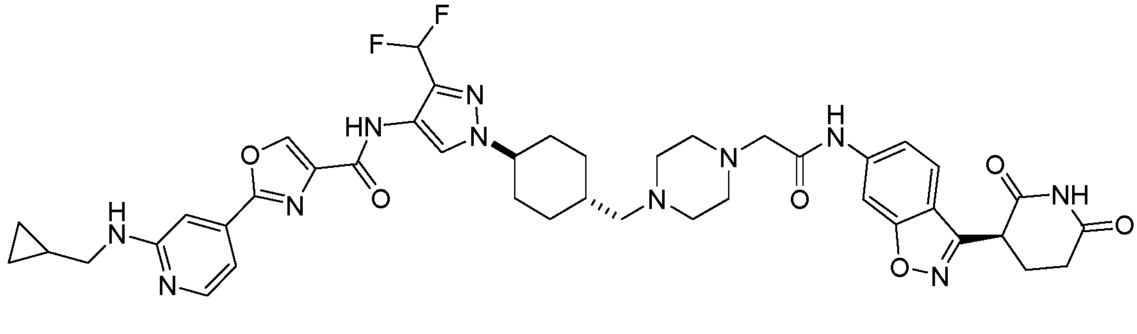

,

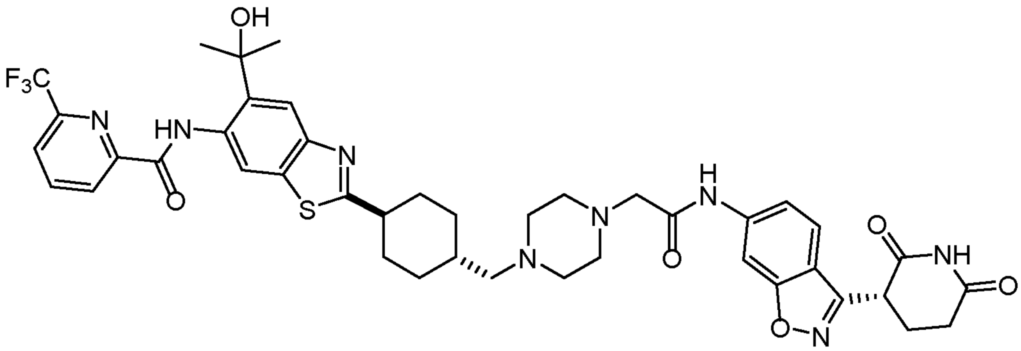

,

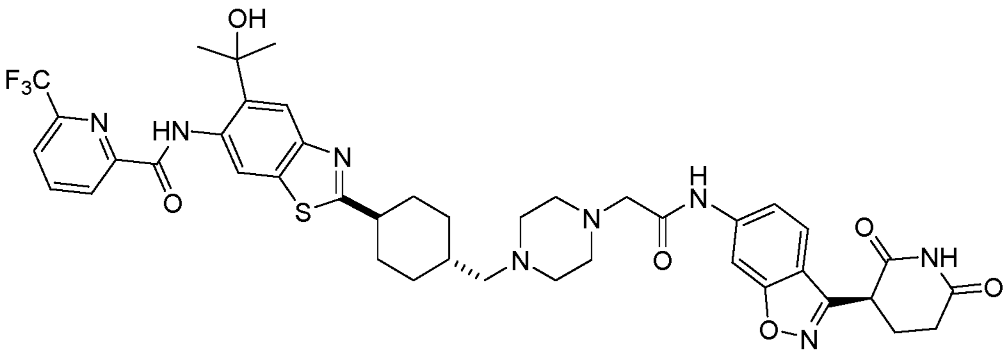

,

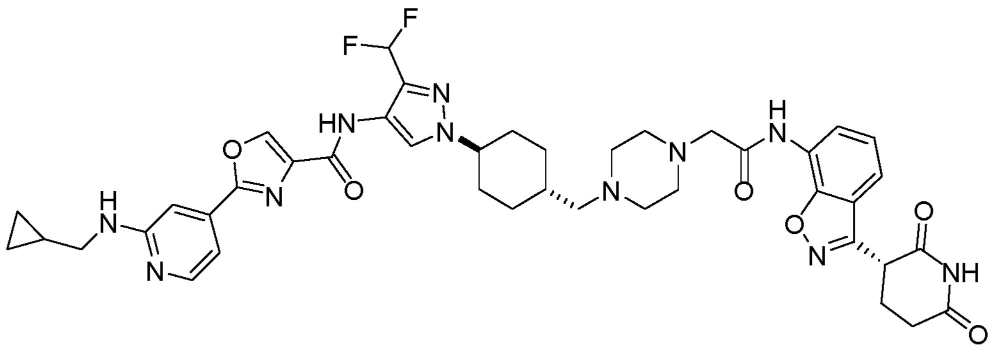

,

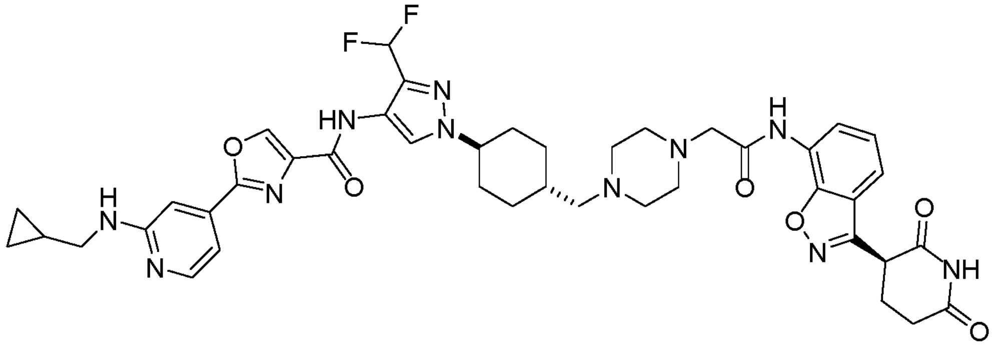

,

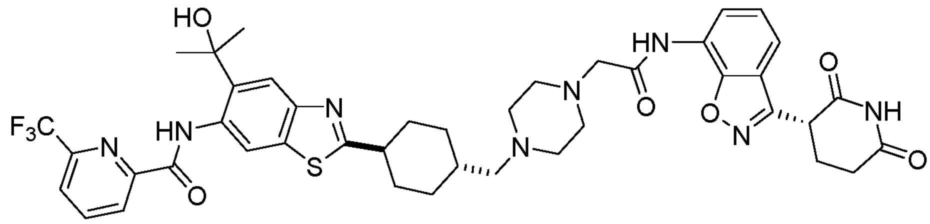

,

and

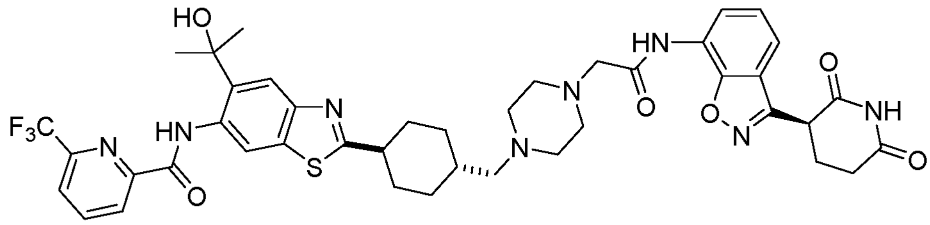

.

**15.** A pharmaceutical composition comprising a therapeutically effective amount of the compound, the stereoisomer thereof, or the pharmaceutically acceptable salt thereof according to any one of claims 1 to 14.

**16.** A use of the compound, the stereoisomer thereof, or the pharmaceutically acceptable salt thereof according to any one of claims 1 to 14, or the pharmaceutical composition according to claim 15 in the manufacture of a medicament for treating tumors associated with proteolysis targeting chimeras of Interleukin-1 Receptor-Associated Kinase 4.

**17.** The use according to claim 16, wherein the tumor associated with proteolysis targeting chimeras of Interleukin-1 Receptor-Associated Kinase 4 is B-cell lymphoma.

## INTERNATIONAL SEARCH REPORT

International application No.
**PCT/CN2022/120267**

**A. CLASSIFICATION OF SUBJECT MATTER**

C07D 413/14(2006.01)i; A61K 31/4545(2006.01)i; A61K 31/496(2006.01)i; A61P 35/00(2006.01)i

According to International Patent Classification (IPC) or to both national classification and IPC

**B. FIELDS SEARCHED**

Minimum documentation searched (classification system followed by classification symbols)

C07D; A61K; A61P

Documentation searched other than minimum documentation to the extent that such documents are included in the fields searched

Electronic data base consulted during the international search (name of data base and, where practicable, search terms used)

CNPAT, CNKI, EPODOC, WPI, STN: 南京明德新药研发有限公司, 哌啶, 白细胞介素-1受体相关激酶, 蛋白降解靶向嵌合体, piperidin+, IRAK, PROTAC, 结构检索

**C. DOCUMENTS CONSIDERED TO BE RELEVANT**

| Category* | Citation of document, with indication, where appropriate, of the relevant passages | Relevant to claim No. |
|---|---|---|
| X | WO 2020264499 A1 (KYMERA THERAPEUTICS, INC.) 30 December 2020 (2020-12-30) see claims 1, 4, and 18-22, and description, paragraph [0014], and table 1 | 1-17 |
| X | WO 2021127283 A2 (KYMERA THERAPEUTICS, INC.) 24 June 2021 (2021-06-24) see claims 1, 4, and 25-28, and description, paragraph [0014], and table 1 | 1-17 |
| X | CN 113423427 A (KYMERA THERAPEUTICS, INC.) 21 September 2021 (2021-09-21) see claims 1, 3, and 20-26, and description, table 1 | 1-17 |
| X | CN 112105385 A (KYMERA THERAPEUTICS, INC.) 18 December 2020 (2020-12-18) see claims 1, 3, and 15-20, and description, table 1 | 1-17 |
| A | WO 2021127190 A1 (KYMERA THERAPEUTICS, INC.) 24 June 2021 (2021-06-24) see claims 1, 3, and 7-12, and description, table 1 | 1-17 |
| A | WO 2021185291 A1 (MEDSHINE DISCOVERY INC.) 23 September 2021 (2021-09-23) see claims 1, 10, and 19-20 | 1-17 |

☐ Further documents are listed in the continuation of Box C. ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **23 November 2022** | **15 December 2022** |
| Name and mailing address of the ISA/CN<br>**China National Intellectual Property Administration (ISA/CN)**<br>**No. 6, Xitucheng Road, Jimenqiao, Haidian District, Beijing 100088, China**<br>Facsimile No. **(86-10)62019451** | Authorized officer<br><br>Telephone No. |

Form PCT/ISA/210 (second sheet) (January 2015)

**INTERNATIONAL SEARCH REPORT**
**Information on patent family members**

International application No.
**PCT/CN2022/120267**

| Patent document cited in search report | Publication date (day/month/year) | Patent family member(s) | Publication date (day/month/year) |
|---|---|---|---|
| WO 2020264499 A1 | 30 December 2020 | EP 3989966 A1 | 04 May 2022 |
| | | JP 2022538192 A | 31 August 2022 |
| | | BR 112021026517 A2 | 10 May 2022 |
| | | CO 2021017893 A2 | 17 January 2022 |
| | | KR 20220032063 A | 15 March 2022 |
| | | CA 3144805 A1 | 30 December 2020 |
| | | CN 114502158 A | 13 May 2022 |
| | | IL 289267 A | 01 February 2022 |
| | | AU 2020302118 A1 | 24 February 2022 |
| WO 2021127283 A2 | 24 June 2021 | US 2022340570 A1 | 27 October 2022 |
| | | EP 4076524 A2 | 26 October 2022 |
| CN 113423427 A | 21 September 2021 | BR 112021010484 A2 | 24 August 2021 |
| | | JP 2022516401 A | 28 February 2022 |
| | | CO 2021007068 A2 | 30 September 2021 |
| | | IL 283471 A | 29 July 2021 |
| | | CA 3119773 A1 | 04 June 2020 |
| | | US 2021323952 A1 | 21 October 2021 |
| | | KR 20210111252 A | 10 September 2021 |
| | | EP 3886904 A1 | 06 October 2021 |
| | | US 2022324854 A1 | 13 October 2022 |
| | | US 11117889 B1 | 14 September 2021 |
| | | WO 2020113233 A1 | 04 June 2020 |
| | | AU 2019389174 A1 | 01 July 2021 |
| | | SG 11202105424 P A | 29 June 2021 |
| CN 112105385 A | 18 December 2020 | AU 2018396142 A1 | 16 July 2020 |
| | | SG 11202005912 P A | 29 July 2020 |
| | | US 2019192668 A1 | 27 June 2019 |
| | | CA 3086763 A1 | 04 July 2019 |
| | | BR 112020012997 A2 | 01 December 2020 |
| | | JP 2021508703 A | 11 March 2021 |
| | | EP 3731869 A1 | 04 November 2020 |
| | | IL 275649 A | 31 August 2020 |
| | | WO 2019133531 A1 | 04 July 2019 |
| | | US 11318205 B1 | 03 May 2022 |
| WO 2021127190 A1 | 24 June 2021 | BR 112022011651 A2 | 23 August 2022 |
| | | AU 2020407200 A1 | 21 July 2022 |
| | | EP 4076520 A1 | 26 October 2022 |
| | | CO 2022008406 A2 | 08 July 2022 |
| | | CN 115052627 A | 13 September 2022 |
| | | CA 3161878 A1 | 24 June 2021 |
| | | TW 202136251 A | 01 October 2021 |
| | | IL 293917 A | 01 August 2022 |
| | | KR 20220145325 A | 28 October 2022 |
| | | US 2021228562 A1 | 29 July 2021 |
| WO 2021185291 A1 | 23 September 2021 | None | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 2021111310410
- CN 2022111046982